# EUROPEAN PATENT APPLICATION

(11) **EP 2 305 717 A1**
(43) Date of publication of application: **06.04.2011**
(21) Application number: 09170853.7
(22) Date of filing: 21.09.2009
(51) Int. Cl.: C07K 16/40, C12N 15/11, C12Q 1/48, C12Q 1/68, G01N 33/50

(54) **Inhibiting TNIK for treating colon cancer**

(71) Applicant: Koninklijke Nederlandse Akademie van Wetenschappen, 1011 JV Amsterdam (NL)
(72) Inventor: Wing Li, Vivian Sze, 3572 SH, Utrecht (NL); Mahmoudi, Tokameh, 3515 XS, Utrecht (NL); Clevers, Johannes Carolus, 3712 AP, Huis ter Heide (NL)
(74) Representative: Swinkels, Bart Willem

(57) **Abstract**

The invention relates to an inhibitor of TNIK and its use for treating cancer.

## Description

### Field of the invention

The invention relates to an inhibitor of TNIK and its use as a medicament for treating cancer.

### Background of the invention

The primary function of the intestinal tract involves the digestion and absorption of nutrients. The intestinal lumen is lined with a specialized simple epithelium, which performs the primary functions of digestion, water and nutrient absorption and forms a barrier against luminal pathogens. The gut is anatomically divided into the small intestine and the colon. The intestinal epithelium is the most vigorously self-renewing tissue of adult mammals (Heath JP. 1996.). The intestinal epithelium is divided into crypts and villi. Proliferative cells reside in the crypts of Lieberkühn. The crypts harbour stem cells and their progeny of transit amplifying cells. Transit amplifying cells spend around two days in the crypt in which they divide 4-5 rounds, before they terminally differentiate into the specialized intestinal epithelial cell types. The primary driving forces behind the proliferation of epithelial cells in the intestinal crypts is the Wnt pathway.

When Wnt signals engage their receptors, a complex series of biochemical events is set in motion, leading to stabilization of the key signalling molecule β-catenin in the cytoplasm. Upon transfer to the nucleus, stabilized β-catenin forms a complex with DNA-binding TCF/LEF transcription factors and serves as a transcriptional co-activator, thus switching on Wnt target genes (Behrens et al, 1996; Molenaar et al, 1996). A primary function of canonical Wnt signalling in adult mammals involves the maintenance of stem and progenitor cells in the intestinal epithelium (Clevers, 2006). Mutational activation of the TCF4/ β-catenin transcriptional program can lead to various types of cancer, most notably of the intestine. In these tumours, loss of the Wnt pathway inhibitors APC and Axin2 (Liu et al, 2000; Rubinfeld et al, 1996) or activating point mutations in β-catenin (Korinek et al, 1997; Morin et al, 1997) lead to the stabilization of β-catenin. The constitutive presence of TCF4/ β-catenin complexes locks the Wnt transcriptional program in the "on" state (Bienz & Clevers, 2000; Korinek et al, 1998), leading to transformation in the gut epithelium. At present, the mechanisms by which the β-catenin/TCF4 complex activates expression of key target genes are incompletely understood. Roles have been proposed for a number of coactivators including CBP/p300 (Hecht et al, 2000; Takemaru & Moon, 2000), Brg1 (Barker et al, 2001) and Pygopus/Bcl9 complexes (Kramps et al, 2002; Mosimann et al, 2009).

There is therefore still a need for identifying a compound which is able to activate the expression of key target genes of the Wnt pathway. Inhibiting such a compound could be used for treating colon cancer.

### Description of the invention

The invention has identified a specific compound able to activate the expression of key target genes of the Wnt pathway: the kinase TNIK (Traf2- and NCK-intracting kinase). Any inhibitor of TNIK is a powerful potential anticancer agent.

In this study, using a proteomics approach, we identified Traf2 and Nck-interacting kinase (TNIK) as a novel protein interacting with Tcf4 in mouse intestinal crypt. TNIK is a member of germinal center kinases (GCKs), and can specifically activate the c-Jun N-terminal kinase (JKN) pathway similar to many GCKs (Fu et al, 1999). Additionally, TNIK has also been reported as an effector of Rap2, to regulate actin cytoskeleton by disrupting F-actin structure, and subsequently inhibits cell spreading (Fu et al, 1999; Taira et al, 2004). However, a potential role for TNIK in transcriptional regulation has never been documented.

Here, we report that TNIK is a critical component of the transcriptional regulatory complex in the Wnt signalling pathway. TNIK is localized in the nuclei of Wnt active intestinal crypts and is recruited to promoters of Wnt target genes in mouse crypts and in colorectal cancer cells in a β-catenin dependent manner. TNIK interacts directly with both β-catenin (encoded by the CTNNB1 gene) and TCF4 (encoded by the TCF7L2 gene) and phosphorylates TCF4 leading to TCF/LEF driven transcriptional activation of Wnt target genes; Exogenous expression of TNIK kinase mutants abrogate TCF/LEF driven transcription, while siRNA depletion of TNIK followed by expression array analysis demonstrates the critical role of TNIK as an essential and specific activator of Wnt target genes. In the application TNIK is used for a human gene or protein while Tnik is used for a mouse ortholog.

In a first aspect of the invention, there is provided an inhibitor of TNIK. An inhibitor of TNIK is a compound which is able to decrease an activity of TNIK and/or to decrease its expression level and/or sub cellular localisation. An inhibitor of TNIK is preferably for use as a medicament. Said medicament is preferably used for treating a cancer and/or a tumor. Preferably for treating a cancer and/or a tumor with a constitutively activated Wnt pathway. Preferred cancers with a constitutively activated Wnt pathway include, but are not restricted to: a gastric carcinoma, a melanoma, a pilomatricoma, a hepatocellular carcinoma, an ovarian carcinoma, a breast fibroma, a medulloblastoma, a Wilm's tumor and colon cancer (Giles et al, 2003). Said medicament is preferably used for treating colon cancer. An activated Wnt pathway is preferably being defined as a detectable activation of said pathway. The activation of said pathway may be assessed using immunohistochemistry for nuclear β-catenin, a hall mark of an activated Wnt pathway or by detecting inactivating mutations in the adenomatous polyposis coli (APC) or AXIN genes, or activating point mutations in the CTNNB1 (β-catenin) gene. This pathway will be said as being constitutively activated when such (mutational) activation is independent on the presence of a Wnt ligand(s) (MacDonald et al, 2009, Dev Cell. Jul;17(1):9-26). A constitutively activated Wnt pathway is defined by comparison to a non-constitutively activated Wnt pathway whose activation is induced by Wnt ligand(s). Throughout the application, TNIK is used to identify a protein or polypeptide, said protein comprising an amino acid sequence that is encoded by a nucleotide sequence selected from:
(a) a nucleotide sequence that has at least 60, 70, 80, 85, 90, 95, 98 or 99% identity with nucleotide sequence SEQ ID NO: 1; and,
(b) a nucleotide sequence that encodes an amino acid sequence that has at least 60, 70, 80, 85, 90, 95, 98 or 99% amino acid identity with an amino acid sequence encoded by a nucleotide sequence selected from SEQ ID NO: 1.

A preferred nucleotide sequence encoding TNIK is represented by SEQ ID NO: 1. A corresponding preferred amino acid sequence of TNIK is represented by SEQ ID NO:2. However, a TNIK protein may also refer to a splice variant of TNIK. 8 splice variants are known in total (Fu et al, 1999). Therefore, in the definition of a TNIK protein as given above SEQ ID NO: 1 may be replaced by SEQ ID NO:3 or 4 or 5 or 6 or 7 or 8 or 9. It follows that SEQ ID NO:2 may be replaced by SEQ ID NO: 10 or 11 or 12 or 13 or 14 or 15 or 16. Therefore throughout the application, TNIK is used to identify a protein TNIK, said protein TNIK comprising an amino acid sequence that is encoded by a nucleotide sequence selected from:
(a) a nucleotide sequence that has at least 60, 70, 80, 85, 90, 95, 98 or 99% identity with nucleotide sequence SEQ ID NO: 1 or 3 or 4 or 5 or 6 or 7 or 8 or 9 or; and,
(b) a nucleotide sequence that encodes an amino acid sequence that has at least 60, 70, 80, 85, 90, 95, 98 or 99% amino acid identity with an amino acid sequence encoded by a nucleotide sequence selected from SEQ ID NO: 1 or 3 or 4 or 5 or 6 or 7 or 8 or 9.

A "decrease of an activity of TNIK or a decrease of the expression level of TNIK" is herein understood to mean any detectable change in a biological activity exerted by TNIK or in the expression level of TNIK as compared to said activity or expression of a wild type TNIK such as the one encoded by SEQ ID NO:1 or the one being represented by SEQ ID NO:2 or 10 or 11 or 12 or 13 or 14 or 15 or 16. The decrease of the level or of the amount of a nucleotide encoding TNIK is preferably assessed using classical molecular biology techniques such as (real time) PCR, arrays or Northern analysis. Alternatively, according to another preferred embodiment, the decrease of the expression level of TNIK is determined directly by quantifying the amount of a TNIK protein. Quantifying a protein amount may be carried out by any known technique such as Western blotting or immunoassay using an antibody raised against said protein. The skilled person will understand that alternatively or in combination with the quantification of a nucleic acid sequence and/or the corresponding polypeptide, a quantification of a substrate or a quantification of the expression of a target gene of TNIK or of any compound known to be associated with a function or activity of TNIK or the quantification of said function or activity of TNIK using a specific assay may be used to assess the decrease of an activity or expression level of TNIK. Several assays are suitable to measure a TNIK activity. They include but are not restricted to: the assessment of the sub cellular localization of a TNIK protein (cytoplasmic vs. nuclear), the assessment of the binding of TNIK to β-catenin and/or TCF4, the assessment of TNIK-dependent phosphorylation of TCF4 and the transcription of specific target genes or the measurement of the transcriptional action of genes via the Wnt pathway using TOPflash reporters. Each of these types of assays are known to the skilled person. The sub cellular localization may be assessed by immunohistochemistry, immunofluorescence (an antibody specific for TNIK is being used and the detected signal will be localized either in the nucleus or in the cytoplasm) or western blotting identifying TNIK using distinct types of cellular extracts (cytosolic versus nuclear extract). The binding of TNIK to β-catenin and/or TCF4 may be assessed by immunoprecipitation or by in vitro binding assay or by Mass Spectrometry. The assessment of the phosphorylation of TCF4 by TNIK may be done by western blotting using an antibody specifically recognizing TNIK phosphorylated amino acids or by an in vitro kinase assay using radioactive P32-ATP to demonstrate phosphorylation of TCF4 by TNIK. TNIK is preferably isolated from a cell that was first treated with a TNIK inhibitor prior to the in vitro kinase assay. The transcriptional activation of specific target genes may be assessed using luciferase reporter experiments or Electrophoretic Mobility Shift assay. Preferred specific target genes are Wnt activated target genes, more preferably TCF7, AXIN2, ZCCHC12 and CCND1 genes. Alternatively the measurement of the transcriptional action of genes via the Wnt pathway may be assessed using TOPflash reporter (Korinek et al, 1997) as has been fully described in the experimental part.

Preferably, a decrease or a down-regulation of the expression level of a nucleotide sequence encoding a TNIK protein means a decrease of at least 5% of the expression level of a nucleotide sequence using arrays or Northern blot. More preferably, a decrease of the expression level of a nucleotide sequence means a decrease of at least 10%, even more preferably at least 20%, at least 30%, at least 40%, at least 50%, at least 70%, at least 90%, at least 100%, or more. Preferably, the expression is no longer detectable. In another preferred embodiment, a decrease of the expression level of a TNIK protein means a decrease of at least 5% of the expression level of a protein using western blotting and/or using ELISA or a suitable assay. More preferably, a decrease of the expression level of a TNIK protein means a decrease of at least 10%, even more preferably at least 20%, at least 30%, at least 40%, at least 50%, at least 70%, at least 90%, at least 150% or more. Preferably, the expression is no longer detectable. In another preferred embodiment, a decrease of a TNIK activity means a decrease of at least 5% of a TNIK activity using a suitable assay as earlier defined herein. More preferably, a decrease of a TNIK activity means a decrease of at least 10%, even more preferably at least 20%, at least 30%, at least 40%, at least 50%, at least 70%, at least 90%, at least 150% or more. Preferably, said activity is no longer detectable

An inhibitor of TNIK may be any compound. The invention also provides a method for identifying additional inhibitors of TNIK (see later herein). Preferably a TNIK inhibitor is a DNA or RNA molecule, a dominant negative molecule of TNIK, an inhibiting antibody raised against TNIK, a small molecule such as a peptide-like molecule (referred to as peptidomimetics) or a non-peptide molecule. Each of these TNIK inhibitors is presented in more details below.

An inhibitor may act at the level of the protein itself, e.g. by providing an antagonist or inhibitor of a TNIK protein to a cell, such as e.g. an inhibiting antibody raised against TNIK (named an antibody herein) or a dominant negative form of TNIK (named DNTNIK herein) or an antisense for TNIK (named antisense molecule herein). An antibody, an antisense molecule or a DNTNIK of the invention may be obtained as described below. Alternatively, an inhibitor may act at the level of the nucleotide encoding TNIK. In this case, the expression level of TNIK is decreased by regulating the expression level of a nucleotide sequence encoding TNIK.

An antisense molecule is a molecule capable of inhibiting the biosynthesis (usually the translation) of a nucleotide sequence encoding TNIK. Decreasing gene expression by providing antisense or interfering RNA molecules is described below herein and is e.g. reviewed by Famulok et al. (2002, Trends Biotechnol., 20(11): 462-466). An antisense molecule may be provided to a cell as such or it may be provided by introducing an expression construct into a cell, whereby said expression construct comprises an antisense nucleotide sequence that is capable of inhibiting the expression of a nucleotide sequence encoding a TNIK protein, and whereby said antisense nucleotide sequence is under control of a promoter capable of driving transcription of said antisense nucleotide sequence in a cell. The expression level of a TNIK protein may also be down-regulated or decreased by introducing an expression construct into a cell, whereby said expression construct comprises a nucleotide sequence encoding a factor capable of trans-repression of an endogenous nucleotide sequence encoding a TNIK protein. Preferably, a nucleotide sequence capable of trans-repression of an endogenous nucleotide sequence is a dominant negative of said endogenous nucleotide sequence as exemplified below.

An antisense or interfering nucleic acid molecule may be introduced into a cell directly "as such", optionally in a suitable formulation, or it may be produced *in situ* in a cell by introducing into a cell an expression construct or nucleic acid construct comprising a (antisense or interfering) nucleotide sequence that is capable of inhibiting the expression of a nucleotide sequence encoding TNIK, whereby, optionally, an antisense or interfering nucleotide sequence is under control of a promoter capable of driving expression of said nucleotide sequence in a cell. Preferably, the cell is an intestinal cell or intestinal cancer cell. Such a nucleic acid construct of the invention comprises or consists of a nucleotide sequence that encodes an RNAi agent, i.e. an RNA molecule that is capable of RNA interference or that is part of an RNA molecule that is capable of RNA interference. Such an RNA molecule is referred to as siRNA (short interfering RNA, including e.g. a short hairpin RNA). A nucleotide sequence that encodes an RNAi agent preferably has sufficient complementarity with a cellular nucleotide sequence encoding TNIK as earlier defined herein to be capable of inhibiting or decreasing the expression of said protein TNIK. Preferred siRNAs as identified in the experimental part had been designed with the use of pre-designed Dharmacon siRNA pools targeting transcripts of the human TNIK (product number L-004542-00). Preferred siRNAs comprise or consist of SEQ ID NO:17-20.

Alternatively or in combination with the antisense approach, one may also use an inactivating approach. In this approach, an inactivating nucleic acid construct is introduced into a cell. Said inactivating construct comprises or consists of a nucleotide molecule which is designed in order to inactivate the expression of the TNIK protein. The skilled person knows how to design an inactivating construct (Capecchi 1989, Science Jun 16;244(4910):1288-92). For example, at least part of a TNIK gene is replaced by a marker such as the neomycine gene.

Alternatively or in combination with the antisense and inactivating approaches, one may also use a dominant negative approach. In this approach, a nucleic acid construct is introduced into a cell, wherein said nucleic construct comprises a dominant negative nucleotide sequence that is capable of inhibiting or down-regulating an activity of a corresponding endogenous TNIK protein, and wherein, optionally, a dominant negative nucleotide sequence is under the control of a promoter capable of driving expression of said dominant negative nucleotide sequence in a cell. In a preferred embodiment described earlier herein, a nucleic acid construct used herein comprises or consists of a dominant negative of a TNIK protein as earlier defined herein. Alternatively, a dominant negative molecule may be directly administered to a subject. The skilled person knows how to design a dominant negative of a kinase. Several strategies are already known for designing a dominant negative of a kinase. A dominant negative kinase is usually a truncated kinase without a catalytic domain(s) or sub-domain(s) or with an inactive catalytic domain(s) or sub-domain(s). An inactive catalytic domain or sub-domain may be generated by introducing a point-mutation(s) in said kinase sub-domain(s) or domain(s). Preferably a kinase sub-domain or domain to be mutated is chosen as being a critical sub-domain or domain for an activity of TNIK. A critical domain or sub-domain is such that when it has been inactivated or deleted, the expressed TNIK has less than 50% of an activity of the TNIK it derives from. An activity of TNIK is preferably measured using one of the assays as earlier identified herein. A dominant negative TNIK is usually still able to bind a substrate and/or a target gene. A preferred sub-domain of TNIK to be deleted or inactivated is selected from sub-domain II (aa: 47-64), VI (aa: 123-164) or VII (aa 165-183). In the experimental part, several dominant negative TNIK have been used: TNIK R152A/D153A, TNIK K54A and TNIK D171A/F172A (using SEQ ID NO:1 as reference). Each of these dominant negative TNIK has a mutation in a distinct catalytic sub-domain, respectively sub-domains VI, II and VII. Each of these dominant negative TNIK is preferably represented by the following amino acid sequence: SEQ ID NO: 21, 23, and 25 respectively. Each of these dominant negative TNIK is preferably encoded by the following nucleic acid molecules represented by the following sequence: SEQ ID NO: 22, 24, and 26. Therefore in a preferred embodiment, an inhibitor or TNIK is a dominant negative TNIK protein, said dominant negative TNIK protein comprising an amino acid sequence that is encoded by a nucleotide sequence selected from:
(a) a nucleotide sequence that has at least 60, 70, 80, 85, 90, 95, 98 or 99% identity with nucleotide sequence SEQ ID NO: 22 or 24 or 26; and,
(b) a nucleotide sequence that encodes an amino acid sequence that has at least 60, 70, 80, 85, 90, 95, 98 or 99% amino acid identity with an amino acid sequence encoded by a nucleotide sequence selected from SEQ ID NO: 22, 24 or 26.
and wherein each of this dominant negative having the mutation R152A/D153A when reference is made to SEQ ID NO:21, TNIK K54A when reference is made to SEQ ID NO:23 and D171A/F172A when reference is made to SEQ ID NO:25. An amino acid used to replace a wild type amino acid can be any other amino acid. Preferably the side chain of the amino acid used to replace an original amino acid has other properties in polarity and or charge (e.g. replace a positively charged residue for a neutral one). Amino acids having similar properties are for example the ones with an aliphatic side chain: glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred amino acid replacements switch from side chain properties e.g.: arginine to alanine; asparagine to methionine; Leucine to threoinine; Leucine to serine; Tryptophan to glutamine etc.

If one of these alternative amino acids is used to design a dominant negative TNIK, the skilled person will preferably test that the hence obtained putative dominant negative has indeed a decreased TNIK activity by comparison to a TNIK it is derived from.

A dominant negative is preferably said to have a decreased TNIK activity by comparison to a TNIK it derives from as earlier defined herein. Alternatively, a TNIK being encoded by SEQ ID NO:1 may be taken as control. A dominant negative of TNIK is a TNIK which has a decreased TNIK activity by comparison to this control TNIK. A preferred TNIK activity in this context is the phosphorylation of TCF4 or the transcriptional activation of specific target genes. Specific target genes are Wnt activated target genes, more preferably TCF7, AXIN2, ZCCHC12 and CCND1 genes.

In a nucleic acid construct of the invention (dominant negative approach or antisense approach), a promoter or a regulatory region preferably is a promoter or regulatory region that is specific for an intestinal cell, more preferably a promoter specific for a cell of an intestinal crypt. A promoter that is specific for a cell of an intestinal crypt is a promoter with a transcription rate that is higher in said cell than in other types of cells. Preferably the promoter's transcription rate in said cell is at least 1.1, 1.5, 2.0 or 5.0 times higher than in other cell. A promoter for use in a DNA construct of the invention is preferably of mammalian origin, more preferably of human origin. For expression in an intestinal stem cell, preferably in an intestinal cancer stem cell for instance a LGR5 promoter can be used. Different Lgr5 knock-in alleles have shown that Lgr5 has an intestinal stem cell specific expression pattern (Barker et al, 2007, Nature. Oct 25;449(7165):1003-7). Also other regulatory regions of genes from the stem cell signature, such as Asc12 can be used (Van der Flier et al, 2009, Cell. Mar 6;136(5):903-12). SEQ ID NO 27 gives the genomic locus of the ASCL2 gene.

In a preferred embodiment, a nucleic acid construct is a viral gene therapy vector selected from gene therapy vectors based on an adenovirus, an adeno-associated virus (AAV), a herpes virus, a pox virus and a retrovirus. A preferred viral gene therapy vector is an AAV or Lentiviral vector. Such vectors are further described herein below.

In a further aspect the invention relates to the use of an inhibitor of TNIK such as a nucleic acid construct as defined herein for decreasing an activity or expression level of a TNIK protein as defined herein, for the manufacture of a medicament for treating a cancer, preferably in a method of the invention as defined herein above.

Accordingly in a further aspect, there is provided a method for treating colon cancer using an inhibitor of TNIK as defined herein. A method preferably comprises decreasing an activity and/ or the expression level of TNIK as defined above in a subject. Preferably, the decrease is sufficient to detect a decrease of the proliferation rate of a colorectal cancer cell and/or restore the normal colonic crypt structure and/or decrease tumor weight or size. A colorectal cancer cell may be isolated from a treated patient and the proliferation rate may be assessed at distinct time points before the onset of the treatment and after the onset of the treatment. Alternatively, colonoscopy or endoscopy could be used to visualize tumor size upon treatment. Alternatively, a colorectal cancer cell line may be used to assess the potential therapeutic value of said inhibitor. Proliferation may be assessed by following ³H incorporation into these cells. Preferably the colorectal cancer cell line is the Ls174T cell line which is publicly available.

A decrease in tumor weight or size may be assessed in situ using colonoscopy or endoscopy or PET imaging or a combination of PET/CT imaging in vivo (Francis et al, 2003, Gut. Nov;52(11):1602-6).The increased uptake of a glucose analogue, ¹⁸F-labeled 2-fluoro-2-deoxy-D-glucose is used as a sensitive imaging technique to detect a tumor in a patient and assess tumor growth. The combination with CT provides a precise and useful anatomical localization of the radioactive tracer uptake.

A method of the invention preferably comprises the step of administering to a subject a therapeutically effective amount of a pharmaceutical composition comprising an inhibitor of TNIK, preferably an inhibiting antibody, a DNA or an RNA, a dominant negative molecule or a nucleic acid construct for decreasing an activity or expression level of a TNIK protein as defined herein. A nucleic acid construct is for inhibiting expression of a TNIK protein of the invention such as an antisense molecule or an RNA molecule capable of RNA interference as defined herein. Alternatively or in combination with both previous embodiments, a nucleic acid construct comprising a dominant negative of an endogenous TNIK protein may be administered into a cell or into a subject. In a method of the invention, a pharmaceutical composition comprising a nucleic acid construct is preferably administered in a cell or in a subject. Preferably, the subject is a mammal. More preferably the mammal is a human being. Each inhibitor may be first applied or administrated in vitro or ex vivo in a cell. More preferably a cell is an intestinal cell, even more preferably a cell of an intestinal crypt, even more preferably a colorectal cancer cell.

In a further preferred method, another treatment, comprising but not restricted to chemotherapy, surgery, radiation or a small molecule inhibitor, such as growth factor specific antibody, a receptor tyrosine kinase inhibitor and/or a kinase inhibitor is combined with the administration of a TNIK inhibitor. Examples of growth factor specific antibodies are bevacizumab (α-VEGF), Rituximab (α-CD20). Examples of tyrosine kinase receptor inhibitors are Erlotinib, Gefitinib, Lapatinib (EGFR inhibitors), SU11248, Midostaurin (VEGFR inhibitors), RO4396686 (FGFR and PDGF inhibitor) and SM16, LY364947 (TGFBR type I inhibitors). Examples of kinase inhibitors are Sorafenib (B-Raf inhibitor), Imatinib, Nilotinib (BCR-Abl inhibitors), Temsirolimus (mTor inhibitor), Desatinib (Src, c-Kit inhibitor).

Accordingly, in a further aspect of the invention, there is provided a use of an inhibitor of TNIK for the manufacture of a medicament for treating cancer. Each of the features of this aspect has already been defined herein.

Accordingly, in a further aspect of the invention, there is provided a method for identification of a compound inhibiting TNIK, the method comprising the steps of:
(a) providing a test cell population capable of expressing a nucleotide sequence encoding TNIK;
(b) contacting the test cell population with the compound;
(c) determining the expression level of the nucleotide sequence or an activity of TNIK in the test cell population contacted with the compound;
(d) comparing the expression, an activity determined in (c) with the expression, an activity of the nucleotide sequence of TNIK in a test cell population that is not contacted with the compound; and,
(e) identifying a compound that produces a decrease in expression level, of an activity of the nucleotide sequence of TNIK, between the test cell population that is contacted with the compound and the test cell population that is not contacted with the compound.

This method is a so-called cell-based method. Preferably, in a method a test cell population comprises intestinal crypt cells, more preferably a cell line such as e.g. the cell line and/or other cells or cell lines described in the Examples herein. A preferred cell line is the Ls174T colorectal cancer cell line which is commercially available from ATCC (CL-188^{™}). A test cell population preferably comprises mammalian cells, more preferably human cells.

Accordingly, in a further aspect of the invention, there is provided a cell-free method for identification of a compound inhibiting TNIK, the method comprising the steps of:
(a) providing a TNIK protein;
(b) contacting it with the compound;
(c) determining an activity of TNIK after it has been contacted with the compound;
(d) comparing an activity determined in (c) with, an activity of TNIK that has not been contacted with the compound; and,
(e) identifying a compound that produces a decrease in an activity of TNIK, between TNIK that has been contacted with the compound and the TNIK that has not been contacted with the compound.

Such activity of TNIK which is assessed in such cell-free method may be an in vitro kinase assay or the binding of a compound preventing interaction between TNIK and/or TCF and/or β-catenin.

In one aspect, the invention also pertains to a substance that has been identified in one of said methods. A substance may first be identified using a cell-free method and subsequently said substance may be tested in a cell-based method as defined earlier may be later tested in a cell-based method as defined herein. A decrease in expression level or of an activity has preferably the same meaning as given earlier herein.

### General technical information

### Sequence identity

"Sequence identity" is herein defined as a relationship between two or more amino acid (polypeptide or protein) sequences or two or more nucleic acid (polynucleotide) sequences, as determined by comparing the sequences. The identity between two nucleic acid sequences is preferably defined by assessing their identity within a whole SEQ ID NO as identified herein or part thereof. Part thereof may mean at least 50% of the length of the SEQ ID NO, or at least 60%, or at least 70%, or at least 80%, or at least 90%.

In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps). Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

### Recombinant techniques and methods for recombinant production of a TNIK polypeptide or protein or a dominant negative TNIK polypeptide or protein named herein a polypeptide

A polypeptide for use in the present invention can be prepared using recombinant techniques, in which a nucleotide sequence encoding a polypeptide of interest is expressed in a suitable host cell. The present invention thus also concerns the use of a vector comprising a nucleic acid molecule represented by a nucleotide sequence as defined above. Preferably a vector is a replicative vector comprising an origin of replication (or autonomously replication sequence) that ensures multiplication of a vector in a suitable host for the vector. Alternatively a vector is capable of integrating into a host cell's genome, e.g. through homologous recombination or otherwise. A particularly preferred vector is an expression vector wherein a nucleotide sequence encoding a polypeptide as defined above, is operably linked to a promoter capable of directing expression of a coding sequence in a host cell for the vector.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" promoter is a promoter that is regulated depending on physiological or developmental conditions. A "tissue specific" promoter is only active in specific types of differentiated cells/tissues, such as preferably intestinal cells or tissues.

An expression vector allows a polypeptide of the invention as defined above to be prepared using recombinant techniques in which a nucleotide sequence encoding a polypeptide of interest is expressed in a suitable cell, e.g. cultured cells or cells of a multicellular organism, such as described in Ausubel et al., "Current Protocols in Molecular Biology", Greene Publishing and Wiley-Interscience, New York (1987) and in Sambrook and Russell (2001, *supra*); both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et al. (1985) Gene 34: 315 (describing cassette mutagenesis).

Typically, a nucleic acid encoding a polypeptide of the invention is used in an expression vector. The phrase "expression vector" generally refers to a nucleotide sequence that is capable of effecting expression of a gene in a host compatible with such sequences. These expression vectors typically include at least a suitable promoter sequence and optionally, a transcription termination signal. Additional factors necessary or helpful in effecting expression can also be used as described herein. A nucleic acid or DNA encoding a polypeptide is incorporated into a DNA construct capable of introduction into and expression in an *in vitro* cell culture. Specifically, a DNA construct is suitable for replication in a prokaryotic host, such as bacteria, e.g., *E. coli,* or can be introduced into a cultured mammalian, plant, insect, e.g., Sf9, yeast, fungi or another eukaryotic cell line.

A DNA construct prepared for introduction into a particular host typically include a replication system recognized by the host, the intended DNA segment encoding a desired polypeptide, and transcriptional and translational initiation and termination regulatory sequences operably linked to a polypeptide-encoding segment. A DNA segment is "operably linked" when it is placed into a functional relationship with another DNA segment. For example, a promoter or enhancer is operably linked to a coding sequence if it stimulates the transcription of the sequence. A DNA for a signal sequence is operably linked to a DNA encoding a polypeptide if it is expressed as a preprotein that participates in the secretion of said polypeptide. Generally, DNA sequences that are operably linked are contiguous, and, in the case of a signal sequence, both contiguous and in reading phase. However, an enhancer needs not be contiguous with a coding sequence whose transcription it controls. Linking is accomplished by ligation at convenient restriction sites or at adapters or linkers inserted in lieu thereof. The selection of an appropriate promoter sequence generally depends upon a host cell selected for the expression of a DNA segment. Examples of suitable promoter sequences include prokaryotic, and eukaryotic promoters well known in the art (see, e.g. Sambrook and Russell, 2001, *supra*). A transcriptional regulatory sequence typically includes a heterologous enhancer or promoter that is recognised by the host. The selection of an appropriate promoter depends upon the host, but promoters such as the trp, lac and phage promoters, tRNA promoters and glycolytic enzyme promoters are known and available (see, e.g. Sambrook and Russell, 2001, *supra*). An expression vector includes the replication system and transcriptional and translational regulatory sequences together with the insertion site for a polypeptide encoding segment can be employed. Examples of workable combinations of cell lines and expression vectors are described in Sambrook and Russell (2001, *supra*) and in Metzger et al. (1988) Nature 334: 31-36. For example, a suitable expression vector can be expressed in, yeast, e.g. *S.cerevisiae, e.g.,* insect cells, *e.g.,* Sf9 cells, mammalian cells, *e.g.,* CHO cells and bacterial cells, *e.g., E. coli.* A host cell may thus be a prokaryotic or eukarotic host cell. A host cell may be a host cell that is suitable for culture in liquid or on solid media. A host cell is preferably used in a method for producing a polypeptide of the invention as defined above. A method comprises the step of culturing a host cell under conditions conducive to the expression of a polypeptide. Optionally a method may comprise recovery of a polypeptide. A polypeptide may e.g. be recovered from the culture medium by standard protein purification techniques, including a variety of chromatography methods known in the art per se.

Alternatively, a host cell is a cell that is part of a multicellular organism such as a transgenic plant or animal, preferably a non-human animal. A transgenic plant comprises in at least a part of its cells a vector as defined above. Methods for generating transgenic plants are e.g. described in U.S. 6,359,196 and in the references cited therein. Such transgenic plant may be used in a method for producing a polypeptide of the invention as defined above, said method comprising the step of recovering a part of a transgenic plant comprising in its cells the vector or a part of a descendant of such transgenic plant, whereby said plant part contains a polypeptide, and, optionally recovery of a polypeptide from said plant part. Such method is also described in U.S. 6,359,196 and in the references cited therein. Similarly, a transgenic animal comprises in its somatic and germ cells a vector as defined above. A transgenic animal preferably is a non-human animal. Methods for generating transgenic animals are e.g. described in WO 01/57079 and in the references cited therein. Such transgenic animal may be used in a method for producing a polypeptide of the invention as defined above, said method comprising the step of recovering a body fluid from a transgenic animal comprising a vector or a female descendant thereof, wherein the body fluid contains a polypeptide, and, optionally recovery of a polypeptide from said body fluid. Such methods are also described in WO 01/57079 and in the references cited therein. A body fluid containing a polypeptide preferably is blood or more preferably milk.

Another method for preparing a polypeptide is to employ an *in vitro* transcription/translation system. DNA encoding a polypeptide is cloned into an expression vector as described *supra.* Said expression vector is then transcribed and translated *in vitro.* A translation product can be used directly or first purified. A polypeptide resulting from *in vitro* translation typically does not contain the post-translation modifications present on a polypeptide synthesised *in vivo,* although due to the inherent presence of microsomes some post-translational modification may occur. Methods for synthesis of polypeptides by *in vitro* translation are described by, for example, Berger & Kimmel, Methods in Enzymology, Volume 152, Guide to Molecular Cloning Techniques, Academic Press, Inc., San Diego, CA, 1987.

### Gene therapy

Some aspects of the invention concern the use of a nucleic acid construct or expression vector comprising a nucleotide sequence as defined above, wherein the vector is a vector that is suitable for gene therapy. Vectors that are suitable for gene therapy are described in Anderson 1998, Nature 392: 25-30; Walther and Stein, 2000, Drugs 60: 249-71; Kay et al., 2001, Nat. Med. 7: 33-40; Russell, 2000, J. Gen. Virol. 81: 2573-604; Amado and Chen, 1999, Science 285: 674-6; Federico, 1999, Curr. Opin. Biotechnol.10: 448-53; Vigna and Naldini, 2000, J. Gene Med. 2: 308-16; Marin et al., 1997, Mol. Med. Today 3: 396-403; Peng and Russell, 1999, Curr. Opin. Biotechnol. 10: 454-7; Sommerfelt, 1999, J. Gen. Virol. 80: 3049-64; Reiser, 2000, Gene Ther. 7: 910-3; and references cited therein.

Particularly suitable gene therapy vectors include Adenoviral and Adeno-associated virus (AAV) vectors. These vectors infect a wide number of dividing and non-dividing cell types including neuronal cells. In addition an adenoviral vector is usually capable of high levels oftransgene expression. However, because of the episomal nature of the adenoviral and AAV vectors after cell entry, these viral vectors are most suited for therapeutic applications requiring only transient expression of a transgene (Russell, 2000, J. Gen. Virol. 81: 2573-2604; Goncalves, 2005, Virol J. 2(1):43) as indicated above. A preferred adenoviral vector is modified to reduce the host response as reviewed by Russell (2000, *supra*). Method for neuronal gene therapy using a AAV vector is described by Wang et al., 2005, J Gene Med. March 9 (Epub ahead of print), Mandel et al., 2004, Curr Opin Mol Ther. 6(5):482-90, and Martin et al., 2004, Eye 18(11):1049-55. For intestinal gene transfer, an AAV serotype 1, 2 and 5 is an effective vector and therefore a preferred AAV serotype.

A preferred retroviral vector for application in the present invention is a lentiviral based expression construct. Lentiviral vectors have the unique ability to infect non-dividing cells (Amado and Chen, 1999 Science 285: 674-6). Methods for the construction and use of lentiviral based expression constructs are described in U.S. Patent No.'s 6,165,782, 6,207,455, 6,218,181, 6,277,633 and 6,323,031 and in Federico (1999, Curr Opin Biotechnol 10: 448-53) and Vigna et al. (2000, J Gene Med 2000; 2: 308-16). Generally, gene therapy vectors will be as the expression vectors described above in the sense that they comprise a nucleotide sequence encoding a polypeptide of the invention to be expressed, whereby a nucleotide sequence is operably linked to the appropriate regulatory sequences as indicated above. Such regulatory sequence will at least comprise a promoter sequence. A suitable promoter for expression of a nucleotide sequence encoding a polypeptide from gene therapy vectors includes e.g. cytomegalovirus (CMV) intermediate early promoter, viral long terminal repeat promoters (LTRs), such as those from murine moloney leukaemia virus (MMLV) rous sarcoma virus, or HTLV-1, the simian virus 40 (SV 40) early promoter and the herpes simplex virus thymidine kinase promoter. Suitable neuronal promoters are described above.

Several inducible promoter systems have been described that may be induced by the administration of small organic or inorganic compounds. Such inducible promoters include those controlled by heavy metals, such as the metallothionine promoter (Brinster et al. 1982 Nature 296: 39-42; Mayo et al. 1982 Cell 29: 99-108), RU-486 (a progesterone antagonist) (Wang et al. 1994 Proc. Natl. Acad. Sci. USA 91: 8180-8184), steroids (Mader and White, 1993 Proc. Natl. Acad. Sci. USA 90: 5603-5607), tetracycline (Gossen and Bujard 1992 Proc. Natl. Acad. Sci. USA 89: 5547-5551; U.S. Pat. No. 5,464,758; Furth et al. 1994 Proc. Natl. Acad. Sci. USA 91: 9302-9306; Howe et al. 1995 J. Biol. Chem. 270: 14168-14174; Resnitzky et al. 1994 Mol. Cell. Biol. 14: 1669-1679; Shockett et al. 1995 Proc. Natl. Acad. Sci. USA 92: 6522-6526) and the tTAER system that is based on the multi-chimeric transactivator composed of a tetR polypeptide, as activation domain of VP16, and a ligand binding domain of an estrogen receptor (Yee et al., 2002, US 6,432,705).

Suitable promoters for nucleotide sequences encoding small RNAs for knock down of specific genes by RNA interference (see below) include, in addition to the above mentioned polymerase II promoters, polymerase III promoters. The RNA polymerase III (pol III) is responsible for the synthesis of a large variety of small nuclear and cytoplasmic non-coding RNAs including SS, U6, adenovirus VA1, Vault, telomerase RNA, and tRNAs. The promoter structures of a large number of genes encoding these RNAs have been determined and it has been found that RNA pol III promoters fall into three types of structures (for a review see Geiduschek and Tocchini-Valentini, 1988 Annu. Rev. Biochem. 57: 873-914; Willis, 1993 Eur. J. Biochem. 212: 1-11; Hernandez, 2001, J. Biol. Chem. 276: 26733-36). Particularly suitable for expression of siRNAs are the type 3 of the RNA pol III promoters, whereby transcription is driven by cis-acting elements found only in the 5'-flanking region, i.e. upstream of the transcription start site. An upstream sequence element includes a traditional TATA box (Mattaj et al., 1988 Cell 55, 435-442), proximal sequence element and a distal sequence element (DSE; Gupta and Reddy, 1991 Nucleic Acids Res. 19, 2073-2075). An example of a gene under the control of the type 3 pol III promoter is a U6 small nuclear RNA (U6 snRNA), 7SK, Y, MRP, H1 and telomerase RNA genes (see e.g. Myslinski et al., 2001, Nucl. Acids Res. 21: 2502-09).

A gene therapy vector may optionally comprise a second or one or more further nucleotide sequence coding for a second or further protein. A second or further protein may be a (selectable) marker protein that allows for the identification, selection and/or screening for a cell containing the expression construct. A suitable marker protein for this purpose is e.g. the fluorescent protein GFP, and the selectable marker genes HSV thymidine kinase (for selection on HAT medium), bacterial hygromycin B phosphotransferase (for selection on hygromycin B), Tn5 aminoglycoside phosphotransferase (for selection on G418), and dihydrofolate reductase (DHFR) (for selection on methotrexate), CD20, the low affinity nerve growth factor gene. Sources for obtaining these marker genes and methods for their use are provided in Sambrook and Russel (2001) "Molecular Cloning: A Laboratory Manual (3rd edition), Cold Spring Harbor Laboratory, Cold Spring Harbor Laboratory Press, New York.

Alternatively, a second or further nucleotide sequence may encode a protein that provides for fail-safe mechanism that allows to cure a subject from a transgenic cell, if deemed necessary. Such a nucleotide sequence, often referred to as a suicide gene, encodes a protein that is capable of converting a pro-drug into a toxic substance that is capable of killing a transgenic cell in which said protein is expressed. Suitable examples of such suicide genes include e.g. the *E.coli* cytosine deaminase gene or one of the thymidine kinase genes from Herpes Simplex Virus, Cytomegalovirus and Varicella-Zoster virus, in which case ganciclovir may be used as prodrug to kill the IL-10 transgenic cells in the subject (see e.g. Clair et al., 1987, Antimicrob. Agents Chemother. 31: 844-849).

A gene therapy vector is preferably formulated in a pharmaceutical composition comprising a suitable pharmaceutical carrier as defined below.

### RNA interference

For knock down of or decreasing the expression of a TNIK polypeptide of the invention, a gene therapy vector or another expression construct is used for the expression of a desired nucleotide sequence that preferably encodes an RNAi agent, i.e. an RNA molecule that is capable of RNA interference or that is part of an RNA molecule that is capable of RNA interference. Such a RNA molecule is referred to as siRNA (short interfering RNA, including e.g. a short hairpin RNA). Alternatively, a siRNA molecule may directly, e.g. in a pharmaceutical composition that is administered to a subject in a need thereof.

A desired nucleotide sequence comprises an antisense code DNA coding for the antisense RNA directed against a region of the target gene mRNA, and/or a sense code DNA coding for the sense RNA directed against the same region of the target gene mRNA. In a DNA construct of the invention, the antisense and sense code DNAs are operably linked to one or more promoters as herein defined above that are capable of expressing the antisense and sense RNAs, respectively. "siRNA" means a small interfering RNA that is a short-length double-stranded RNA that are not toxic in mammalian cells (Elbashir et al., 2001, Nature 411: 494-98; Caplen et al., 2001, Proc. Natl. Acad. Sci. USA 98: 9742-47). The length is not necessarily limited to 21 to 23 nucleotides. There is no particular limitation in the length of siRNA as long as it does not show toxicity. "siRNAs" can be, e.g. at least 15, 18 or 21 nucleotides and up to 25, 30, 35 or 49 nucleotides long. Alternatively, the double-stranded RNA portion of a final transcription product of siRNA to be expressed can be, e.g. at least 15, 18 or 21 nucleotides and up to 25, 30, 35 or 49 nucleotides long.

"Antisense RNA" is an RNA strand having a sequence complementary to a target gene mRNA, and thought to induce RNAi by binding to the target gene mRNA. "Sense RNA" has a sequence complementary to the antisense RNA, and annealed to its complementary antisense RNA to form siRNA. The term "target gene" in this context refers to a gene whose expression is to be silenced due to siRNA to be expressed by the present system, and can be arbitrarily selected. As this target gene, for example, genes whose sequences are known but whose functions remain to be elucidated, and genes whose expressions are thought to be causative of diseases are preferably selected. A target gene may be one whose genome sequence has not been fully elucidated, as long as a partial sequence of mRNA of the gene having at least 15 nucleotides or more, which is a length capable of binding to one of the strands (antisense RNA strand) of siRNA, has been determined. Therefore, genes, expressed sequence tags (ESTs) and portions of mRNA, of which some sequence (preferably at least 15 nucleotides) has been elucidated, may be selected as the "target gene" even if their full length sequences have not been determined.

The double-stranded RNA portions of siRNAs in which two RNA strands pair up are not limited to the completely paired ones, and may contain non-pairing portions due to mismatch (the corresponding nucleotides are not complementary), bulge (lacking in the corresponding complementary nucleotide on one strand), and the like. Non-pairing portions can be contained to the extent that they do not interfere with siRNA formation. The "bulge" used herein preferably comprise 1 to 2 non-pairing nucleotides, and the double-stranded RNA region of siRNAs in which two RNA strands pair up contains preferably 1 to 7, more preferably 1 to 5 bulges. In addition, the "mismatch" used herein is contained in the double-stranded RNA region of siRNAs in which two RNA strands pair up, preferably 1 to 7, more preferably 1 to 5, in number. In a preferable mismatch, one of the nucleotides is guanine, and the other is uracil. Such a mismatch is due to a mutation from C to T, G to A, or mixtures thereof in DNA coding for sense RNA, but not particularly limited to them. Furthermore, in the present invention, the double-stranded RNA region of siRNAs in which two RNA strands pair up may contain both bulge and mismatched, which sum up to, preferably 1 to 7, more preferably 1 to 5 in number. Such non-pairing portions (mismatches or bulges, etc.) can suppress the below-described recombination between antisense and sense code DNAs and make the siRNA expression system as described below stable. Furthermore, although it is difficult to sequence stem loop DNA containing no non-pairing portion in the double-stranded RNA region of siRNAs in which two RNA strands pair up, the sequencing is enabled by introducing mismatches or bulges as described above. Moreover, siRNAs containing mismatches or bulges in the pairing double-stranded RNA region have the advantage of being stable in *E. coli* or animal cells.

The terminal structure of siRNA may be either blunt or cohesive (overhanging) as long as siRNA enables to silence the target gene expression due to its RNAi effect. The cohesive (overhanging) end structure is not limited only to the 3' overhang, and the 5' overhanging structure may be included as long as it is capable of inducing the RNAi effect. In addition, the number of overhanging nucleotide is not limited to the already reported 2 or 3, but can be any numbers as long as the overhang is capable of inducing the RNAi effect. For example, the overhang consists of 1 to 8, preferably 2 to 4 nucleotides. Herein, the total length of siRNA having cohesive end structure is expressed as the sum of the length of the paired double-stranded portion and that of a pair comprising overhanging single-strands at both ends. For example, in the case of 19 bp double-stranded RNA portion with 4 nucleotide overhangs at both ends, the total length is expressed as 23 bp. Furthermore, since this overhanging sequence has low specificity to a target gene, it is not necessarily complementary (antisense) or identical (sense) to the target gene sequence. Furthermore, as long as siRNA is able to maintain its gene silencing effect on the target gene, siRNA may contain a low molecular weight RNA (which may be a natural RNA molecule such as tRNA, rRNA or viral RNA, or an artificial RNA molecule), for example, in the overhanging portion at its one end.

In addition, the terminal structure of the "siRNA" is necessarily the cut off structure at both ends as described above, and may have a stem-loop structure in which ends of one side of double-stranded RNA are connected by a linker RNA (a "shRNA"). The length of the double-stranded RNA region (stem-loop portion) can be, e.g. at least 15, 18 or 21 nucleotides and up to 25, 30, 35 or 49 nucleotides long. Alternatively, the length of the double-stranded RNA region that is a final transcription product of siRNAs to be expressed is, e.g. at least 15, 18 or 21 nucleotides and up to 25, 30, 35 or 49 nucleotides long. Furthermore, there is no particular limitation in the length of the linker as long as it has a length so as not to hinder the pairing of the stem portion. For example, for stable pairing of the stem portion and suppression of the recombination between DNAs coding for the portion, the linker portion may have a clover-leaf tRNA structure. Even though the linker has a length that hinders pairing of the stem portion, it is possible, for example, to construct the linker portion to include introns so that the introns are excised during processing of precursor RNA into mature RNA, thereby allowing pairing of the stem portion. In the case of a stem-loop siRNA, either end (head or tail) of RNA with no loop structure may have a low molecular weight RNA. As described above, this low molecular weight RNA may be a natural RNA molecule such as tRNA, rRNA, snRNA or viral RNA, or an artificial RNA molecule.

To express antisense and sense RNAs from the antisense and sense code DNAs respectively, a DNA construct of the present invention comprises a promoter as defined above. The number and the location of the promoter in a construct can in principle be arbitrarily selected as long as it is capable of expressing antisense and sense code DNAs. As a simple example of a DNA construct of the invention, a tandem expression system can be formed, in which a promoter is located upstream of both antisense and sense code DNAs. This tandem expression system is capable of producing siRNAs having the aforementioned cut off structure on both ends. In the stem-loop siRNA expression system (stem expression system), antisense and sense code DNAs are arranged in the opposite direction, and these DNAs are connected via a linker DNA to construct a unit. A promoter is linked to one side of this unit to construct a stem-loop siRNA expression system. Herein, there is no particular limitation in the length and sequence of the linker DNA, which may have any length and sequence as long as its sequence is not the termination sequence, and its length and sequence do not hinder the stem portion pairing during the mature RNA production as described above. As an example, DNA coding for the above-mentioned tRNA and such can be used as a linker

### DNA.

In both cases of tandem and stem-loop expression systems, the 5' end may be have a sequence capable of promoting the transcription from the promoter. More specifically, in the case of tandem siRNA, the efficiency of siRNA production may be improved by adding a sequence capable of promoting the transcription from the promoters at the 5' ends of antisense and sense code DNAs. In the case of stem-loop siRNA, such a sequence can be added at the 5' end of the above-described unit. A transcript from such a sequence may be used in a state of being attached to siRNA as long as the target gene silencing by siRNA is not hindered. If this state hinders the gene silencing, it is preferable to perform trimming of the transcript using a trimming means (for example, ribozyme as are known in the art). It will be clear to the skilled person that the antisense and sense RNAs may be expressed in the same vector or in different vectors. To avoid the addition of excess sequences downstream of the sense and antisense RNAs, it is preferred to place a terminator of transcription at the 3' ends of the respective strands (strands coding for antisense and sense RNAs). The terminator may be a sequence of four or more consecutive adenine (A) nucleotides.

### Antibodies

Some aspects of the invention concern the use of an antibody or antibody-fragment that specifically binds to a TNIK protein as defined above and that is able to inhibit an activity of TNIK. Said antibody is designated as an inhibiting-antibody. Methods for generating antibodies or antibody-fragments that specifically bind to a given polypeptide are described in e.g. Harlow and Lane (1988, Antibodies: A Laboratory Manual, Cold Spring Harbor Laboratory Press, Cold Spring Harbor, NY) and WO 91/19818; WO 91/18989; WO 92/01047; WO 92/06204; WO 92/18619; and US 6,420,113 and references cited therein. The term "specific binding," as used herein, includes both low and high affinity specific binding. Specific binding can be exhibited, e.g., by a low affinity antibody or antibody-fragment having a Kd of at least 10⁻⁴ M. Specific binding also can be exhibited by a high affinity antibody or antibody-fragment, for example, an antibody or antibody-fragment having a Kd of at least about of 10⁻⁷ M, at least 10⁻⁸ M, at least 10⁻⁹ M, at least 10⁻¹⁰ M, or can have a Kd of at least 10⁻¹¹ M or 10⁻¹² M or greater. A preferred assay to assess the inhibition of a TNIK activity by a TNIK antibody is the assessment of the phosphorylation of TCF4 as defined earlier. An antibody is preferably a "human antibody". A human antibody means an antibody in which the variable and constant domain sequences are derived from human sequences. A human antibody provides a substantial advantage in a use of the present invention, as it is expected to minimize the immunogenic and allergic responses that are associated with use of non-human antibodies in a human patient.

For treating a human subject, an antibody would preferably be a chimeric, deimmunised, humanized or human antibody. Such antibodies can reduce immunogenicity and thus avoid human anti-mouse antibody (HAMA) response. It is preferable that the antibody be IgG4, IgG2, or other genetically mutated IgG or IgM which does not augment antibody-dependent cellular cytotoxicity (S. M. Canfield and S. L. Morrison, J. Exp. Med., 1991: 173: 1483-1491) and complement mediated cytolysis (Y. Xu et al., J. Biol. Chem., 1994: 269: 3468-3474; V. L. Pulito et al., J. Immunol., 1996; 156: 2840-2850).

A chimeric antibody may be produced by recombinant processes well known in the art, and has an animal variable region and a human constant region. A humanized antibody usually has a greater degree of human peptide sequences than do chimeric antibodies. In a humanized antibody, only the complementarity determining regions (CDRs), which are responsible for antigen binding and specificity are animal derived and have an amino acid sequence corresponding to the animal antibody, and substantially all of the remaining portions of the molecule (except, in some cases, small portions of the framework regions within the variable region) are human derived and correspond in amino acid sequence to a human antibody (see L. Riechmann et al., Nature, 1988; 332:323-327; G. Winter, United States Patent No. C. Queen et al., U. S. patent number 5,530, 101).

A deimmunised antibody is an antibody in which the T and B cell epitopes have been eliminated, as described in International Patent Application PCT/GB98/01473. They have reduced immunogenicity when applied *in vivo.*

A human antibody can be made by several different ways, including by use of human immunoglobulin expression libraries (Stratagene Corp., La Jolla, California) to produce fragments of human antibodies VH, VL, Fv, Fd, Fab, or (Fab')2, and using these fragments to construct whole human antibodies using techniques similar to those for producing chimeric antibodies. Alternatively, these fragments may be used on their own as inhibitor. Human antibodies can also be produced in transgenic mice with a human immunoglobulin genome. Such mice are available from Abgenix. Inc., Fremont, California, and Medarex, Inc., Annandale, New Jersey.

One can also create single peptide chain binding molecule in which the heavy and light chain Fv regions are connected. Single chain antibodies ("ScFv") and the method of their construction are described in U. S. Patent No. 4,946,778. Alternatively, Fab can be constructed and expressed by similar means (M. J. Evans et al., J.Immunol. Meth., 1995; 184:123-138). All of the wholly and partially human antibodies are less immunogenic than wholly murine MAbs, and the fragments and single chain antibodies are also less immunogenic. All these types of antibodies are therefore less likely to evoke an immune or allergic response. Consequently, they are better suited for *in vivo* administration in a human subject than wholly animal antibodies, especially when repeated or long-term administration is necessary. In addition, the smaller size of the antibody fragment may help improve tissue bioavailability, which may be critical for better dose accumulation in acute disease indications, such as tumor treatment.

### Peptidomimetics

A small molecule or peptide-like molecule (referred to as peptidomimetics) or a non-peptide molecule that specifically binds to a protein TNIK, that is able to inhibit an activity of TNIK and that may be applied in any of the methods of the invention as defined herein as an antagonist of a protein TNIK of the invention and they may be identified using methods known in the art per se, as e.g. described in detail in US 6,180,084 which incorporated herein by reference. Such methods include e.g. screening libraries of peptidomimetics, peptides, DNA or cDNA expression libraries, combinatorial chemistry and, particularly useful, phage display libraries. These libraries may be screened for an antagonist of a TNIK protein by contacting the libraries with a substantially purified TNIK polypeptide of the invention, a fragment thereof or a structural analogue thereof. A preferred assay to assess the inhibition of a TNIK activity by a peptide-like molecule (referred to as peptidomimetics) or a non-peptide is the assessment of the phosphorylation of TCF4 as defined earlier.

### Pharmaceutical composition

The invention further relates to a pharmaceutical preparation or composition comprising as active ingredient an inhibitor of TNIK, preferably at least one of an antibody, a dominant negative of TNIK, a nucleic acid, an antisense molecule or a nucleic acid construct or a gene therapy vector as defined above. The composition preferably at least comprises a pharmaceutically acceptable carrier in addition to an active ingredient.

This composition is preferably for treating cancer.

In some embodiments, a nucleic acid or nucleic acid construct or antibody or dominant negative of the invention as purified from mammalian, insect or microbial cell cultures, from milk of transgenic mammals or other source is administered in purified form together with a pharmaceutical carrier as a pharmaceutical composition. A method of producing a pharmaceutical composition comprising a polypeptide is described in US Patents No.'s 5,789,543 and 6,207,718. The preferred form depends on the intended mode of administration and therapeutic application.

A pharmaceutical carrier can be any compatible, non-toxic substance suitable to deliver an antibody, a dominant negative molecule or a nucleic acid or a gene therapy vector to the patient. Sterile water, alcohol, fats, waxes, and inert solids may be used as the carrier. A pharmaceutically acceptable adjuvant, buffering agent, dispersing agent, and the like, may also be incorporated into a pharmaceutical composition.

The concentration of an antibody or dominant negative or nucleic acid of nucleic acid construct of the invention in a pharmaceutical composition can vary widely, i.e., from less than about 0.1 % by weight, usually being at least about 1% by weight to as much as 20% by weight or more.

For oral administration, an active ingredient can be administered in solid dosage forms, such as capsules, tablets, and powders, or in liquid dosage forms, such as elixirs, syrups, and suspensions. Active component(s) can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate and the like. An example of an additional inactive ingredient that may be added to provide desirable colour, taste, stability, buffering capacity, dispersion or other known desirable features are red iron oxide, silica gel, sodium lauryl sulfate, titanium dioxide, edible white ink and the like. A similar diluent can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain colouring and flavouring to increase patient acceptance.

An antibody or dominant negative molecule or nucleic acid or nucleic acid construct or gene therapy vector is preferably administered parentally. An antibody or dominant negative molecule or nucleic acid or nucleic acid construct or vector for a preparation for parental administration must be sterile. Sterilisation is readily accomplished by filtration through sterile filtration membranes, prior to or following lyophilisation and reconstitution. The parental route for administration of an antibody or dominant negative molecule or nucleic acid or nucleic acid construct or vector is in accord with known methods, e.g. injection or infusion by intravenous, intraperitoneal, intramuscular, intraarterial, intralesional, intracranial, intrathecal, transdermal, nasal, buccal, rectal, or vaginal routes. An antibody or dominant negative molecule or nucleic acid or nucleic acid construct or vector is administered continuously by infusion or by bolus injection. A typical composition for intravenous infusion could be made up to contain 10 to 50 ml of sterile 0.9% NaCl or 5% glucose optionally supplemented with a 20% albumin solution and 1 to 50 µg of a polypeptide, antibody or dominant negatif or nucleic acid or nucleic acid construct or vector. A typical pharmaceutical composition for intramuscular injection would be made up to contain, for example, 1 - 10 ml of sterile buffered water and 1 to 100 µg of an antibody or dominant negative molecule or nucleic acid or nucleic acid construct or vector of the invention. Methods for preparing a parenterally administrable composition is well known in the art and described in more detail in various sources, including, for example, Remington's Pharmaceutical Science (15th ed., Mack Publishing, Easton, PA, 1980) (incorporated by reference in its entirety for all purposes).

A composition comprising a TNIK inhibitor may comprise an excipient that will aid in delivery of each of the constituents as defined herein to a cell and/or into a cell, preferably a cancer cell or cancer stem cell or intestinal cancer cell or a cancer stem cell. Preferred are excipients capable of forming complexes, nanoparticles, micelles, vesicles and/or liposomes that deliver each constituent as defined herein, complexed or trapped in a vesicle or liposome through a cell membrane. Many of these excipients are known in the art. Suitable excipients comprise polyethylenimine (PEI), or similar cationic polymers, including polypropyleneimine or polyethylenimine copolymers (PECs) and derivatives, synthetic amphiphils (SAINT-18), lipofectinTM, DOTAP and/or viral capsid proteins that are capable of self assembly into particles that can deliver each constitutent as defined herein to a cell, preferably a cancer cell or cancer stem cell or intestinal cancer cell or a cancer stem cell. Such excipients have been shown to efficiently deliver nucleic acids for example to a wide variety of cultured cells. Their high transfection potential is combined with an excepted low to moderate toxicity in terms of overall cell survival. The ease of structural modification can be used to allow further modifications and the analysis of their further (*in vivo*) nucleic acid transfer characteristics and toxicity.

Lipofectin represents an example of a liposomal transfection agent. It consists of two lipid components, a cationic lipid N-[1-(2,3 dioleoyloxy)propyl]-N,N,N-trimethylammonium chloride (DOTMA) (cp. DOTAP which is the methylsulfate salt) and a neutral lipid dioleoylphosphatidylethanolamine (DOPE). The neutral component mediates the intracellular release. Another group of delivery systems are polymeric nanoparticles.

Polycations such like diethylaminoethylaminoethyl (DEAE)-dextran, which are well known as DNA transfection reagent can be combined with butylcyanoacrylate (PBCA) and hexylcyanoacrylate (PHCA) to formulate cationic nanoparticles that can deliver each constituent as defined herein, preferably a nucleic acid across cell membranes into cells.

In addition to these common nanoparticle materials, the cationic peptide protamine offers an alternative approach to formulate an inhibitor with colloids. This colloidal nanoparticle system can form so called proticles, which can be prepared by a simple self-assembly process to package and mediate intracellular release of an inhibitor. The skilled person may select and adapt any of the above or other commercially available alternative excipients and delivery systems to package and deliver an inhibitor for use in the current invention to deliver it for the treatment of cancer in humans.

In preferred embodiment, an inhibitor is formulated in a so-called slow release formulation or slow release vehicle. Such formulations are also named formulation with a delayed or controlled release. A controlled release formulation is a formulation that will release at least 20%, at least 25%, at least 30%, at least 35%, at least 40%, at least 45%, at least 50%, at least 55%, at least 60%, at least 65%, at least 70%, at least 75%, at least 80% of its active ingredient in a controlled fashion, i.e. a TNIK inhibitor within a day, a week, two weeks, three weeks, a month, or longer. Several types of slow release formulations are already known such as mineral oil (e.g. Montanide ISA 51) or Poly-lactic-co-glycolic acid (PLGA) or polymer based formulations. An example of a polymer-based formulation is a gel composition comprising charged polymers as described in WO 2005/110377 or a composition comprising a dextran hydrogel as described in WO 02/17884 or WO 2005/051414 or US 3,710,795.

For therapeutic applications, a pharmaceutical composition is administered to a patient suffering from cancer in an amount sufficient to reduce the severity of symptoms and/or prevent or arrest further development of symptoms. An amount adequate to accomplish this is defined as a "therapeutically-" or "prophylactically-effective dose". Such effective dosages will depend on the severity of the condition and on the general state of the patient's health. In general, a therapeutically- or prophylactically-effective dose preferably is a dose, which is sufficient to reverse the symptoms to the average levels found in normal unaffected healthy individuals. Preferably, the symptom is the proliferation rate of cancer cells and/or the tumor mass.

In a present method of the invention, an antibody or dominant negative molecule or nucleic acid or nucleic acid construct or vector is usually administered at a dosage of about 1 µg/kg patient body weight or more per week to a patient. Often dosages are greater than 10 µg/kg per week. A dosage regime can range from 10 µg/kg per week to at least 1 mg/kg per week. Typically a dosage regime may be 10 µg/kg per week, 20 µg/kg per week, 30 µg/kg per week, 40 µg/kg week, 60 µg/kg week, 80 µg/kg per week and 120 µg/kg per week. In a preferred regime 10 µg/kg, 20 µg/kg or 40 µg/kg is administered once, twice or three times weekly. Treatment is preferably administered by parenteral route.

In addition, an inhibitor could be covalently or non-covalently linked to a targeting ligand specifically designed to facilitate the uptake in to a cell, cytoplasm and/or its nucleus. Such ligand could comprise (i) a molecule (including but not limited to peptide(-like) structures) recognising cell, tissue or organ specific elements facilitating cellular uptake and/or (ii) a molecule able to facilitate the uptake in to a cell and/or the intracellular release of an inhibitor from vesicles, e.g. endosomes or lysosomes.

Therefore, in a preferred embodiment, an inhibitor is formulated in a composition or a medicament or a composition which is provided with at least an excipient and/or a targeting ligand for delivery and/or a delivery device thereof to a cell and/or enhancing its intracellular delivery. Accordingly, the invention also encompasses a pharmaceutically acceptable composition comprising an inhibitor and further comprising at least one excipient and/or a targeting ligand for delivery and/or a delivery device of said inhibitor to a cell and/or enhancing its intracellular delivery.

Depending on their identity, the skilled person will know which type of formulation is the most appropriate for each constituent as defined herein.

In a preferred embodiment, a targeting part is added to a TNIK inhibitor to ensure said TNIK inhibitor is targeted to cancer cells, preferably to intestinal cancer cells or cancer stem cells. This may lower possible side effects and/or may improve the specificity of said inhibitor. A TNIK inhibitor may be targeted to a cell that has a constitutively active Wnt pathway, preferably a tumor cell, more preferably a colon tumor cell, or more preferably a colon cancer stem cell. A targeting part may be an antibody that recognizes a protein expressed by the cell it needs to be targeted to, or a ligand specific for a receptor that is expressed by the cell it needs to be targeted to, or derivatives thereof. Examples of such antibodies may be an α-Her2/Neu antibody targeting breast tumor cells, or an α-Lgr5 antibody, targeting Lgr5 positive cancer stem cells, or α-CD133 antibody for targeting CD133 positive cancer stem cells. A targeting part may be used in this context when the targeting of an inhibitor linked to this targeting part to cancer cells is increased of at least 5%, 10%, 15%, 20%, 25%, 30%, 35%, 40%, 45%, 50%, 55%, 60%, 65%, 70%, 75%, 80%, 85%, 90%, 95%, 100% or more compared to the targeting of corresponding inhibitor without targeting part. The targeting to cancer cells or cancer stem cells may be assessed using in vivo imaging techniques. To detect whether TNIK has been targeted, an inhibitor of TNIK may be radiolabeled or fluorescently labeled. A targeted compound may be conjugated to a substance that allows radioactive and fluorescent imaging, positron emission tomography (PET) scanning, magnetic resonance imaging (MRI) scanning, or X-ray/ computed tomography (CT) scanning. Examples of radioactive substances are radioactive labels such as Indium-11, Technetium-99m, Iodine-131 or Fluorine-19.

Examples of fluorescent substances are the fluorescent dye fluorescein isothiocyanate or aliphatic biodegradable photoluminescent polymers. The fluorescent or photoluminescent probes may also be present in a formulation that comprises a TNIK inhibitor, such as a liposomal formulation or a nanoparticle

In this document and in its claims, the verb "to comprise" and its conjugations is used in its non-limiting sense to mean that items following the word are included, but items not specifically mentioned are not excluded. In addition the verb "to consist" may be replaced by "to consist essentially of" meaning that an inhibitor, a product or a composition as defined herein may comprise additional component(s) than the ones specifically identified, said additional component(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one".

All patent and literature references cited in the present specification are hereby incorporated by reference in their entirety.

The following examples are offered for illustrative purposes only, and are not intended to limit the scope of the present invention in any way.

### Description of the figures

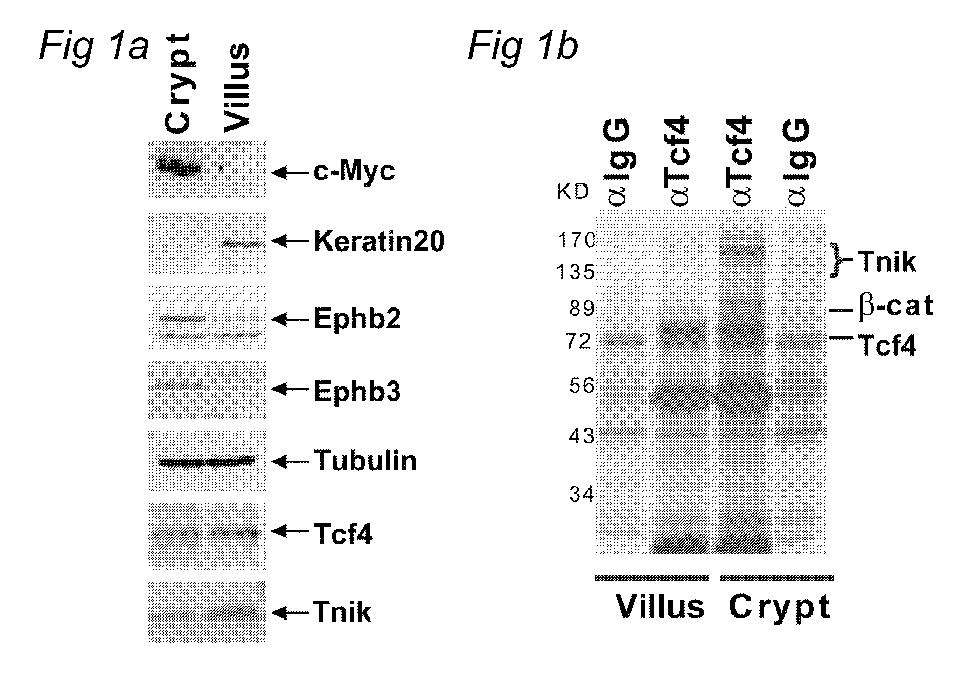

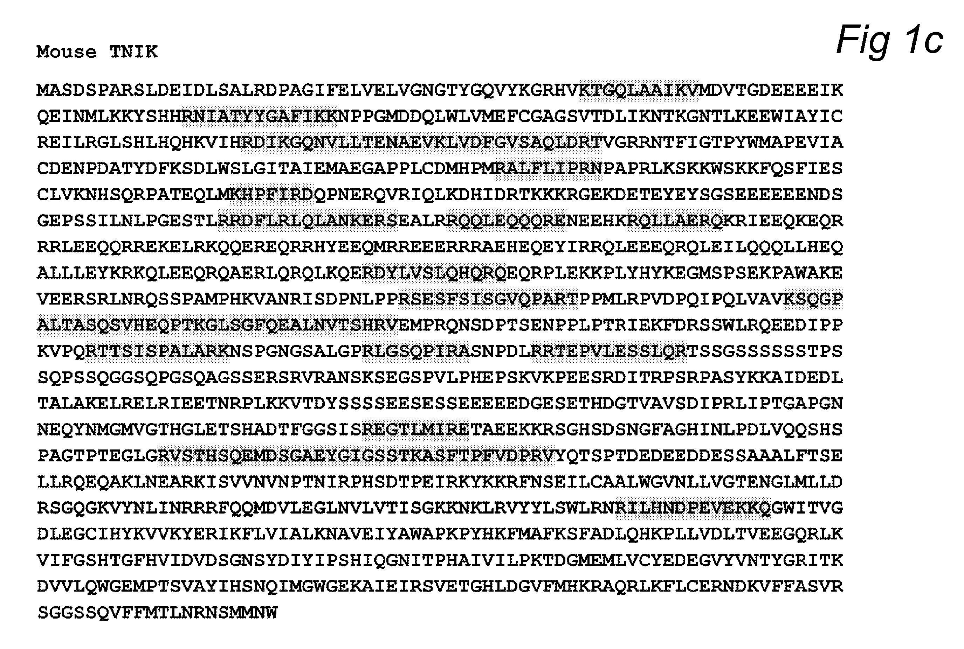

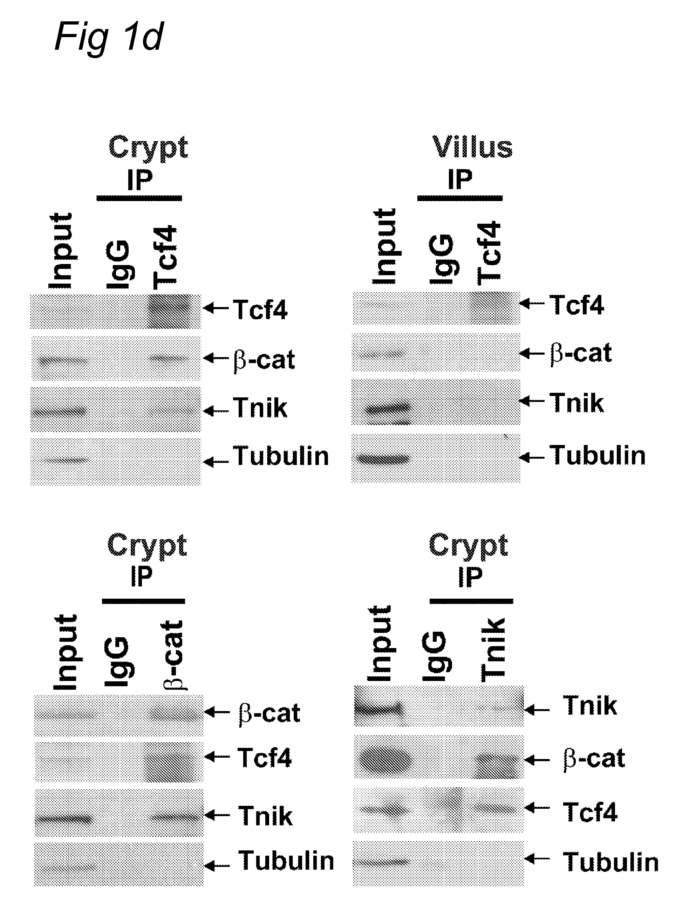

**Figure 1****. Tnik Co-immunoprecipitates with Tcf4 in the Mouse Small Intestinal Crypt.** (A) Biochemical purification of crypt and villus fractions from mouse small intestine used in Mass Spec experiment. Western blot analysis using antibodies directed against genes expressed specifically in the crypt (c-Myc, Ephb2 and Ephb3), villus (Keratin 20), as well as Tubulin as control were used to assess the quality of the purified fractions. (B) Silverstained gel of Tcf4 containing complexes immunoprecipitated from purified crypt and villus fractions using antibody directed against Tcf4. The Tcf4-interacting proteins are indicated by arrows and were identified by mass spectrometry (see Material and Methods for details). (C) Peptide coverage of Tnik in MS experiment. Amino acid sequences of Tnik peptides detected in the Tcf4 immunoprecipitate from crypt lysates are high-lighted in grey. (D) Cell lysates from purified crypt and villus fractions were immunoprecipitated with antibodies directed against endogenous Tcf4, β-catenin and Tnik as indicated and analyzed by Western blotting with the indicated antibodies.

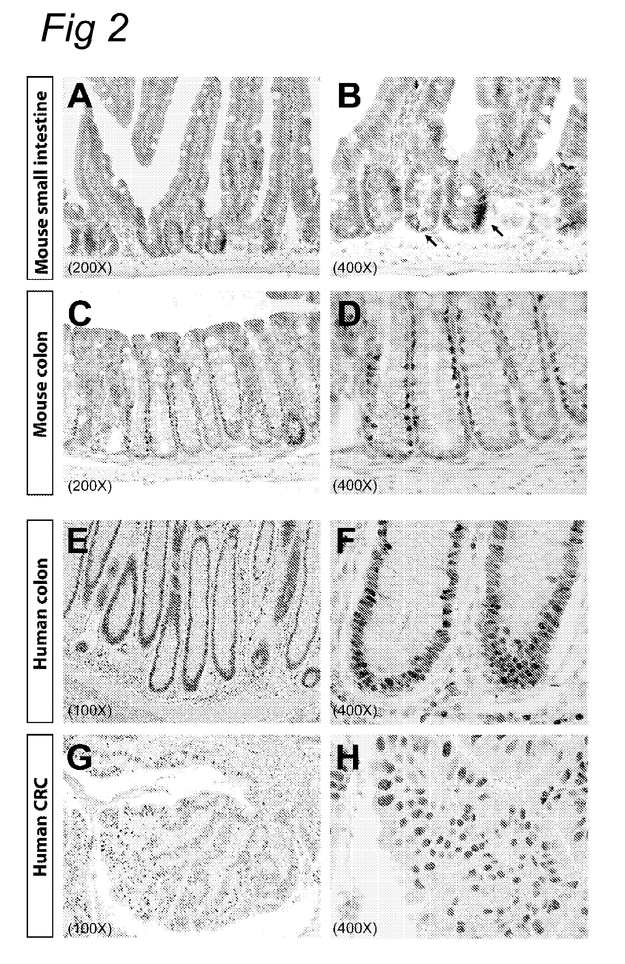

**Figure 2****. Nuclear localization of Tnik protein in Wnt activated intestinal crypt.** Immunohistochemistry of Tnik in mouse small intestine (A-B), mouse colon (C-D), human normal colon (E-F) and human colorectal tissues (G-H). (B) Nuclear localization of Tnik protein in mouse intestinal crypt was indicated by arrows. Corresponding magnification was show on the bottom left of the figures.

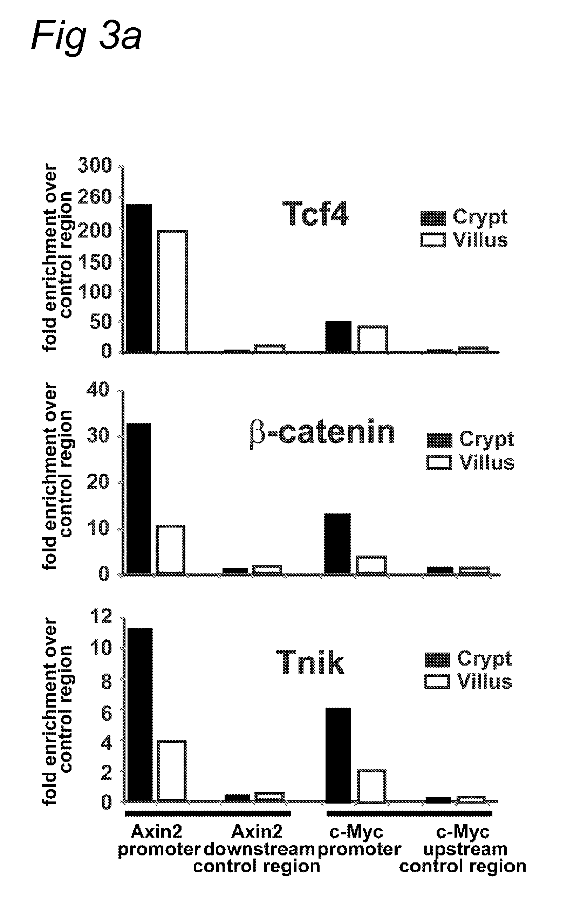

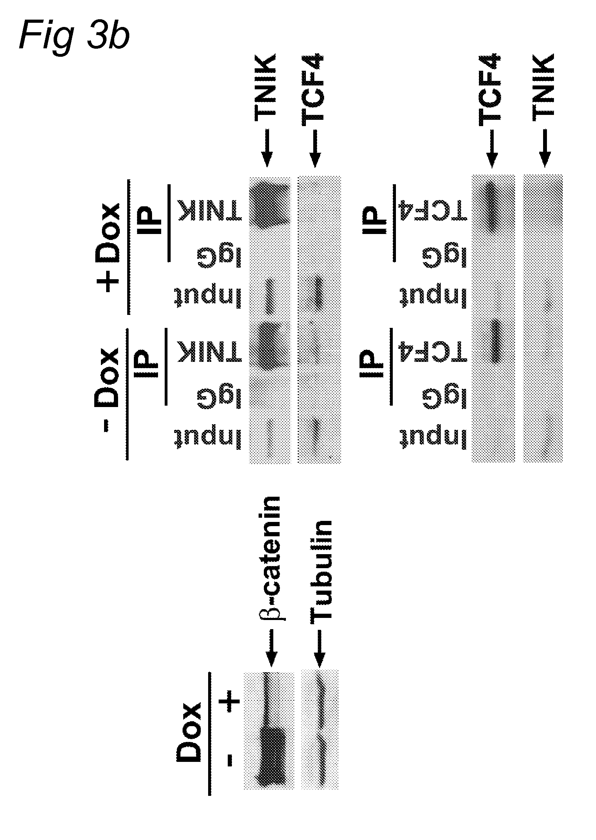

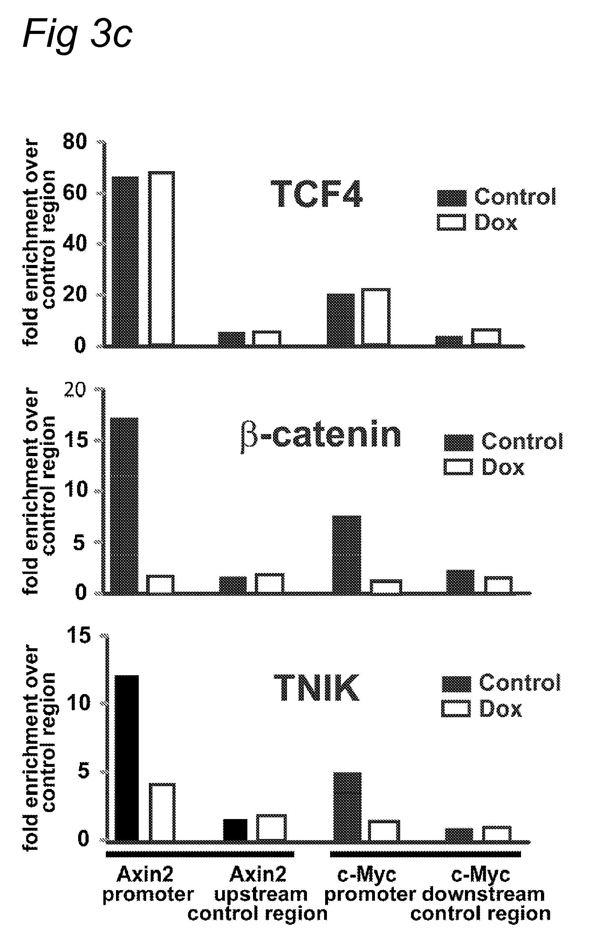

**Figure 3****. Tnik is Recruited to Wnt Target Genes in Mouse Crypts and CRC Cells in β-catenin-dependent manner.** (A) Purified crypt and villus fractions from mouse small intestine were subjected to chromatin immunoprecipitation using antibodies directed against Tcf4, β-catenin and Tnik. Formaldehyde cross-linked chromatin was immunoprecipitated with the specified antibodies followed by qPCR using primers specific for the Axin2 and c-Myc proximal promoters as well as Axin2 downstream and c-Myc upstream control regions as indicated. Results are presented as relative enrichment over the non-bound downstream SP5 control region and are representative of three independent experiments. (B) TNIK interaction with TCF4 is mediated by β-catenin. Western blot analysis of β-catenin depletion in Ls174T cells expressing doxycycline (Dox) inducible β-catenin shRNA (left panel). Immunoprecipitated TNIK-protein complexes from untreated or doxycycline-treated cells were resolved by SDS-PAGE followed by western blotting using antibodies directed against TNIK and TCF4 (top right panel), and the result was further confirmed by the reverse IP (bottom right panel). (C) Chromatin immunoprecipitation experiments in Ls174T cells uninduced or induced with Dox using antibodies specific for TCF4, β-catenin, and TNIK. The immunoprecipitated DNA was analyzed by qPCR using primer pairs specific for the Axin2 and c-Myc promoters, and Axin2 upstream and c-Myc downstream control regions. Results are presented as relative enrichment over the non-bound second exon of the myoglobin gene and are representative of three independent experiments.

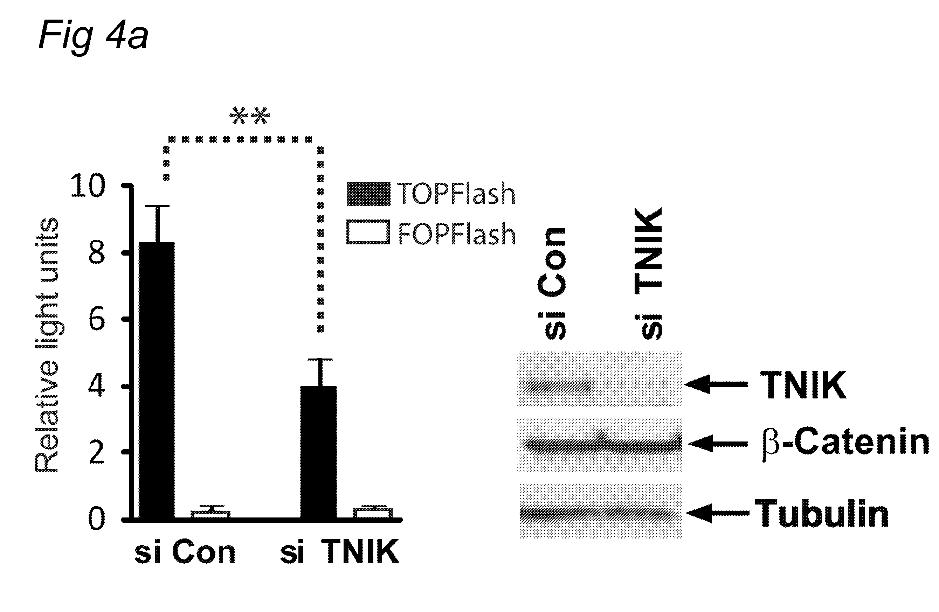

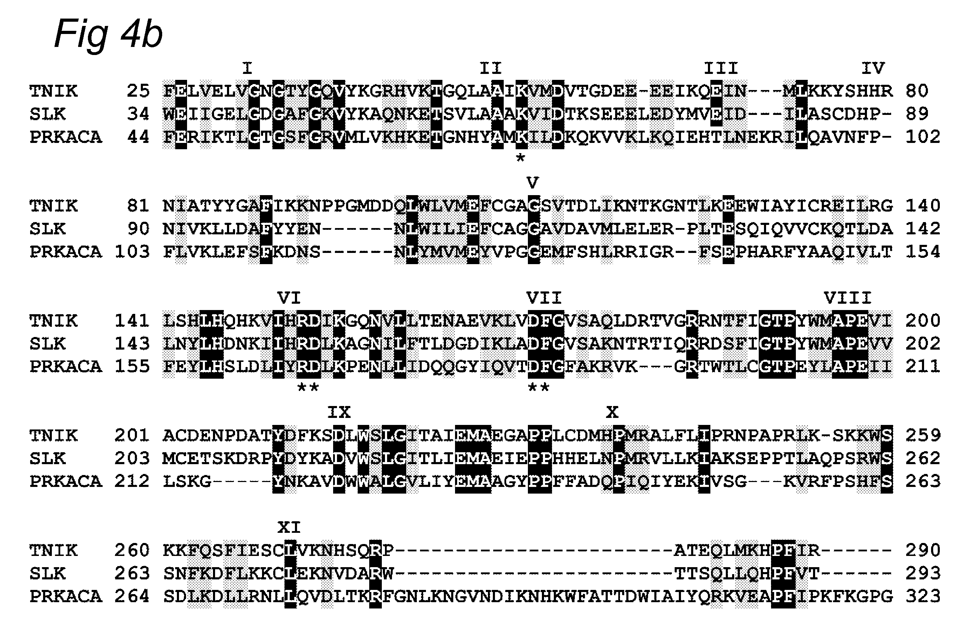

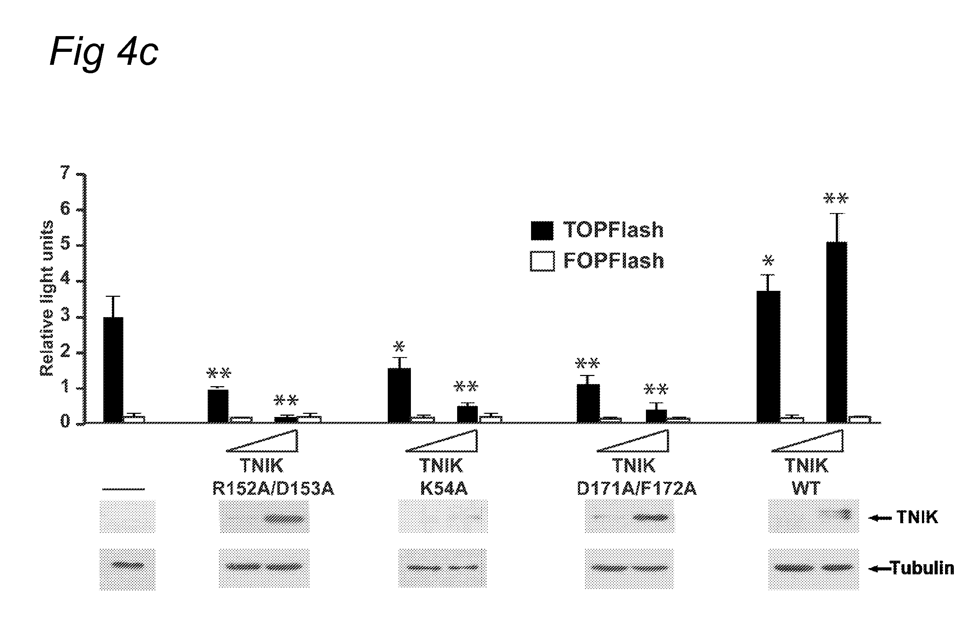

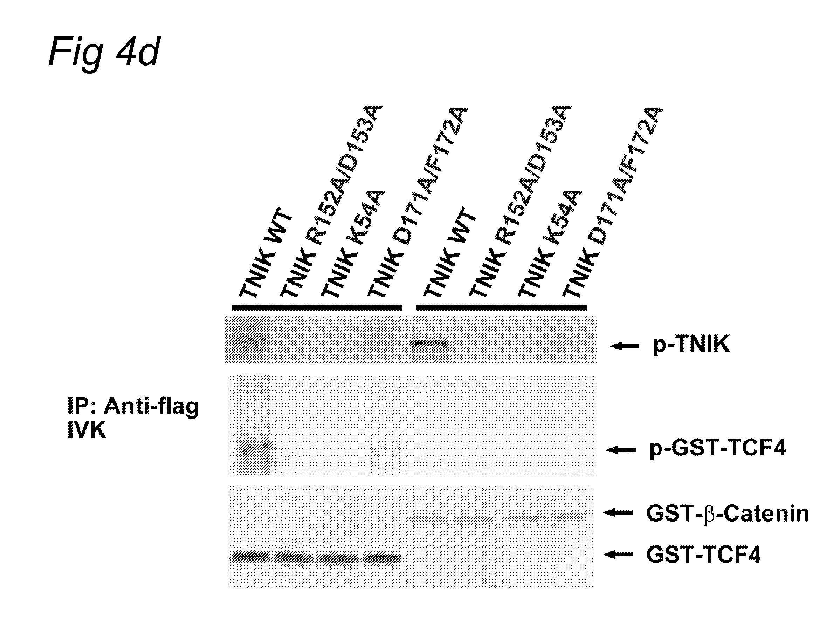

**Figure 4****. TNIK Kinase Activity is Required for TCF/LEF Mediated Transcription.** (A) siRNA mediated depletion of TNIK reduces β-catenin /TCF driven transcription in Ls174T CRCs. Activity of the TOPFlash (black bars) and FOPFlash (white bars) 96 hours post siRNA transfection is shown. Error bars represent standard deviation from three independent experiments. Expression of TNIK, β-catenin, and control Tubulin was analyzed by western blotting after depletion of TNIK (left panel). (B) Amino acid alignment of the N-terminal kinase domain of human TNIK with Ser/Thr kinases SLK and PRKACA. Identical amino acids are shown in black and conserved amino acids are highlighted in grey. The subdomains are indicated above the sequence by romain numbering (I-XI). Asterisks indicate the conserved amino acids mutated to generate TNIK mutants used in C. (C) Expression of TNIK kinase mutants (second, third and fourth panels) abrogates β-catenin /TCF driven transcription while over expression of WT TNIK (last panel) specifically increases β-catenin /TCF driven transcription in Ls174T CRCs. Black bars indicate TOPFlash activity while white bars indicate FOPFlash activity 24 hours post transfection of expression vectors. Error bars represent standard deviation from three independent experiments. Western blot analysis indicates expression of Flag-tagged WT and mutant TNIK proteins using M2 Flag antisera (bottom panel) and expression of Tubulin as a loading control. * *p-value*<0.05; ** *p-value*<0.01. (D) Phosphorylation of TCF4 by TNIK was demonstrated by *in vitro* kinase assay. WT TNIK was able to phosphorylate TCF4 but not β-catenin, whereas the phosphorylation was largely suppressed in all the TNIK kinase mutants (middle panel). The top panel shows that wt TNIK was phosphorylated and thereby active, whereas the deletion mutants were not. GST-tagged protein input was shown at the bottom.

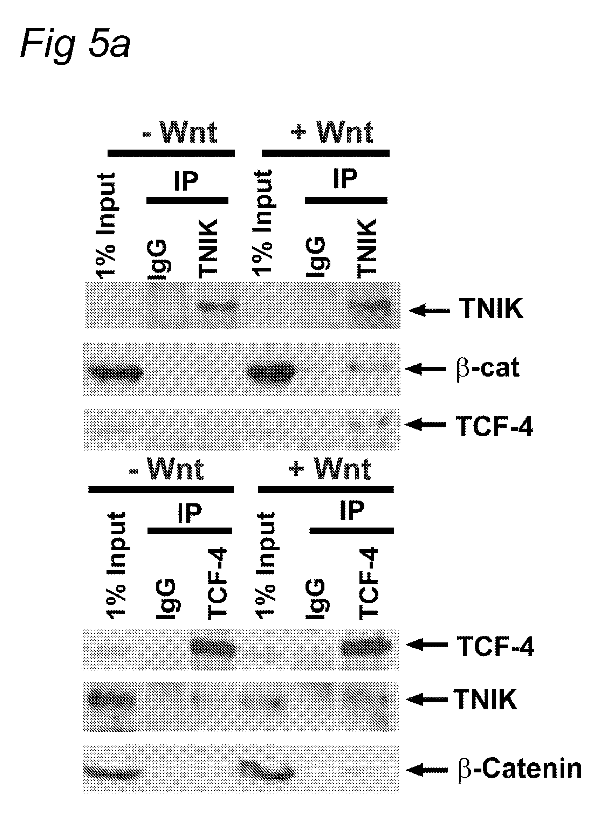

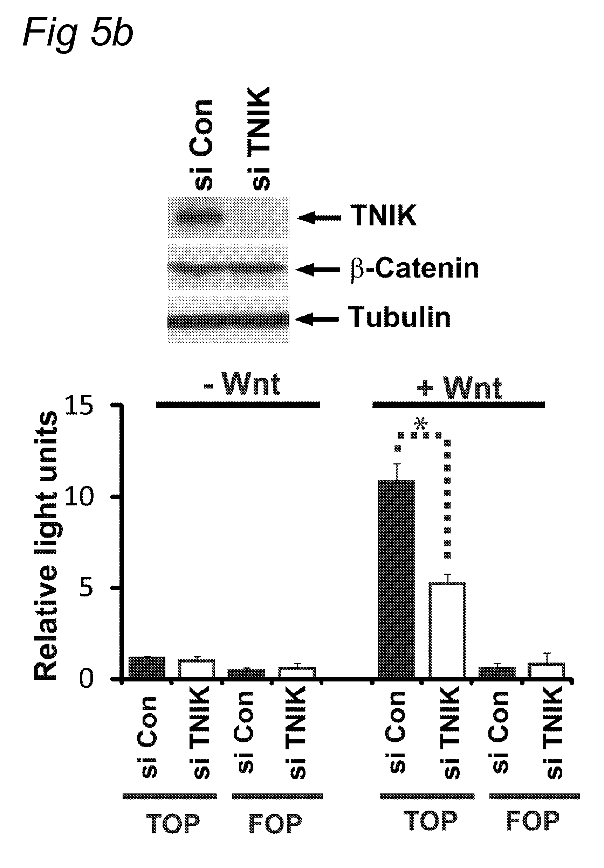

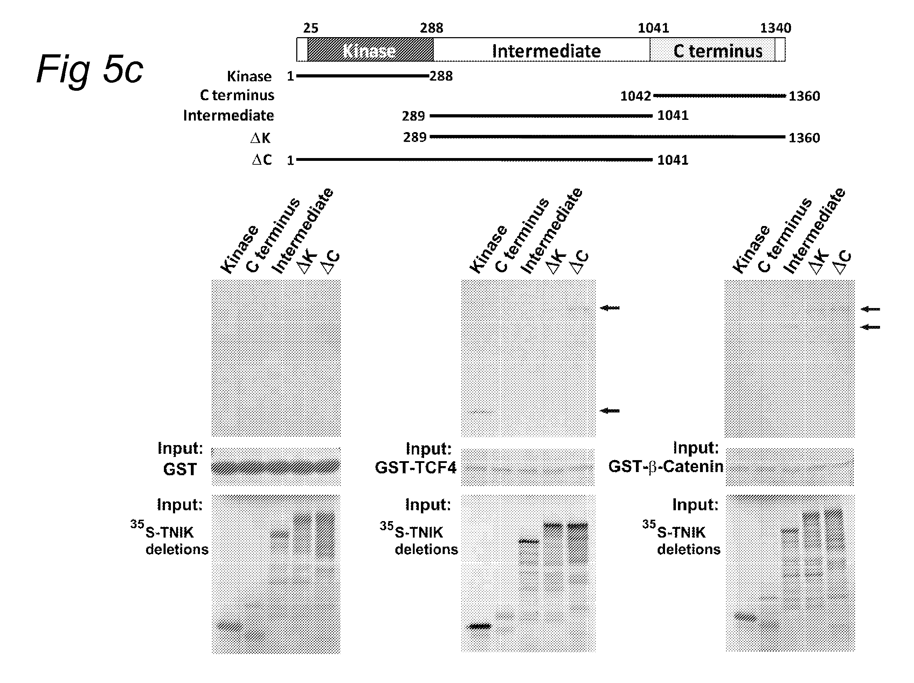

**Figure 5****. TNIK is Interacting with TCF4 in** β**-catenin Dependent Manner.** (A) TNIK interacts with TCF4 and β-catenin upon Wnt stimulation in HEK293T cells. Lysates from cells stimulated with either Wnt3A or control conditioned media as indicated for 8 hours were used to immunoprecipitate TCF4 (bottom panel) or TNIK (top panel) and their associated proteins. Immunoprecipitated protein complexes were resolved by SDS-PAGE followed by western blotting using antibodies directed against TNIK, β-catenin and TCF4 as indicated. (B) siRNA mediated depletion of TNIK reduces β-catenin /TCF driven transcription in HEK293T cells. Expression of TNIK and control Tubulin was analyzed by western blotting after depletion of TNIK (top panel). Activity of the TOPFlash and FOPFlash 12 hrs post Wnt stimulation is shown (bottom panel). Error bars represent standard deviation from three independent experiments. ** p-value=0.006.* (C) Schematic summary of different TNIK deletion mutants (top panel). All five TNIK deletion mutants were in vitro translated with S³⁵ labelling, and were incubated with GST-tagged TCF4 or β-catenin, followed by a GST pull down to examine the direct interaction. GST protein alone was used as control (bottom left panel). Only TNIK kinase and ΔC domains were binding to GST-TCF4 (bottom middle panel), whereas TNIK intermediate, ΔK and ΔC domains were binding to GST-β-catenin (bottom right panel).

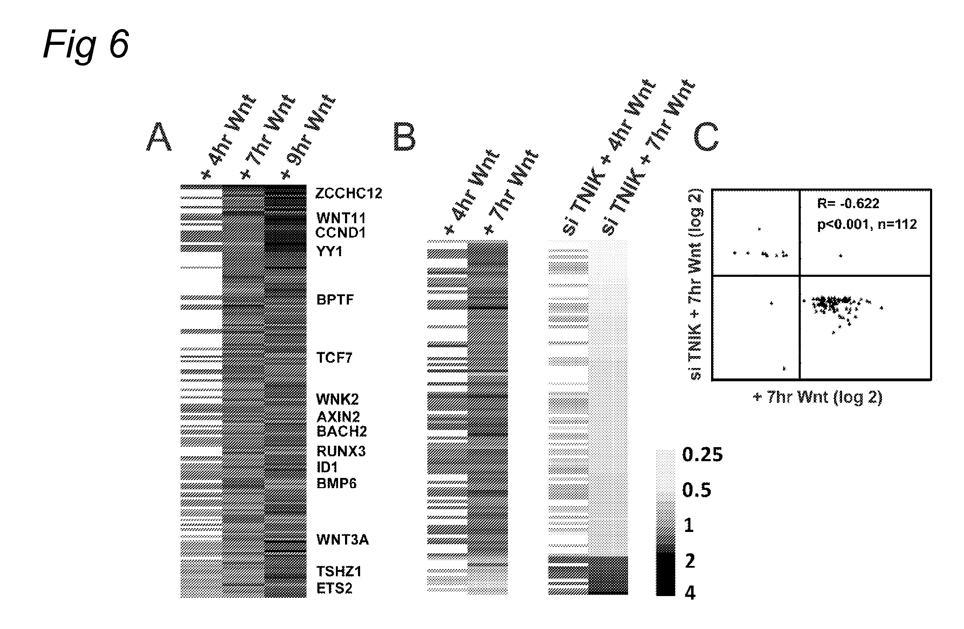

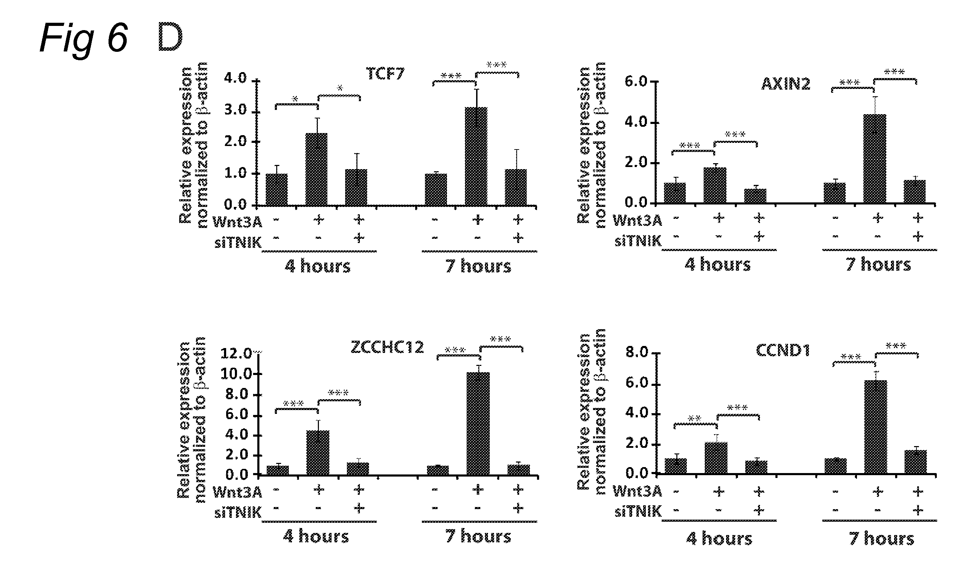

**Figure 6****. TNIK is an Essential Activator and Specifically Regulates Wnt Target Genes in HEK293T Cells.** (A) Expression pattern of 172 Wnt induced genes selected against overlapping probes with 1.5 fold change in either 7 or 9 hour Wnt induction. Representative Wnt induced genes were listed. (B) Differential expression pattern of genes after TNIK suppression in HEK293T cells upon 4 and 7 hours Wnt induction with greater than 1.5 fold variation at 7 hour time point. The corresponding expression pattern upon Wnt induction without TNIK suppression is shown on the left. Grey, down-regulated after TNIK suppression; Black, upregulated after TNIK suppression; White, missing data. (C) Significant negative correlation of the overlapping probes between 7 hours WNT induction and TNIK suppression selected by 1.5 fold change after TNIK suppression. X-axis: fold change (log2) after 7 hours Wnt induction; Y-axis: fold change (log2) after TNIK suppression. (D) Quantitative RT-PCR validation of four selected candidate genes. * *p-value*<0.05; ** *p-value*<0.01; *** *p-value*<0.001.

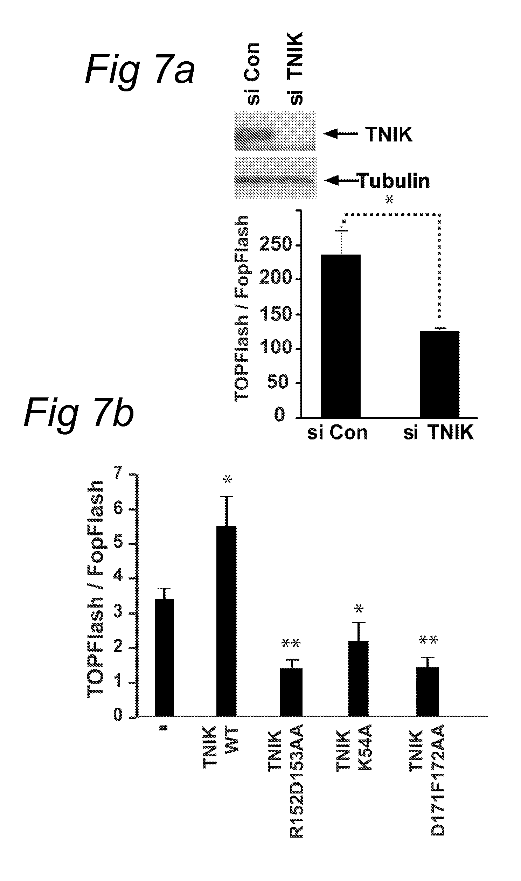

**Figure 7****. TNIK expression modulates TCF/LEF transcription regulation.** (A) Suppression of TOPFlash activity upon TNIK depletion in SW480 cells. (B) Over-expression of WT TNIK specifically increases β-catenin/TCF driven transcription in DLD1 CRC cells whereas expression of the three TNIK kinase mutants abrogates β-catenin/TCF driven transcription. Black bars indicate ratio of TOP/FOPFIash activity. * *p-value<*0.05; ** *p-value*<0.01.

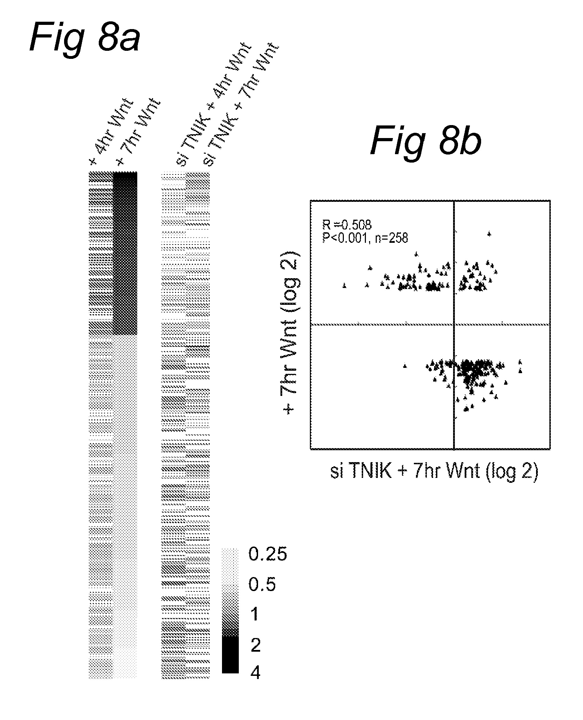

**Figure 8****. Comparison of expression profile changes between Wnt induction and TNIK depletion.** (A) Differential expression pattern of Wnt regulated genes in HEK293T cells upon 4 and 7 hours Wnt induction with greater than 1.5 fold variation at 7 hour time point. The corresponding expression pattern after TNIK suppression is shown on the right. Black, up-regulated by Wnt; grey, down-regulated by Wnt; White, missing data. (B) Significant negative correlation of the overlapping clones between 7 hours Wnt induction and TNIK suppression selected by 1.5 fold change after 7 hours Wnt induction. X-axis: fold change (log2) after TNIK suppression; Y-axis: fold change (log2) after 7 hours Wnt induction.

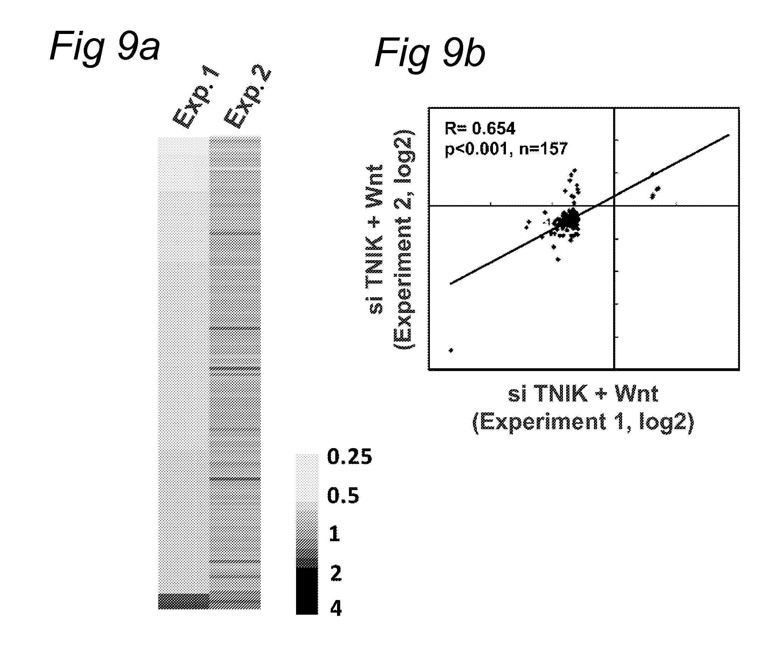

**Figure 9****. Comparison of independent biological replicates of si TNIK +7hr Wnt stimulation.** (A) Differential gene expression pattern after TNIK suppression in HEK293T cells with Wnt induction in two independent experiments with greater than 1.5 fold variation at 7 hour time point. (B) Pearson correlation of the 157 overlapping probes between the two biological replicates. Trendline representing the positive correlation is shown.

### Examples

### Tnik Co-immunoprecipitates with Tcf4 in the Mouse Small Intestinal Crypt

As an unbiased approach towards the identification of components of the endogenous TCF4 complex in murine small intestinal crypts and villi, we applied the combination of affinity purification and mass spectrometry (MS). We first developed a fractionation method to separate proliferative crypt epithelium from differentiated villus epithelium. The quality of the fractionation was assessed by western blot analysis of known proteins differentially expressed between the two compartments. The TCF4/β-catenin target genes/crypt markers c-Myc (He et al, 1998), EPHB2 and EPHB3 (van de Wetering et al, 2002) were strongly enriched in the crypt fraction, while the villus marker Keratin 20 (Calnek & Quaroni, 1993) was present exclusively in the purified villus fraction (Figure 1A). Tcf4 was detected in both crypt and villus fractions, as was tubulin.

To identify potential Tcf4 co-regulators in a physiological *in vivo* setting, we immunopurified Tcf4 from crypt and villus fractions using a TCF4 antibody as well as a non-immune IgG as control. The immunoprecipitates were subjected to SDS-PAGE and silver staining (Figure 1B), followed by mass spectrometric identification. Tcf4 and β-catenin were readily identified in the crypt fraction while Tcf4, but not β-catenin, was found in the villus fraction (see after table 2 the information identifying the peptides identified using MS). None of these proteins were observed in the IgG control. This implied that the approach allowed us to specifically isolate Wnt-activated Tcf4 complexes from crypts and inactive Tcf4 from villi. Amongst the proteins specifically co-precipitating with Tcf4/β-catenin from the crypt fraction, the serine/threonine kinase Tnik stood out in that a large portion of the protein sequence was covered by the MS-identified peptides (Figure 1C and see after table 2 the information identifying the peptides identified using MS). To confirm the interaction, we immunoprecipitated Tcf4 from crypt and villus fractions and probed for its association with endogenous Tnik by western blotting using a Tnik antibody. Despite the presence of Tnik protein in both fractions (Figure 1A), Tcf4 interacted with Tnik specifically in the crypt, but not the differentiated villus fraction (Figure 1D top panels). Immunoprecipitation (IP) of β-catenin from crypts followed by western blot analysis also detected Tnik (Figure 1D bottom left). The same endogenous interactions were confirmed in reverse, using the Tnik antibody for IP (Figure 1D bottom right). These results demonstrated that Tnik specifically interacts with the Tcf4/β-catenin complex in murine small intestinal crypts.

### Nuclear Localization of Tnik in Wnt Activated Intestinal Crypt

Tnik has been previously reported to localize in the cytoplasm and interact with and phosphorylate cytoskeletal structures (Fu et al, 1999). The presence of Tnik in the Tcf4 transcriptional complex suggested nuclear localization of Tnik. We next examined the expression of TNIK in mouse and human intestinal tissue using immunohistochemistry. Tnik was detected in both mouse small intestinal and colonic epithelia by immunohistochemistry (IHC) (Figure 2A-D). Nuclear localization of Tnik was detected specifically in Wnt activated intestinal crypt (Figure 2B), which consolidates our mass spectrometry findings on the specific interaction of Tnik with Tcf4 in mouse intestinal crypt but not villus. Using a mouse monoclonal antibody against the intermediate part of human TNIK, we further confirmed the nuclear localization of human TNIK in normal colonic epithelia as well as colorectal cancer tissues (Figure 2E-H). These results demonstrate that in addition to the reported cytoplasmic expression, Tnik is strongly enriched in the cell nuclei of Wnt activated intestinal crypts and colorectal cancer cells.

### Tnik is Recruited to the Proximal Promoters of Wnt Target Genes in Mouse Small Intestinal Crypts in vivo

We have previously determined TCF4 binding sites by genome-wide chromatin IP (ChIP) in human colon cancer cells (Hatzis et al, 2008). To test whether Tnik is associated with such Tcf4/β-catenin response elements *in vivo,* we performed ChIP on purified intestinal crypt and villus fractions with antibodies specific for Tcf4, β-catenin and Tnik. Quantitative PCR (qPCR) analysis of the immunoprecipitated material was performed for homologous elements in two murine intestinal Wnt target genes, the Axin2 and c-Myc proximal promoters and up/downstream control regions (Figure 3A). As expected, Tcf4 was bound specifically to the target gene promoters in both the crypt and villus, while β-catenin was enriched on the target promoters specifically in the crypt-proliferative compartment (Figure 3A). While not enriched on up/downstream control regions, Tnik was specifically recruited to the promoters of the two Wnt targets genes in the crypt, but not the villus fraction (Figure 3A).

### TNIK is Recruited to Wnt Target Genes in a β-catenin Dependent Manner

To examine whether the Tcf4-Tnik association was mediated by β-catenin, we used a stable transfectant of the human colon cancer cell line Ls174T which expresses a shRNA targeting β-catenin in response to doxycycline treatment (van de Wetering et al, 2003). Of note, Ls174T cells harbour a stabilizing oncogenic mutation in β-catenin, resulting in the constitutive presence of β-catenin/TCF4 nuclear complexes. In the transfected cells, the β-catenin protein was strongly reduced 72 hours post-doxycycline treatment (Figure 3B left panel). We immunoprecipitated TNIK from the Ls174T cells with and without doxycycline treatment and probed for the presence of TCF4. TCF4 is bound to TNIK in these cells demonstrating conservation of the TCF4-TNIK association across mammalian species (Figure 3B right top panel). However, depletion of β-catenin resulted in loss of the TNIK/TCF4 interaction, implying that β-catenin serves as a bridge. Conversely, immunoprecipitation of TCF4 in β-catenin depleted cells resulted in loss of TCF4-TNIK interaction (Figure 3B bottom right panel).

Using the same system, we examined the recruitment of TNIK to the promoters of TCF4 target genes Axin2 and c-Myc in vivo in the presence or absence of β-catenin (Figure 3C). TCF4 was bound to the Axin2 and c-Myc proximal promoters regardless of β-catenin status. As expected, β-catenin disappeared from the target gene promoters upon β-catenin knockdown. Importantly, while specifically present on the Axin2 and c-Myc promoters, TNIK enrichment over these targets was decreased upon β-catenin depletion (Figure 3C). Thus, TNIK recruitment to TCF4 target genes Axin2 and c-Myc occurs in a β-catenin dependent manner.

### The Kinase Activity of TNIK is Required for TCF/LEF Transcriptional Activation

The specific association of TNIK with β-catenin/TCF4 in crypts and Ls174T cells was suggestive of a co-activator function for TNIK. To test this, we depleted TNIK from Ls174T cells using transient siRNA transfection and examined the effect on transcriptional activity of a Tcf reporter TOPFlash (Figure 4A). Removal of TNIK by siRNA resulted in specific suppression of TOPFlash activity but not of the mutant FOPFlash control. Similar suppression of TOPFlash activity was observed upon TNIK depletion in another colorectal cancer cell line, SW480 (Figure 7A). Conversely, over-expression of wild type (WT) Flag-TNIK resulted in a dosage dependent specific increase in TOPFlash activity (Figure 4C right panel). The N-terminal kinase domain of TNIK is highly conserved among serine/threonine protein kinases (e.g. Ste20 family and protein kinase A) and contains several characterized subdomains that fold into a catalytic core structure (Hanks & Hunter, 1995) (Figure 4B). To examine the role of the kinase activity of TNIK in TCF/LEF mediated transcriptional activation, we generated dominant negative TNIK kinase mutants TNIK R152A/D153A (SEQ ID NO:21 encoded by SEQ ID NO:22), TNIK K54A(SEQ ID NO:23 encoded by SEQ ID NO:24), and TNIK D171A/F172A (SEQ ID NO:25 encoded by SEQ ID NO:26), harbouring mutations in the distinct conserved subdomains VI, II, and VII respectively, essential to catalytic activity. Expression of increasing amounts of dominant negative TNIK kinase mutants in Ls174T colorectal cancer cells was examined by western blotting (Figure 4C bottom panels). As previously reported, WT TNIK migrates as two bands comprising phosphorylated and unphosphorylated forms (Figure 4C right bottom) (Taira et al, 2004) All TNIK kinase mutants lacked the lower mobility phosphorylated form of WT TNIK, consistent with their inability to autophosphorylate. As shown in Figure 4C, expression of all TNIK kinase mutants abolished Tcf-reporter TOPFlash transcription activity in a dose dependent manner. The results were further confirmed in another colorectal cancer cell line DLD1 (Figure 7B). Thus, while depletion of TNIK by siRNA decreases TOPFlash activity, exogenous expression of kinase mutant TNIK results in an almost complete inhibition of TOPFlash reporter activity.

We next tested whether TNIK can phosphorylate TCF4 or β-catenin using in vitro kinase assay. We immunoprecipitated wild type or kinase mutant TNIK from 293T cells and performed in vitro kinase assays using GST-TCF4 and GST-β-catenin as substrate as shown in Figure 4D. As reported previously, wild type TNIK was capable of autophosphorylation (Fu et al, 1999), while the kinase mutants failed to autophosphorylate (Figure 4D, top panel). Importantly, wild type TNIK and not the kinase mutants specifically phosphorylated GST-TCF4, identifying TCF4 as a substrate for TNIK kinase activity (Figure 4D). Taken together, these results demonstrate that the kinase activity of TNIK is essential to its role as a TCF4/ β-catenin co-activator.

### TNIK interacts directly with both TCF4 and β-catenin.

To confirm the role of TNIK in regulation of the Wnt target gene expression, we employed the HEK293T cell line in which the Wnt pathway is present, yet not mutationally activated. We examined the interaction between TNIK and TCF4 in HEK293T cells treated with either control or Wnt3A conditioned media for 8 hours. TNIK was immunoprecipitated from HEK293T lysates and probed for interaction with TCF4 and β-catenin by Western blotting (Figure 5A top panel). While absent from the TNIK complex in the absence of Wnt signalling, both TCF4 and β-catenin specifically associated with TNIK in response to Wnt treatment. Conversely, immunoprecipitation of TCF4 from HEK293T lysates (either untreated or treated with Wnt) demonstrated a Wnt-induced interaction with TNIK (Figure 5A bottom panel). As expected, the interaction between β-catenin and TCF4 with TNIK was Wnt-dependent.

Next, we examined the effect of siRNA mediated knock-down of TNIK on TCF/β-catenin mediated TOP/FOP transcriptional activation in the presence and absence of Wnt. Significant depletion of TNIK was accomplished at 72 hours post siRNA treatment (Figure 5B top panel). Removal of TNIK resulted in specific suppression of Wnt-dependent TOPFlash activity (Figure 5B bottom panel). We concluded that TNIK is required for optimal TCF4/ β-catenin transcriptional activation in response to Wnt.

We next probed whether TNIK can directly associate with TCF4/ β-catenin using in vitro binding assays. We generated in vitro translated radiolabeled TNIK deletion mutants (shown in Figure 5C) and probed their direct binding to GST-fused TCF4 and GST-β-catenin immobilized on glutathione beads. The TNIK kinase domain and the kinase domain-containing ΔC bound to TCF4 (Figure 5C, bottom middle panel) but not to control GST beads (Figure 5C, bottom left panel) while the ΔK TNIK was incapable of binding TCF4 (Figure 5C, bottom middle panel). Thus, the TNIK kinase domain directly contacts TCF4. β-catenin also displayed direct binding to TNIK. The TNIK Intermediate domain and deletion mutants ΔC and ΔK, which contain the Intermediate region all interacted with β-catenin, pointing to this region of TNIK as the β-catenin interaction interface (Figure 5C, bottom right panel). Thus, different domains of TNIK directly contact both TCF4 and β-catenin.

### TNIK is an Essential and Specific Activator of Wnt Target Genes in HEK293T Cells

To determine the role for TNIK in the control of endogenous Wnt target gene expression, we studied the effect of TNIK depletion in HEK293T cell line stimulated with Wnt3A by microarray analysis. We first characterized the Wnt target gene program of HEK293T cells by inducing the cells with Wnt3A-conditioned medium followed by microarray expression analysis at three early time points, i.e. 4 hours, 7 hours and 9 hours. This resulted in 172 overlapping probes representing 144 unique genes (Figure 6A and Table 1) selected against 1.5 fold increase at either 7 or 9 hour time points of Wnt induction. While some of the Wnt-induced genes were previously described as Wnt target genes, such as TCF7 (Roose et al, 1999), CCND1 (Tetsu & McCormick, 1999) and AXIN2 (Jho et al, 2002; Lustig et al, 2002; Yan et al, 2001) in other cell/tissue types (http://www.stanford,edu/∼musse/Wntwindow.html), the large majority were unique to the HEK293 kidney cells. These include various transcription factors such as BACH2, ID1, YY1, ETS2 and RUNX3. To increase the likelihood of examining direct transcriptional effects, we then focused on the 4 and 7 hour time points to examine Wnt induced expression profile changes upon TNIK depletion. By comparing the expression pattern of Wnt induction in TNIK depleted cells to the Wnt activated genes with greater than 1.5 fold increase at 7 hour Wnt induction, depletion of TNIK by siRNA resulted in a consistent opposite pattern in both 4 hour and 7 hour time points (Figure 8A). The negative association between the overlapping genes of 7 hours Wnt induction in the presence or absence of TNIK (+7hr Wnt and si TNIK +7hr Wnt) was statistically significant (Pearson correlation coefficient, -0.508; p < 0.001; Figure 8B), in which genes up-regulated upon Wnt induction were down-regulated after TNIK suppression and vice versa. These results suggest an essential role for TNIK in the regulation of Wnt target gene expression.

To understand if TNIK is essential to Wnt pathway regulation, we filtered out 315 probes, representing 304 unique genes (Table 2, Figure 6B) with 1.5 fold variation in Wnt induced TNIK depleted cells (si TNIK +7hr Wnt). Surprisingly, 91.4% (288 out of 315) of the probes were down-regulated upon 7 hour Wnt induction upon TNIK depletion, demonstrating that TNIK serves preferentially as an activator in the Wnt signalling pathway. In addition, 98% of the observed overlapping genes down-regulated upon TNIK depletion were up-regulated after 7 hour Wnt induction (Pearson correlation coefficient, -0.622; p < 0.001, Figure 6C) indicating that TNIK is an essential component in the Wnt signalling pathway. The results were reproduced by independent biological replicates with good correlation (Pearson correlation coefficient, 0.654; p < 0.001, Figure 9). To validate the microarray data, we chose four representative genes, *AXIN2, TCF7, CCND1* and *ZCCHC12* for quantitative RT-PCR validation. All the selected genes were upregulated in response to Wnt stimulation at 4 and 7 hours. Expression of selected target genes was abrogated to basal levels upon TNIK depletion, highlighting the essential role played by TNIK in activation of Wnt target genes (Figure 6D). In summary, our data demonstrate that TNIK serves as an essential, specific activator in regulating the Wnt target gene signature.

Nuclear Wnt signalling requires the concerted action of numerous co-activating transcriptional complexes, many of which are enzymes such as CBP (Takemaru & Moon, 2000), p300 (Hecht et al, 2000), MLL (Mosimann et al, 2009; Sierra et al, 2006). However, most of the co-activating enzymes implicated in TCF gene activation are pleiotropic factors involved in additional cellular processes and transcriptional pathways, and thus are not specific to regulation of Wnt signalling. In fact, despite the passage of a decade after the discovery of TCF4 and β-catenin as the molecular effectors of the Wnt signal, few transcriptional activators unique to the Wnt transcriptional regulation have been found. Here we report the identification of TNIK, a member of the STE20 germinal center kinase family (Fu et al, 1999), as a TCF4-interacting protein in the crypt proliferative compartment of the mouse small intestine as well as in human colorectal cancer cells. TNIK is specifically recruited in a β-catenin dependent manner to the Wnt target genes Axin2 and c-Myc. Expression of dominant negative TNIK kinase mutants abrogate TCF/LEF reporter activity highlighting the essential role of TNIK kinase activity in TCF4/β-catenin transcription regulation. Distinct regions of TNIK directly contact TCF4 and β-catenin and TNIK specifically phosphorylates TCF4. Examination of the function of TNIK by siRNA mediated knock-down followed by gene expression array analysis identifies TNIK as an essential and specific co-activator of the Wnt target gene transcription program as over 90% of genes down-regulated in response to TNIK depletion were Wnt target genes.

It will be of interest to probe the role for TNIK in the multitude of biological phenomena controlled by Wnt signalling. The current data imply TNIK as a potential target for the generation of small molecule inhibitors to specifically block the Wnt pathway in disease states such as colorectal cancer.

### MATERIALS AND METHODS

### Biochemical fractionation of mouse crypt and villus.

The small intestine of four 6-12 week old BALB C mice sacrificed by CO₂ chambers and cervical dislocation was removed, flushed with ice-cold PBS, and cut open longitudinally to expose crypts and villi. Small intestine was cut into small 1-2 cm pieces and crypt and villus fractions were isolated using incubations in a mild PBS-EDTA/EGTA chelation solution combined with vigorous shaking followed by further purification using 70µm (for villi) and 40µm (for crypts) nylon cell strainer (Falcoln). Briefly, intestine pieces were washed several times in cold PBS^{+/+} (Ca⁺⁺/Mg⁺⁺) and incubated in PBS⁰EDTA-EGTA (no Ca⁺⁺/Mg⁺⁺ + 1mM EDTA + 1mM EGTA) 10 min on rotator. Buffer was decanted followed by addition of fresh PBS⁰ and vigorous shaking approximately 10-15 times. Incubation in PBS⁰EDTA-EGTA, decanting and shaking in PBS⁰ was repeated. Fractions were put through a 70µm strainer. Whole villus structures remain on top of cell strainer and are collected while the flow through (F/T) discarded. Incubation, shaking and separation through cell strainer was repeated and fractions 2-5 collected containing pure villus structures. From fraction 5, the F/T of the cell strainer containing intact crypts was collected and further purified by passage through a 40µm cell strainer. This was repeated until the mesenchyme was stripped of epithelial cells. Typically, fractions 2-4 contained pure villi while fractions 6-8 F/T contained pure crypts. Purified crypts and villi were washed twice and kept in PBS^{+/+} for up to one hour before further use.

### DNA constructs

Details of cloning procedures are available upon request. Briefly, using a PCR-based strategy a sequence encoding the FLAG-epitope was added at the end of the TCF4, β-catenin and TNIK coding sequences and cloned into pcDNA3.1 (Invitrogen). For expression of GST-fusion proteins, the coding sequences of TCF4 and β-catenin were cloned in pGEX6P-1. For in vitro transcripition/translation of TNIK deletions, indicated regions of TNIK coding sequence were cloned in pcDNA3.1 (Invitrogen).

### Immunoprecipitation

Total cellular extracts from Ls174T cells, HEK293T cells or primary mouse crypt or villus material were prepared in PLB buffer (1%Triton X-100, 2mM EDTA, 1mM DTT, 5%glycerol in PBS) supplemented with protease inhibitors (PIs) (Complete, Roche Molecular Biochemicals). Cellular lysates were pre-cleared with IgG-agarose beads (Sigma) for 6h at 4°C. Immunoprecipitations of endogenous complexes from mouse crypt and villus, Ls174T and HEK293T cells were carried out overnight at 4°C with anti-TCF4 (Santa Cruz), anti-β-catenin (BD transductions), or anti-TNIK (Santa Cruz) (2µg/mL) in combination with a 50% protein G-Agarose slurry (Sigma). Immunoprecipitated material was washed three times in PLB supplemented with PIs. Bound proteins were subjected to SDS-PAGE and Western blot analysis or Mass Spectrometry Analysis.

### In vitro binding assay

³⁵S-labeled reticulocyte expressed TNIK deletion mutants were generated using the TNT quick coupled transcription translation kit (Promega). Bacterially expressed GST, GST-TCF4 and GST-β-catenin were immobilized and purified on Glutathione beads for 2 hr at 4°C followed by extensive washes with HEMG buffer (25 mm HEPES-KOH (pH 7.6), 0.1 mm EDTA, 12.5 mm MgCl₂, 10% glycerol, 1 mm DTT, 0.01% Nonidet P-40 (NP-40), protease and phosphatase inhibitors) containing 0.5-0.1 M KC1. Beads were incubated for 2 hr with ³⁵S-labeled reticulocyte expressed TNIK deletion mutants, washed extensively with HEMG buffer containing 0.1M KCl and analyzed by SDS-PAGE followed by autoradiography.

### In vitro kinase assay

Immunoprecipitated wild type or mutant Flag-TNIK was incubated with purified GST-TCF4 and GST-β-catenin fusion proteins. Phosphorylation reactions were performed in 30 µl kinase buffer (20 mM Hepes (pH 7.5), 20 mM MgCl₂, 0.1 mM orthovanadate, and 2 mM dithiothreitol) supplemented with 20 µM ATP and 5 µCi of [γ-³²P]ATP at 30 °C for 30 min. Reactions were stopped by the addition of 4x Laemmli sample buffer and resolved by SDS-PAGE followed by autoradiography.

### Mass Spectrometry Analysis

In-gel digestion: Gel bands putatively containing TCF4, β-catenin and TNIK were sliced-out from the gels of all four pull downs (i.e. αIgG villus, αTCF4 villus,αIgG crypt, αTCF4 crypt), Figure 1B, and subjected to in-gel digestion as described previously (Shevchenko et al, 1996). Protein reduction and alkylation was performed with DTT (60 °C, 1 hour) and Iodoacetamide (dark, room temperature, 30 min), respectively. Digestion was performed with trypsin over night at 37 °C. Peptides were extracted with 10 % FA.

NanoLC-MS/MS: The extracted peptides were subjected to nanoscale liquid chromatography tandem mass spectrometry (nanoLC-MS/MS) analysis, performed on an Agilent 1100 HPLC system (Agilent technologies) connected to an LTQ Linear Ion Trap Mass Spectrometer combined with either an Orbitrap (ThermoFisher, Waltham, MA) or an Fourier Transform Ion Cyclotron Resonance cell (ThermoFisher, Waltham, MA). The nanoLC was equipped with a 20 mm x 100 µm i.d. Aqua C18 trap column (Phenomenex, Torrance, CA) and a 400 mm x 50 µm i.d. Reprosil C18 analytical column (Dr Maisch, Ammerbuch-Entringen, Germany) (Pinkse et al, 2008). Trapping was performed at a flow of 5 µl/min for 10 min and the fractions were eluted using a 45 min linear gradient from 0 to 40 % solvent B (0.1 M acetic acid in 80 % ACN (v/v), in which solvent A was 0.1 M acetic acid), 40 to 100 % solvent B in 2 min and 100 % B for 2.5 min. The analytical flow rate was 100 nl/min and the column effluent was directly introduced into the ESI source of the MS using a standard coated fused silica emitter (New Objective, Woburn, MA, USA) (o.d. 360µm, tip i.d. 10µm) biased to 1.7kV. The mass spectrometer was operated in positive ion mode and in data-dependent mode to automatically switch between MS and MSMS. The three most intense ions in the survey scan were fragmented in the linear ion trap using collisional induced dissociation. The target ion setting was 5e5 for the Orbitrap, with a maximum fill-time of 250ms and 1e6 for the FTICR, with a maximum fill-time of 250ms. Fragment ion spectra were acquired in the LTQ with an AGC value of 3e4 and a max injection time of 500ms.

Protein Identification: Raw MS data were converted to peak lists using Bioworks Browser software, version 3.1.1. Spectra were searched against the IPI (International Protein Index) Human database version 3.36 (69012 sequences; 29002682 residues) or against the IPI mouse database version 3.36 (51326 sequences; 23682061 residues) using Mascot software version 2.2.0 (www.matrixscience.com), with trypsin set as enzyme. The database search was made with the following parameters set to consider a peptide tolerance of ± 15 ppm, a fragment tolerance of ± 0.9 Da, allowing 3 missed cleavages, Carbamidomethyl (C) as fixed modification, Oxidation (M).

### Immunohistochemistry

Tissues were fixed in 10% formalin, paraffin embedded, and sectioned. Antibodies rabbit α-Tnik (1:1000, Santa Cruz) and mouse α-Tnik, clone 3D4 (1:500, Abnova) were used. Peroxidase conjugated secondary antibodies used were mouse or rabbit EnVision+ (DAKO).

### RNA Interference

Pre-designed Dharmacon siRNA pools targeting transcripts of the human TNIK (L-004542-00) and non-target control siRNA pool (D-001810-10-20) were used to knock down TNIK in Ls174T colorectal cancer and HEK293T cells. siRNA was delivered into Ls174T, SW480 and HEK 293T cells using the siLentFect Lipid Reagent (BioRad). siRNA (15nM) was used to transfect 2X10⁵ cells, and protein levels were examined by western blot analysis 72 hours after transfection. The sequences for SMARTpool si-TNIK are: GAACAUACGGGCAAGUUUA (SEQ ID NO:17), UAAGCGAGCUCAAAGGUUA (SEQ ID NO:18), CGACAUACCCAGACUGAUA (SEQ ID NO:19), and GACCGAAGCUCUUGGUUAC (SEQ ID NO:20).

### Transient Transfection and Luciferase Assays

Ls174T CRC and HEK 293T cells were seeded at a density of 2 x 10⁵ cells/12-well plate. siRNA targeting the human TNIK gene or control non-targeting siRNA was delivered the next day with siLentFect Lipid Reagent (BioRad) according to the manufacturer's instructions. After 72 hours, cells were transfected with plasmid constructs pGL-TOP and pGL-FOP optimal and mutated TCF luciferase-reporter constructs as described previously(van de Wetering et al, 2002). The cells were lysed after 24 h using luciferase lysis buffer (Promega Corp.), and luciferase activities were measured using the Dual-Luciferase reporter assay system. Transfection efficiency was normalized using the cotransfected *Renilla* luciferase activity as an internal control. The data shown are of two independent triplicate experiments as the mean ± S.D. of the ratio between the TOP/FOP and *Renilla* reporters.

### Chromatin immunoprecipitation

Ls174T CRC cells or purified mouse crypt and villus fractions were fixed by adding formaldehyde to a final concentration of 1% for 30 min at room temperature. Glycine was added to a final concentration of 125mM to quench the reaction. Cells were washed twice with buffer B (0.25%Triton-X 100, 1mM EDTA, 0.5mM EGTA, 20mM Hepes, pH7.6) and buffer C (150mM NaCl, 1mM EDTA, 0.5mM EGTA, 20mM Hepes, pH 7.6). Cells were resuspended in 0.3%SDS, 1%Triton-X 100, 0.15M NaCl, 1mM EDTA, 0.5mM EGTA, 20mM Hepes, pH7.6) and sheared using a BioRuptor sonicator (Cosmo Bio Co., Ltd) with twelve 30-sec pulses at the maximum setting. 5µg of the indicated antibody TCF4 (Santa Cruz), β-catenin (BD Transduction), TNIK (Santa Cruz) was incubated with the sheared cross-linked chromatin and BSA blocked protein-G beads overnight at 4°C. Approximately 20 million cells were used per IP. IPs were washed twice with each buffer (0.1% SDS, 0.1% deoxycholate, 1%Triton-X 100, 150mM NaCl, 1mM EDTA, 0.5mM EGTA, 20mM Hepes pH7.6), buffer2 (0.1%SDS, 0.1%deoxycholate, 1%Triton-X100, 0.5M NaCl, 1mM EDTA, 0.5mM EGTA, 20mM Hepes pH7.6), buffer3 (250mM LiCl, 0.5% deoxycholate, 0.5% NP-40, 1mM EDTA, 0.5mM EGTA, 20mM Hepes, pH7.6), buffer4 (1mM EDTA, 0.5mM EGTA, 20mM Hepes, pH7.6). Immunoprecipitated complexes were eluted in 1%SDS, 0.1M NaHCO₃ for 20min, and incubated overnight at 65°C for removal of crosslinks in presence of 200mM NaCl. DNA was phenol:chloroform extracted, chloroform:isoamylalcohol extracted, ethanol precipitated. Input and immunoprecipitated DNA were subjected to Sybergreen qPCR with primers overlapping the promoters, upstream/downstream regulatory regions of mouse and human Axin2 and c-Myc genes.

### Quantitative PCR (qPCR)

ChIP experiments were analyzed by qPCR in an iCycler iQ real-time PCR detection system (BioRad) using iQ Sybergreen Supermix (BioRad). ChIP values were normalized as a percentage of input, and presented as fold enrichment over the percentage of input immunoprecipitated over the second exon of the myoglobin gene (human samples) and the Sp5 downstream region (mouse samples) as control. The following primer pairs were used for examining recruitment to the human Axin2 promoter: 5'GTTACCTGGAAGACGAAGG3'; 5'TCTGGAGGCGTTCAGTTG3', human Axin2 upstream control region: 5'GCCAGAGTCAAGCCAGTAGT3'; 5'TAGCCTAATGTGGAGTGGAT3', mouse Axin2 promoter: 5'CCACCAAGACCTACATAC3'; 5'CCACTCCTCACATATTCC3', mouse Axin2 downstream control region: 5'CTACCGTGTTACTCTGACTATTCTTC3'; 5'GCGGCTCAGTGGCTAAGG3', human c-Myc promoter: 5'ACTCAGTCTGGGTGGAAGG3'; 5'GGAATGATAGAGGCATAAGGAGTATC3', human c-Myc downstream control region: 5'CAGCAAGATAGCAGAGGAAG3'; 5'TGGAGATGGATTGAATGAAGG3', mouse c-Myc promoter: 5'CTCACTGGAACTTACAATCTG3'; 5'CAACGCCCAAAGGAAATC3', mouse c-Myc upstream control region:5'ACTCAAGAACTGGTAGAC3'; 5'CTAAGTGGGTAAGGTAGG3', second exon of human Myoglobin gene: 5'AAGTTTGACAAGTTCAAGGACC3'; 5'TGGCACCATGCTTCTTTAAGTC3', mouse Sp5 downstream control region: 5'TGGAGTTACAGGCAGTGGTG3'; 5'ATTGATTTCTTTAGGTTGGGTGGAG3' .

### Site-directed Mutagenesis

TNIK kinase mutants TNIK R152A/D153A, TNIK K54A, and TNIK D171A/F172A were generated using the Quick Change Site-Directed Mutagenesis kit (Stratagene). Wild type Full length Flag tagged TNIK expression vector was used as a template for PCR based mutagenesis using the following primers: TNIK R152A/D153A Forward primer 5'CCAGCATAAAGTGATTCATGCAGCTATTAAAGGGCAAAATGTCTTG3', TNIK R152A/D153A Reverse primer 5'CAAGACATTTTGCCCTTTAATAGCTGCATGAATCACTTTATGCTGG3', TNIK K54A For 5'GCCAGCTTGCAGCCATCGCGGTTATGGATGTCACAGG3', TNIK K54A Rev 5'CCTGTGACATCCATAACCGCGATGGCTGCAAGCTGGC3', TNIK D171A/F172A Forward primer 5'GCAGAAGTTAAACTAGTGGCCGCTGGAGTCAGTGCTCAGC3', TNIK D171A/F172A Reverse primer 5'GCTGAGCACTGACTCCAGCGGCCACTAGTTTAACTTCTGC3' .

### Microarray analysis

Total RNA was extracted from HEK293T cells after siRNA transfection (siTNIK or siNon target control) and induced with Wnt3A expressing conditioned medium (Wnt3A) or control medium (CM) for 4, 7 or 9 hours using RNeasy Mini Kit (Qiagen) according to ATCC instruction. 2µg of RNA from each sample was labelled by Quick Amp Labelling Kit, two colour (Agilent) and corresponding pairs of samples were hybridized to 4X44K Whole Human Genome dual colour Microarrays (Agilent, G4112F). Six pair of samples:
sicontrol+Wnt3A 4hours / sicontrol+CM 4hours (+4hr Wnt); sicontrol+Wnt3A 7hours / sicontrol+CM 7hours (+7hr Wnt); sicontrol+Wnt3A 9hours / sicontrol+CM 9hours (+9hr Wnt); siTNIK+Wnt3A 4hours / sicontrol+Wnt3A 4hours (siTNIK+4hr Wnt); siTNIK+Wnt3A 7hours / sicontrol+Wnt3A 7hours (siTNIK+7hr Wnt, Experiment1); siTNIK+Wnt3A 7hours / sicontrol+Wnt3A 7hours (siTNIK+7hr Wnt, Experiment2) were hybridized in two dye swap experiments resulting in twelve individual arrays.

Microarray signal and background information were retrieved using Feature Extraction program (V.9.5.3, Agilent Technologies). For each pair of experiment, fluorescent signals in either channel with greater than 2 times above the local background and showing the same trend of fold change in the corresponding dye swap experiments were considered as well measured and average was taken. Overlapping genes with greater than 1.5 fold variations in either +7hr Wnt or +9hr Wnt (144 unique genes, Table 1) were selected as Wnt regulated genes in HEK293T cells, whereas genes with greater than 1.5 fold variation (304 unique genes, Table 2) in siTNIK +7hr Wnt were selected as TNIK regulated genes. Array data is available at Gene Expression Omnibus under accession number GSE17623.

### Statistical analysis

Comparison of differences between TOPFlash activities was performed using two-tailed student t-test (where equal variance between groups was assumed). To examine the correlation of expression fold change in corresponding microarray experiments, Pearson correlation was performed whereas R represent correlation coefficient and P value<0.05 was considered as significant.

### REFERENCES

Barker N, Hurlstone A, Musisi H, Miles A, Bienz M, Clevers H (2001) The chromatin remodelling factor Brg-1 interacts with beta-catenin to promote target gene activation. EMBO J 20(17): 4935-4943
Behrens J, von Kries JP, Kuhl M, Bruhn L, Wedlich D, Grosschedl R, Birchmeier W (1996) Functional interaction of beta-catenin with the transcription factor LEF-1. Nature 382(6592): 638-642
Bienz M, Clevers H (2000) Linking colorectal cancer to Wnt signalling. Cell 103(2): 311-320
Calnek D, Quaroni A (1993) Differential localization by in situ hybridization of distinct keratin mRNA species during intestinal epithelial cell development and differentiation. Differentiation 53(2): 95-104
Clevers H (2006) Wnt/beta-catenin signalling in development and disease. Cell 127(3): 469-480
Fu CA, Shen M, Huang BC, Lasaga J, Payan DG, Luo Y (1999) TNIK, a novel member of the germinal center kinase family that activates the c-Jun N-terminal kinase pathway and regulates the cytoskeleton. J Biol Chem 274(43): 30729-30737
Hanks SK, Hunter T (1995) Protein kinases 6. The eukaryotic protein kinase superfamily: kinase (catalytic) domain structure and classification. FASEB J 9(8): 576-596
Hatzis P, van der Flier LG, van Driel MA, Guryev V, Nielsen F, Denissov S, Nijman IJ, Koster J, Santo EE, Welboren W, Versteeg R, Cuppen E, van de Wetering M, Clevers H, Stunnenberg HG (2008) Genome-wide pattern of TCF7L2/TCF4 chromatin occupancy in colorectal cancer cells. Mol Cell Biol 28(8): 2732-2744
He TC, Sparks AB, Rago C, Hermeking H, Zawel L, da Costa LT, Morin PJ, Vogelstein B, Kinzler KW (1998) Identification of c-MYC as a target of the APC pathway. Science 281(5382): 1509-1512
Heath JP. 1996.). EPITHELIAL CELL MIGRATION IN THE INTESTINE. Cell Biology International 20: 139-46
Hecht A, Vleminckx K, Stemmler MP, van Roy F, Kemler R (2000) The p300/CBP acetyltransferases function as transcriptional coactivators of beta-catenin in vertebrates. Embo J 19(8): 1839-1850
Jho EH, Zhang T, Domon C, Joo CK, Freund JN, Costantini F (2002) Wnt/beta-catenin/Tcf signalling induces the transcription of Axin2, a negative regulator of the signalling pathway. Mol Cell Biol 22(4): 1172-1183
Korinek V, Barker N, Moerer P, van Donselaar E, Huls G, Peters PJ, Clevers H (1998) Depletion of epithelial stem-cell compartments in the small intestine of mice lacking Tcf-4. Nat Genet 19(4): 379-383
Korinek V, Barker N, Morin PJ, van Wichen D, de Weger R, Kinzler KW, Vogelstein B, Clevers H (1997) Constitutive transcriptional activation by a beta-catenin-Tcf complex in APC-/- colon carcinoma. Science 275(5307): 1784-1787
Kramps T, Peter O, Brunner E, Nellen D, Froesch B, Chatterjee S, Murone M, Zullig S, Basler K (2002) Wnt/wingless signalling requires BCL9/legless-mediated recruitment ofpygopus to the nuclear beta-catenin-TCF complex. Cell 109(1): 47-60
Liu W, Dong X, Mai M, Seelan RS, Taniguchi K, Krishnadath KK, Halling KC, Cunningham JM, Boardman LA, Qian C, Christensen E, Schmidt SS, Roche PC, Smith DI, Thibodeau SN (2000) Mutations in AXIN2 cause colorectal cancer with defective mismatch repair by activating beta-catenin/TCF signalling. Nat Genet 26(2): 146-147
Lustig B, Jerchow B, Sachs M, Weiler S, Pietsch T, Karsten U, van de Wetering M, Clevers H, Schlag PM, Birchmeier W, Behrens J (2002) Negative feedback loop of Wnt signalling through upregulation of conductin/axin2 in colorectal and liver tumours. Mol Cell Biol 22(4): 1184-1193
Molenaar M, van de Wetering M, Oosterwegel M, Peterson-Maduro J, Godsave S, Korinek V, Roose J, Destree O, Clevers H (1996) XTcf-3 transcription factor mediates beta-catenin-induced axis formation in Xenopus embryos. Cell 86(3): 391-399
Morin PJ, Sparks AB, Korinek V, Barker N, Clevers H, Vogelstein B, Kinzler KW (1997) Activation of beta-catenin-Tcf signalling in colon cancer by mutations in beta-catenin or APC. Science 275(5307): 1787-1790
Mosimann C, Hausmann G, Basler K (2009) Beta-catenin hits chromatin: regulation of Wnt target gene activation. Nat Rev Mol Cell Biol 10(4): 276-286
Pinkse MW, Mohammed S, Gouw JW, van Breukelen B, Vos HR, Heck AJ (2008) Highly robust, automated, and sensitive online TiO2-based phosphoproteomics applied to study endogenous phosphorylation in Drosophila melanogaster. J Proteome Res 7(2): 687-697
Roose J, Huls G, van Beest M, Moerer P, van der Horn K, Goldschmeding R, Logtenberg T, Clevers H (1999) Synergy between tumour suppressor APC and the beta-catenin-Tcf4 target Tcfl. Science 285(5435): 1923-1926
Rubinfeld B, Albert I, Porfiri E, Fiol C, Munemitsu S, Polakis P (1996) Binding of GSK3beta to the APC-beta-catenin complex and regulation of complex assembly. Science 272(5264): 1023-1026
Shevchenko A, Wilm M, Vorm O, Mann M (1996) Mass spectrometric sequencing of proteins silver-stained polyacrylamide gels. Anal Chem 68(5): 850-858
Sierra J, Yoshida T, Joazeiro CA, Jones KA (2006) The APC tumour suppressor counteracts beta-catenin activation and H3K4 methylation at Wnt target genes. Genes Dev 20(5): 586-600
Taira K, Umikawa M, Takei K, Myagmar BE, Shinzato M, Machida N, Uezato H, Nonaka S, Kariya K (2004) The Traf2- and Nck-interacting kinase as a putative effector of Rap2 to regulate actin cytoskeleton. J Biol Chem 279(47): 49488-49496
Takemaru KI, Moon RT (2000) The transcriptional coactivator CBP interacts with beta-catenin to activate gene expression. J Cell Biol 149(2): 249-254
Tetsu O, McCormick F (1999) Beta-catenin regulates expression of cyclin D1 in colon carcinoma cells. Nature 398(6726): 422-426
van de Wetering M, Oving I, Muncan V, Pon Fong MT, Brantjes H, van Leenen D, Holstege FC, Brummelkamp TR, Agami R, Clevers H (2003) Specific inhibition of gene expression using a stably integrated, inducible small-interfering-RNA vector. EMBO Rep 4(6): 609-615
van de Wetering M, Sancho E, Verweij C, de Lau W, Oving I, Hurlstone A, van der Horn K, Batlle E, Coudreuse D, Haramis AP, Tjon-Pon-Fong M, Moerer P, van den Born M, Soete G, Pals S, Eilers M, Medema R, Clevers H (2002) The beta-catenin/TCF-4 complex imposes a crypt progenitor phenotype on colorectal cancer cells. Cell 111(2): 241-250
Yan D, Wiesmann M, Rohan M, Chan V, Jefferson AB, Guo L, Sakamoto D, Caothien RH, Fuller JH, Reinhard C, Garcia PD, Randazzo FM, Escobedo J, Fantl WJ, Williams LT (2001) Elevated expression of axin2 and hnkd mRNA provides evidence that Wnt/beta -catenin signalling is activated in human colon tumours. Proc Natl Acad Sci U S A 98(26): 14973-14978

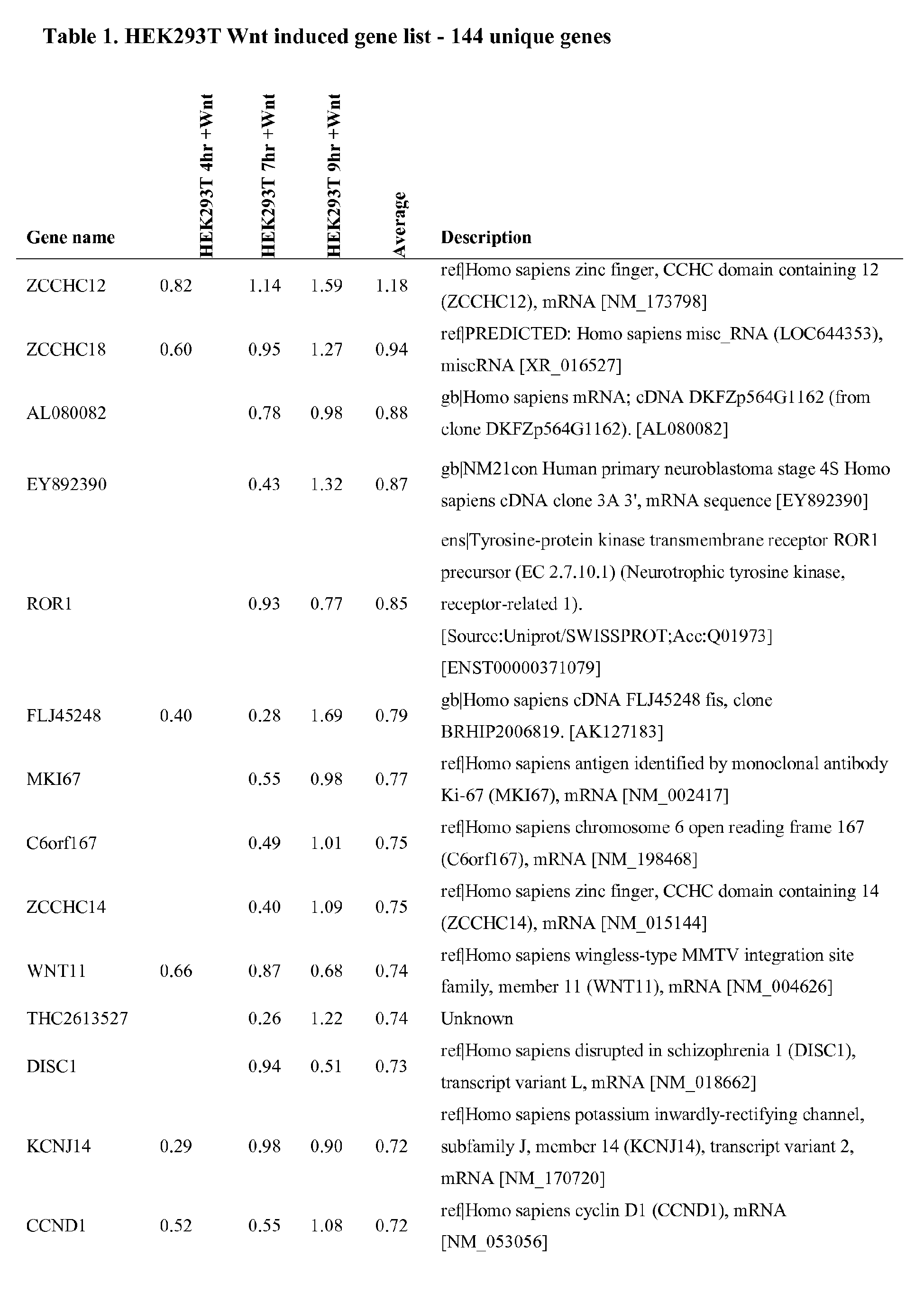

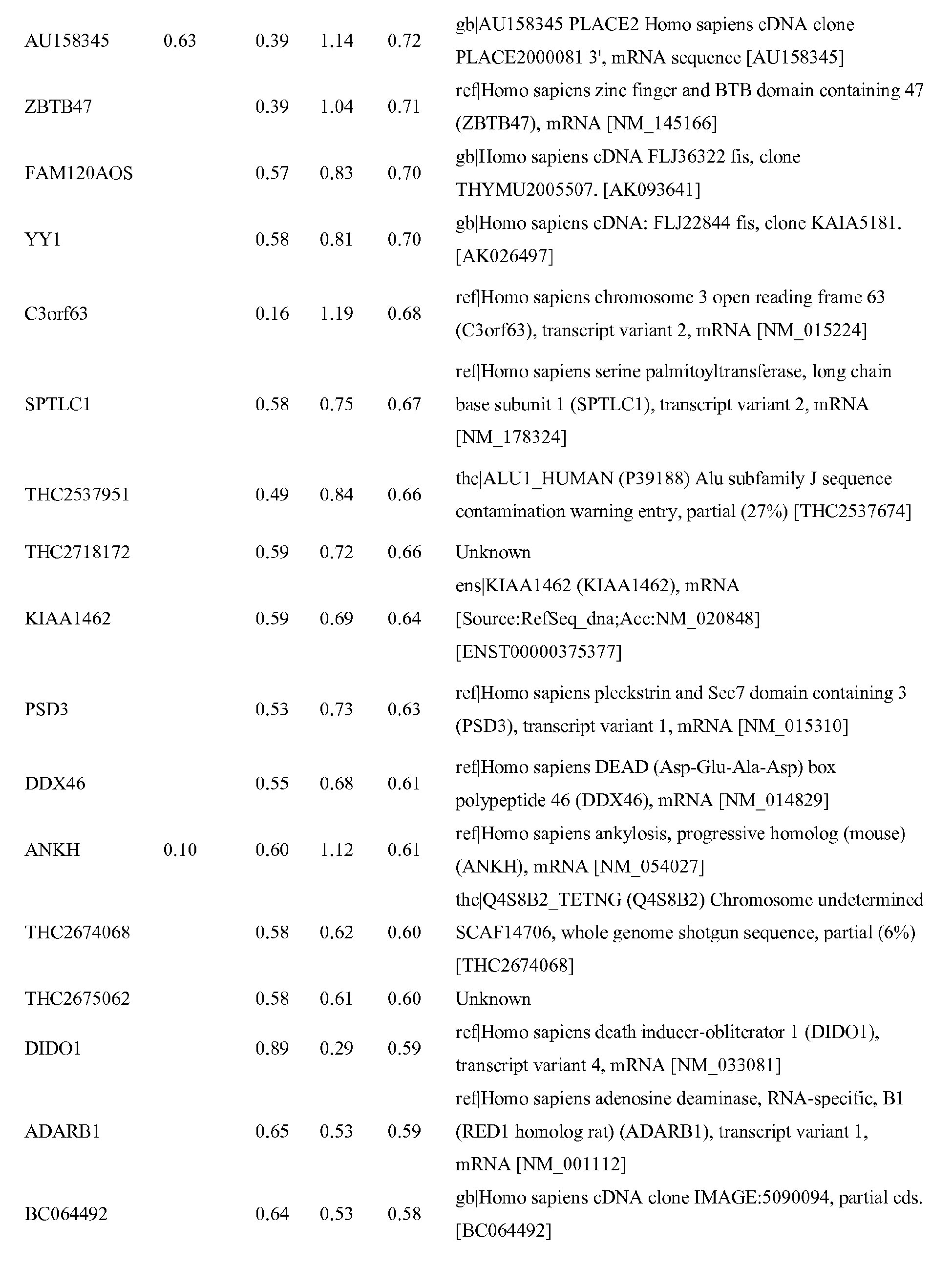

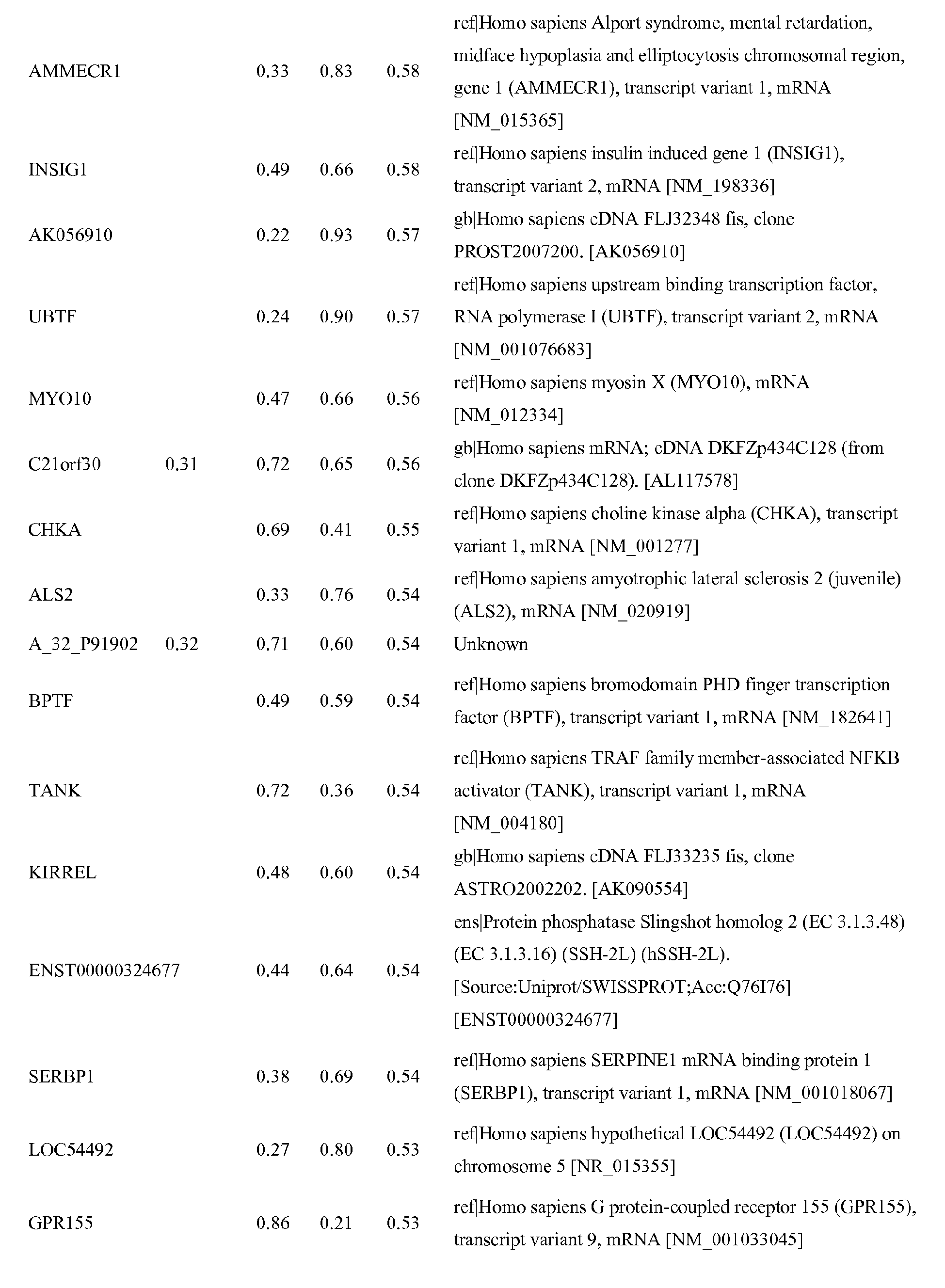

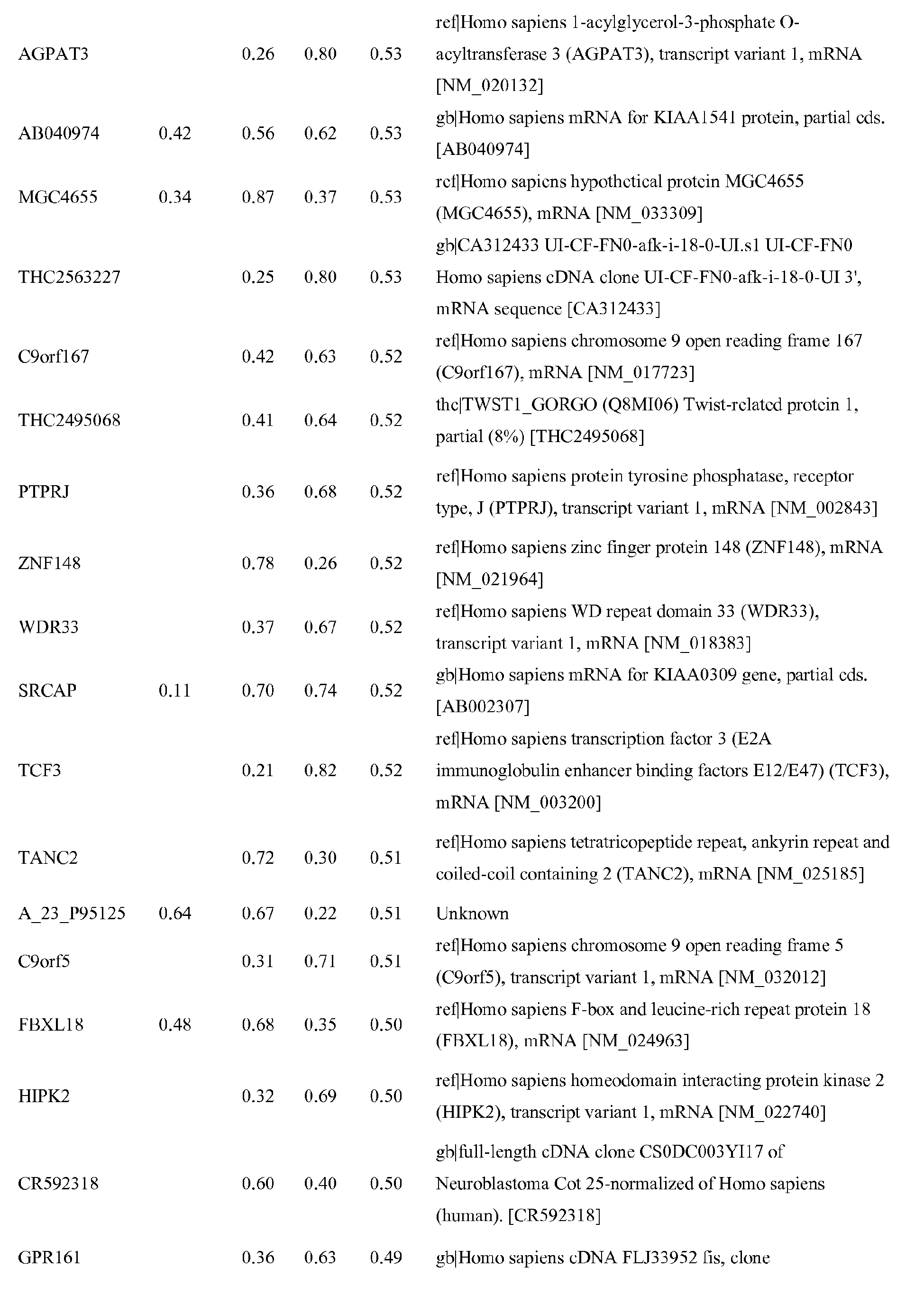

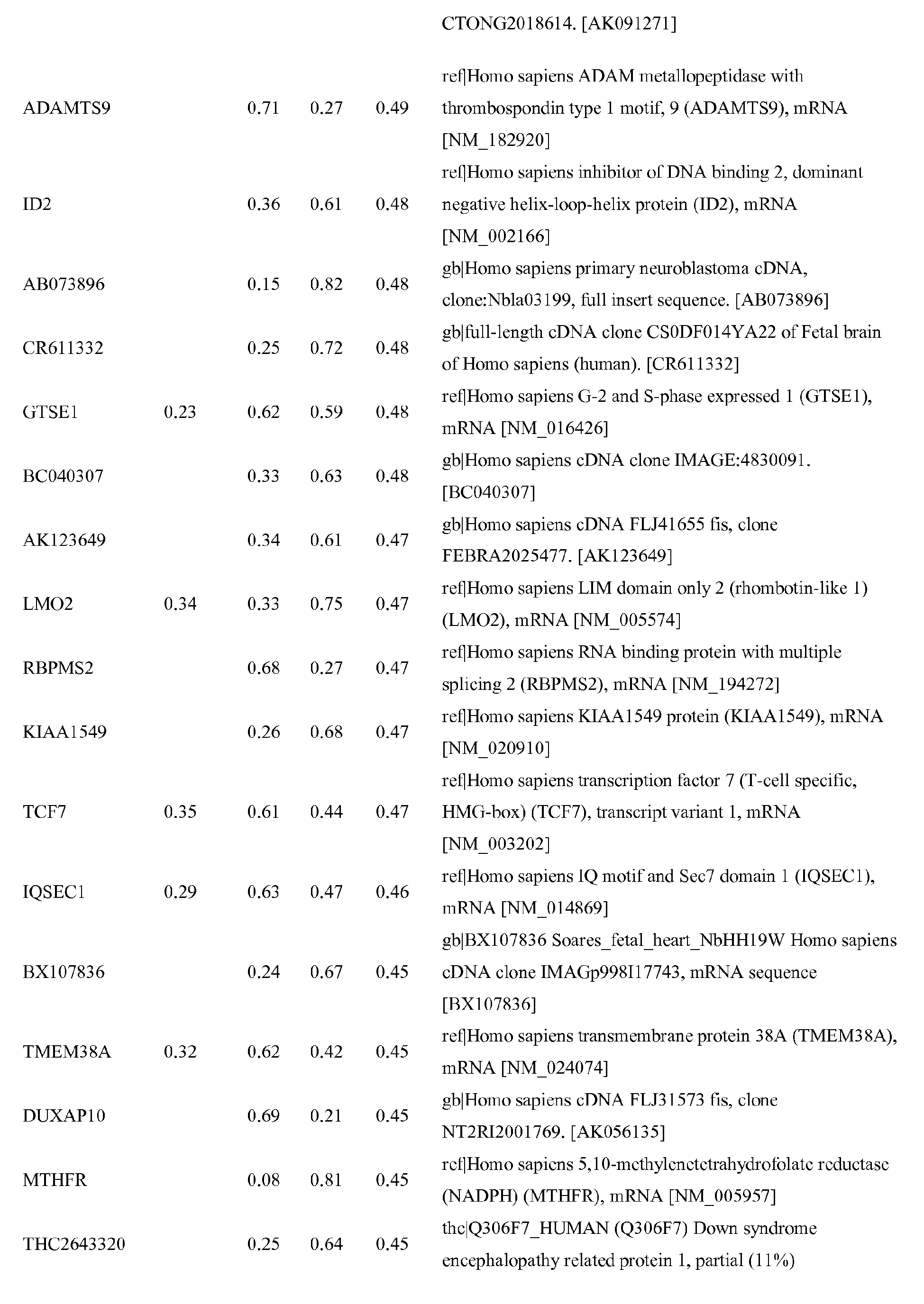

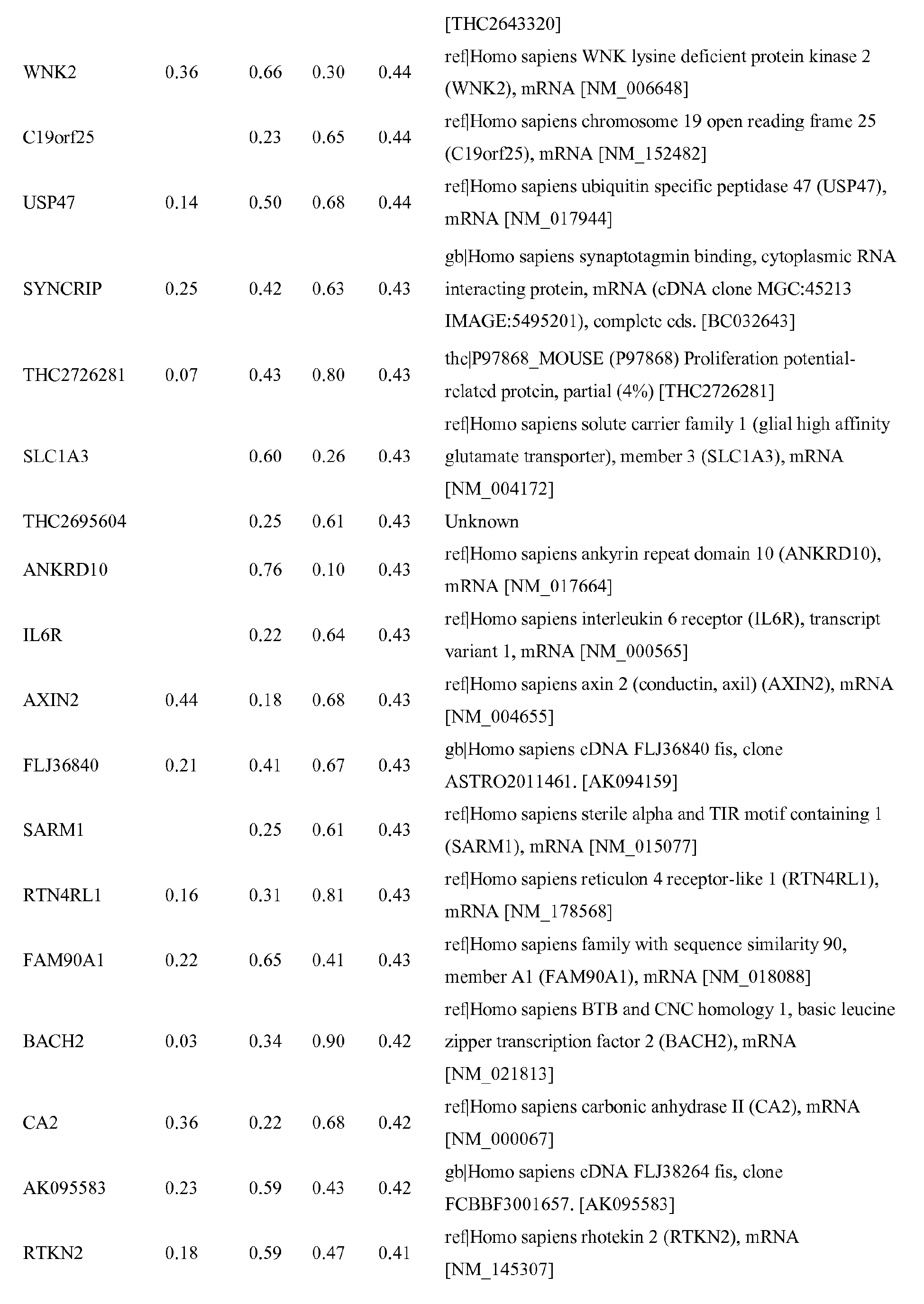

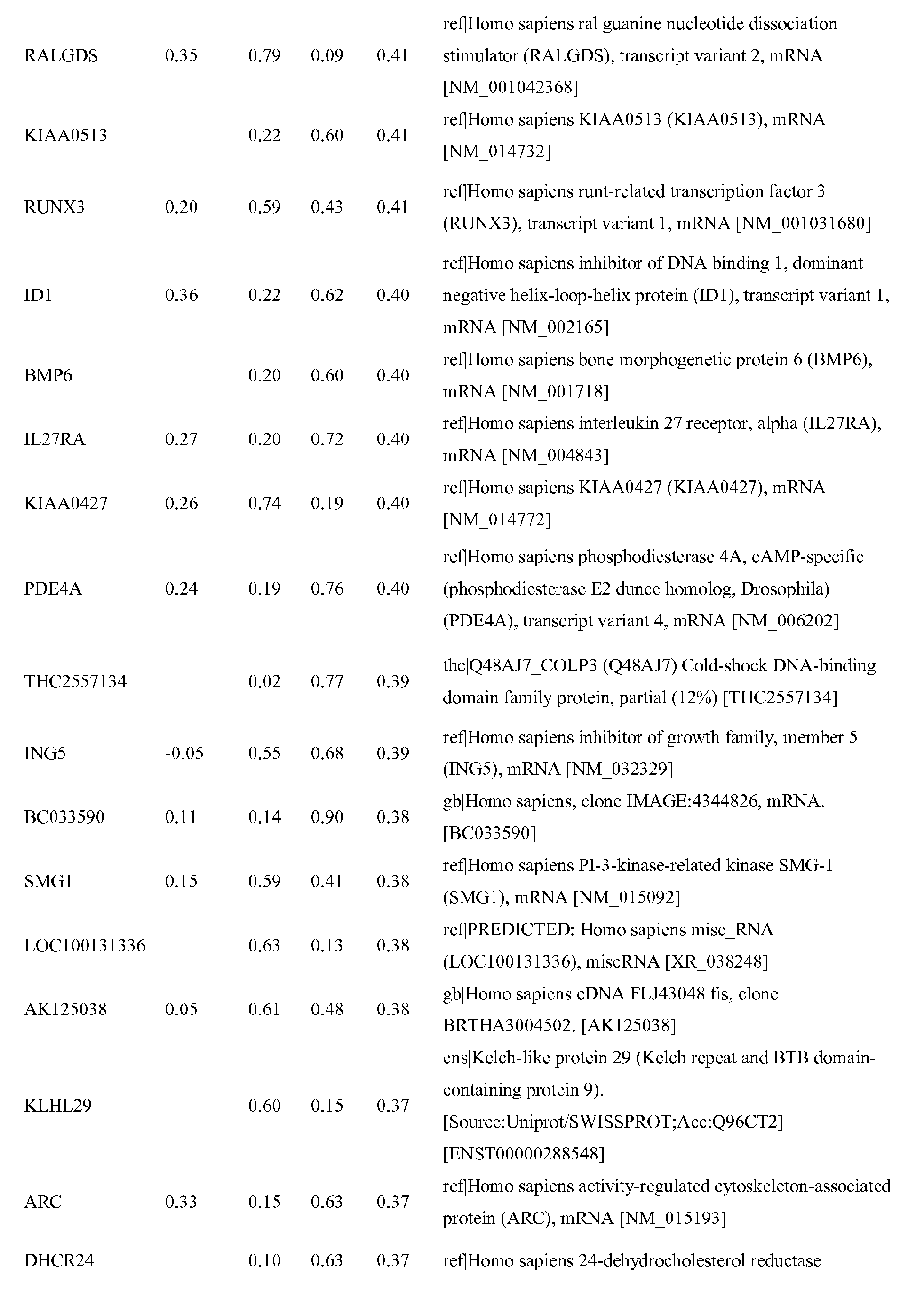

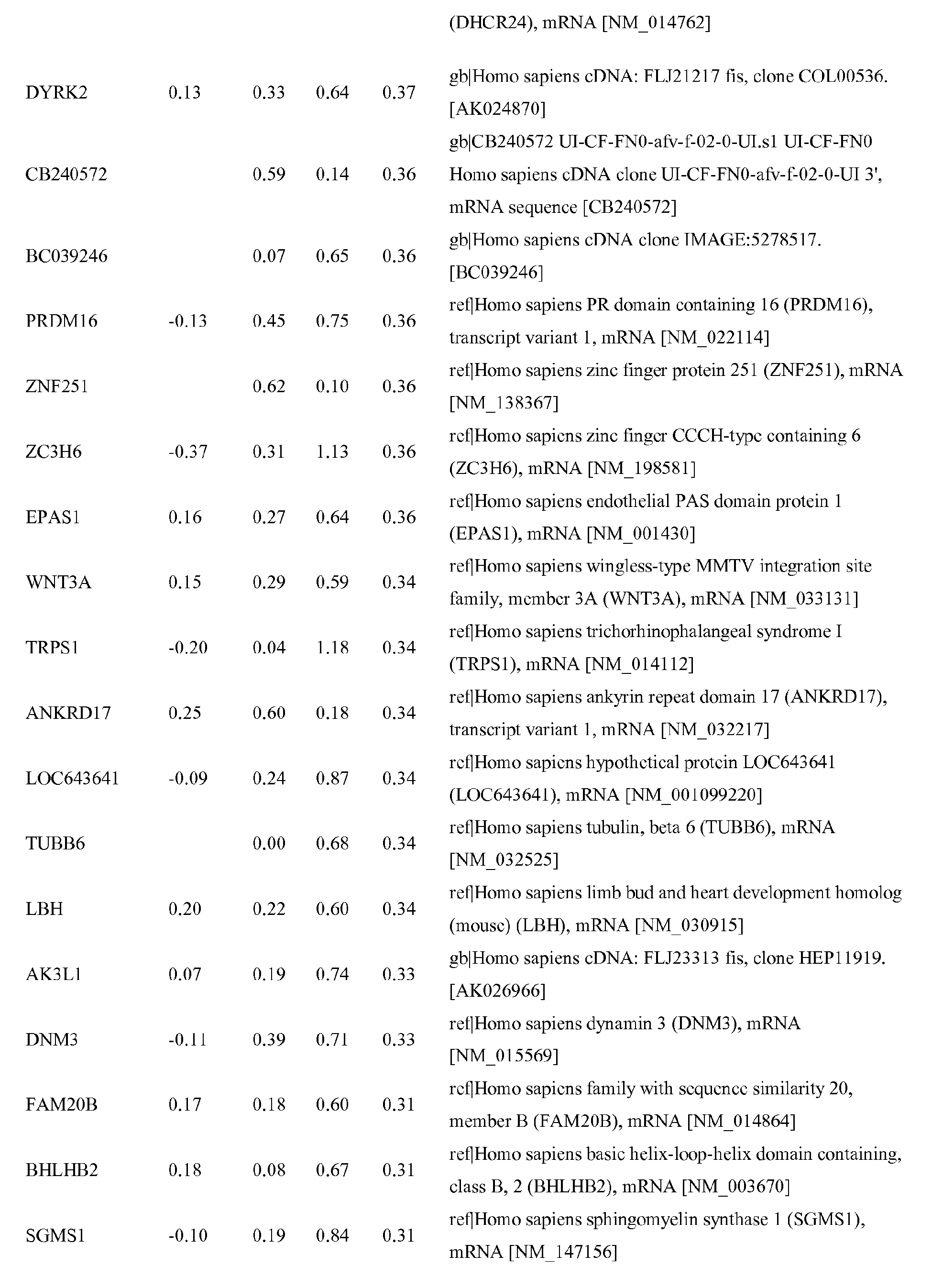

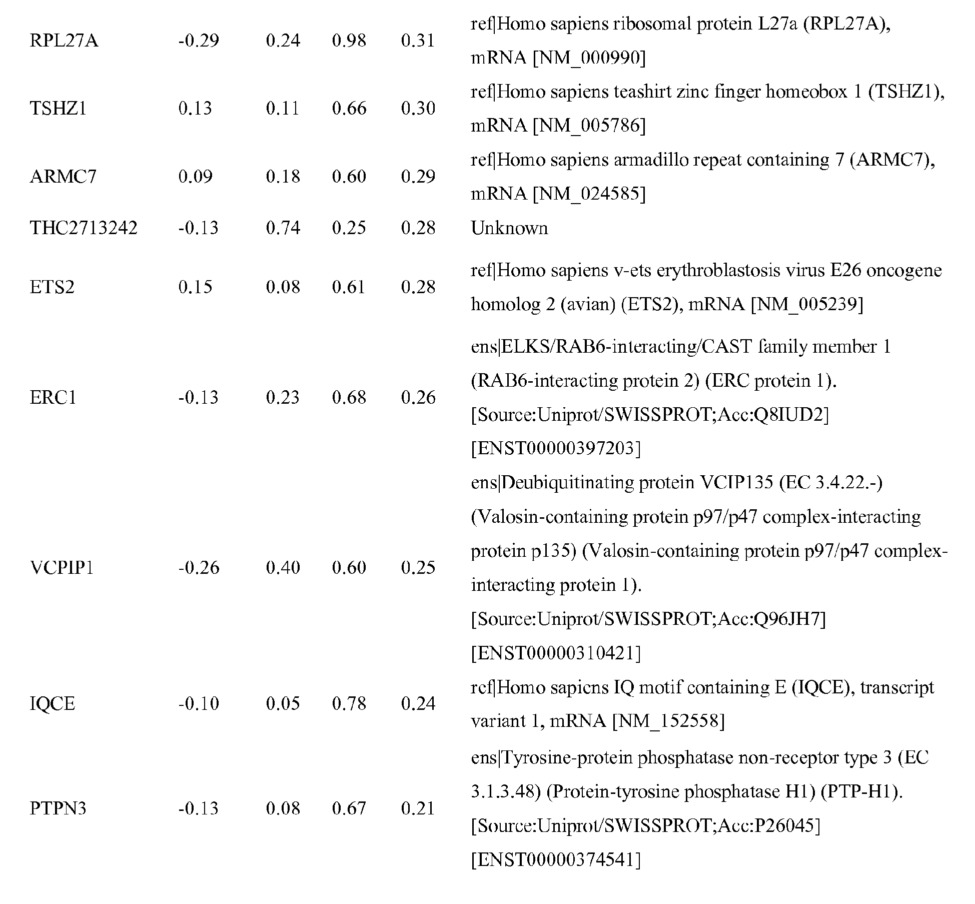

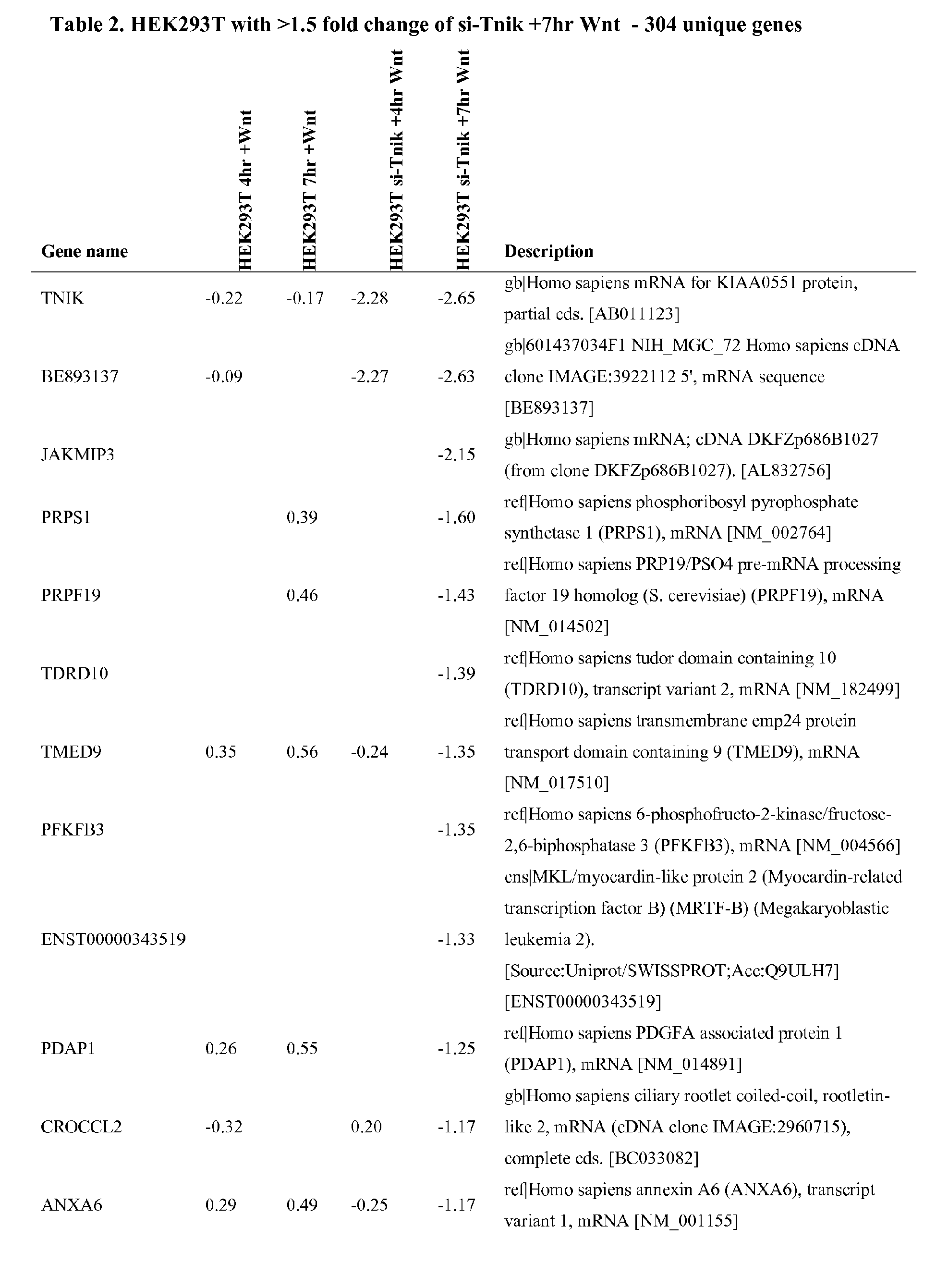

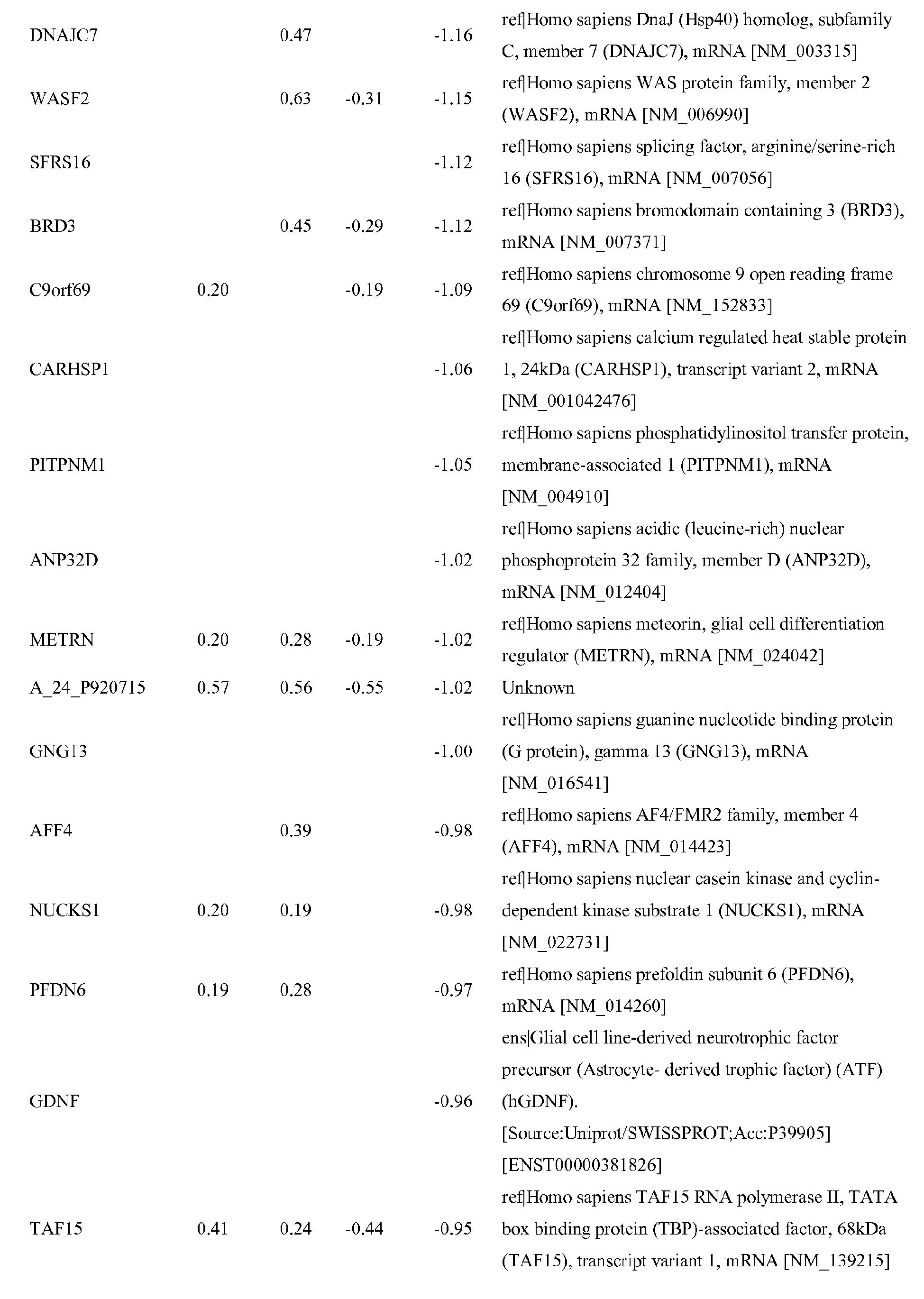

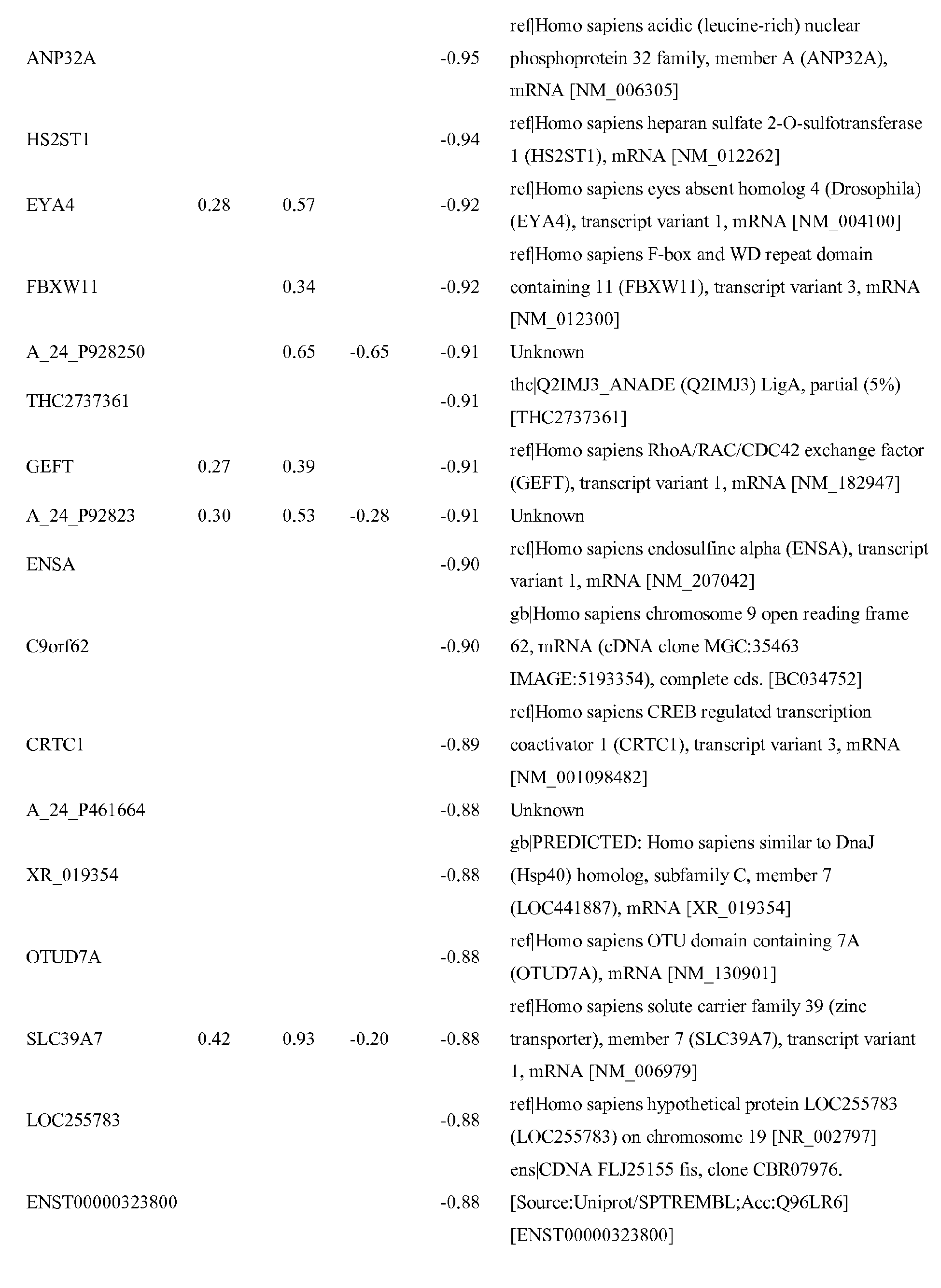

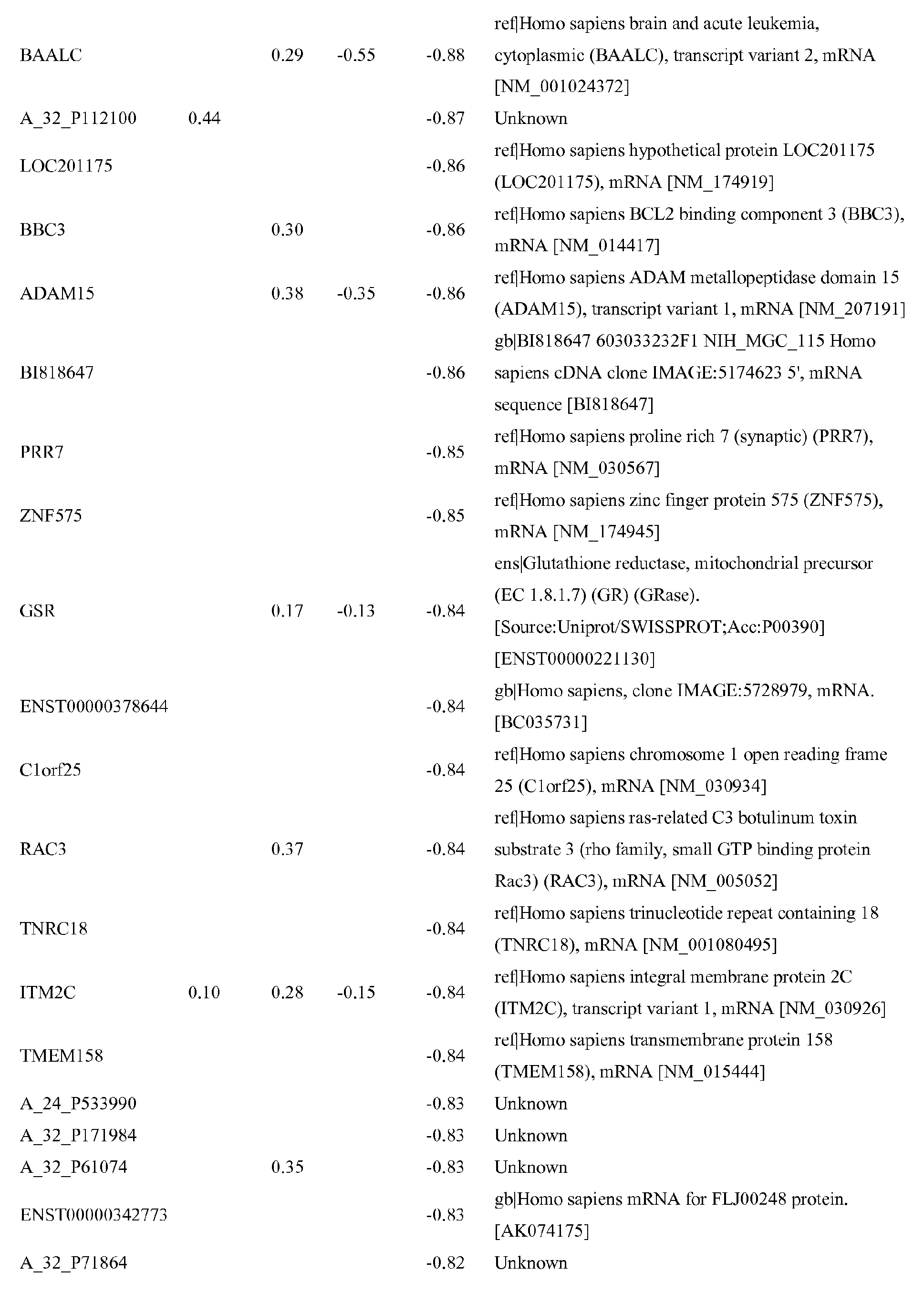

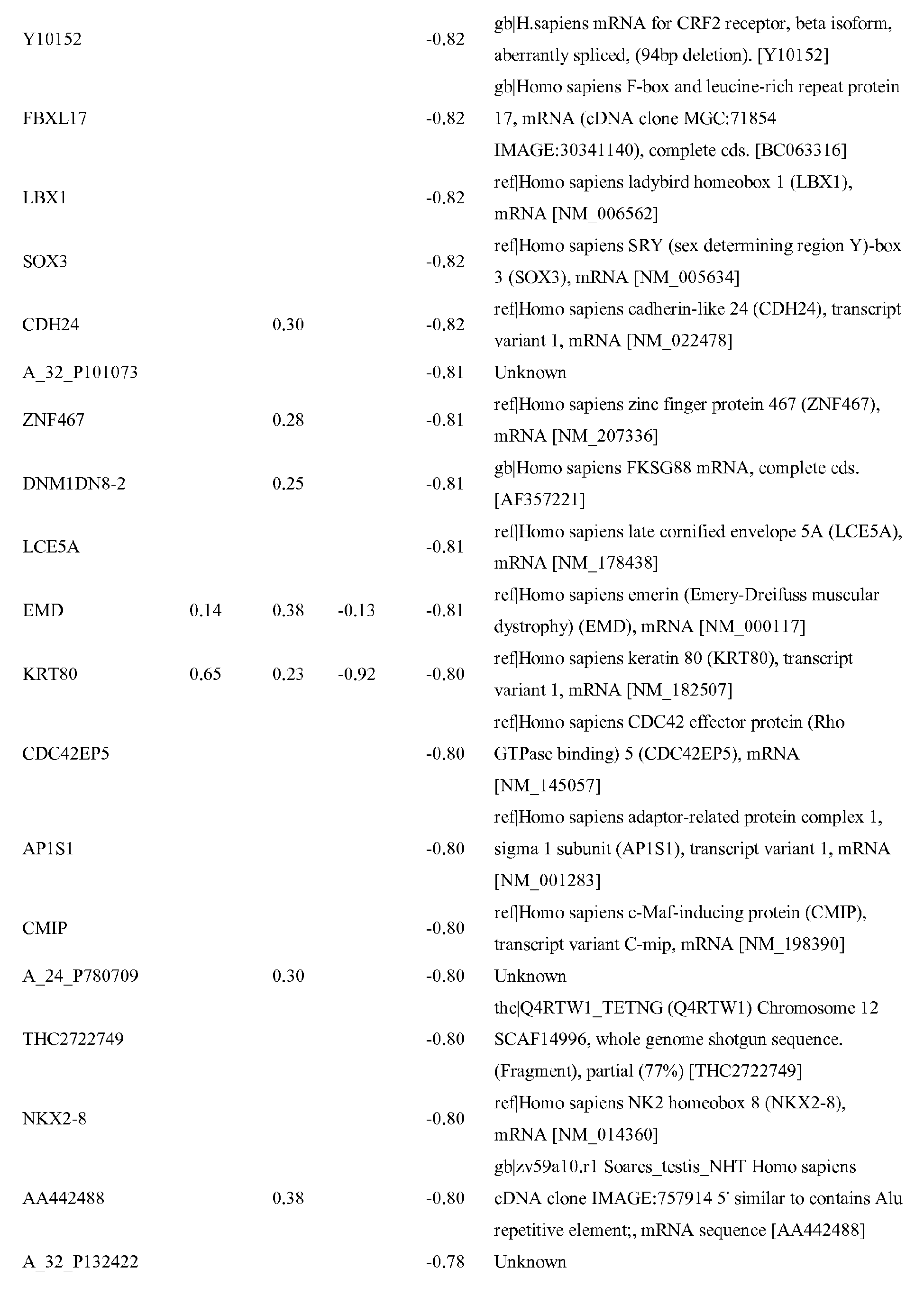

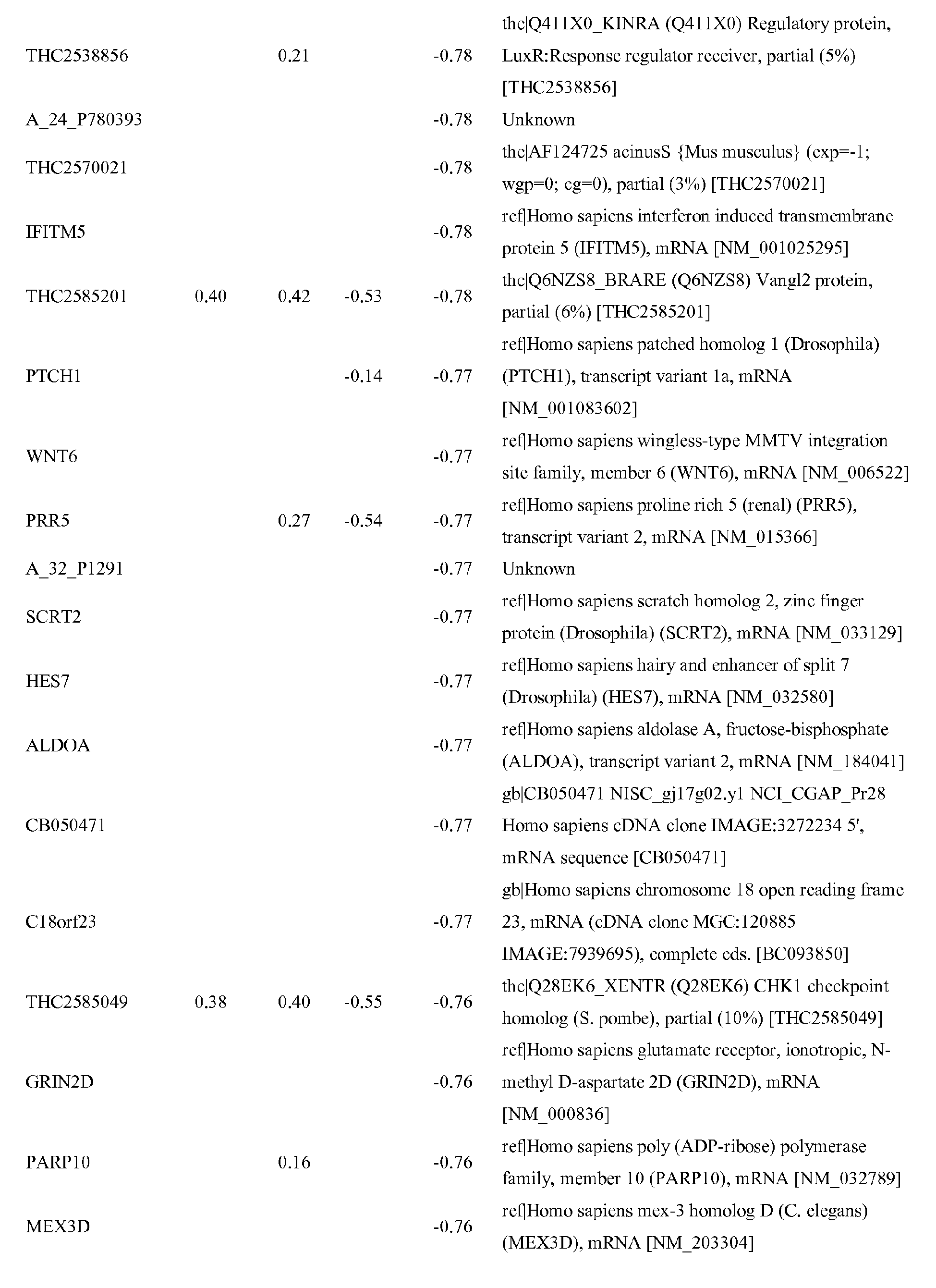

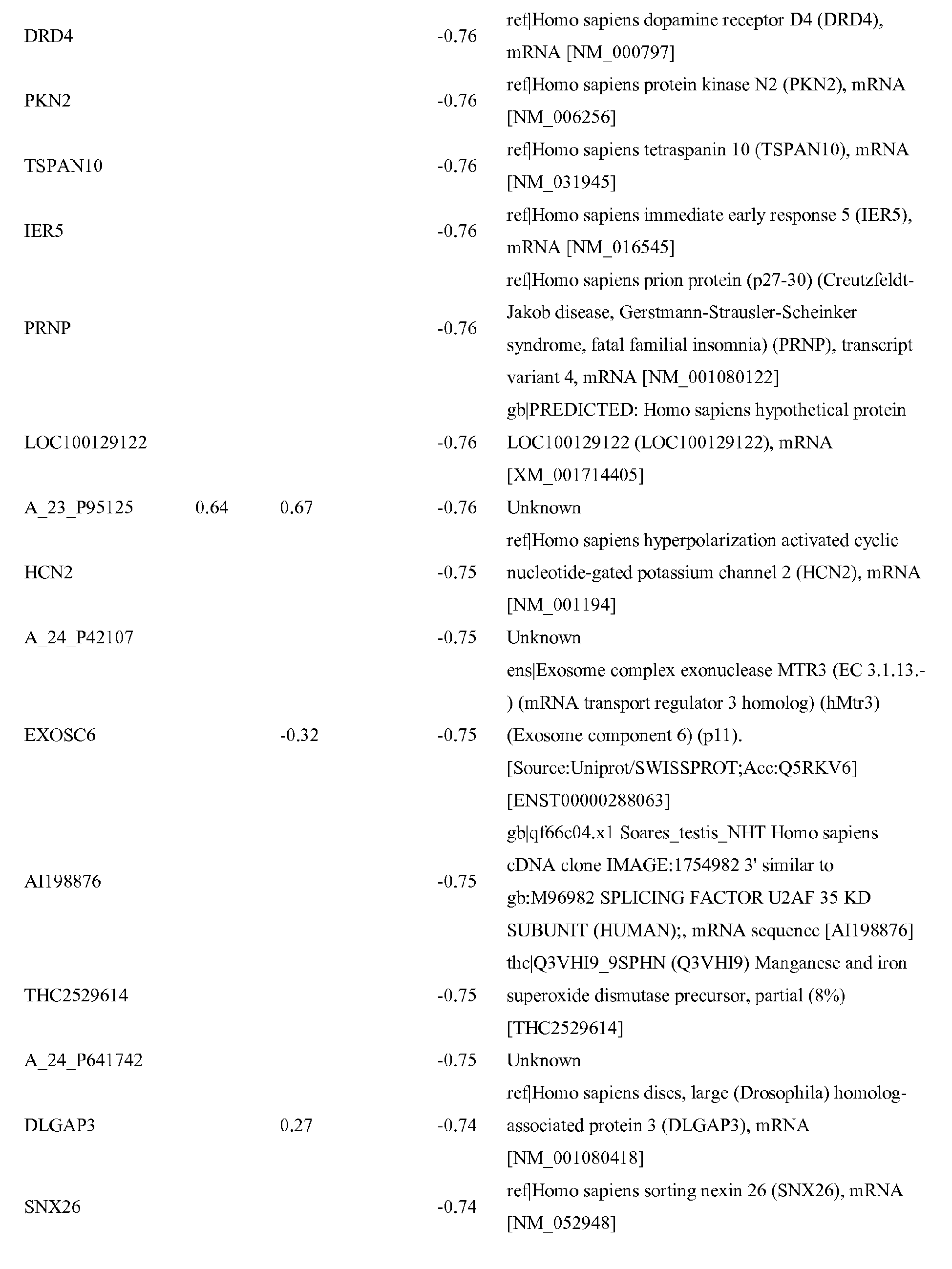

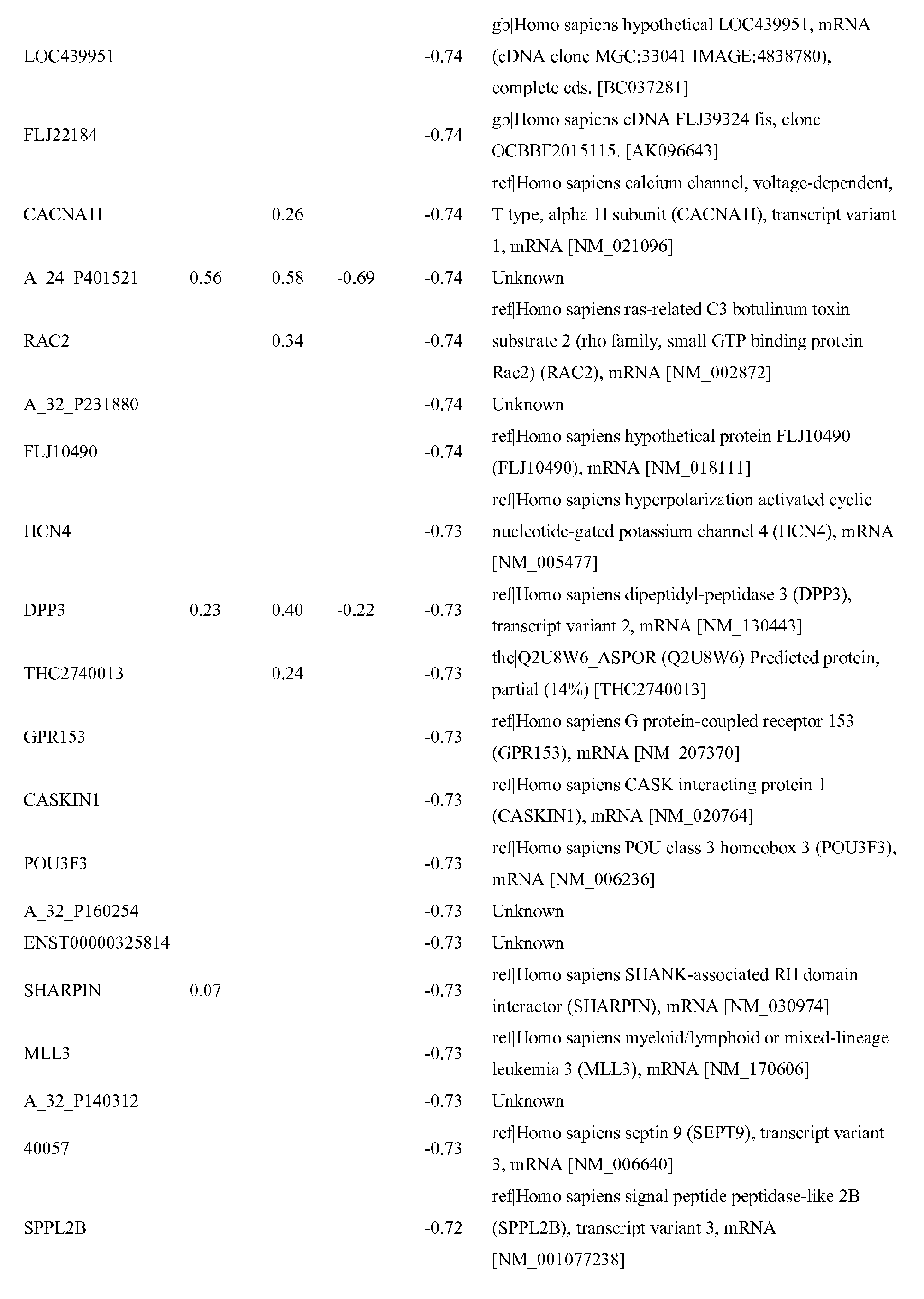

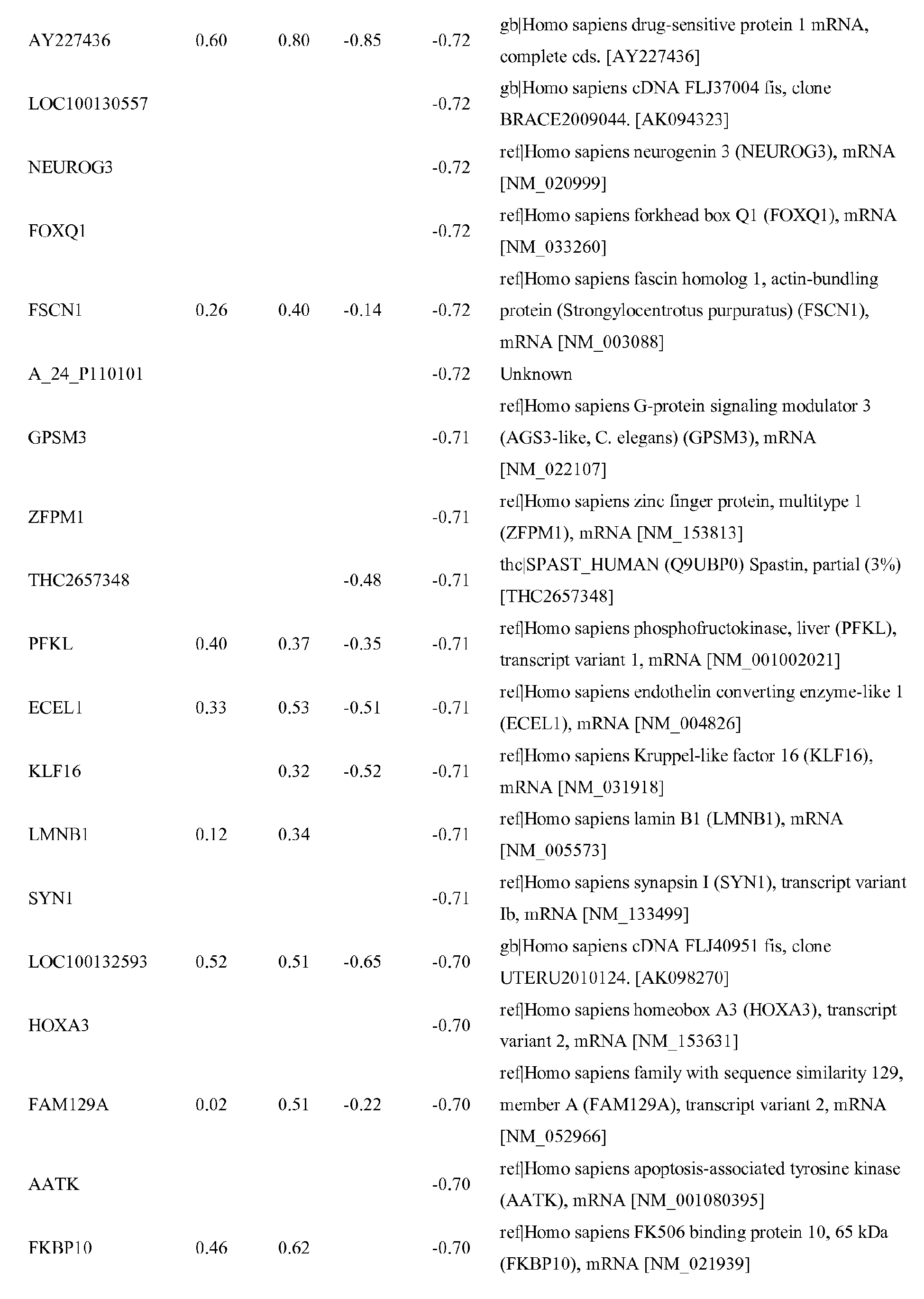

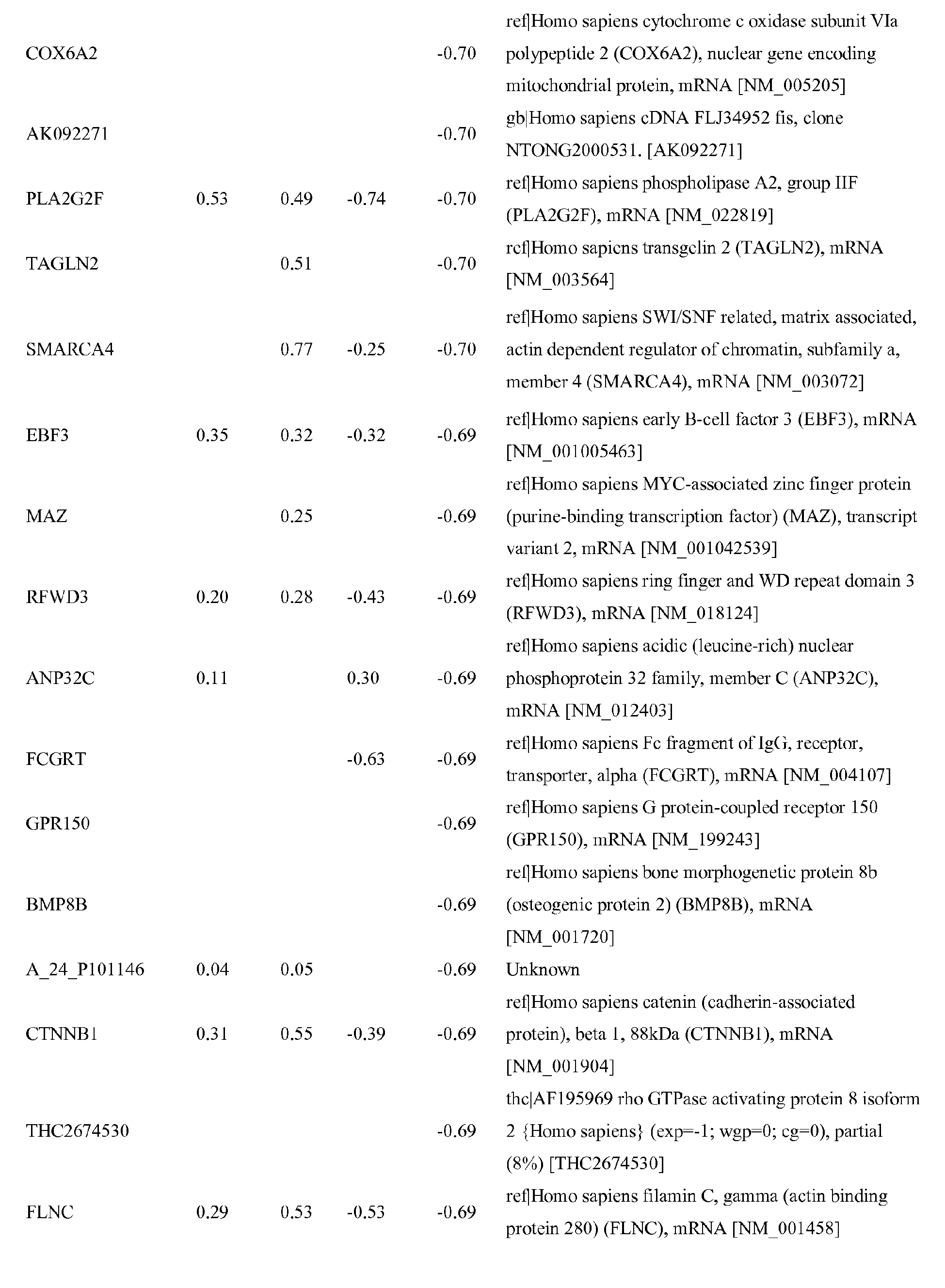

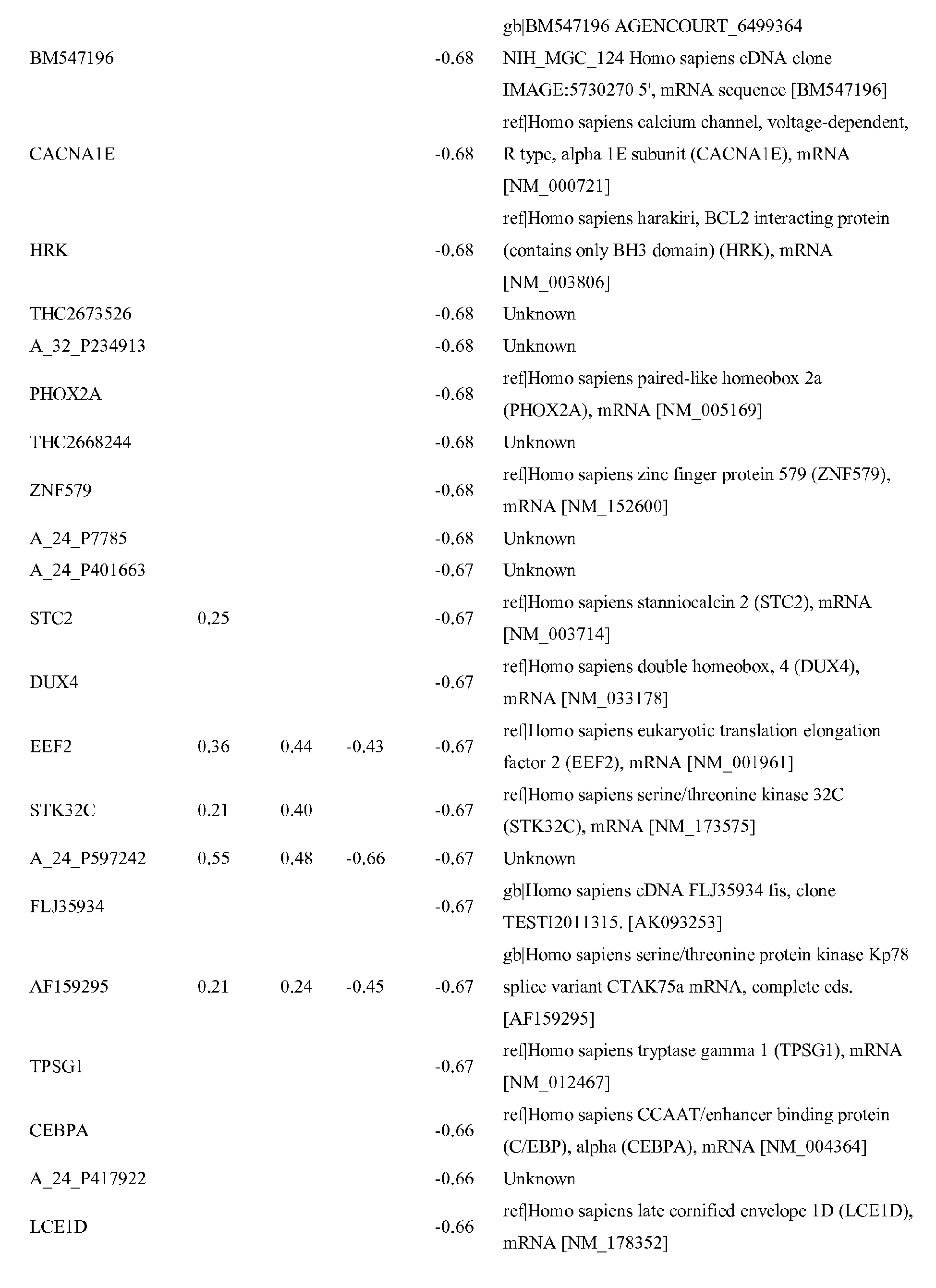

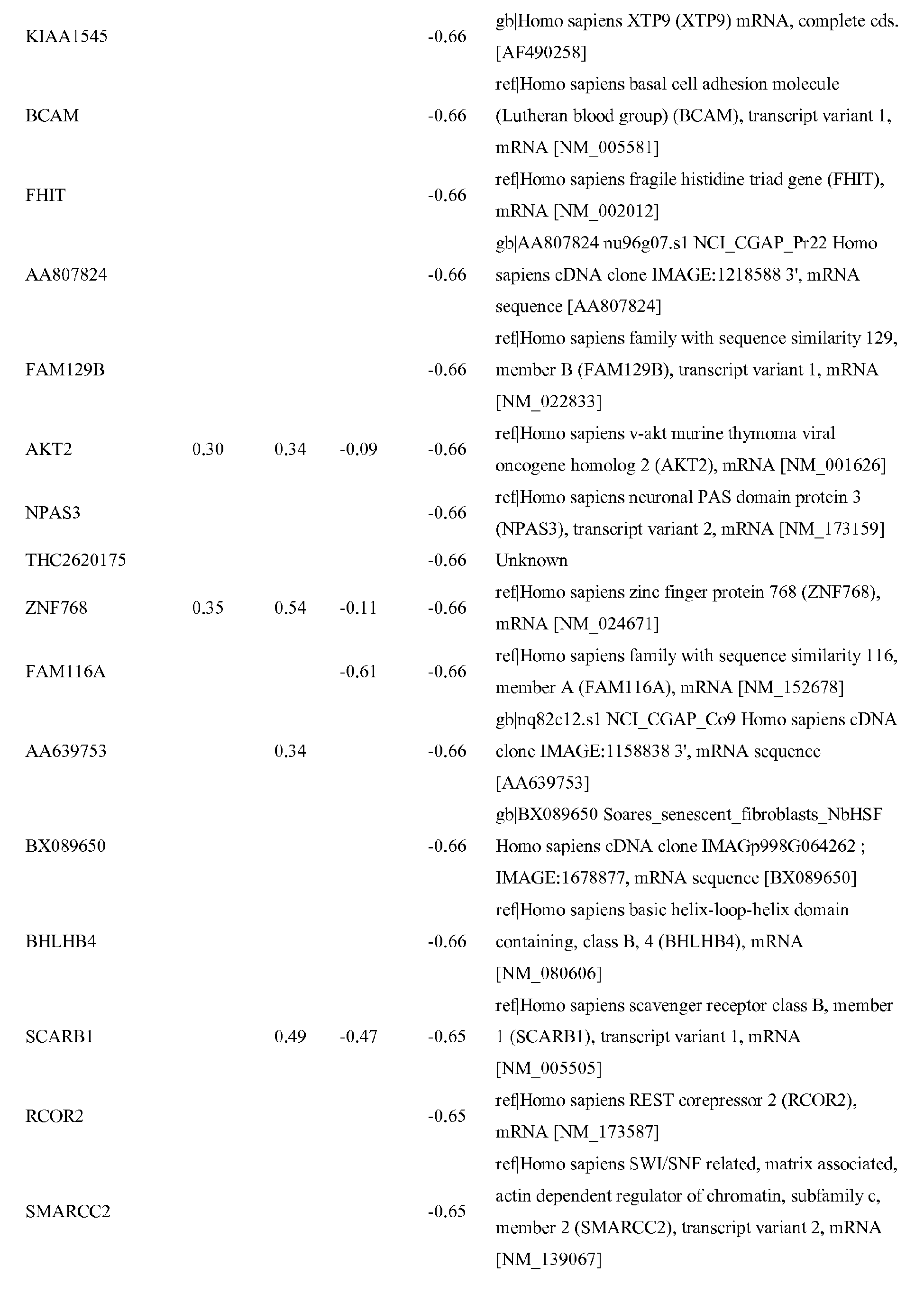

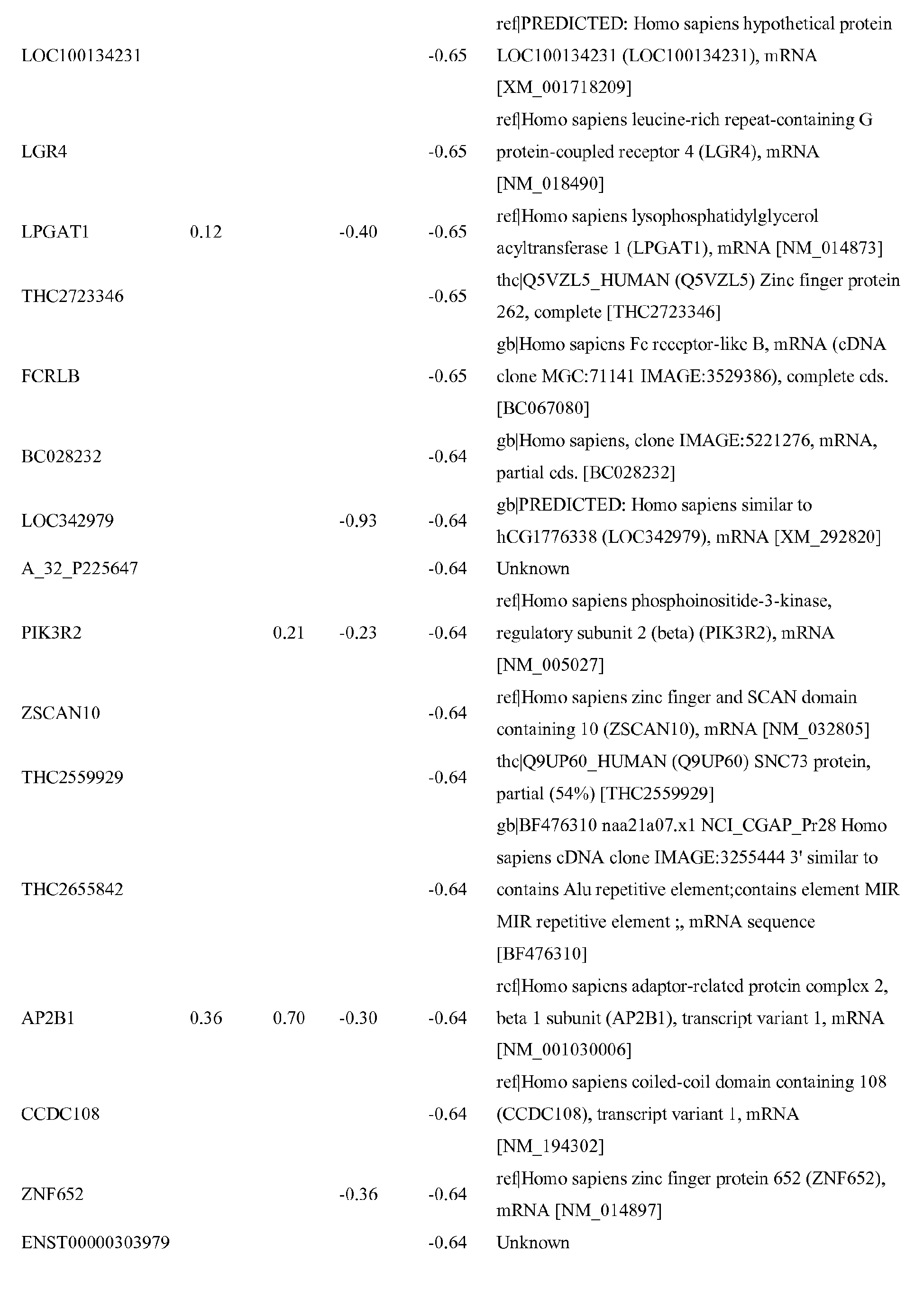

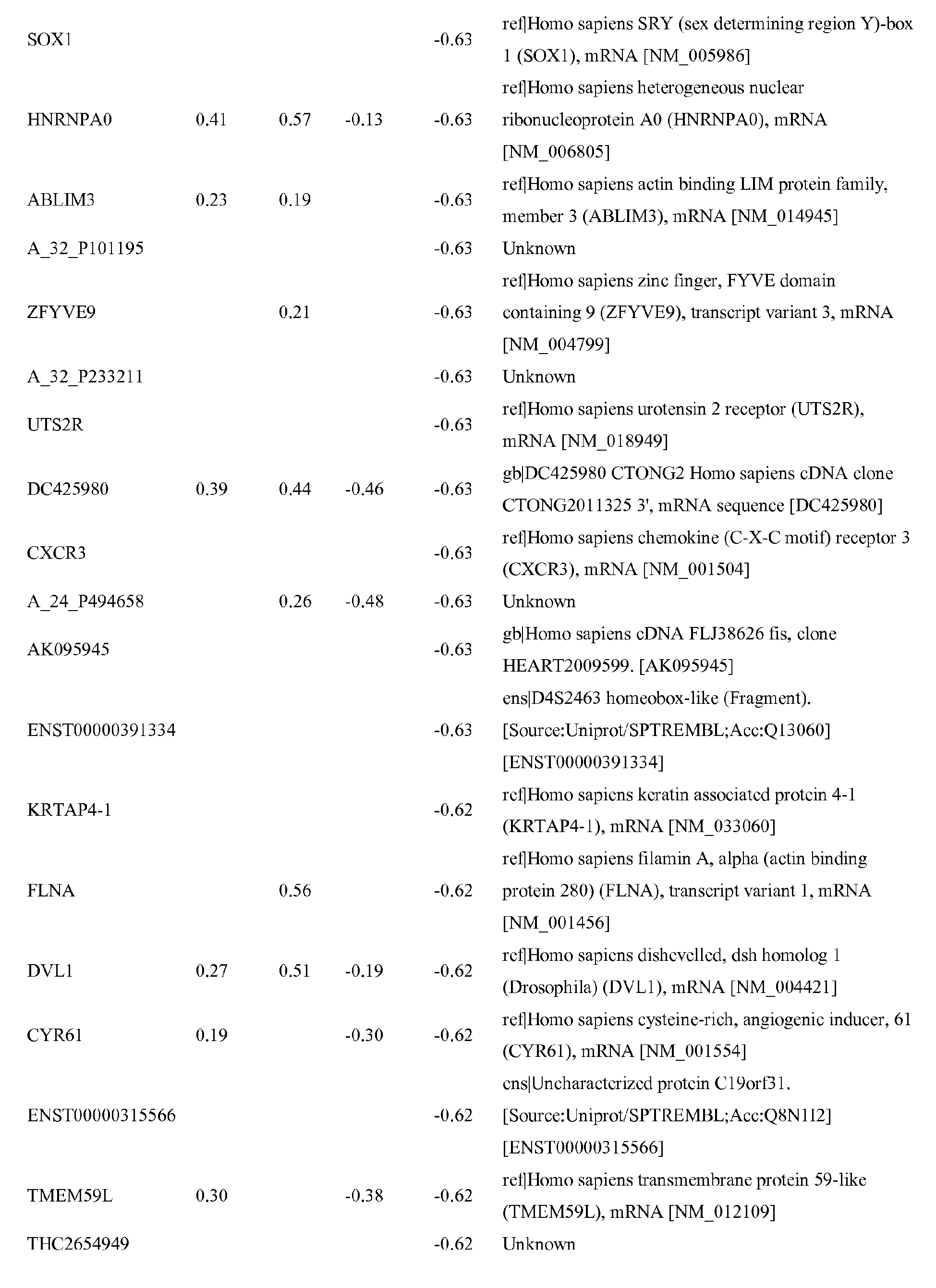

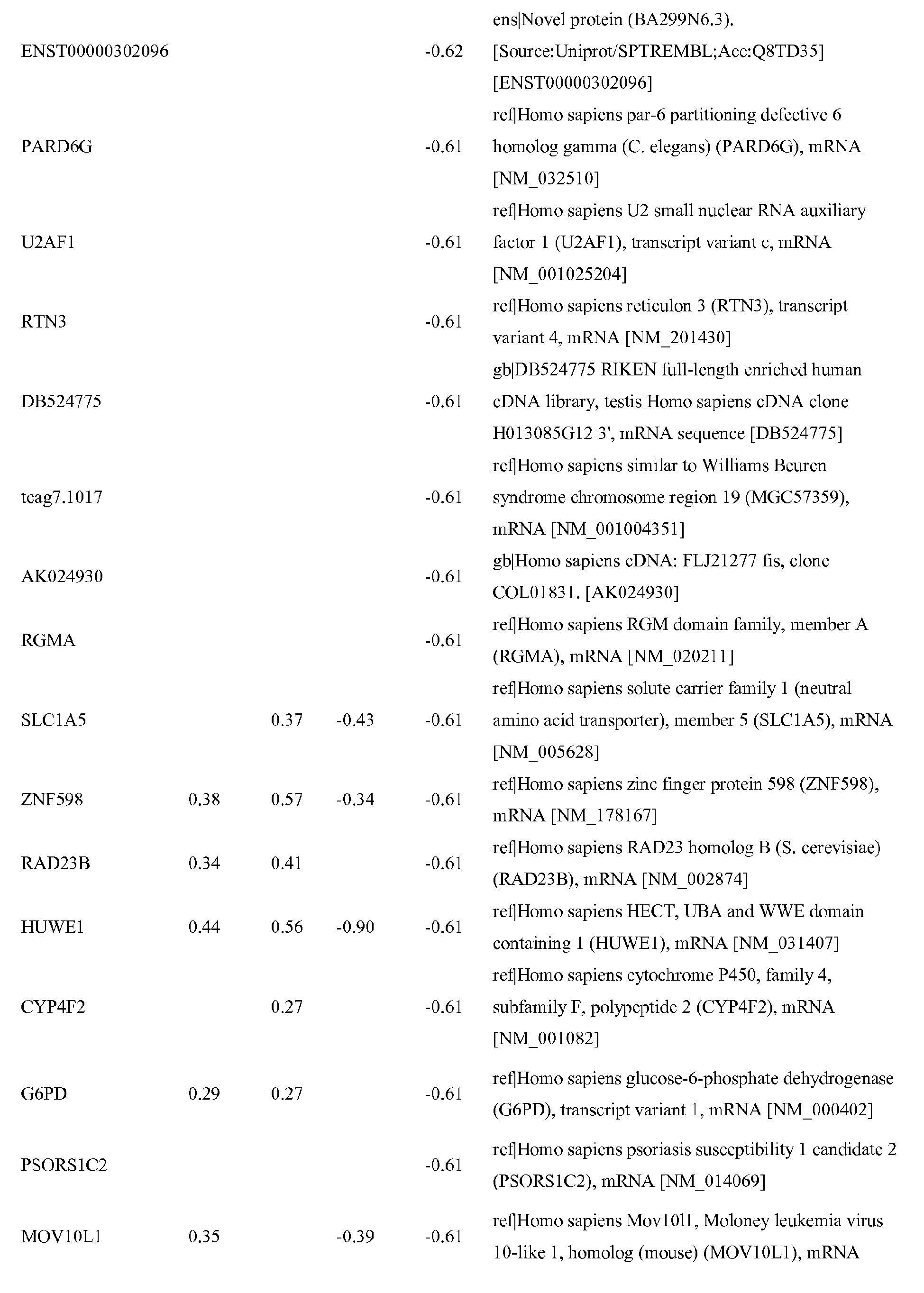

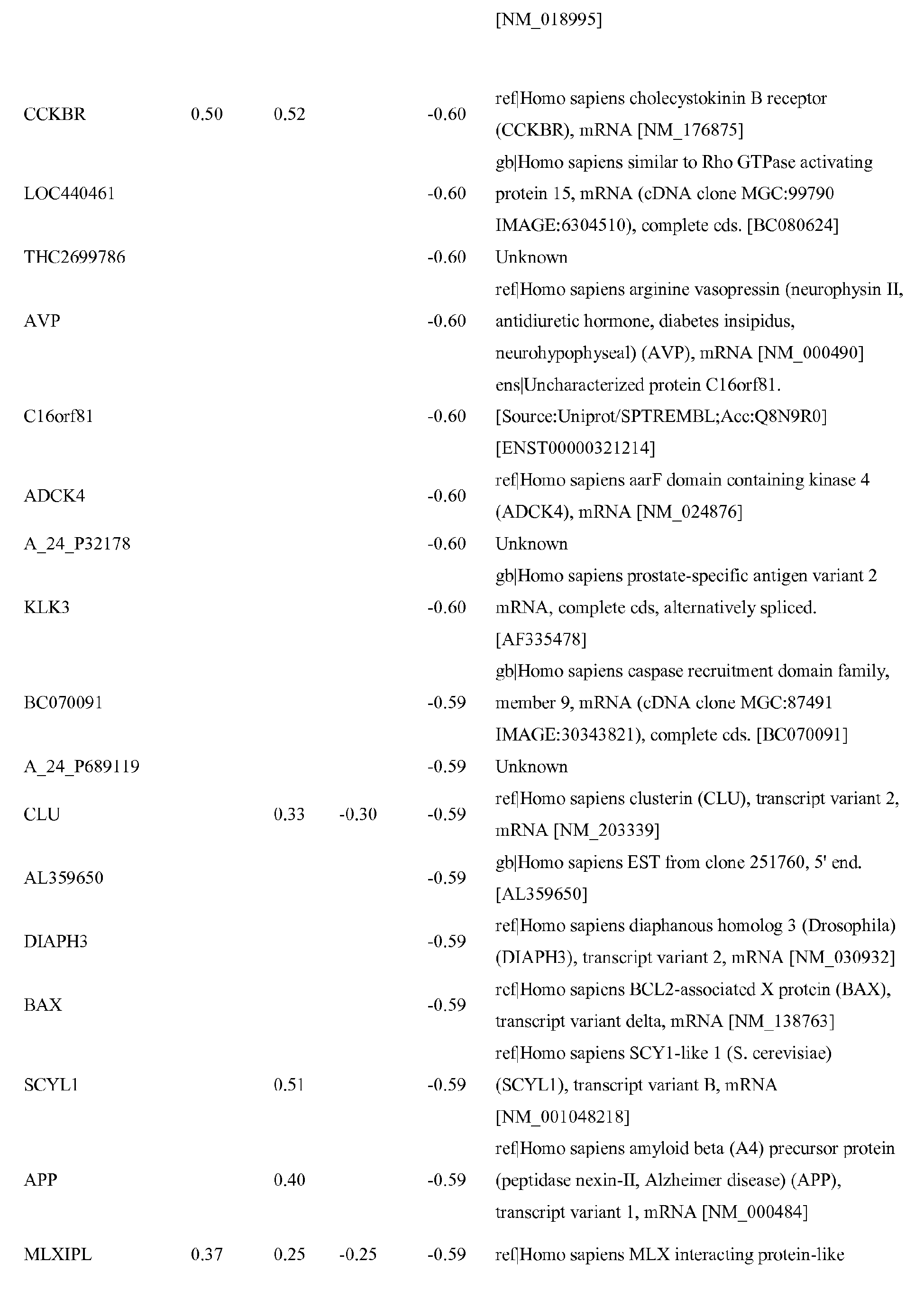

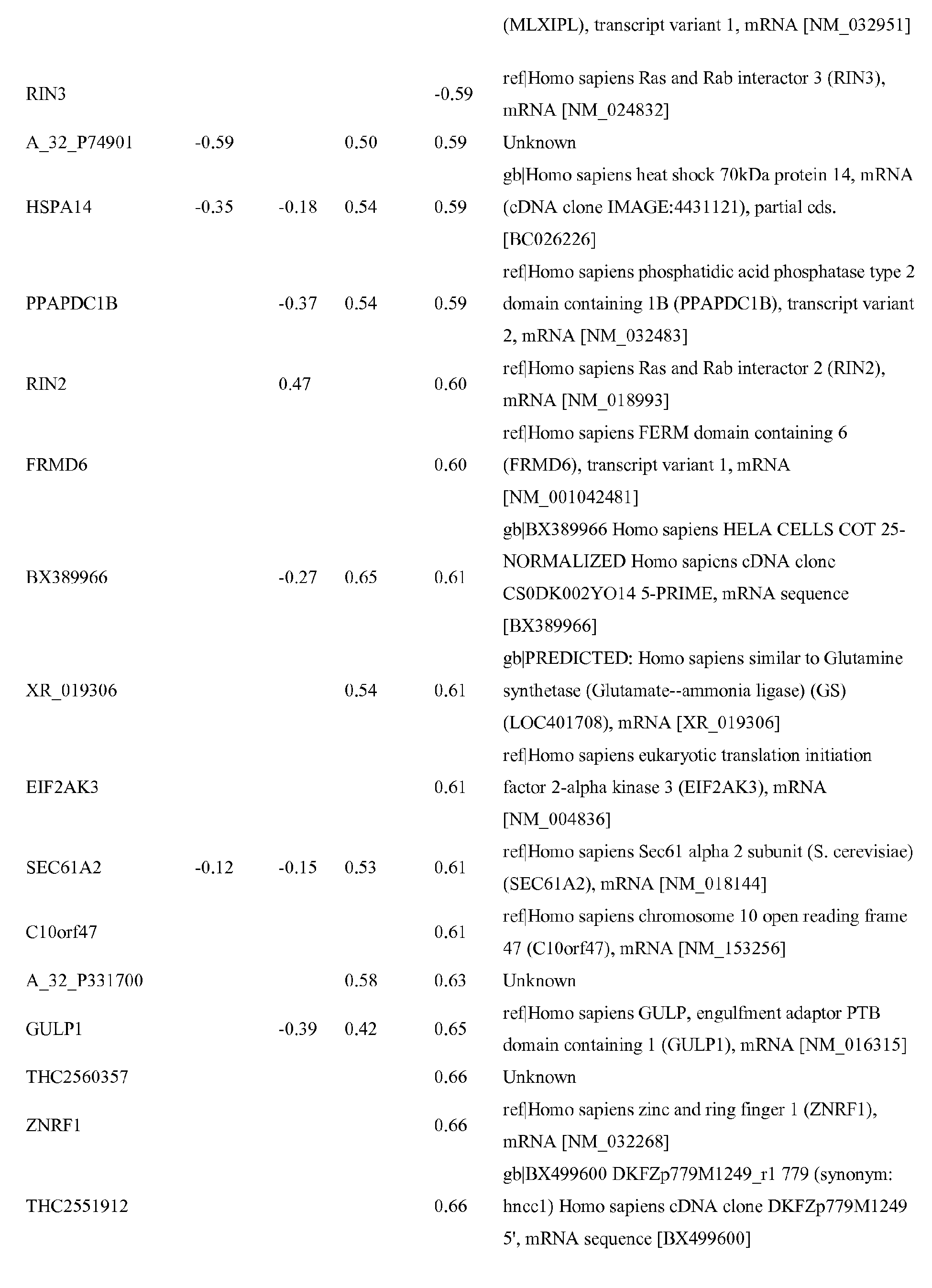

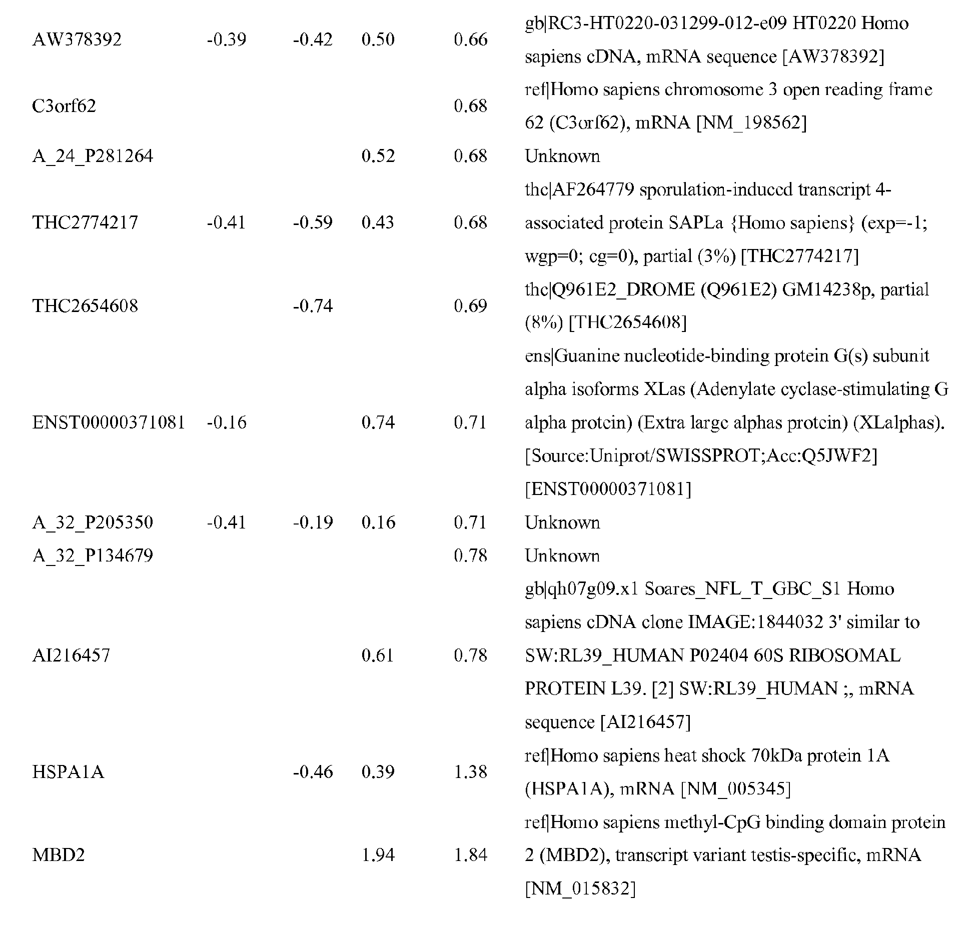

### Supplemental information: Peptides identified and coverage of Tcf4, β-catenin and Tnik in Tcf4 complexes from mouse crypt and villus.

### (A) Tcf4 in crypt (top) and villus (bottom). (B) β-catenin in crypt. (C) TNIK in crypt.

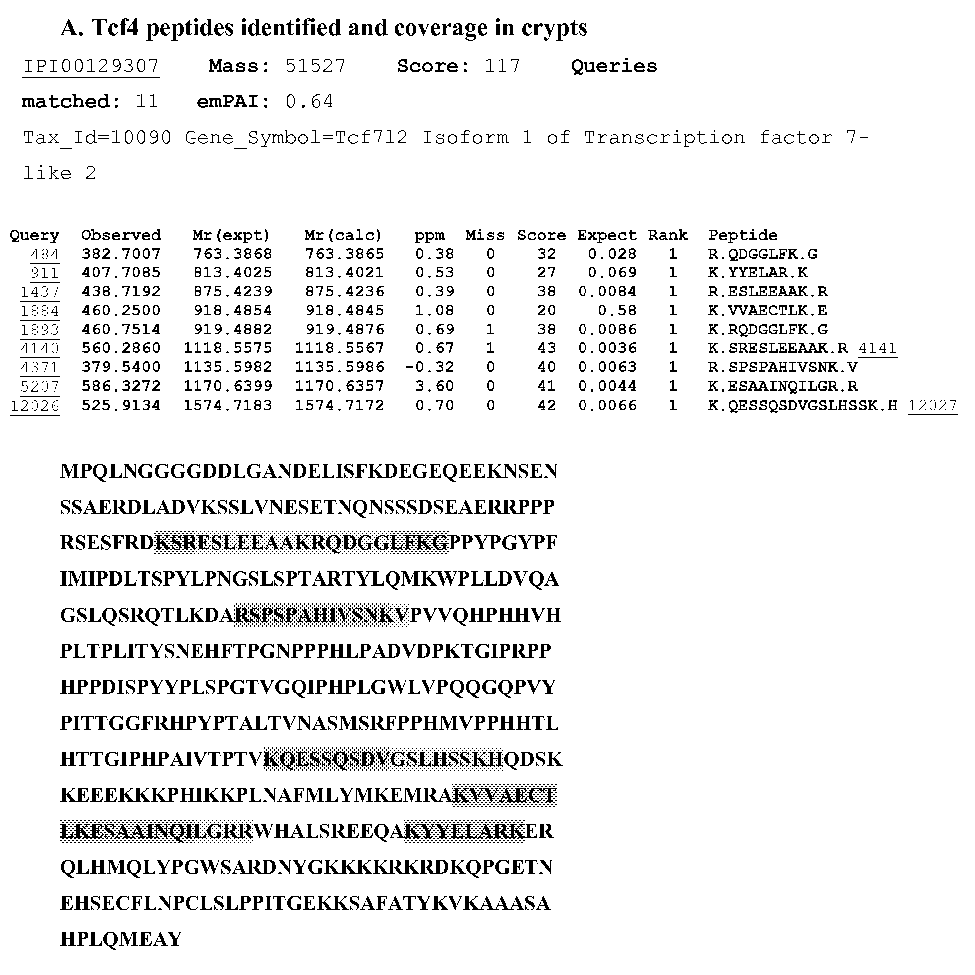

### A. Tcf4 peptides identified and coverage in villus

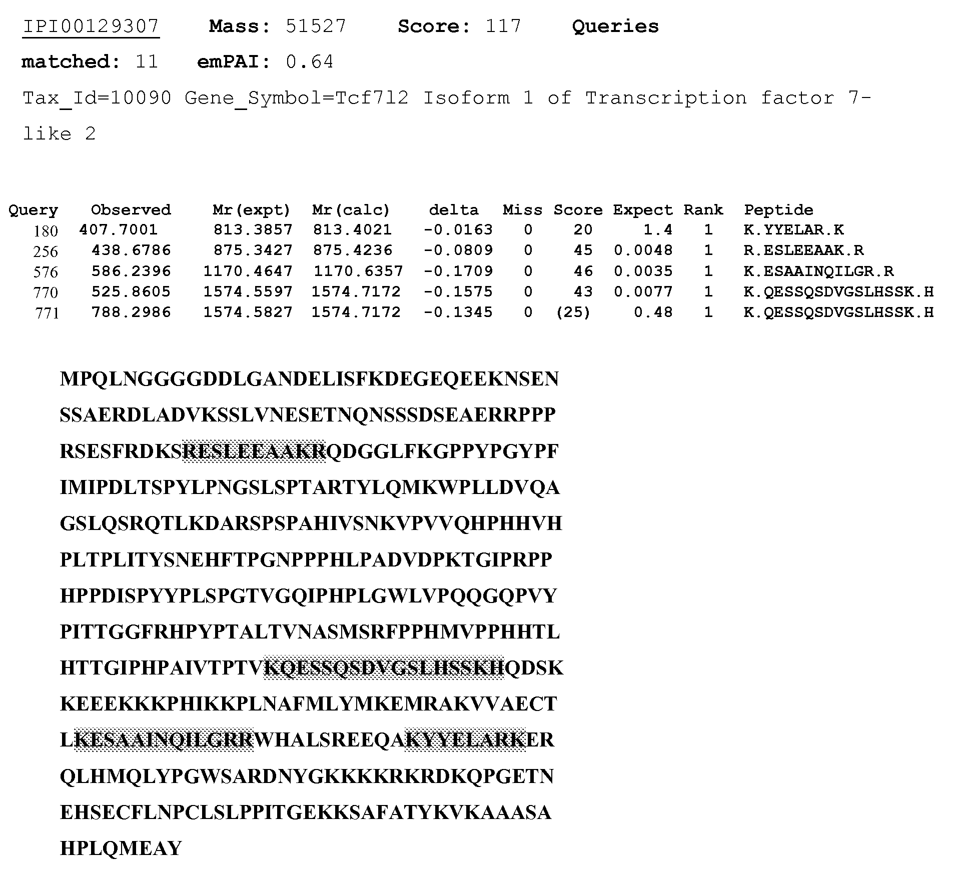

### B. β-catenin peptides identified and coverage in crypts

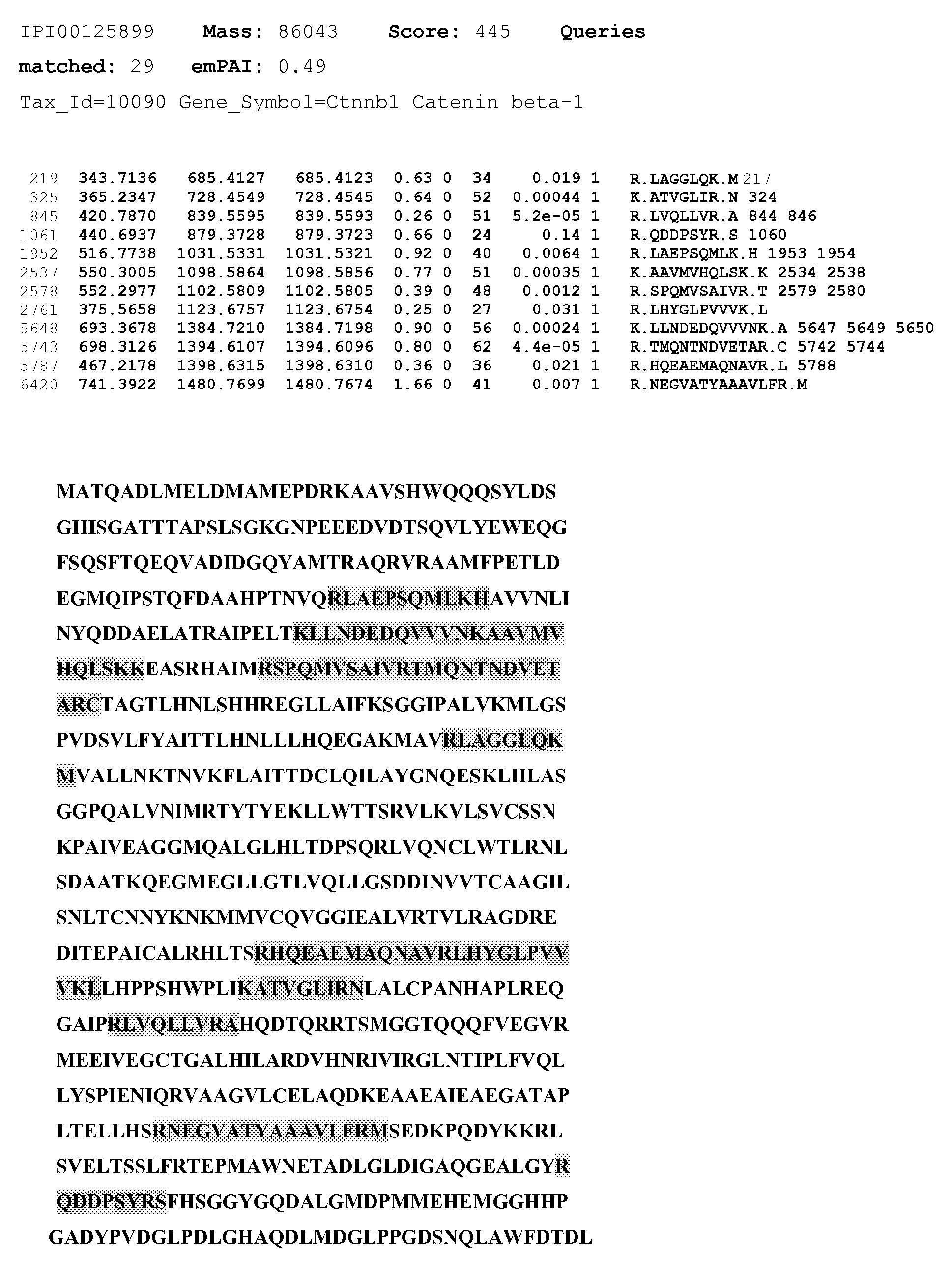

### C.Tnik peptides identified and coverage in crypts

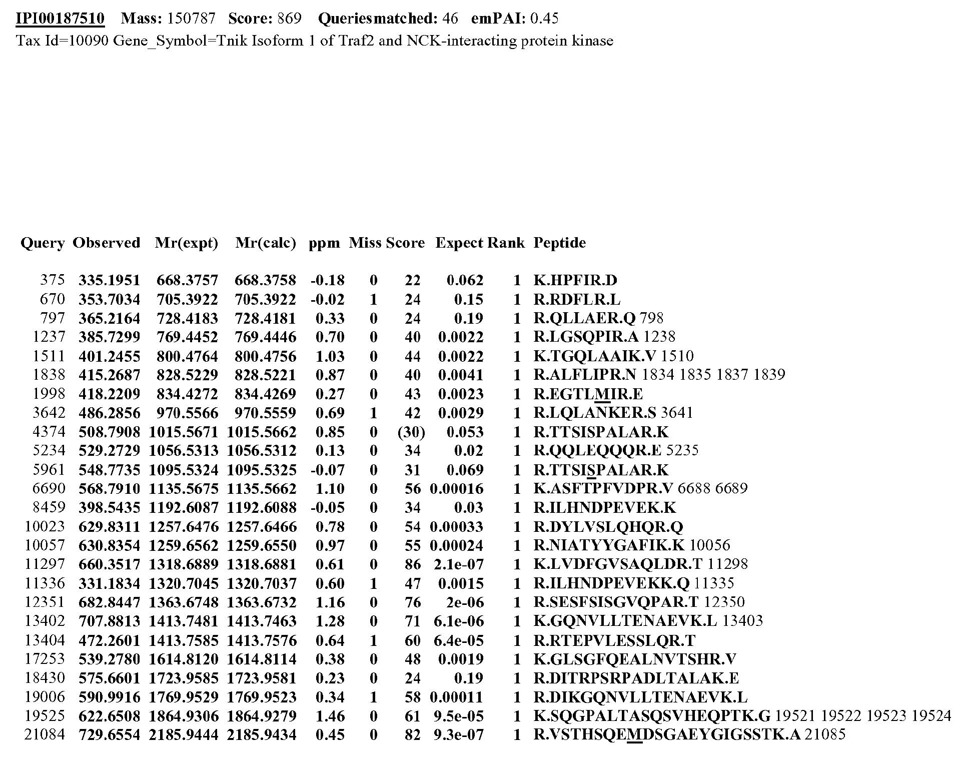

## Claims

1. An inhibitor of TNIK.

2. An inhibitor of TNIK for use as a medicament.

3. An inhibitor of TNIK according to claim 2, wherein the medicament is for treating cancer or a tumor with a constitutively activated Wnt pathway.

4. An inhibitor according to claim 3 wherein the cancer is colon cancer.

5. An inhibitor of TNIK according to any one of the preceding claims wherein the inhibitor is a DNA or RNA molecule, a small molecule, a dominant negative TNIK, an inhibiting antibody raised against TNIK.

6. An inhibitor of TNIK according to claim 5, wherein the inhibitor is an inactivating RNA molecule, preferably a siRNA.

7. A composition comprising an inhibitor of TNIK as defined in any one of the preceding claims.

8. Use of an inhibitor of TNIK as defined in any one of claims 1 to 6 or a composition as defined in claim 7 for the manufacture of a medicament for treating cancer.

9. A method for treating cancer, wherein use is made of an inhibitor of TNIK as defined in any one of claims 1 to 6 or a composition as defined in claim 7.

10. A method for identification of a compound inhibiting TNIK, the method comprising the steps of:
a) providing a test cell population capable of expressing a nucleotide sequence encoding TNIK;
b) contacting the test cell population with the compound;
c) determining the expression level of the nucleotide sequence or the activity of TNIK in the test cell population contacted with the compound;
d) comparing the expression, activity level determined in (c) with the expression, or activity of the nucleotide sequence of TNIK in a test cell population that is not contacted with the compound; and,
e) identifying a compound that produces a difference in expression level, activity of TNIK, between the test cell population that is contacted with the compound and the test cell population that is not contacted with the compound.


SEQUENCE LISTING

<110>  Koninklijke Nederlandse Akademie van Wetenschappen

<120>  Inhibiting TNIK for treating cancer

<130>  P6027752EP

<160>  52

<170>  PatentIn version 3.3

<210>  1
<211>  4083
<212>  DNA
<213>  homo sapiens

<400>  1
   atggcgagcg actccccggc tcgaagcctg gatgaaatag atctctcggc tctgagggac      60
   cctgcaggga tctttgaatt ggtggaactt gttggaaatg gaacatacgg gcaagtttat     120
   aagggtcgtc atgtcaaaac gggccagctt gcagccatca aggttatgga tgtcacaggg     180
   gatgaagagg aagaaatcaa acaagaaatt aacatgttga agaaatattc tcatcaccgg     240
   aatattgcta catactatgg tgcttttatc aaaaagaacc caccaggcat ggatgaccaa     300
   ctttggttgg tgatggagtt ttgtggtgct ggctctgtca ccgacctgat caagaacaca     360
   aaaggtaaca cgttgaaaga ggagtggatt gcatacatct gcagggaaat cttacggggg     420
   ctgagtcacc tgcaccagca taaagtgatt catcgagata ttaaagggca aaatgtcttg     480
   ctgactgaaa atgcagaagt taaactagtg gactttggag tcagtgctca gcttgatcga     540
   acagtgggca ggaggaatac tttcattgga actccctact ggatggcacc agaagttatt     600
   gcctgtgatg aaaacccaga tgccacatat gatttcaaga gtgacttgtg gtctttgggt     660
   atcaccgcca ttgaaatggc agaaggtgct ccccctctct gtgacatgca ccccatgaga     720
   gctctcttcc tcatcccccg gaatccagcg cctcggctga agtctaagaa gtggtcaaaa     780
   aaattccagt catttattga gagctgcttg gtaaagaatc acagccagcg accagcaaca     840
   gaacaattga tgaagcatcc atttatacga gaccaaccta atgagcgaca ggtccgcatt     900
   caactcaagg accatattga tagaacaaag aagaagcgag gagaaaaaga tgagacagag     960
   tatgagtaca gtggaagtga ggaagaagag gaggagaatg actcaggaga gcccagctcc    1020
   atcctgaatc tgccagggga gtcgacgctg cggagggact ttctgaggct gcagctggcc    1080
   aacaaggagc gttctgaggc cctacggagg cagcagctgg agcagcagca gcgggagaat    1140
   gaggagcaca agcggcagct gctggccgag cgtcagaagc gcatcgagga gcagaaagag    1200
   cagaggcggc ggctggagga gcaacaaagg cgagagaagg agctgcggaa gcagcaggag    1260
   agggagcagc gccggcacta tgaggagcag atgcgccggg aggaggagag gaggcgtgcg    1320
   gagcatgaac aggaatacat caggcgacag ttagaggagg agcagagaca gttagagatc    1380
   ttgcagcagc agctactgca tgaacaagct ctacttctgg aatataagcg caaacaattg    1440
   gaagaacaga gacaagcaga aagactgcag aggcagctaa agcaagaaag agactactta    1500
   gtttcccttc agcatcagcg gcaggagcag aggcctgtgg agaagaagcc actgtaccat    1560
   tacaaagaag gaatgagtcc tagtgagaag ccagcatggg ccaaggaggt agaagaacgg 1620
   tcaaggctca accggcaaag ttcccctgcc atgcctcaca aggttgccaa caggatatct 1680
   gaccccaacc tgcccccaag gtcggagtcc ttcagcatta gtggagttca gcctgctcga 1740
   acacccccca tgctcagacc agtcgatccc cagatcccac atctggtagc tgtaaaatcc 1800
   cagggacctg ccttgaccgc ctcccagtca gtgcacgagc agcccacaaa gggcctctct 1860
   gggtttcagg aggctctgaa cgtgacctcc caccgcgtgg agatgccacg ccagaactca 1920
   gatcccacct cggaaaatcc tcctctcccc actcgcattg aaaagtttga ccgaagctct 1980
   tggttacgac aggaagaaga cattccacca aaggtgcctc aaagaacaac ttctatatcc 2040
   ccagcattag ccagaaagaa ttctcctggg aatggtagtg ctctgggacc cagactagga 2100
   tctcaaccca tcagagcaag caaccctgat ctccggagaa ctgagcccat cttggagagc 2160
   cccttgcaga ggaccagcag tggcagttcc tccagctcca gcacccctag ctcccagccc 2220
   agctcccaag gaggctccca gcctggatca caagcaggat ccagtgaacg caccagagtt 2280
   cgagccaaca gtaagtcaga aggatcacct gtgcttcccc atgagcctgc caaggtgaaa 2340
   ccagaagaat ccagggacat tacccggccc agtcgaccag ctagctacaa aaaagctata 2400
   gatgaggatc tgacggcatt agccaaagaa ctaagagaac tccggattga agaaacaaac 2460
   cgcccaatga agaaggtgac tgattactcc tcctccagtg aggagtcaga aagtagcgag 2520
   gaagaggagg aagatggaga gagcgagacc catgatggga cagtggctgt cagcgacata 2580
   cccagactga taccaacagg agctccaggc agcaacgagc agtacaatgt gggaatggtg 2640
   gggacgcatg ggctggagac ctctcatgcg gacagtttca gcggcagtat ttcaagagaa 2700
   ggaaccttga tgattagaga gacgtctgga gagaagaagc gatctggcca cagtgacagc 2760
   aatggctttg ctggccacat caacctccct gacctggtgc agcagagcca ttctccagct 2820
   ggaaccccga ctgagggact ggggcgcgtc tcaacccatt cccaggagat ggactctggg 2880
   actgaatatg gcatggggag cagcaccaaa gcctccttca ccccctttgt ggaccccaga 2940
   gtataccaga cgtctcccac tgatgaagat gaagaggatg aggaatcatc agccgcagct 3000
   ctgtttacta gcgaacttct taggcaagaa caggccaaac tcaatgaagc aagaaagatt 3060
   tcggtggtaa atgtaaaccc aaccaacatt cggcctcata gcgacacacc agaaatcaga 3120
   aaatacaaga aacgattcaa ctcagaaata ctttgtgcag ctctgtgggg tgtaaacctt 3180
   ctggtgggga ctgaaaatgg cctgatgctt ttggaccgaa gtgggcaagg caaagtctat 3240
   aatctgatca accggaggcg atttcagcag atggatgtgc tagagggact gaatgtcctt 3300
   gtgacaattt caggaaagaa gaataagcta cgagtttact atctttcatg gttaagaaac 3360
   agaatactac ataatgaccc agaagtagaa aagaaacaag gctggatcac tgttggggac 3420
   ttggaaggct gtatacatta taaagttgtt aaatatgaaa ggatcaaatt tttggtgatt 3480
   gccttaaaga atgctgtgga aatatatgct tgggctccta aaccgtatca taaattcatg 3540
   gcatttaagt cttttgcaga tctccagcac aagcctctgc tagttgatct cacggtagaa 3600
   gaaggtcaaa gattaaaggt tatttttggt tcacacactg gtttccatgt aattgatgtt 3660
   gattcaggaa actcttatga tatctacata ccatctcata ttcagggcaa tatcactcct 3720
   catgctattg tcatcttgcc taaaacagat ggaatggaaa tgcttgtttg ctatgaggat 3780
   gagggggtgt atgtaaacac ctatggccgg ataactaagg atgtggtgct ccaatgggga 3840
   gaaatgccca cgtctgtggc ctacattcat tccaatcaga taatgggctg gggcgagaaa 3900
   gctattgaga tccggtcagt ggaaacagga catttggatg gagtatttat gcataagcga 3960
   gctcaaaggt taaagtttct atgtgaaaga aatgataagg tattttttgc atccgtgcga 4020
   tctggaggaa gtagccaagt gtttttcatg accctcaaca gaaattccat gatgaactgg 4080
   taa 4083

<210> 2
<211> 1360
<212> PRT
<213> homo sapiens

<400> 2
   Met Ala Ser Asp Ser Pro Ala Arg Ser Leu Asp Glu Ile Asp Leu Ser
   1 5 10 15
   Ala Leu Arg Asp Pro Ala Gly Ile Phe Glu Leu Val Glu Leu Val Gly
   20 25 30
   Asn Gly Thr Tyr Gly Gln Val Tyr Lys Gly Arg His Val Lys Thr Gly
   35 40 45
   Gln Leu Ala Ala Ile Lys Val Met Asp Val Thr Gly Asp Glu Glu Glu
   50 55 60
   Glu Ile Lys Gln Glu Ile Asn Met Leu Lys Lys Tyr Ser His His Arg
   65 70 75 80
   Asn Ile Ala Thr Tyr Tyr Gly Ala Phe Ile Lys Lys Asn Pro Pro Gly
   85 90 95
   Met Asp Asp Gln Leu Trp Leu Val Met Glu Phe Cys Gly Ala Gly Ser
   100 105 110
   Val Thr Asp Leu Ile Lys Asn Thr Lys Gly Asn Thr Leu Lys Glu Glu
   115 120 125
   Trp Ile Ala Tyr Ile Cys Arg Glu Ile Leu Arg Gly Leu Ser His Leu
   130 135 140
   His Gln His Lys Val Ile His Arg Asp Ile Lys Gly Gln Asn Val Leu
   145 150 155 160
   Leu Thr Glu Asn Ala Glu Val Lys Leu Val Asp Phe Gly Val Ser Ala
   165 170 175
   Gln Leu Asp Arg Thr Val Gly Arg Arg Asn Thr Phe Ile Gly Thr Pro
   180 185 190
   Tyr Trp Met Ala Pro Glu Val Ile Ala Cys Asp Glu Asn Pro Asp Ala
   195 200 205
   Thr Tyr Asp Phe Lys Ser Asp Leu Trp Ser Leu Gly Ile Thr Ala Ile
   210 215 220
   Glu Met Ala Glu Gly Ala Pro Pro Leu Cys Asp Met His Pro Met Arg
   225 230 235 240
   Ala Leu Phe Leu Ile Pro Arg Asn Pro Ala Pro Arg Leu Lys Ser Lys
   245 250 255
   Lys Trp Ser Lys Lys Phe Gln Ser Phe Ile Glu Ser Cys Leu Val Lys
   260 265 270
   Asn His Ser Gln Arg Pro Ala Thr Glu Gln Leu Met Lys His Pro Phe
   275 280 285
   Ile Arg Asp Gln Pro Asn Glu Arg Gln Val Arg Ile Gln Leu Lys Asp
   290 295 300
   His Ile Asp Arg Thr Lys Lys Lys Arg Gly Glu Lys Asp Glu Thr Glu
   305 310 315 320
   Tyr Glu Tyr Ser Gly Ser Glu Glu Glu Glu Glu Glu Asn Asp Ser Gly
   325 330 335
   Glu Pro Ser Ser Ile Leu Asn Leu Pro Gly Glu Ser Thr Leu Arg Arg
   340 345 350
   Asp Phe Leu Arg Leu Gln Leu Ala Asn Lys Glu Arg Ser Glu Ala Leu
   355 360 365
   Arg Arg Gln Gln Leu Glu Gln Gln Gln Arg Glu Asn Glu Glu His Lys
   370 375 380
   Arg Gln Leu Leu Ala Glu Arg Gln Lys Arg Ile Glu Glu Gln Lys Glu
   385 390 395 400
   Gln Arg Arg Arg Leu Glu Glu Gln Gln Arg Arg Glu Lys Glu Leu Arg
   405 410 415
   Lys Gln Gln Glu Arg Glu Gln Arg Arg His Tyr Glu Glu Gln Met Arg
   420 425 430
   Arg Glu Glu Glu Arg Arg Arg Ala Glu His Glu Gln Glu Tyr Ile Arg
   435 440 445
   Arg Gln Leu Glu Glu Glu Gln Arg Gln Leu Glu Ile Leu Gln Gln Gln
       450                 455                 460
   Leu Leu His Glu Gln Ala Leu Leu Leu Glu Tyr Lys Arg Lys Gln Leu
   465                 470                 475                 480
   Glu Glu Gln Arg Gln Ala Glu Arg Leu Gln Arg Gln Leu Lys Gln Glu
                   485                 490                 495
   Arg Asp Tyr Leu Val Ser Leu Gln His Gln Arg Gln Glu Gln Arg Pro
               500                 505                 510
   Val Glu Lys Lys Pro Leu Tyr His Tyr Lys Glu Gly Met Ser Pro Ser
           515                 520                 525
   Glu Lys Pro Ala Trp Ala Lys Glu Val Glu Glu Arg Ser Arg Leu Asn
       530                 535                 540
   Arg Gln Ser Ser Pro Ala Met Pro His Lys Val Ala Asn Arg Ile Ser
   545                 550                 555                 560
   Asp Pro Asn Leu Pro Pro Arg Ser Glu Ser Phe Ser Ile Ser Gly Val
                   565                 570                 575
   Gln Pro Ala Arg Thr Pro Pro Met Leu Arg Pro Val Asp Pro Gln Ile
               580                 585                 590
   Pro His Leu Val Ala Val Lys Ser Gln Gly Pro Ala Leu Thr Ala Ser
           595                 600                 605
   Gln Ser Val His Glu Gln Pro Thr Lys Gly Leu Ser Gly Phe Gln Glu
       610                 615                 620
   Ala Leu Asn Val Thr Ser His Arg Val Glu Met Pro Arg Gln Asn Ser
   625                 630                 635                 640
   Asp Pro Thr Ser Glu Asn Pro Pro Leu Pro Thr Arg Ile Glu Lys Phe
                   645                 650                 655
   Asp Arg Ser Ser Trp Leu Arg Gln Glu Glu Asp Ile Pro Pro Lys Val
               660                 665                 670
   Pro Gln Arg Thr Thr Ser Ile Ser Pro Ala Leu Ala Arg Lys Asn Ser
           675                 680                 685
   Pro Gly Asn Gly Ser Ala Leu Gly Pro Arg Leu Gly Ser Gln Pro Ile
       690                 695                 700
   Arg Ala Ser Asn Pro Asp Leu Arg Arg Thr Glu Pro Ile Leu Glu Ser
   705                 710                 715                 720
   Pro Leu Gln Arg Thr Ser Ser Gly Ser Ser Ser Ser Ser Ser Thr Pro
   725 730 735
   Ser Ser Gln Pro Ser Ser Gln Gly Gly Ser Gln Pro Gly Ser Gln Ala
   740 745 750
   Gly Ser Ser Glu Arg Thr Arg Val Arg Ala Asn Ser Lys Ser Glu Gly
   755 760 765
   Ser Pro Val Leu Pro His Glu Pro Ala Lys Val Lys Pro Glu Glu Ser
   770 775 780
   Arg Asp Ile Thr Arg Pro Ser Arg Pro Ala Ser Tyr Lys Lys Ala Ile
   785 790 795 800
   Asp Glu Asp Leu Thr Ala Leu Ala Lys Glu Leu Arg Glu Leu Arg Ile
   805 810 815
   Glu Glu Thr Asn Arg Pro Met Lys Lys Val Thr Asp Tyr Ser Ser Ser
   820 825 830
   Ser Glu Glu Ser Glu Ser Ser Glu Glu Glu Glu Glu Asp Gly Glu Ser
   835 840 845
   Glu Thr His Asp Gly Thr Val Ala Val Ser Asp Ile Pro Arg Leu Ile
   850 855 860
   Pro Thr Gly Ala Pro Gly Ser Asn Glu Gln Tyr Asn Val Gly Met Val
   865 870 875 880
   Gly Thr His Gly Leu Glu Thr Ser His Ala Asp Ser Phe Ser Gly Ser
   885 890 895
   Ile Ser Arg Glu Gly Thr Leu Met Ile Arg Glu Thr Ser Gly Glu Lys
   900 905 910
   Lys Arg Ser Gly His Ser Asp Ser Asn Gly Phe Ala Gly His Ile Asn
   915 920 925
   Leu Pro Asp Leu Val Gln Gln Ser His Ser Pro Ala Gly Thr Pro Thr
   930 935 940
   Glu Gly Leu Gly Arg Val Ser Thr His Ser Gln Glu Met Asp Ser Gly
   945 950 955 960
   Thr Glu Tyr Gly Met Gly Ser Ser Thr Lys Ala Ser Phe Thr Pro Phe
   965 970 975
   Val Asp Pro Arg Val Tyr Gln Thr Ser Pro Thr Asp Glu Asp Glu Glu
   980 985 990
   Asp Glu Glu Ser Ser Ala Ala Ala Leu Phe Thr Ser Glu Leu Leu Arg
   995 1000 1005
   Gln Glu Gln Ala Lys Leu Asn Glu Ala Arg Lys Ile Ser Val Val
       1010                1015                1020
   Asn Val Asn Pro Thr Asn Ile Arg Pro His Ser Asp Thr Pro Glu
       1025                1030                1035
   Ile Arg Lys Tyr Lys Lys Arg Phe Asn Ser Glu Ile Leu Cys Ala
       1040                1045                1050
   Ala Leu Trp Gly Val Asn Leu Leu Val Gly Thr Glu Asn Gly Leu
       1055                1060                1065
   Met Leu Leu Asp Arg Ser Gly Gln Gly Lys Val Tyr Asn Leu Ile
       1070                1075                1080
   Asn Arg Arg Arg Phe Gln Gln Met Asp Val Leu Glu Gly Leu Asn
       1085                1090                1095
   Val Leu Val Thr Ile Ser Gly Lys Lys Asn Lys Leu Arg Val Tyr
       1100                1105                1110
   Tyr Leu Ser Trp Leu Arg Asn Arg Ile Leu His Asn Asp Pro Glu
       1115                1120                1125
   Val Glu Lys Lys Gln Gly Trp Ile Thr Val Gly Asp Leu Glu Gly
       1130                1135                1140
   Cys Ile His Tyr Lys Val Val Lys Tyr Glu Arg Ile Lys Phe Leu
       1145                1150                1155
   Val Ile Ala Leu Lys Asn Ala Val Glu Ile Tyr Ala Trp Ala Pro
       1160                1165                1170
   Lys Pro Tyr His Lys Phe Met Ala Phe Lys Ser Phe Ala Asp Leu
       1175                1180                1185
   Gln His Lys Pro Leu Leu Val Asp Leu Thr Val Glu Glu Gly Gln
       1190                1195                1200
   Arg Leu Lys Val Ile Phe Gly Ser His Thr Gly Phe His Val Ile
       1205                1210                1215
   Asp Val Asp Ser Gly Asn Ser Tyr Asp Ile Tyr Ile Pro Ser His
       1220                1225                1230
   Ile Gln Gly Asn Ile Thr Pro His Ala Ile Val Ile Leu Pro Lys
       1235                1240                1245
   Thr Asp Gly Met Glu Met Leu Val Cys Tyr Glu Asp Glu Gly Val
       1250                1255                1260
   Tyr Val Asn Thr Tyr Gly Arg Ile Thr Lys Asp Val Val Leu Gln
       1265                1270                1275
   Trp Gly Glu Met Pro Thr Ser Val Ala Tyr Ile His Ser Asn Gln
       1280                1285                1290
   Ile Met Gly Trp Gly Glu Lys Ala Ile Glu Ile Arg Ser Val Glu
       1295                1300                1305
   Thr Gly His Leu Asp Gly Val Phe Met His Lys Arg Ala Gln Arg
       1310                1315                1320
   Leu Lys Phe Leu Cys Glu Arg Asn Asp Lys Val Phe Phe Ala Ser
       1325                1330                1335
   Val Arg Ser Gly Gly Ser Ser Gln Val Phe Phe Met Thr Leu Asn
       1340                1345                1350
   Arg Asn Ser Met Met Asn Trp
       1355                1360

<210>  3
<211>  4059
<212>  DNA
<213>  homo sapiens

<400>  3
atggcgagcg actccccggc tcgaagcctg gatgaaatag atctctcggc tctgagggac      60

cccgcaggga tctttgaatt ggtggaactt gttggaaatg gaacatacgg gcaagtttat     120

aagggtcgtc atgtcaaaac gggccagctt gcagccatca aggttatgga tgtcacaggg     180

gatgaagagg aagaaatcaa acaagaaatt aacatgttga agaaatattc tcatcaccgg     240

aatattgcta catactatgg tgcttttatc aaaaagaacc caccaggcat ggatgaccaa     300

ctttggttgg tgatggagtt ttgtggtgct ggctctgtca ccgacctgat caagaacaca     360

aaaggtaaca cgttgaaaga ggagtggatt gcatacatct gcagggaaat cttacggggg     420

ctgagtcacc tgcaccagca taaagtgatt catcgagata ttaaagggca aaatgtcttg     480

ctgactgaaa atgcagaagt taaactagtg gactttggag tcagtgctca gcttgatcga     540

acagtgggca ggaggaatac tttcattgga actccctact ggatggcacc agaagttatt     600

gcctgtgatg aaaacccaga tgccacatat gatttcaaga gtgacttgtg gtctttgggt     660

atcaccgcca ttgaaatggc agaaggtgct ccccctctct gtgacatgca ccccatgaga     720

gctctcttcc tcatcccccg gaacccagcg cctcggctga agtctaagaa gtggtcaaaa     780

aaattccagt catttattga gagctgcttg gtaaagaatc acagccagcg accagcaaca     840

gaacaattga tgaagcatcc atttatacga gaccaaccta atgagcgaca ggtccgcatt     900

caactcaagg accatattga tagaacaaag aagaagcgag gagaaaaaga tgagacagag     960

tatgagtaca gtggaagtga ggaagaagag gaggagaatg actcaggaga gcccagctcc    1020

atcctgaatc tgccagggga gtcgacgctg cggagggact ttctgaggct gcagctggcc 1080
aacaaggagc gttctgaggc cctacggagg cagcagctgg agcagcagca gcgggagaat 1140
gaggagcaca agcggcagct gctggccgag cgtcagaagc gcatcgagga gcagaaagag 1200
cagaggcggc ggctggagga gcaacaaagg cgagagaagg agctgcggaa gcagcaggag 1260
agggagcagc gccggcacta tgaggagcag atgcgccggg aggaggagag gaggcgtgcg 1320
gagcatgaac aggaatacat caggcgacag ttagaggagg agcagagaca gttagagatc 1380
ttgcagcagc agctactgca tgaacaagct ctacttctgg aatataagcg caaacaattg 1440
gaagaacaga gacaagcaga aagactgcag aggcagctaa agcaagaaag agactactta 1500
gtttcccttc agcatcagcg gcaggagcag aggcctgtgg agaagaagcc actgtaccat 1560
tacaaagaag gaatgagtcc tagtgagaag ccagcatggg ccaaggaggt agaagaacgg 1620
tcaaggctca accggcaaag ttcccctgcc atgcctcaca aggttgccaa caggatatct 1680
gaccccaacc tgcccccaag gtcggagtcc ttcagcatta gtggagttca gcctgctcga 1740
acacccccca tgctcagacc agtcgatccc cagatcccac atctggtagc tgtaaaatcc 1800
cagggacctg ccttgaccgc ctcccagtca gtgcacgagc agcccacaaa gggcctctct 1860
gggtttcagg aggctctgaa cgtgacctcc caccgcgtgg agatgccacg ccagaactca 1920
gatcccacct cggaaaatcc tcctctcccc actcgcattg aaaagtttga ccgaagctct 1980
tggttacgac aggaagaaga cattccacca aaggtgcctc aaagaacaac ttctatatcc 2040
ccagcattag ccagaaagaa ttctcctggg aatggtagtg ctctgggacc cagactagga 2100
tctcaaccca tcagagcaag caaccctgat ctccggagaa ctgagcccat cttggagagc 2160
cccttgcaga ggaccagcag tggcagttcc tccagctcca gcacccctag ctcccagccc 2220
agctcccaag gaggctccca gcctggatca caagcaggat ccagtgaacg caccagagtt 2280
cgagccaaca gtaagtcaga aggatcacct gtgctccccc atgagcctgc caaggtgaaa 2340
ccagaagaat ccagggacat tacccggccc agtcgaccag ctgatctgac ggcattagcc 2400
aaagaactaa gagaactccg gattgaagaa acaaaccgcc caatgaagaa ggtgactgat 2460
tactcctcct ccagtgagga gtcagaaagt agcgaggaag aggaggaaga tggagagagc 2520
gagacccatg atgggacagt ggctgtcagc gacataccca gactgatacc aacaggagct 2580
ccaggcagca acgagcagta caatgtggga atggtgggga cgcatgggct ggagacctct 2640
catgcggaca gtttcagcgg cagtatttca agagaaggaa ccttgatgat tagagagacg 2700
tctggagaga agaagcgatc tggccacagt gacagcaatg gctttgctgg ccacatcaac 2760
ctccctgacc tggtgcagca gagccattct ccagctggaa ccccgactga gggactgggg 2820
cgcgtctcaa cccattccca ggagatggac tctgggactg aatatggcat ggggagcagc 2880
accaaagcct ccttcacccc ctttgtggac cccagagtat accagacgtc tcccactgat 2940
gaagatgaag aggatgagga atcatcagcc gcagctctgt ttactagcga acttcttagg 3000
caagaacagg ccaaactcaa tgaagcaaga aagatttcgg tggtaaatgt aaacccaacc 3060
aacattcggc ctcatagcga cacaccagaa atcagaaaat acaagaaacg attcaactca 3120

gaaatacttt gtgcagctct gtggggtgta aaccttctgg tggggactga aaatggcctg 3180
atgcttttgg accgaagtgg gcaaggcaaa gtctataatc tgatcaaccg gaggcgattt 3240
cagcagatgg atgtgctaga gggactgaat gtccttgtga caatttcagg aaagaagaat 3300
aagctacgag tttactatct ttcatggtta agaaacagaa tactacataa tgacccagaa 3360
gtagaaaaga aacaaggctg gatcactgtt ggggacttgg aaggctgtat acattataaa 3420
gttgttaaat atgaaaggat caaatttttg gtgattgcct taaagaatgc tgtggaaata 3480
tatgcttggg ctcctaaacc gtatcataaa ttcatggcat ttaagtcttt tgcagatctc 3540
cagcacaagc ctctgctagt tgatctcacg gtagaagaag gtcaaagatt aaaggttatt 3600
tttggttcac acactggttt ccatgtaatt gatgttgatt caggaaactc ttatgatatc 3660
tacataccat ctcatattca gggcaatatc actcctcatg ctattgtcat cttgcctaaa 3720
acagatggaa tggaaatgct tgtttgctat gaggatgagg gggtgtatgt aaacacctat 3780
ggccggataa ctaaggatgt ggtgctccaa tggggagaaa tgcccacgtc tgtggcctac 3840
attcattcca atcagataat gggctggggc gagaaagcta ttgagatccg gtcagtggaa 3900
acaggacatt tggatggagt atttatgcat aagcgagctc aaaggttaaa gtttctatgt 3960
gaaagaaatg ataaggtatt ttttgcatcc gtgcgatctg gaggaagtag ccaagtgttt 4020
ttcatgaccc tcaacagaaa ttccatgatg aactggtaa 4059

<210> 4
<211> 3996
<212> DNA
<213> homo sapiens

<400> 4
atggcgagcg actccccggc tcgaagcctg gatgaaatag atctctcggc tctgagggac 60
cccgcaggga tctttgaatt ggtggaactt gttggaaatg gaacatacgg gcaagtttat 120
aagggtcgtc atgtcaaaac gggccagctt gcagccatca aggttatgga tgtcacaggg 180
gatgaagagg aagaaatcaa acaagaaatt aacatgttga agaaatattc tcatcaccgg 240
aatattgcta catactatgg tgcttttatc aaaaagaacc caccaggcat ggatgaccaa 300
ctttggttgg tgatggagtt ttgtggtgct ggctctgtca ccgacctgat caagaacaca 360
aaaggtaaca cgttgaaaga ggagtggatt gcatacatct gcagggaaat cttacggggg 420
ctgagtcacc tgcaccagca taaagtgatt catcgagata ttaaagggca aaatgtcttg 480
ctgactgaaa atgcagaagt taaactagtg gactttggag tcagtgctca gcttgatcga 540
acagtgggca ggaggaatac tttcattgga actccctact ggatggcacc agaagttatt 600
gcctgtgatg aaaacccaga tgccacatat gatttcaaga gtgacttgtg gtctttgggt 660
atcaccgcca ttgaaatggc agaaggtgct ccccctctct gtgacatgca ccccatgaga 720
gctctcttcc tcatcccccg gaacccagcg cctcggctga agtctaagaa gtggtcaaaa 780
aaattccagt catttattga gagctgcttg gtaaagaatc acagccagcg accagcaaca 840
gaacaattga tgaagcatcc atttatacga gaccaaccta atgagcgaca ggtccgcatt 900

caactcaagg accatattga tagaacaaag aagaagcgag gagaaaaaga tgagacagag 960
tatgagtaca gtggaagtga ggaagaagag gaggagaatg actcaggaga gcccagctcc 1020
atcctgaatc tgccagggga gtcgacgctg cggagggact ttctgaggct gcagctggcc 1080
aacaaggagc gttctgaggc cctacggagg cagcagctgg agcagcagca gcgggagaat 1140
gaggagcaca agcggcagct gctggccgag cgtcagaagc gcatcgagga gcagaaagag 1200
cagaggcggc ggctggagga gcaacaaagg cgagagaagg agctgcggaa gcagcaggag 1260
agggagcagc gccggcacta tgaggagcag atgcgccggg aggaggagag gaggcgtgcg 1320
gagcatgaac aggaatataa gcgcaaacaa ttggaagaac agagacaagc agaaagactg 1380
cagaggcagc taaagcaaga aagagactac ttagtttccc ttcagcatca gcggcaggag 1440
cagaggcctg tggagaagaa gccactgtac cattacaaag aaggaatgag tcctagtgag 1500
aagccagcat gggccaagga ggtagaagaa cggtcaaggc tcaaccggca aagttcccct 1560
gccatgcctc acaaggttgc caacaggata tctgacccca acctgccccc aaggtcggag 1620
tccttcagca ttagtggagt tcagcctgct cgaacacccc ccatgctcag accagtcgat 1680
ccccagatcc cacatctggt agctgtaaaa tcccagggac ctgccttgac cgcctcccag 1740
tcagtgcacg agcagcccac aaagggcctc tctgggtttc aggaggctct gaacgtgacc 1800
tcccaccgcg tggagatgcc acgccagaac tcagatccca cctcggaaaa tcctcctctc 1860
cccactcgca ttgaaaagtt tgaccgaagc tcttggttac gacaggaaga agacattcca 1920
ccaaaggtgc ctcaaagaac aacttctata tccccagcat tagccagaaa gaattctcct 1980
gggaatggta gtgctctggg acccagacta ggatctcaac ccatcagagc aagcaaccct 2040
gatctccgga gaactgagcc catcttggag agccccttgc agaggaccag cagtggcagt 2100
tcctccagct ccagcacccc tagctcccag cccagctccc aaggaggctc ccagcctgga 2160
tcacaagcag gatccagtga acgcaccaga gttcgagcca acagtaagtc agaaggatca 2220
cctgtgctcc cccatgagcc tgccaaggtg aaaccagaag aatccaggga cattacccgg 2280
cccagtcgac cagctagcta caaaaaagct atagatgagg atctgacggc attagccaaa 2340
gaactaagag aactccggat tgaagaaaca aaccgcccaa tgaagaaggt gactgattac 2400
tcctcctcca gtgaggagtc agaaagtagc gaggaagagg aggaagatgg agagagcgag 2460
acccatgatg ggacagtggc tgtcagcgac atacccagac tgataccaac aggagctcca 2520
ggcagcaacg agcagtacaa tgtgggaatg gtggggacgc atgggctgga gacctctcat 2580
gcggacagtt tcagcggcag tatttcaaga gaaggaacct tgatgattag agagacgtct 2640
ggagagaaga agcgatctgg ccacagtgac agcaatggct ttgctggcca catcaacctc 2700
cctgacctgg tgcagcagag ccattctcca gctggaaccc cgactgaggg actggggcgc 2760
gtctcaaccc attcccagga gatggactct gggactgaat atggcatggg gagcagcacc 2820
aaagcctcct tcaccccctt tgtggacccc agagtatacc agacgtctcc cactgatgaa 2880
gatgaagagg atgaggaatc atcagccgca gctctgttta ctagcgaact tcttaggcaa 2940

gaacaggcca aactcaatga agcaagaaag atttcggtgg taaatgtaaa cccaaccaac 3000
attcggcctc atagcgacac accagaaatc agaaaataca agaaacgatt caactcagaa 3060
atactttgtg cagctctgtg gggtgtaaac cttctggtgg ggactgaaaa tggcctgatg 3120
cttttggacc gaagtgggca aggcaaagtc tataatctga tcaaccggag gcgatttcag 3180
cagatggatg tgctagaggg actgaatgtc cttgtgacaa tttcaggaaa gaagaataag 3240
ctacgagttt actatctttc atggttaaga aacagaatac tacataatga cccagaagta 3300
gaaaagaaac aaggctggat cactgttggg gacttggaag gctgtataca ttataaagtt 3360
gttaaatatg aaaggatcaa atttttggtg attgccttaa agaatgctgt ggaaatatat 3420
gcttgggctc ctaaaccgta tcataaattc atggcattta agtcttttgc agatctccag 3480
cacaagcctc tgctagttga tctcacggta gaagaaggtc aaagattaaa ggttattttt 3540
ggttcacaca ctggtttcca tgtaattgat gttgattcag gaaactctta tgatatctac 3600
ataccatctc atattcaggg caatatcact cctcatgcta ttgtcatctt gcctaaaaca 3660
gatggaatgg aaatgcttgt ttgctatgag gatgaggggg tgtatgtaaa cacctatggc 3720
cggataacta aggatgtggt gctccaatgg ggagaaatgc ccacgtctgt ggcctacatt 3780
cattccaatc agataatggg ctggggcgag aaagctattg agatccggtc agtggaaaca 3840
ggacatttgg atggagtatt tatgcataag cgagctcaaa ggttaaagtt tctatgtgaa 3900
agaaatgata aggtattttt tgcatccgtg cgatctggag gaagtagcca agtgtttttc 3960
atgaccctca acagaaattc catgatgaac tggtaa 3996

<210> 5
<211> 3972
<212> DNA
<213> homo sapiens

<400> 5
atggcgagcg actccccggc tcgaagcctg gatgaaatag atctctcggc tctgagggac 60
cccgcaggga tctttgaatt ggtggaactt gttggaaatg gaacatacgg gcaagtttat 120
aagggtcgtc atgtcaaaac gggccagctt gcagccatca aggttatgga tgtcacaggg 180
gatgaagagg aagaaatcaa acaagaaatt aacatgttga agaaatattc tcatcaccgg 240
aatattgcta catactatgg tgcttttatc aaaaagaacc caccaggcat ggatgaccaa 300
ctttggttgg tgatggagtt ttgtggtgct ggctctgtca ccgacctgat caagaacaca 360
aaaggtaaca cgttgaaaga ggagtggatt gcatacatct gcagggaaat cttacggggg 420
ctgagtcacc tgcaccagca taaagtgatt catcgagata ttaaagggca aaatgtcttg 480
ctgactgaaa atgcagaagt taaactagtg gactttggag tcagtgctca gcttgatcga 540
acagtgggca ggaggaatac tttcattgga actccctact ggatggcacc agaagttatt 600
gcctgtgatg aaaacccaga tgccacatat gatttcaaga gtgacttgtg gtctttgggt 660
atcaccgcca ttgaaatggc agaaggtgct ccccctctct gtgacatgca ccccatgaga 720
gctctcttcc tcatcccccg gaacccagcg cctcggctga agtctaagaa gtggtcaaaa 780

aaattccagt catttattga gagctgcttg gtaaagaatc acagccagcg accagcaaca 840
gaacaattga tgaagcatcc atttatacga gaccaaccta atgagcgaca ggtccgcatt 900
caactcaagg accatattga tagaacaaag aagaagcgag gagaaaaaga tgagacagag 960
tatgagtaca gtggaagtga ggaagaagag gaggagaatg actcaggaga gcccagctcc 1020
atcctgaatc tgccagggga gtcgacgctg cggagggact ttctgaggct gcagctggcc 1080
aacaaggagc gttctgaggc cctacggagg cagcagctgg agcagcagca gcgggagaat 1140
gaggagcaca agcggcagct gctggccgag cgtcagaagc gcatcgagga gcagaaagag 1200
cagaggcggc ggctggagga gcaacaaagg cgagagaagg agctgcggaa gcagcaggag 1260
agggagcagc gccggcacta tgaggagcag atgcgccggg aggaggagag gaggcgtgcg 1320
gagcatgaac aggaatataa gcgcaaacaa ttggaagaac agagacaagc agaaagactg 1380
cagaggcagc taaagcaaga aagagactac ttagtttccc ttcagcatca gcggcaggag 1440
cagaggcctg tggagaagaa gccactgtac cattacaaag aaggaatgag tcctagtgag 1500
aagccagcat gggccaagga ggtagaagaa cggtcaaggc tcaaccggca aagttcccct 1560
gccatgcctc acaaggttgc caacaggata tctgacccca acctgccccc aaggtcggag 1620
tccttcagca ttagtggagt tcagcctgct cgaacacccc ccatgctcag accagtcgat 1680
ccccagatcc cacatctggt agctgtaaaa tcccagggac ctgccttgac cgcctcccag 1740
tcagtgcacg agcagcccac aaagggcctc tctgggtttc aggaggctct gaacgtgacc 1800
tcccaccgcg tggagatgcc acgccagaac tcagatccca cctcggaaaa tcctcctctc 1860
cccactcgca ttgaaaagtt tgaccgaagc tcttggttac gacaggaaga agacattcca 1920
ccaaaggtgc ctcaaagaac aacttctata tccccagcat tagccagaaa gaattctcct 1980
gggaatggta gtgctctggg acccagacta ggatctcaac ccatcagagc aagcaaccct 2040
gatctccgga gaactgagcc catcttggag agccccttgc agaggaccag cagtggcagt 2100
tcctccagct ccagcacccc tagctcccag cccagctccc aaggaggctc ccagcctgga 2160
tcacaagcag gatccagtga acgcaccaga gttcgagcca acagtaagtc agaaggatca 2220
cctgtgctcc cccatgagcc tgccaaggtg aaaccagaag aatccaggga cattacccgg 2280
cccagtcgac cagctgatct gacggcatta gccaaagaac taagagaact ccggattgaa 2340
gaaacaaacc gcccaatgaa gaaggtgact gattactcct cctccagtga ggagtcagaa 2400
agtagcgagg aagaggagga agatggagag agcgagaccc atgatgggac agtggctgtc 2460
agcgacatac ccagactgat accaacagga gctccaggca gcaacgagca gtacaatgtg 2520
ggaatggtgg ggacgcatgg gctggagacc tctcatgcgg acagtttcag cggcagtatt 2580
tcaagagaag gaaccttgat gattagagag acgtctggag agaagaagcg atctggccac 2640
agtgacagca atggctttgc tggccacatc aacctccctg acctggtgca gcagagccat 2700
tctccagctg gaaccccgac tgagggactg gggcgcgtct caacccattc ccaggagatg 2760
gactctggga ctgaatatgg catggggagc agcaccaaag cctccttcac cccctttgtg 2820
gaccccagag tataccagac gtctcccact gatgaagatg aagaggatga ggaatcatca 2880

gccgcagctc tgtttactag cgaacttctt aggcaagaac aggccaaact caatgaagca 2940

agaaagattt cggtggtaaa tgtaaaccca accaacattc ggcctcatag cgacacacca 3000

gaaatcagaa aatacaagaa acgattcaac tcagaaatac tttgtgcagc tctgtggggt 3060

gtaaaccttc tggtggggac tgaaaatggc ctgatgcttt tggaccgaag tgggcaaggc 3120

aaagtctata atctgatcaa ccggaggcga tttcagcaga tggatgtgct agagggactg 3180

aatgtccttg tgacaatttc aggaaagaag aataagctac gagtttacta tctttcatgg 3240

ttaagaaaca gaatactaca taatgaccca gaagtagaaa agaaacaagg ctggatcact 3300

gttggggact tggaaggctg tatacattat aaagttgtta aatatgaaag gatcaaattt 3360

ttggtgattg ccttaaagaa tgctgtggaa atatatgctt gggctcctaa accgtatcat 3420

aaattcatgg catttaagtc ttttgcagat ctccagcaca agcctctgct agttgatctc 3480

acggtagaag aaggtcaaag attaaaggtt atttttggtt cacacactgg tttccatgta 3540

attgatgttg attcaggaaa ctcttatgat atctacatac catctcatat tcagggcaat 3600

atcactcctc atgctattgt catcttgcct aaaacagatg gaatggaaat gcttgtttgc 3660

tatgaggatg agggggtgta tgtaaacacc tatggccgga taactaagga tgtggtgctc 3720

caatggggag aaatgcccac gtctgtggcc tacattcatt ccaatcagat aatgggctgg 3780

ggcgagaaag ctattgagat ccggtcagtg gaaacaggac atttggatgg agtatttatg 3840

cataagcgag ctcaaaggtt aaagtttcta tgtgaaagaa atgataaggt attttttgca 3900

tccgtgcgat ctggaggaag tagccaagtg tttttcatga ccctcaacag aaattccatg 3960

atgaactggt aa 3972

<210> 6
<211> 3918
<212> DNA
<213> homo sapiens

<400> 6
atggcgagcg actccccggc tcgaagcctg gatgaaatag atctctcggc tctgagggac 60

cccgcaggga tctttgaatt ggtggaactt gttggaaatg gaacatacgg gcaagtttat 120

aagggtcgtc atgtcaaaac gggccagctt gcagccatca aggttatgga tgtcacaggg 180

gatgaagagg aagaaatcaa acaagaaatt aacatgttga agaaatattc tcatcaccgg 240

aatattgcta catactatgg tgcttttatc aaaaagaacc caccaggcat ggatgaccaa 300

ctttggttgg tgatggagtt ttgtggtgct ggctctgtca ccgacctgat caagaacaca 360

aaaggtaaca cgttgaaaga ggagtggatt gcatacatct gcagggaaat cttacggggg 420

ctgagtcacc tgcaccagca taaagtgatt catcgagata ttaaagggca aaatgtcttg 480

ctgactgaaa atgcagaagt taaactagtg gactttggag tcagtgctca gcttgatcga 540

acagtgggca ggaggaatac tttcattgga actccctact ggatggcacc agaagttatt 600

gcctgtgatg aaaacccaga tgccacatat gatttcaaga gtgacttgtg gtctttgggt 660

atcaccgcca ttgaaatggc agaaggtgct ccccctctct gtgacatgca ccccatgaga 720

gctctcttcc tcatcccccg gaacccagcg cctcggctga agtctaagaa gtggtcaaaa 780
aaattccagt catttattga gagctgcttg gtaaagaatc acagccagcg accagcaaca 840
gaacaattga tgaagcatcc atttatacga gaccaaccta atgagcgaca ggtccgcatt 900
caactcaagg accatattga tagaacaaag aagaagcgag gagaaaaaga tgagacagag 960
tatgagtaca gtggaagtga ggaagaagag gaggagaatg actcaggaga gcccagctcc 1020
atcctgaatc tgccagggga gtcgacgctg cggagggact ttctgaggct gcagctggcc 1080
aacaaggagc gttctgaggc cctacggagg cagcagctgg agcagcagca gcgggagaat 1140
gaggagcaca agcggcagct gctggccgag cgtcagaagc gcatcgagga gcagaaagag 1200
cagaggcggc ggctggagga gcaacaaagg cgagagaagg agctgcggaa gcagcaggag 1260
agggagcagc gccggcacta tgaggagcag atgcgccggg aggaggagag gaggcgtgcg 1320
gagcatgaac aggaatacat caggcgacag ttagaggagg agcagagaca gttagagatc 1380
ttgcagcagc agctactgca tgaacaagct ctacttctgg aatataagcg caaacaattg 1440
gaagaacaga gacaagcaga aagactgcag aggcagctaa agcaagaaag agactactta 1500
gtttcccttc agcatcagcg gcaggagcag aggcctgtgg agaagaagcc actgtaccat 1560
tacaaagaag gaatgagtcc tagtgagaag ccagcatggg ccaaggagat cccacatctg 1620
gtagctgtaa aatcccaggg acctgccttg accgcctccc agtcagtgca cgagcagccc 1680
acaaagggcc tctctgggtt tcaggaggct ctgaacgtga cctcccaccg cgtggagatg 1740
ccacgccaga actcagatcc cacctcggaa aatcctcctc tccccactcg cattgaaaag 1800
tttgaccgaa gctcttggtt acgacaggaa gaagacattc caccaaaggt gcctcaaaga 1860
acaacttcta tatccccagc attagccaga aagaattctc ctgggaatgg tagtgctctg 1920
ggacccagac taggatctca acccatcaga gcaagcaacc ctgatctccg gagaactgag 1980
cccatcttgg agagcccctt gcagaggacc agcagtggca gttcctccag ctccagcacc 2040
cctagctccc agcccagctc ccaaggaggc tcccagcctg gatcacaagc aggatccagt 2100
gaacgcacca gagttcgagc caacagtaag tcagaaggat cacctgtgct cccccatgag 2160
cctgccaagg tgaaaccaga agaatccagg gacattaccc ggcccagtcg accagctagc 2220
tacaaaaaag ctatagatga ggatctgacg gcattagcca aagaactaag agaactccgg 2280
attgaagaaa caaaccgccc aatgaagaag gtgactgatt actcctcctc cagtgaggag 2340
tcagaaagta gcgaggaaga ggaggaagat ggagagagcg agacccatga tgggacagtg 2400
gctgtcagcg acatacccag actgatacca acaggagctc caggcagcaa cgagcagtac 2460
aatgtgggaa tggtggggac gcatgggctg gagacctctc atgcggacag tttcagcggc 2520
agtatttcaa gagaaggaac cttgatgatt agagagacgt ctggagagaa gaagcgatct 2580
ggccacagtg acagcaatgg ctttgctggc cacatcaacc tccctgacct ggtgcagcag 2640
agccattctc cagctggaac ccgactgagg gactggggc gcgtctcaac ccattcccag 2700
gagatggact ctgggactga atatggcatg gggagcagca ccaaagcctc cttcaccccc 2760

tttgtggacc ccagagtata ccagacgtct cccactgatg aagatgaaga ggatgaggaa 2820

tcatcagccg cagctctgtt tactagcgaa cttcttaggc aagaacaggc caaactcaat 2880

gaagcaagaa agatttcggt ggtaaatgta aacccaacca acattcggcc tcatagcgac 2940

acaccagaaa tcagaaaata caagaaacga ttcaactcag aaatactttg tgcagctctg 3000

tggggtgtaa accttctggt ggggactgaa aatggcctga tgcttttgga ccgaagtggg 3060

caaggcaaag tctataatct gatcaaccgg aggcgatttc agcagatgga tgtgctagag 3120

ggactgaatg tccttgtgac aatttcagga aagaagaata agctacgagt ttactatctt 3180

tcatggttaa gaaacagaat actacataat gacccagaag tagaaaagaa acaaggctgg 3240

atcactgttg gggacttgga aggctgtata cattataaag ttgttaaata tgaaaggatc 3300

aaatttttgg tgattgcctt aaagaatgct gtggaaatat atgcttgggc tcctaaaccg 3360

tatcataaat tcatggcatt taagtctttt gcagatctcc agcacaagcc tctgctagtt 3420

gatctcacgg tagaagaagg tcaaagatta aaggttattt ttggttcaca cactggtttc 3480

catgtaattg atgttgattc aggaaactct tatgatatct acataccatc tcatattcag 3540

ggcaatatca ctcctcatgc tattgtcatc ttgcctaaaa cagatggaat ggaaatgctt 3600

gtttgctatg aggatgaggg ggtgtatgta aacacctatg gccggataac taaggatgtg 3660

gtgctccaat ggggagaaat gcccacgtct gtggcctaca ttcattccaa tcagataatg 3720

ggctggggcg agaaagctat tgagatccgg tcagtggaaa caggacattt ggatggagta 3780

tttatgcata agcgagctca aaggttaaag tttctatgtg aaagaaatga taaggtattt 3840

tttgcatccg tgcgatctgg aggaagtagc caagtgtttt tcatgaccct caacagaaat 3900

tccatgatga actggtaa 3918

<210> 7
<211> 3894
<212> DNA
<213> homo sapiens

<400> 7
   atggcgagcg actccccggc tcgaagcctg gatgaaatag atctctcggc tctgagggac 60
   cccgcaggga tctttgaatt ggtggaactt gttggaaatg gaacatacgg gcaagtttat 120
   aagggtcgtc atgtcaaaac gggccagctt gcagccatca aggttatgga tgtcacaggg 180
   gatgaagagg aagaaatcaa acaagaaatt aacatgttga agaaatattc tcatcaccgg 240
   aatattgcta catactatgg tgcttttatc aaaaagaacc caccaggcat ggatgaccaa 300
   ctttggttgg tgatggagtt ttgtggtgct ggctctgtca ccgacctgat caagaacaca 360
   aaaggtaaca cgttgaaaga ggagtggatt gcatacatct gcagggaaat cttacggggg 420
   ctgagtcacc tgcaccagca taaagtgatt catcgagata ttaaagggca aaatgtcttg 480
   ctgactgaaa atgcagaagt taaactagtg gactttggag tcagtgctca gcttgatcga 540
   acagtgggca ggaggaatac tttcattgga actccctact ggatggcacc agaagttatt 600
   gcctgtgatg aaaacccaga tgccacatat gatttcaaga gtgacttgtg gtctttgggt 660
   atcaccgcca ttgaaatggc agaaggtgct ccccctctct gtgacatgca ccccatgaga 720
   gctctcttcc tcatcccccg gaacccagcg cctcggctga agtctaagaa gtggtcaaaa 780
   aaattccagt catttattga gagctgcttg gtaaagaatc acagccagcg accagcaaca 840
   gaacaattga tgaagcatcc atttatacga gaccaaccta atgagcgaca ggtccgcatt 900
   caactcaagg accatattga tagaacaaag aagaagcgag gagaaaaaga tgagacagag 960
   tatgagtaca gtggaagtga ggaagaagag gaggagaatg actcaggaga gcccagctcc 1020
   atcctgaatc tgccagggga gtcgacgctg cggagggact ttctgaggct gcagctggcc 1080
   aacaaggagc gttctgaggc cctacggagg cagcagctgg agcagcagca gcgggagaat 1140
   gaggagcaca agcggcagct gctggccgag cgtcagaagc gcatcgagga gcagaaagag 1200
   cagaggcggc ggctggagga gcaacaaagg cgagagaagg agctgcggaa gcagcaggag 1260
   agggagcagc gccggcacta tgaggagcag atgcgccggg aggaggagag gaggcgtgcg 1320
   gagcatgaac aggaatacat caggcgacag ttagaggagg agcagagaca gttagagatc 1380
   ttgcagcagc agctactgca tgaacaagct ctacttctgg aatataagcg caaacaattg 1440
   gaagaacaga gacaagcaga aagactgcag aggcagctaa agcaagaaag agactactta 1500
   gtttcccttc agcatcagcg gcaggagcag aggcctgtgg agaagaagcc actgtaccat 1560
   tacaaagaag gaatgagtcc tagtgagaag ccagcatggg ccaaggagat cccacatctg 1620
   gtagctgtaa aatcccaggg acctgccttg accgcctccc agtcagtgca cgagcagccc 1680
   acaaagggcc tctctgggtt tcaggaggct ctgaacgtga cctcccaccg cgtggagatg 1740
   ccacgccaga actcagatcc cacctcggaa aatcctcctc tccccactcg cattgaaaag 1800
   tttgaccgaa gctcttggtt acgacaggaa gaagacattc caccaaaggt gcctcaaaga 1860
   acaacttcta tatccccagc attagccaga aagaattctc ctgggaatgg tagtgctctg 1920
   ggacccagac taggatctca acccatcaga gcaagcaacc ctgatctccg gagaactgag 1980
   cccatcttgg agagcccctt gcagaggacc agcagtggca gttcctccag ctccagcacc 2040
   cctagctccc agcccagctc ccaaggaggc tcccagcctg gatcacaagc aggatccagt 2100
   gaacgcacca gagttcgagc caacagtaag tcagaaggat cacctgtgct cccccatgag 2160
   cctgccaagg tgaaaccaga agaatccagg gacattaccc ggcccagtcg accagctgat 2220
   ctgacggcat tagccaaaga actaagagaa ctccggattg aagaaacaaa ccgcccaatg 2280
   aagaaggtga ctgattactc ctcctccagt gaggagtcag aaagtagcga ggaagaggag 2340
   gaagatggag agagcgagac ccatgatggg acagtggctg tcagcgacat acccagactg 2400
   ataccaacag gagctccagg cagcaacgag cagtacaatg tgggaatggt ggggacgcat 2460
   gggctggaga cctctcatgc ggacagtttc agcggcagta tttcaagaga aggaaccttg 2520
   atgattagag agacgtctgg agagaagaag cgatctggcc acagtgacag caatggcttt 2580
   gctggccaca tcaacctccc tgacctggtg cagcagagcc attctccagc tggaaccccg 2640
   actgagggac tggggcgcgt ctcaacccat tcccaggaga tggactctgg gactgaatat 2700
   ggcatgggga gcagcaccaa agcctccttc accccctttg tggaccccag agtataccag 2760
   acgtctccca ctgatgaaga tgaagaggat gaggaatcat cagccgcagc tctgtttact 2820
   agcgaacttc ttaggcaaga acaggccaaa ctcaatgaag caagaaagat ttcggtggta 2880
   aatgtaaacc caaccaacat tcggcctcat agcgacacac cagaaatcag aaaatacaag   2940
   aaacgattca actcagaaat actttgtgca gctctgtggg gtgtaaacct tctggtgggg   3000
   actgaaaatg gcctgatgct tttggaccga agtgggcaag gcaaagtcta taatctgatc 3060
   aaccggaggc gatttcagca gatggatgtg ctagagggac tgaatgtcct tgtgacaatt 3120
   tcaggaaaga agaataagct acgagtttac tatctttcat ggttaagaaa cagaatacta 3180
   cataatgacc cagaagtaga aaagaaacaa ggctggatca ctgttgggga cttggaaggc 3240
   tgtatacatt ataaagttgt taaatatgaa aggatcaaat ttttggtgat tgccttaaag 3300
   aatgctgtgg aaatatatgc ttgggctcct aaaccgtatc ataaattcat ggcatttaag 3360
   tcttttgcag atctccagca caagcctctg ctagttgatc tcacggtaga agaaggtcaa 3420
   agattaaagg ttatttttgg ttcacacact ggtttccatg taattgatgt tgattcagga 3480
   aactcttatg atatctacat accatctcat attcagggca atatcactcc tcatgctatt 3540
   gtcatcttgc ctaaaacaga tggaatggaa atgcttgttt gctatgagga tgagggggtg 3600
   tatgtaaaca cctatggccg gataactaag gatgtggtgc tccaatgggg agaaatgccc 3660
   acgtctgtgg cctacattca ttccaatcag ataatgggct ggggcgagaa agctattgag 3720
   atccggtcag tggaaacagg acatttggat ggagtattta tgcataagcg agctcaaagg 3780
   ttaaagtttc tatgtgaaag aaatgataag gtattttttg catccgtgcg atctggagga 3840
   agtagccaag tgtttttcat gaccctcaac agaaattcca tgatgaactg gtaa 3894

<210> 8
<211> 3831
<212> DNA
<213> homo sapiens

<400> 8
atggcgagcg actccccggc tcgaagcctg gatgaaatag atctctcggc tctgagggac 60

cccgcaggga tctttgaatt ggtggaactt gttggaaatg gaacatacgg gcaagtttat 120

aagggtcgtc atgtcaaaac gggccagctt gcagccatca aggttatgga tgtcacaggg 180

gatgaagagg aagaaatcaa acaagaaatt aacatgttga agaaatattc tcatcaccgg 240

aatattgcta catactatgg tgcttttatc aaaaagaacc caccaggcat ggatgaccaa 300

ctttggttgg tgatggagtt ttgtggtgct ggctctgtca ccgacctgat caagaacaca 360

aaaggtaaca cgttgaaaga ggagtggatt gcatacatct gcagggaaat cttacggggg 420

ctgagtcacc tgcaccagca taaagtgatt catcgagata ttaaagggca aaatgtcttg 480

ctgactgaaa atgcagaagt taaactagtg gactttggag tcagtgctca gcttgatcga 540

acagtgggca ggaggaatac tttcattgga actccctact ggatggcacc agaagttatt 600

gcctgtgatg aaaacccaga tgccacatat gatttcaaga gtgacttgtg gtctttgggt 660

atcaccgcca ttgaaatggc agaaggtgct ccccctctct gtgacatgca ccccatgaga 720

gctctcttcc tcatcccccg gaacccagcg cctcggctga agtctaagaa gtggtcaaaa 780
aaattccagt catttattga gagctgcttg gtaaagaatc acagccagcg accagcaaca 840
gaacaattga tgaagcatcc atttatacga gaccaaccta atgagcgaca ggtccgcatt 900
caactcaagg accatattga tagaacaaag aagaagcgag gagaaaaaga tgagacagag 960
tatgagtaca gtggaagtga ggaagaagag gaggagaatg actcaggaga gcccagctcc 1020
atcctgaatc tgccagggga gtcgacgctg cggagggact ttctgaggct gcagctggcc 1080
aacaaggagc gttctgaggc cctacggagg cagcagctgg agcagcagca gcgggagaat 1140
gaggagcaca agcggcagct gctggccgag cgtcagaagc gcatcgagga gcagaaagag 1200
cagaggcggc ggctggagga gcaacaaagg cgagagaagg agctgcggaa gcagcaggag 1260
agggagcagc gccggcacta tgaggagcag atgcgccggg aggaggagag gaggcgtgcg 1320
gagcatgaac aggaatataa gcgcaaacaa ttggaagaac agagacaagc agaaagactg 1380
cagaggcagc taaagcaaga aagagactac ttagtttccc ttcagcatca gcggcaggag 1440
cagaggcctg tggagaagaa gccactgtac cattacaaag aaggaatgag tcctagtgag 1500
aagccagcat gggccaagga gatcccacat ctggtagctg taaaatccca gggacctgcc 1560
ttgaccgcct cccagtcagt gcacgagcag cccacaaagg gcctctctgg gtttcaggag 1620
gctctgaacg tgacctccca ccgcgtggag atgccacgcc agaactcaga tcccacctcg 1680
gaaaatcctc ctctccccac tcgcattgaa aagtttgacc gaagctcttg gttacgacag 1740
gaagaagaca ttccaccaaa ggtgcctcaa agaacaactt ctatatcccc agcattagcc 1800
agaaagaatt ctcctgggaa tggtagtgct ctgggaccca gactaggatc tcaacccatc 1860
agagcaagca accctgatct ccggagaact gagcccatct tggagagccc cttgcagagg 1920
accagcagtg gcagttcctc cagctccagc acccctagct cccagcccag ctcccaagga 1980
ggctcccagc ctggatcaca agcaggatcc agtgaacgca ccagagttcg agccaacagt 2040
aagtcagaag gatcacctgt gctcccccat gagcctgcca aggtgaaacc agaagaatcc 2100
agggacatta cccggcccag tcgaccagct agctacaaaa aagctataga tgaggatctg 2160
acggcattag ccaaagaact aagagaactc cggattgaag aaacaaaccg cccaatgaag 2220
aaggtgactg attactcctc ctccagtgag gagtcagaaa gtagcgagga agaggaggaa 2280
gatggagaga gcgagaccca tgatgggaca gtggctgtca gcgacatacc cagactgata 2340
ccaacaggag ctccaggcag caacgagcag tacaatgtgg gaatggtggg gacgcatggg 2400
ctggagacct ctcatgcgga cagtttcagc ggcagtattt caagagaagg aaccttgatg 2460
attagagaga cgtctggaga gaagaagcga tctggccaca gtgacagcaa tggctttgct 2520
ggccacatca acctccctga cctggtgcag cagagccatt ctccagctgg aaccccgact 2580
gagggactgg ggcgcgtctc aacccattcc caggagatgg actctgggac tgaatatggc 2640
atggggagca gcaccaaagc ctccttcacc ccctttgtgg accccagagt ataccagacg 2700
tctcccactg atgaagatga agaggatgag gaatcatcag ccgcagctct gtttactagc 2760

gaacttctta ggcaagaaca ggccaaactc aatgaagcaa gaaagatttc ggtggtaaat 2820

gtaaacccaa ccaacattcg gcctcatagc gacacaccag aaatcagaaa atacaagaaa 2880

cgattcaact cagaaatact ttgtgcagct ctgtggggtg taaaccttct ggtggggact 2940

gaaaatggcc tgatgctttt ggaccgaagt gggcaaggca aagtctataa tctgatcaac 3000

cggaggcgat ttcagcagat ggatgtgcta gagggactga atgtccttgt gacaatttca 3060

ggaaagaaga ataagctacg agtttactat ctttcatggt taagaaacag aatactacat 3120

aatgacccag aagtagaaaa gaaacaaggc tggatcactg ttggggactt ggaaggctgt 3180

atacattata aagttgttaa atatgaaagg atcaaatttt tggtgattgc cttaaagaat 3240

gctgtggaaa tatatgcttg ggctcctaaa ccgtatcata aattcatggc atttaagtct 3300

tttgcagatc tccagcacaa gcctctgcta gttgatctca cggtagaaga aggtcaaaga 3360

ttaaaggtta tttttggttc acacactggt ttccatgtaa ttgatgttga ttcaggaaac 3420

tcttatgata tctacatacc atctcatatt cagggcaata tcactcctca tgctattgtc 3480

atcttgccta aaacagatgg aatggaaatg cttgtttgct atgaggatga gggggtgtat 3540

gtaaacacct atggccggat aactaaggat gtggtgctcc aatggggaga aatgcccacg 3600

tctgtggcct acattcattc caatcagata atgggctggg gcgagaaagc tattgagatc 3660

cggtcagtgg aaacaggaca tttggatgga gtatttatgc ataagcgagc tcaaaggtta 3720

aagtttctat gtgaaagaaa tgataaggta ttttttgcat ccgtgcgatc tggaggaagt 3780

agccaagtgt ttttcatgac cctcaacaga aattccatga tgaactggta a 3831

<210> 9
<211> 3807
<212> DNA
<213> homo sapiens

<400> 9
atggcgagcg actccccggc tcgaagcctg gatgaaatag atctctcggc tctgagggac 60

cccgcaggga tctttgaatt ggtggaactt gttggaaatg gaacatacgg gcaagtttat 120

aagggtcgtc atgtcaaaac gggccagctt gcagccatca aggttatgga tgtcacaggg 180

gatgaagagg aagaaatcaa acaagaaatt aacatgttga agaaatattc tcatcaccgg 240

aatattgcta catactatgg tgcttttatc aaaaagaacc caccaggcat ggatgaccaa 300

ctttggttgg tgatggagtt ttgtggtgct ggctctgtca ccgacctgat caagaacaca 360

aaaggtaaca cgttgaaaga ggagtggatt gcatacatct gcagggaaat cttacggggg 420

ctgagtcacc tgcaccagca taaagtgatt catcgagata ttaaagggca aaatgtcttg 480

ctgactgaaa atgcagaagt taaactagtg gactttggag tcagtgctca gcttgatcga 540

acagtgggca ggaggaatac tttcattgga actccctact ggatggcacc agaagttatt 600

gcctgtgatg aaaacccaga tgccacatat gatttcaaga gtgacttgtg gtctttgggt 660

atcaccgcca ttgaaatggc agaaggtgct ccccctctct gtgacatgca ccccatgaga 720

gctctcttcc tcatcccccg gaacccagcg cctcggctga agtctaagaa gtggtcaaaa 780

aaattccagt catttattga gagctgcttg gtaaagaatc acagccagcg accagcaaca 840
gaacaattga tgaagcatcc atttatacga gaccaaccta atgagcgaca ggtccgcatt 900
caactcaagg accatattga tagaacaaag aagaagcgag gagaaaaaga tgagacagag 960
tatgagtaca gtggaagtga ggaagaagag gaggagaatg actcaggaga gcccagctcc 1020
atcctgaatc tgccagggga gtcgacgctg cggagggact ttctgaggct gcagctggcc 1080
aacaaggagc gttctgaggc cctacggagg cagcagctgg agcagcagca gcgggagaat 1140
gaggagcaca agcggcagct gctggccgag cgtcagaagc gcatcgagga gcagaaagag 1200
cagaggcggc ggctggagga gcaacaaagg cgagagaagg agctgcggaa gcagcaggag 1260
agggagcagc gccggcacta tgaggagcag atgcgccggg aggaggagag gaggcgtgcg 1320
gagcatgaac aggaatataa gcgcaaacaa ttggaagaac agagacaagc agaaagactg 1380
cagaggcagc taaagcaaga aagagactac ttagtttccc ttcagcatca gcggcaggag 1440
cagaggcctg tggagaagaa gccactgtac cattacaaag aaggaatgag tcctagtgag 1500
aagccagcat gggccaagga gatcccacat ctggtagctg taaaatccca gggacctgcc 1560
ttgaccgcct cccagtcagt gcacgagcag cccacaaagg gcctctctgg gtttcaggag 1620
gctctgaacg tgacctccca ccgcgtggag atgccacgcc agaactcaga tcccacctcg 1680
gaaaatcctc ctctccccac tcgcattgaa aagtttgacc gaagctcttg gttacgacag 1740
gaagaagaca ttccaccaaa ggtgcctcaa agaacaactt ctatatcccc agcattagcc 1800
agaaagaatt ctcctgggaa tggtagtgct ctgggaccca gactaggatc tcaacccatc 1860
agagcaagca accctgatct ccggagaact gagcccatct tggagagccc cttgcagagg 1920
accagcagtg gcagttcctc cagctccagc acccctagct cccagcccag ctcccaagga 1980
ggctcccagc ctggatcaca agcaggatcc agtgaacgca ccagagttcg agccaacagt 2040
aagtcagaag gatcacctgt gctcccccat gagcctgcca aggtgaaacc agaagaatcc 2100
agggacatta cccggcccag tcgaccagct gatctgacgg cattagccaa agaactaaga 2160
gaactccgga ttgaagaaac aaaccgccca atgaagaagg tgactgatta ctcctcctcc 2220
agtgaggagt cagaaagtag cgaggaagag gaggaagatg gagagagcga gacccatgat 2280
gggacagtgg ctgtcagcga catacccaga ctgataccaa caggagctcc aggcagcaac 2340
gagcagtaca atgtgggaat ggtggggacg catgggctgg agacctctca tgcggacagt 2400
ttcagcggca gtatttcaag agaaggaacc ttgatgatta gagagacgtc tggagagaag 2460
aagcgatctg gccacagtga cagcaatggc tttgctggcc acatcaacct ccctgacctg 2520
gtgcagcaga gccattctcc agctggaacc ccgactgagg gactggggcg cgtctcaacc 2580
cattcccagg agatggactc tgggactgaa tatggcatgg ggagcagcac caaagcctcc 2640
ttcaccccct ttgtggaccc cagagtatac cagacgtctc ccactgatga agatgaagag 2700
gatgaggaat catcagccgc agctctgttt actagcgaac ttcttaggca agaacaggcc 2760
aaactcaatg aagcaagaaa gatttcggtg gtaaatgtaa acccaaccaa cattcggcct 2820
catagcgaca caccagaaat cagaaaatac aagaaacgat tcaactcaga aatactttgt 2880

gcagctctgt ggggtgtaaa ccttctggtg gggactgaaa atggcctgat gcttttggac 2940

cgaagtgggc aaggcaaagt ctataatctg atcaaccgga ggcgatttca gcagatggat 3000

gtgctagagg gactgaatgt ccttgtgaca atttcaggaa agaagaataa gctacgagtt 3060

tactatcttt catggttaag aaacagaata ctacataatg acccagaagt agaaaagaaa 3120

caaggctgga tcactgttgg ggacttggaa ggctgtatac attataaagt tgttaaatat 3180

gaaaggatca aatttttggt gattgcctta aagaatgctg tggaaatata tgcttgggct 3240

cctaaaccgt atcataaatt catggcattt aagtcttttg cagatctcca gcacaagcct 3300

ctgctagttg atctcacggt agaagaaggt caaagattaa aggttatttt tggttcacac 3360

actggtttcc atgtaattga tgttgattca ggaaactctt atgatatcta cataccatct 3420

catattcagg gcaatatcac tcctcatgct attgtcatct tgcctaaaac agatggaatg 3480

gaaatgcttg tttgctatga ggatgagggg gtgtatgtaa acacctatgg ccggataact 3540

aaggatgtgg tgctccaatg gggagaaatg cccacgtctg tggcctacat tcattccaat 3600

cagataatgg gctggggcga gaaagctatt gagatccggt cagtggaaac aggacatttg 3660

gatggagtat ttatgcataa gcgagctcaa aggttaaagt ttctatgtga aagaaatgat 3720

aaggtatttt ttgcatccgt gcgatctgga ggaagtagcc aagtgttttt catgaccctc 3780

aacagaaatt ccatgatgaa ctggtaa 3807

<210> 10
<211> 1352
<212> PRT
<213> homo sapiens

<400> 10

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys
                245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu

385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Ile Arg
        435                 440                 445

Arg Gln Leu Glu Glu Glu Gln Arg Gln Leu Glu Ile Leu Gln Gln Gln
    450                 455                 460

Leu Leu His Glu Gln Ala Leu Leu Leu Glu Tyr Lys Arg Lys Gln Leu
465                 470                 475                 480

Glu Glu Gln Arg Gln Ala Glu Arg Leu Gln Arg Gln Leu Lys Gln Glu
                485                 490                 495

Arg Asp Tyr Leu Val Ser Leu Gln His Gln Arg Gln Glu Gln Arg Pro
            500                 505                 510

Val Glu Lys Lys Pro Leu Tyr His Tyr Lys Glu Gly Met Ser Pro Ser
        515                 520                 525

Glu Lys Pro Ala Trp Ala Lys Glu Val Glu Glu Arg Ser Arg Leu Asn
    530                 535                 540

Arg Gln Ser Ser Pro Ala Met Pro His Lys Val Ala Asn Arg Ile Ser
545                 550                 555                 560

Asp Pro Asn Leu Pro Pro Arg Ser Glu Ser Phe Ser Ile Ser Gly Val
                565                 570                 575

Gln Pro Ala Arg Thr Pro Pro Met Leu Arg Pro Val Asp Pro Gln Ile
            580                 585                 590

Pro His Leu Val Ala Val Lys Ser Gln Gly Pro Ala Leu Thr Ala Ser
        595                 600                 605

Gln Ser Val His Glu Gln Pro Thr Lys Gly Leu Ser Gly Phe Gln Glu
    610                 615                 620

Ala Leu Asn Val Thr Ser His Arg Val Glu Met Pro Arg Gln Asn Ser
625                 630                 635                 640

Asp Pro Thr Ser Glu Asn Pro Pro Leu Pro Thr Arg Ile Glu Lys Phe
                645                 650                 655

Asp Arg Ser Ser Trp Leu Arg Gln Glu Glu Asp Ile Pro Pro Lys Val
            660                 665                 670

Pro Gln Arg Thr Thr Ser Ile Ser Pro Ala Leu Ala Arg Lys Asn Ser
        675                 680                 685

Pro Gly Asn Gly Ser Ala Leu Gly Pro Arg Leu Gly Ser Gln Pro Ile
    690                 695                 700

Arg Ala Ser Asn Pro Asp Leu Arg Arg Thr Glu Pro Ile Leu Glu Ser
705                 710                 715                 720

Pro Leu Gln Arg Thr Ser Ser Gly Ser Ser Ser Ser Ser Ser Thr Pro
                725                 730                 735

Ser Ser Gln Pro Ser Ser Gln Gly Gly Ser Gln Pro Gly Ser Gln Ala
            740                 745                 750

Gly Ser Ser Glu Arg Thr Arg Val Arg Ala Asn Ser Lys Ser Glu Gly
        755                 760                 765

Ser Pro Val Leu Pro His Glu Pro Ala Lys Val Lys Pro Glu Glu Ser
    770                 775                 780

Arg Asp Ile Thr Arg Pro Ser Arg Pro Ala Asp Leu Thr Ala Leu Ala
785                 790                 795                 800

Lys Glu Leu Arg Glu Leu Arg Ile Glu Glu Thr Asn Arg Pro Met Lys
                805                 810                 815

Lys Val Thr Asp Tyr Ser Ser Ser Ser Glu Glu Ser Glu Ser Ser Glu
            820                 825                 830

Glu Glu Glu Glu Asp Gly Glu Ser Glu Thr His Asp Gly Thr Val Ala
        835                 840                 845

Val Ser Asp Ile Pro Arg Leu Ile Pro Thr Gly Ala Pro Gly Ser Asn
    850                 855                 860

Glu Gln Tyr Asn Val Gly Met Val Gly Thr His Gly Leu Glu Thr Ser
865                 870                 875                 880

His Ala Asp Ser Phe Ser Gly Ser Ile Ser Arg Glu Gly Thr Leu Met
                885                 890                 895

Ile Arg Glu Thr Ser Gly Glu Lys Lys Arg Ser Gly His Ser Asp Ser
            900                 905                 910

Asn Gly Phe Ala Gly His Ile Asn Leu Pro Asp Leu Val Gln Gln Ser
        915                 920                 925

His Ser Pro Ala Gly Thr Pro Thr Glu Gly Leu Gly Arg Val Ser Thr
    930                 935                 940

His Ser Gln Glu Met Asp Ser Gly Thr Glu Tyr Gly Met Gly Ser Ser
945                 950                 955                 960

Thr Lys Ala Ser Phe Thr Pro Phe Val Asp Pro Arg Val Tyr Gln Thr
                965                 970                 975

Ser Pro Thr Asp Glu Asp Glu Glu Asp Glu Glu Ser Ser Ala Ala Ala
            980                 985                 990

Leu Phe Thr Ser Glu Leu Leu Arg  Gln Glu Gln Ala Lys  Leu Asn Glu
        995                 1000                 1005

Ala Arg  Lys Ile Ser Val Val  Asn Val Asn Pro Thr  Asn Ile Arg
    1010                 1015                 1020

Pro His  Ser Asp Thr Pro Glu  Ile Arg Lys Tyr Lys  Lys Arg Phe
    1025                 1030                 1035

Asn Ser  Glu Ile Leu Cys Ala  Ala Leu Trp Gly Val  Asn Leu Leu
    1040                 1045                 1050

Val Gly  Thr Glu Asn Gly Leu  Met Leu Leu Asp Arg  Ser Gly Gln
    1055                 1060                 1065

Gly Lys  Val Tyr Asn Leu Ile  Asn Arg Arg Arg Phe  Gln Gln Met
    1070                 1075                 1080

Asp Val  Leu Glu Gly Leu Asn  Val Leu Val Thr Ile  Ser Gly Lys
    1085                 1090                 1095

Lys Asn  Lys Leu Arg Val Tyr  Tyr Leu Ser Trp Leu  Arg Asn Arg
    1100                 1105                 1110

Ile Leu  His Asn Asp Pro Glu  Val Glu Lys Lys Gln  Gly Trp Ile
    1115                 1120                 1125

Thr Val  Gly Asp Leu Glu Gly  Cys Ile His Tyr Lys  Val Val Lys
    1130                 1135                 1140

Tyr Glu  Arg Ile Lys Phe Leu  Val Ile Ala Leu Lys  Asn Ala Val
    1145                 1150                 1155

Glu Ile  Tyr Ala Trp Ala Pro  Lys Pro Tyr His Lys  Phe Met Ala
    1160                 1165                 1170

Phe Lys  Ser Phe Ala Asp Leu  Gln His Lys Pro Leu  Leu Val Asp
    1175                 1180                 1185

Leu Thr  Val Glu Glu Gly Gln  Arg Leu Lys Val Ile  Phe Gly Ser
    1190                 1195                 1200

His Thr Gly Phe His Val Ile Asp Val Asp Ser Gly Asn Ser Tyr
    1205                1210                1215

Asp Ile Tyr Ile Pro Ser His Ile Gln Gly Asn Ile Thr Pro His
    1220                1225                1230

Ala Ile Val Ile Leu Pro Lys Thr Asp Gly Met Glu Met Leu Val
    1235                1240                1245

Cys Tyr Glu Asp Glu Gly Val Tyr Val Asn Thr Tyr Gly Arg Ile
    1250                1255                1260

Thr Lys Asp Val Val Leu Gln Trp Gly Glu Met Pro Thr Ser Val
    1265                1270                1275

Ala Tyr Ile His Ser Asn Gln Ile Met Gly Trp Gly Glu Lys Ala
    1280                1285                1290

Ile Glu Ile Arg Ser Val Glu Thr Gly His Leu Asp Gly Val Phe
    1295                1300                1305

Met His Lys Arg Ala Gln Arg Leu Lys Phe Leu Cys Glu Arg Asn
    1310                1315                1320

Asp Lys Val Phe Phe Ala Ser Val Arg Ser Gly Gly Ser Ser Gln
    1325                1330                1335

Val Phe Phe Met Thr Leu Asn Arg Asn Ser Met Met Asn Trp
    1340                1345                1350

<210>  11
<211>  1331
<212>  PRT
<213>  homo sapiens

<400>  11

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys
                245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Lys Arg
        435                 440                 445

Lys Gln Leu Glu Glu Gln Arg Gln Ala Glu Arg Leu Gln Arg Gln Leu
    450                 455                 460

Lys Gln Glu Arg Asp Tyr Leu Val Ser Leu Gln His Gln Arg Gln Glu
465                 470                 475                 480

Gln Arg Pro Val Glu Lys Lys Pro Leu Tyr His Tyr Lys Glu Gly Met
                485                 490                 495

Ser Pro Ser Glu Lys Pro Ala Trp Ala Lys Glu Val Glu Glu Arg Ser
            500                 505                 510

Arg Leu Asn Arg Gln Ser Ser Pro Ala Met Pro His Lys Val Ala Asn
        515                 520                 525

Arg Ile Ser Asp Pro Asn Leu Pro Pro Arg Ser Glu Ser Phe Ser Ile
    530                 535                 540

Ser Gly Val Gln Pro Ala Arg Thr Pro Pro Met Leu Arg Pro Val Asp
545                 550                 555                 560

Pro Gln Ile Pro His Leu Val Ala Val Lys Ser Gln Gly Pro Ala Leu
                565                 570                 575

Thr Ala Ser Gln Ser Val His Glu Gln Pro Thr Lys Gly Leu Ser Gly
            580                 585                 590

Phe Gln Glu Ala Leu Asn Val Thr Ser His Arg Val Glu Met Pro Arg
        595                 600                 605

Gln Asn Ser Asp Pro Thr Ser Glu Asn Pro Pro Leu Pro Thr Arg Ile
    610                 615                 620

Glu Lys Phe Asp Arg Ser Ser Trp Leu Arg Gln Glu Glu Asp Ile Pro
625                 630                 635                 640

Pro Lys Val Pro Gln Arg Thr Thr Ser Ile Ser Pro Ala Leu Ala Arg
                645                 650                 655

Lys Asn Ser Pro Gly Asn Gly Ser Ala Leu Gly Pro Arg Leu Gly Ser
            660                 665                 670

Gln Pro Ile Arg Ala Ser Asn Pro Asp Leu Arg Arg Thr Glu Pro Ile
        675                 680                 685

Leu Glu Ser Pro Leu Gln Arg Thr Ser Ser Gly Ser Ser Ser Ser Ser
    690                 695                 700

Ser Thr Pro Ser Ser Gln Pro Ser Ser Gln Gly Gly Ser Gln Pro Gly
705                 710                 715                 720

Ser Gln Ala Gly Ser Ser Glu Arg Thr Arg Val Arg Ala Asn Ser Lys
                725                 730                 735

Ser Glu Gly Ser Pro Val Leu Pro His Glu Pro Ala Lys Val Lys Pro
            740                 745                 750

Glu Glu Ser Arg Asp Ile Thr Arg Pro Ser Arg Pro Ala Ser Tyr Lys
        755                 760                 765

Lys Ala Ile Asp Glu Asp Leu Thr Ala Leu Ala Lys Glu Leu Arg Glu
    770                 775                 780

Leu Arg Ile Glu Glu Thr Asn Arg Pro Met Lys Lys Val Thr Asp Tyr
785                 790                 795                 800

Ser Ser Ser Ser Glu Glu Ser Glu Ser Ser Glu Glu Glu Glu Glu Asp
                805                 810                 815

Gly Glu Ser Glu Thr His Asp Gly Thr Val Ala Val Ser Asp Ile Pro
            820                 825                 830

Arg Leu Ile Pro Thr Gly Ala Pro Gly Ser Asn Glu Gln Tyr Asn Val
        835                 840                 845

Gly Met Val Gly Thr His Gly Leu Glu Thr Ser His Ala Asp Ser Phe
    850                 855                 860

Ser Gly Ser Ile Ser Arg Glu Gly Thr Leu Met Ile Arg Glu Thr Ser
865                 870                 875                 880

Gly Glu Lys Lys Arg Ser Gly His Ser Asp Ser Asn Gly Phe Ala Gly
                885                 890                 895

His Ile Asn Leu Pro Asp Leu Val Gln Gln Ser His Ser Pro Ala Gly
            900                 905                 910

Thr Pro Thr Glu Gly Leu Gly Arg Val Ser Thr His Ser Gln Glu Met

        915                 920                 925

Asp Ser Gly Thr Glu Tyr Gly Met Gly Ser Ser Thr Lys Ala Ser Phe
    930                 935                 940

Thr Pro Phe Val Asp Pro Arg Val Tyr Gln Thr Ser Pro Thr Asp Glu
945                 950                 955                 960

Asp Glu Glu Asp Glu Glu Ser Ser Ala Ala Ala Leu Phe Thr Ser Glu
                965                 970                 975

Leu Leu Arg Gln Glu Gln Ala Lys Leu Asn Glu Ala Arg Lys Ile Ser
            980                 985                 990

Val Val Asn Val Asn Pro Thr Asn Ile Arg Pro His Ser Asp Thr Pro
        995                 1000                1005

Glu Ile Arg Lys Tyr Lys Lys Arg Phe Asn Ser Glu Ile Leu Cys
    1010                1015                1020

Ala Ala Leu Trp Gly Val Asn Leu Leu Val Gly Thr Glu Asn Gly
    1025                1030                1035

Leu Met Leu Leu Asp Arg Ser Gly Gln Gly Lys Val Tyr Asn Leu
    1040                1045                1050

Ile Asn Arg Arg Arg Phe Gln Gln Met Asp Val Leu Glu Gly Leu
    1055                1060                1065

Asn Val Leu Val Thr Ile Ser Gly Lys Lys Asn Lys Leu Arg Val
    1070                1075                1080

Tyr Tyr Leu Ser Trp Leu Arg Asn Arg Ile Leu His Asn Asp Pro
    1085                1090                1095

Glu Val Glu Lys Lys Gln Gly Trp Ile Thr Val Gly Asp Leu Glu
    1100                1105                1110

Gly Cys Ile His Tyr Lys Val Val Lys Tyr Glu Arg Ile Lys Phe
    1115                1120                1125

Leu Val Ile Ala Leu Lys Asn Ala Val Glu Ile Tyr Ala Trp Ala
    1130                1135                1140

Pro Lys Pro Tyr His Lys Phe Met Ala Phe Lys Ser Phe Ala Asp
    1145                1150                1155

Leu Gln His Lys Pro Leu Leu Val Asp Leu Thr Val Glu Glu Gly
    1160                1165                1170

Gln Arg Leu Lys Val Ile Phe Gly Ser His Thr Gly Phe His Val
    1175                1180                1185

Ile Asp Val Asp Ser Gly Asn Ser Tyr Asp Ile Tyr Ile Pro Ser
    1190                1195                1200

His Ile Gln Gly Asn Ile Thr Pro His Ala Ile Val Ile Leu Pro
    1205                1210                1215

Lys Thr Asp Gly Met Glu Met Leu Val Cys Tyr Glu Asp Glu Gly
    1220                1225                1230

Val Tyr Val Asn Thr Tyr Gly Arg Ile Thr Lys Asp Val Val Leu
    1235                1240                1245

Gln Trp Gly Glu Met Pro Thr Ser Val Ala Tyr Ile His Ser Asn
    1250                1255                1260

Gln Ile Met Gly Trp Gly Glu Lys Ala Ile Glu Ile Arg Ser Val
    1265                1270                1275

Glu Thr Gly His Leu Asp Gly Val Phe Met His Lys Arg Ala Gln
    1280                1285                1290

Arg Leu Lys Phe Leu Cys Glu Arg Asn Asp Lys Val Phe Phe Ala
    1295                1300                1305

Ser Val Arg Ser Gly Gly Ser Ser Gln Val Phe Phe Met Thr Leu
    1310                1315                1320

Asn Arg Asn Ser Met Met Asn Trp
    1325                1330

<210>  12
<211>  1323
<212>  PRT
<213>  homo sapiens

<400>  12
   Met Ala Ser Asp Ser Pro Ala Arg Ser Leu Asp Glu Ile Asp Leu Ser
   1               5                   10                  15
   Ala Leu Arg Asp Pro Ala Gly Ile Phe Glu Leu Val Glu Leu Val Gly
               20                  25                  30
   Asn Gly Thr Tyr Gly Gln Val Tyr Lys Gly Arg His Val Lys Thr Gly
           35                  40                  45
   Gln Leu Ala Ala Ile Lys Val Met Asp Val Thr Gly Asp Glu Glu Glu
       50                  55                  60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Asn Pro Pro Gly
                   85                  90                  95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
       290                 295                 300
   His Ile Asp Arg Thr Lys Lys Lys Arg Gly Glu Lys Asp Glu Thr Glu
   305                 310                 315                 320
   Tyr Glu Tyr Ser Gly Ser Glu Glu Glu Glu Glu Glu Asn Asp Ser Gly
                   325                 330                 335
   Glu Pro Ser Ser Ile Leu Asn Leu Pro Gly Glu Ser Thr Leu Arg Arg
               340                 345                 350
   Asp Phe Leu Arg Leu Gln Leu Ala Asn Lys Glu Arg Ser Glu Ala Leu
   355                 360                 365
   Arg Arg Gln Gln Leu Glu Gln Gln Gln Arg Glu Asn Glu Glu His Lys
       370                 375                 380
   Arg Gln Leu Leu Ala Glu Arg Gln Lys Arg Ile Glu Glu Gln Lys Glu
   385                 390                 395                 400
   Gln Arg Arg Arg Leu Glu Glu Gln Gln Arg Arg Glu Lys Glu Leu Arg
                   405                 410                 415
   Lys Gln Gln Glu Arg Glu Gln Arg Arg His Tyr Glu Glu Gln Met Arg
               420                 425                 430
   Arg Glu Glu Glu Arg Arg Arg Ala Glu His Glu Gln Glu Tyr Lys Arg
           435                 440                 445
   Lys Gln Leu Glu Glu Gln Arg Gln Ala Glu Arg Leu Gln Arg Gln Leu
       450                 455                 460
   Lys Gln Glu Arg Asp Tyr Leu Val Ser Leu Gln His Gln Arg Gln Glu
   465                 470                 475                 480
   Gln Arg Pro Val Glu Lys Lys Pro Leu Tyr His Tyr Lys Glu Gly Met
                   485                 490                 495
   Ser Pro Ser Glu Lys Pro Ala Trp Ala Lys Glu Val Glu Glu Arg Ser
               500                 505                 510
   Arg Leu Asn Arg Gln Ser Ser Pro Ala Met Pro His Lys Val Ala Asn
           515                 520                 525
   Arg Ile Ser Asp Pro Asn Leu Pro Pro Arg Ser Glu Ser Phe Ser Ile
       530                 535                 540
   Ser Gly Val Gln Pro Ala Arg Thr Pro Pro Met Leu Arg Pro Val Asp
   545                 550                 555                 560
   Pro Gln Ile Pro His Leu Val Ala Val Lys Ser Gln Gly Pro Ala Leu
                   565                 570                 575
   Thr Ala Ser Gln Ser Val His Glu Gln Pro Thr Lys Gly Leu Ser Gly
               580                 585                 590
   Phe Gln Glu Ala Leu Asn Val Thr Ser His Arg Val Glu Met Pro Arg
           595                 600                 605
   Gln Asn Ser Asp Pro Thr Ser Glu Asn Pro Pro Leu Pro Thr Arg Ile
       610                 615                 620
   Glu Lys Phe Asp Arg Ser Ser Trp Leu Arg Gln Glu Glu Asp Ile Pro
   625                 630                 635                 640
   Pro Lys Val Pro Gln Arg Thr Thr Ser Ile Ser Pro Ala Leu Ala Arg
                   645                 650                 655
   Lys Asn Ser Pro Gly Asn Gly Ser Ala Leu Gly Pro Arg Leu Gly Ser
               660                 665                 670
   Gln Pro Ile Arg Ala Ser Asn Pro Asp Leu Arg Arg Thr Glu Pro Ile
           675                 680                 685
   Leu Glu Ser Pro Leu Gln Arg Thr Ser Ser Gly Ser Ser Ser Ser Ser
       690                 695                 700
   Ser Thr Pro Ser Ser Gln Pro Ser Ser Gln Gly Gly Ser Gln Pro Gly
   705                 710                 715                 720
   Ser Gln Ala Gly Ser Ser Glu Arg Thr Arg Val Arg Ala Asn Ser Lys
                   725                 730                 735
   Ser Glu Gly Ser Pro Val Leu Pro His Glu Pro Ala Lys Val Lys Pro
               740                 745                 750
   Glu Glu Ser Arg Asp Ile Thr Arg Pro Ser Arg Pro Ala Asp Leu Thr
           755                 760                 765
   Ala Leu Ala Lys Glu Leu Arg Glu Leu Arg Ile Glu Glu Thr Asn Arg
       770                 775                 780
   Pro Met Lys Lys Val Thr Asp Tyr Ser Ser Ser Ser Glu Glu Ser Glu
   785                 790                 795                 800
   Ser Ser Glu Glu Glu Glu Glu Asp Gly Glu Ser Glu Thr His Asp Gly
                   805                 810                 815
   Thr Val Ala Val Ser Asp Ile Pro Arg Leu Ile Pro Thr Gly Ala Pro
               820                 825                 830
   Gly Ser Asn Glu Gln Tyr Asn Val Gly Met Val Gly Thr His Gly Leu
           835                 840                 845
   Glu Thr Ser His Ala Asp Ser Phe Ser Gly Ser Ile Ser Arg Glu Gly
       850                 855                 860
   Thr Leu Met Ile Arg Glu Thr Ser Gly Glu Lys Lys Arg Ser Gly His
   865                 870                 875                 880
   Ser Asp Ser Asn Gly Phe Ala Gly His Ile Asn Leu Pro Asp Leu Val
                   885                 890                 895
   Gln Gln Ser His Ser Pro Ala Gly Thr Pro Thr Glu Gly Leu Gly Arg
               900                 905                 910
   Val Ser Thr His Ser Gln Glu Met Asp Ser Gly Thr Glu Tyr Gly Met
           915                 920                 925
   Gly Ser Ser Thr Lys Ala Ser Phe Thr Pro Phe Val Asp Pro Arg Val
       930                 935                 940
   Tyr Gln Thr Ser Pro Thr Asp Glu Asp Glu Glu Asp Glu Glu Ser Ser
   945                 950                 955                 960
   Ala Ala Ala Leu Phe Thr Ser Glu Leu Leu Arg Gln Glu Gln Ala Lys
                   965                 970                 975
   Leu Asn Glu Ala Arg Lys Ile Ser Val Val Asn Val Asn Pro Thr Asn
               980                 985                 990
   Ile Arg Pro His Ser Asp Thr Pro Glu Ile Arg Lys Tyr Lys Lys Arg
           995                 1000                1005
   Phe Asn Ser Glu Ile Leu Cys Ala Ala Leu Trp Gly Val Asn Leu
       1010                1015                1020
   Leu Val Gly Thr Glu Asn Gly Leu Met Leu Leu Asp Arg Ser Gly
       1025                1030                1035
   Gln Gly Lys Val Tyr Asn Leu Ile Asn Arg Arg Arg Phe Gln Gln
       1040                1045                1050
   Met Asp Val Leu Glu Gly Leu Asn Val Leu Val Thr Ile Ser Gly
       1055                1060                1065
   Lys Lys Asn Lys Leu Arg Val Tyr Tyr Leu Ser Trp Leu Arg Asn
       1070                1075                1080
   Arg Ile Leu His Asn Asp Pro Glu Val Glu Lys Lys Gln Gly Trp
       1085                1090                1095
   Ile Thr Val Gly Asp Leu Glu Gly Cys Ile His Tyr Lys Val Val
       1100                1105                1110
   Lys Tyr Glu Arg Ile Lys Phe Leu Val Ile Ala Leu Lys Asn Ala
       1115                1120                1125
   Val Glu Ile Tyr Ala Trp Ala Pro Lys Pro Tyr His Lys Phe Met
       1130                1135                1140
   Ala Phe Lys Ser Phe Ala Asp Leu Gln His Lys Pro Leu Leu Val
       1145                1150                1155
   Asp Leu Thr Val Glu Glu Gly Gln Arg Leu Lys Val Ile Phe Gly
       1160                1165                1170
   Ser His Thr Gly Phe His Val Ile Asp Val Asp Ser Gly Asn Ser
   1175 1180 1185
   Tyr Asp Ile Tyr Ile Pro Ser His Ile Gln Gly Asn Ile Thr Pro
   1190 1195 1200
   His Ala Ile Val Ile Leu Pro Lys Thr Asp Gly Met Glu Met Leu
   1205 1210 1215
   Val Cys Tyr Glu Asp Glu Gly Val Tyr Val Asn Thr Tyr Gly Arg
   1220 1225 1230
   Ile Thr Lys Asp Val Val Leu Gln Trp Gly Glu Met Pro Thr Ser
   1235 1240 1245
   Val Ala Tyr Ile His Ser Asn Gln Ile Met Gly Trp Gly Glu Lys
   1250 1255 1260
   Ala Ile Glu Ile Arg Ser Val Glu Thr Gly His Leu Asp Gly Val
   1265 1270 1275
   Phe Met His Lys Arg Ala Gln Arg Leu Lys Phe Leu Cys Glu Arg
   1280 1285 1290
   Asn Asp Lys Val Phe Phe Ala Ser Val Arg Ser Gly Gly Ser Ser
   1295 1300 1305
   Gln Val Phe Phe Met Thr Leu Asn Arg Asn Ser Met Met Asn Trp
   1310 1315 1320

<210>  13
<211>  1305
<212>  PRT
<213>  homo sapiens

<400>  13
   Met Ala Ser Asp Ser Pro Ala Arg Ser Leu Asp Glu Ile Asp Leu Ser
   1 5 10 15
   Ala Leu Arg Asp Pro Ala Gly Ile Phe Glu Leu Val Glu Leu Val Gly
   20 25 30
   Asn Gly Thr Tyr Gly Gln Val Tyr Lys Gly Arg His Val Lys Thr Gly
   35 40 45
   Gln Leu Ala Ala Ile Lys Val Met Asp Val Thr Gly Asp Glu Glu Glu
   50 55 60
   Glu Ile Lys Gln Glu Ile Asn Met Leu Lys Lys Tyr Ser His His Arg
   65 70 75 80
   Asn Ile Ala Thr Tyr Tyr Gly Ala Phe Ile Lys Lys Asn Pro Pro Gly
   85 90 95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
       290                 295                 300
   His Ile Asp Arg Thr Lys Lys Lys Arg Gly Glu Lys Asp Glu Thr Glu
   305                 310                 315                 320
   Tyr Glu Tyr Ser Gly Ser Glu Glu Glu Glu Glu Glu Asn Asp Ser Gly
                   325                 330                 335
   Glu Pro Ser Ser Ile Leu Asn Leu Pro Gly Glu Ser Thr Leu Arg Arg
               340                 345                 350
   Asp Phe Leu Arg Leu Gln Leu Ala Asn Lys Glu Arg Ser Glu Ala Leu
           355                 360                 365
   Arg Arg Gln Gln Leu Glu Gln Gln Gln Arg Glu Asn Glu Glu His Lys
       370                 375                 380
   Arg Gln Leu Leu Ala Glu Arg Gln Lys Arg Ile Glu Glu Gln Lys Glu
   385                 390                 395                 400
   Gln Arg Arg Arg Leu Glu Glu Gln Gln Arg Arg Glu Lys Glu Leu Arg
                   405                 410                 415
   Lys Gln Gln Glu Arg Glu Gln Arg Arg His Tyr Glu Glu Gln Met Arg
               420                 425                 430
   Arg Glu Glu Glu Arg Arg Arg Ala Glu His Glu Gln Glu Tyr Ile Arg
           435                 440                 445
   Arg Gln Leu Glu Glu Glu Gln Arg Gln Leu Glu Ile Leu Gln Gln Gln
       450                 455                 460
   Leu Leu His Glu Gln Ala Leu Leu Leu Glu Tyr Lys Arg Lys Gln Leu
   465                 470                 475                 480
   Glu Glu Gln Arg Gln Ala Glu Arg Leu Gln Arg Gln Leu Lys Gln Glu
                   485                 490                 495
   Arg Asp Tyr Leu Val Ser Leu Gln His Gln Arg Gln Glu Gln Arg Pro
               500                 505                 510
   Val Glu Lys Lys Pro Leu Tyr His Tyr Lys Glu Gly Met Ser Pro Ser
           515                 520                 525
   Glu Lys Pro Ala Trp Ala Lys Glu Ile Pro His Leu Val Ala Val Lys
       530                 535                 540
   Ser Gln Gly Pro Ala Leu Thr Ala Ser Gln Ser Val His Glu Gln Pro
   545                 550                 555                 560
   Thr Lys Gly Leu Ser Gly Phe Gln Glu Ala Leu Asn Val Thr Ser His
                   565                 570                 575
   Arg Val Glu Met Pro Arg Gln Asn Ser Asp Pro Thr Ser Glu Asn Pro
               580                 585                 590
   Pro Leu Pro Thr Arg Ile Glu Lys Phe Asp Arg Ser Ser Trp Leu Arg
           595                 600                 605
   Gln Glu Glu Asp Ile Pro Pro Lys Val Pro Gln Arg Thr Thr Ser Ile
       610                 615                 620
   Ser Pro Ala Leu Ala Arg Lys Asn Ser Pro Gly Asn Gly Ser Ala Leu
   625                 630                 635                 640
   Gly Pro Arg Leu Gly Ser Gln Pro Ile Arg Ala Ser Asn Pro Asp Leu
                   645                 650                 655
   Arg Arg Thr Glu Pro Ile Leu Glu Ser Pro Leu Gln Arg Thr Ser Ser
               660                 665                 670
   Gly Ser Ser Ser Ser Ser Ser Thr Pro Ser Ser Gln Pro Ser Ser Gln
           675                 680                 685
   Gly Gly Ser Gln Pro Gly Ser Gln Ala Gly Ser Ser Glu Arg Thr Arg
       690                 695                 700
   Val Arg Ala Asn Ser Lys Ser Glu Gly Ser Pro Val Leu Pro His Glu
   705                 710                 715                 720
   Pro Ala Lys Val Lys Pro Glu Glu Ser Arg Asp Ile Thr Arg Pro Ser
                   725                 730                 735
   Arg Pro Ala Ser Tyr Lys Lys Ala Ile Asp Glu Asp Leu Thr Ala Leu
               740                 745                 750
   Ala Lys Glu Leu Arg Glu Leu Arg Ile Glu Glu Thr Asn Arg Pro Met
           755                 760                 765
   Lys Lys Val Thr Asp Tyr Ser Ser Ser Ser Glu Glu Ser Glu Ser Ser
       770                 775                 780
   Glu Glu Glu Glu Glu Asp Gly Glu Ser Glu Thr His Asp Gly Thr Val
   785                 790                 795                 800
   Ala Val Ser Asp Ile Pro Arg Leu Ile Pro Thr Gly Ala Pro Gly Ser
                   805                 810                 815
   Asn Glu Gln Tyr Asn Val Gly Met Val Gly Thr His Gly Leu Glu Thr
               820                 825                 830
   Ser His Ala Asp Ser Phe Ser Gly Ser Ile Ser Arg Glu Gly Thr Leu
           835                 840                 845
   Met Ile Arg Glu Thr Ser Gly Glu Lys Lys Arg Ser Gly His Ser Asp
       850                 855                 860
   Ser Asn Gly Phe Ala Gly His Ile Asn Leu Pro Asp Leu Val Gln Gln
   865                 870                 875                 880
   Ser His Ser Pro Ala Gly Thr Pro Thr Glu Gly Leu Gly Arg Val Ser
                   885                 890                 895
   Thr His Ser Gln Glu Met Asp Ser Gly Thr Glu Tyr Gly Met Gly Ser
               900                 905                 910
   Ser Thr Lys Ala Ser Phe Thr Pro Phe Val Asp Pro Arg Val Tyr Gln
   915 920 925
   Thr Ser Pro Thr Asp Glu Asp Glu Glu Asp Glu Glu Ser Ser Ala Ala
   930 935 940
   Ala Leu Phe Thr Ser Glu Leu Leu Arg Gln Glu Gln Ala Lys Leu Asn
   945 950 955 960
   Glu Ala Arg Lys Ile Ser Val Val Asn Val Asn Pro Thr Asn Ile Arg
   965 970 975
   Pro His Ser Asp Thr Pro Glu Ile Arg Lys Tyr Lys Lys Arg Phe Asn
   980 985 990
   Ser Glu Ile Leu Cys Ala Ala Leu Trp Gly Val Asn Leu Leu Val Gly
   995 1000 1005
   Thr Glu Asn Gly Leu Met Leu Leu Asp Arg Ser Gly Gln Gly Lys
   1010 1015 1020
   Val Tyr Asn Leu Ile Asn Arg Arg Arg Phe Gln Gln Met Asp Val
   1025 1030 1035
   Leu Glu Gly Leu Asn Val Leu Val Thr Ile Ser Gly Lys Lys Asn
   1040 1045 1050
   Lys Leu Arg Val Tyr Tyr Leu Ser Trp Leu Arg Asn Arg Ile Leu
   1055 1060 1065
   His Asn Asp Pro Glu Val Glu Lys Lys Gln Gly Trp Ile Thr Val
   1070 1075 1080
   Gly Asp Leu Glu Gly Cys Ile His Tyr Lys Val Val Lys Tyr Glu
   1085 1090 1095
   Arg Ile Lys Phe Leu Val Ile Ala Leu Lys Asn Ala Val Glu Ile
   1100 1105 1110
   Tyr Ala Trp Ala Pro Lys Pro Tyr His Lys Phe Met Ala Phe Lys
   1115 1120 1125
   Ser Phe Ala Asp Leu Gln His Lys Pro Leu Leu Val Asp Leu Thr
   1130 1135 1140
   Val Glu Glu Gly Gln Arg Leu Lys Val Ile Phe Gly Ser His Thr
   1145 1150 1155
   Gly Phe His Val Ile Asp Val Asp Ser Gly Asn Ser Tyr Asp Ile
   1160 1165 1170
   Tyr Ile Pro Ser His Ile Gln Gly Asn Ile Thr Pro His Ala Ile
   1175 1180 1185
   Val Ile Leu Pro Lys Thr Asp Gly Met Glu Met Leu Val Cys Tyr
       1190                1195                1200
   Glu Asp Glu Gly Val Tyr Val Asn Thr Tyr Gly Arg Ile Thr Lys
       1205                1210                1215
   Asp Val Val Leu Gln Trp Gly Glu Met Pro Thr Ser Val Ala Tyr
       1220                1225                1230
   Ile His Ser Asn Gln Ile Met Gly Trp Gly Glu Lys Ala Ile Glu
       1235                1240                1245
   Ile Arg Ser Val Glu Thr Gly His Leu Asp Gly Val Phe Met His
       1250                1255                1260
   Lys Arg Ala Gln Arg Leu Lys Phe Leu Cys Glu Arg Asn Asp Lys
       1265                1270                1275
   Val Phe Phe Ala Ser Val Arg Ser Gly Gly Ser Ser Gln Val Phe
       1280                1285                1290
   Phe Met Thr Leu Asn Arg Asn Ser Met Met Asn Trp
       1295                1300                1305

<210>  14
<211>  1297
<212>  PRT
<213>  homo sapiens

<400>  14

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys
                245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu

385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Ile Arg
        435                 440                 445

Arg Gln Leu Glu Glu Glu Gln Arg Gln Leu Glu Ile Leu Gln Gln Gln
    450                 455                 460

Leu Leu His Glu Gln Ala Leu Leu Leu Glu Tyr Lys Arg Lys Gln Leu
465                 470                 475                 480

Glu Glu Gln Arg Gln Ala Glu Arg Leu Gln Arg Gln Leu Lys Gln Glu
                485                 490                 495

Arg Asp Tyr Leu Val Ser Leu Gln His Gln Arg Gln Glu Gln Arg Pro
            500                 505                 510

Val Glu Lys Lys Pro Leu Tyr His Tyr Lys Glu Gly Met Ser Pro Ser
        515                 520                 525

Glu Lys Pro Ala Trp Ala Lys Glu Ile Pro His Leu Val Ala Val Lys
    530                 535                 540

Ser Gln Gly Pro Ala Leu Thr Ala Ser Gln Ser Val His Glu Gln Pro
545                 550                 555                 560

Thr Lys Gly Leu Ser Gly Phe Gln Glu Ala Leu Asn Val Thr Ser His
                565                 570                 575

Arg Val Glu Met Pro Arg Gln Asn Ser Asp Pro Thr Ser Glu Asn Pro
            580                 585                 590

Pro Leu Pro Thr Arg Ile Glu Lys Phe Asp Arg Ser Ser Trp Leu Arg
        595                 600                 605

Gln Glu Glu Asp Ile Pro Pro Lys Val Pro Gln Arg Thr Thr Ser Ile
    610                 615                 620

Ser Pro Ala Leu Ala Arg Lys Asn Ser Pro Gly Asn Gly Ser Ala Leu
625                 630                 635                 640

Gly Pro Arg Leu Gly Ser Gln Pro Ile Arg Ala Ser Asn Pro Asp Leu
                645                 650                 655

Arg Arg Thr Glu Pro Ile Leu Glu Ser Pro Leu Gln Arg Thr Ser Ser
            660                 665                 670

Gly Ser Ser Ser Ser Ser Ser Thr Pro Ser Ser Gln Pro Ser Ser Gln
        675                 680                 685

Gly Gly Ser Gln Pro Gly Ser Gln Ala Gly Ser Ser Glu Arg Thr Arg
    690                 695                 700

Val Arg Ala Asn Ser Lys Ser Glu Gly Ser Pro Val Leu Pro His Glu
705                 710                 715                 720

Pro Ala Lys Val Lys Pro Glu Glu Ser Arg Asp Ile Thr Arg Pro Ser
                725                 730                 735

Arg Pro Ala Asp Leu Thr Ala Leu Ala Lys Glu Leu Arg Glu Leu Arg
            740                 745                 750

Ile Glu Glu Thr Asn Arg Pro Met Lys Lys Val Thr Asp Tyr Ser Ser
        755                 760                 765

Ser Ser Glu Glu Ser Glu Ser Ser Glu Glu Glu Glu Glu Asp Gly Glu
    770                 775                 780

Ser Glu Thr His Asp Gly Thr Val Ala Val Ser Asp Ile Pro Arg Leu
785                 790                 795                 800

Ile Pro Thr Gly Ala Pro Gly Ser Asn Glu Gln Tyr Asn Val Gly Met
                805                 810                 815

Val Gly Thr His Gly Leu Glu Thr Ser His Ala Asp Ser Phe Ser Gly
            820                 825                 830

Ser Ile Ser Arg Glu Gly Thr Leu Met Ile Arg Glu Thr Ser Gly Glu
        835                 840                 845

Lys Lys Arg Ser Gly His Ser Asp Ser Asn Gly Phe Ala Gly His Ile
    850                 855                 860

Asn Leu Pro Asp Leu Val Gln Gln Ser His Ser Pro Ala Gly Thr Pro
865                 870                 875                 880

Thr Glu Gly Leu Gly Arg Val Ser Thr His Ser Gln Glu Met Asp Ser
                885                 890                 895

Gly Thr Glu Tyr Gly Met Gly Ser Ser Thr Lys Ala Ser Phe Thr Pro
            900                 905                 910

Phe Val Asp Pro Arg Val Tyr Gln Thr Ser Pro Thr Asp Glu Asp Glu
        915                 920                 925

Glu Asp Glu Glu Ser Ser Ala Ala Ala Leu Phe Thr Ser Glu Leu Leu
    930                 935                 940

Arg Gln Glu Gln Ala Lys Leu Asn Glu Ala Arg Lys Ile Ser Val Val
945                 950                 955                 960

Asn Val Asn Pro Thr Asn Ile Arg Pro His Ser Asp Thr Pro Glu Ile
                965                 970                 975

Arg Lys Tyr Lys Lys Arg Phe Asn Ser Glu Ile Leu Cys Ala Ala Leu
            980                 985                 990

Trp Gly Val Asn Leu Leu Val Gly  Thr Glu Asn Gly Leu  Met Leu Leu
        995                 1000                 1005

Asp Arg  Ser Gly Gln Gly Lys  Val Tyr Asn Leu Ile  Asn Arg Arg
    1010                 1015                 1020

Arg Phe  Gln Gln Met Asp Val  Leu Glu Gly Leu Asn  Val Leu Val
    1025                 1030                 1035

Thr Ile  Ser Gly Lys Lys Asn  Lys Leu Arg Val Tyr  Tyr Leu Ser
    1040                 1045                 1050

Trp Leu  Arg Asn Arg Ile Leu  His Asn Asp Pro Glu  Val Glu Lys
    1055                 1060                 1065

Lys Gln  Gly Trp Ile Thr Val  Gly Asp Leu Glu Gly  Cys Ile His
    1070                 1075                 1080

Tyr Lys  Val Val Lys Tyr Glu  Arg Ile Lys Phe Leu  Val Ile Ala
    1085                 1090                 1095

Leu Lys  Asn Ala Val Glu Ile  Tyr Ala Trp Ala Pro  Lys Pro Tyr
    1100                 1105                 1110

His Lys  Phe Met Ala Phe Lys  Ser Phe Ala Asp Leu  Gln His Lys
    1115                 1120                 1125

Pro Leu  Leu Val Asp Leu Thr  Val Glu Glu Gly Gln  Arg Leu Lys
    1130                 1135                 1140

Val Ile  Phe Gly Ser His Thr  Gly Phe His Val Ile  Asp Val Asp
    1145                 1150                 1155

Ser Gly  Asn Ser Tyr Asp Ile  Tyr Ile Pro Ser His  Ile Gln Gly
    1160                 1165                 1170

Asn Ile  Thr Pro His Ala Ile  Val Ile Leu Pro Lys  Thr Asp Gly
    1175                 1180                 1185

Met Glu  Met Leu Val Cys Tyr  Glu Asp Glu Gly Val  Tyr Val Asn
    1190                 1195                 1200

Thr Tyr Gly Arg Ile Thr Lys Asp Val Val Leu Gln Trp Gly Glu
    1205                1210                1215

Met Pro Thr Ser Val Ala Tyr Ile His Ser Asn Gln Ile Met Gly
    1220                1225                1230

Trp Gly Glu Lys Ala Ile Glu Ile Arg Ser Val Glu Thr Gly His
    1235                1240                1245

Leu Asp Gly Val Phe Met His Lys Arg Ala Gln Arg Leu Lys Phe
    1250                1255                1260

Leu Cys Glu Arg Asn Asp Lys Val Phe Phe Ala Ser Val Arg Ser
    1265                1270                1275

Gly Gly Ser Ser Gln Val Phe Phe Met Thr Leu Asn Arg Asn Ser
    1280                1285                1290

Met Met Asn Trp
    1295

<210>  15
<211>  1276
<212>  PRT
<213>  homo sapiens

<400>  15

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys
                245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Lys Arg
        435                 440                 445

Lys Gln Leu Glu Glu Gln Arg Gln Ala Glu Arg Leu Gln Arg Gln Leu
    450                 455                 460

Lys Gln Glu Arg Asp Tyr Leu Val Ser Leu Gln His Gln Arg Gln Glu
465                 470                 475                 480

Gln Arg Pro Val Glu Lys Lys Pro Leu Tyr His Tyr Lys Glu Gly Met
                485                 490                 495

Ser Pro Ser Glu Lys Pro Ala Trp Ala Lys Glu Ile Pro His Leu Val
            500                 505                 510

Ala Val Lys Ser Gln Gly Pro Ala Leu Thr Ala Ser Gln Ser Val His
        515                 520                 525

Glu Gln Pro Thr Lys Gly Leu Ser Gly Phe Gln Glu Ala Leu Asn Val
    530                 535                 540

Thr Ser His Arg Val Glu Met Pro Arg Gln Asn Ser Asp Pro Thr Ser
545                 550                 555                 560

Glu Asn Pro Pro Leu Pro Thr Arg Ile Glu Lys Phe Asp Arg Ser Ser
                565                 570                 575

Trp Leu Arg Gln Glu Glu Asp Ile Pro Pro Lys Val Pro Gln Arg Thr
            580                 585                 590

Thr Ser Ile Ser Pro Ala Leu Ala Arg Lys Asn Ser Pro Gly Asn Gly
        595                 600                 605

Ser Ala Leu Gly Pro Arg Leu Gly Ser Gln Pro Ile Arg Ala Ser Asn
    610                 615                 620

Pro Asp Leu Arg Arg Thr Glu Pro Ile Leu Glu Ser Pro Leu Gln Arg
625                 630                 635                 640

Thr Ser Ser Gly Ser Ser Ser Ser Ser Ser Thr Pro Ser Ser Gln Pro
                645                 650                 655

Ser Ser Gln Gly Gly Ser Gln Pro Gly Ser Gln Ala Gly Ser Ser Glu
            660                 665                 670

Arg Thr Arg Val Arg Ala Asn Ser Lys Ser Glu Gly Ser Pro Val Leu
        675                 680                 685

Pro His Glu Pro Ala Lys Val Lys Pro Glu Glu Ser Arg Asp Ile Thr
    690                 695                 700

Arg Pro Ser Arg Pro Ala Ser Tyr Lys Lys Ala Ile Asp Glu Asp Leu
705                 710                 715                 720

Thr Ala Leu Ala Lys Glu Leu Arg Glu Leu Arg Ile Glu Glu Thr Asn
                725                 730                 735

Arg Pro Met Lys Lys Val Thr Asp Tyr Ser Ser Ser Ser Glu Glu Ser
            740                 745                 750

Glu Ser Ser Glu Glu Glu Glu Glu Asp Gly Glu Ser Glu Thr His Asp
        755                 760                 765

Gly Thr Val Ala Val Ser Asp Ile Pro Arg Leu Ile Pro Thr Gly Ala
    770                 775                 780

Pro Gly Ser Asn Glu Gln Tyr Asn Val Gly Met Val Gly Thr His Gly
785                 790                 795                 800

Leu Glu Thr Ser His Ala Asp Ser Phe Ser Gly Ser Ile Ser Arg Glu
                805                 810                 815

Gly Thr Leu Met Ile Arg Glu Thr Ser Gly Glu Lys Lys Arg Ser Gly
            820                 825                 830

His Ser Asp Ser Asn Gly Phe Ala Gly His Ile Asn Leu Pro Asp Leu
        835                 840                 845

Val Gln Gln Ser His Ser Pro Ala Gly Thr Pro Thr Glu Gly Leu Gly
    850                 855                 860

Arg Val Ser Thr His Ser Gln Glu Met Asp Ser Gly Thr Glu Tyr Gly
865                 870                 875                 880

Met Gly Ser Ser Thr Lys Ala Ser Phe Thr Pro Phe Val Asp Pro Arg
                885                 890                 895

Val Tyr Gln Thr Ser Pro Thr Asp Glu Asp Glu Glu Asp Glu Glu Ser
            900                 905                 910

Ser Ala Ala Ala Leu Phe Thr Ser Glu Leu Leu Arg Gln Glu Gln Ala
        915                 920                 925

Lys Leu Asn Glu Ala Arg Lys Ile Ser Val Val Asn Val Asn Pro Thr
    930                 935                 940

Asn Ile Arg Pro His Ser Asp Thr Pro Glu Ile Arg Lys Tyr Lys Lys
945                 950                 955                 960

Arg Phe Asn Ser Glu Ile Leu Cys Ala Ala Leu Trp Gly Val Asn Leu

                965                 970                 975

Leu Val Gly Thr Glu Asn Gly Leu Met Leu Leu Asp Arg Ser Gly Gln
            980                 985                 990

Gly Lys Val Tyr Asn Leu Ile Asn Arg Arg Arg Phe Gln Gln Met Asp
        995                 1000                1005

Val Leu Glu Gly Leu Asn Val Leu Val Thr Ile Ser Gly Lys Lys
    1010                1015                1020

Asn Lys Leu Arg Val Tyr Tyr Leu Ser Trp Leu Arg Asn Arg Ile
    1025                1030                1035

Leu His Asn Asp Pro Glu Val Glu Lys Lys Gln Gly Trp Ile Thr
    1040                1045                1050

Val Gly Asp Leu Glu Gly Cys Ile His Tyr Lys Val Val Lys Tyr
    1055                1060                1065

Glu Arg Ile Lys Phe Leu Val Ile Ala Leu Lys Asn Ala Val Glu
    1070                1075                1080

Ile Tyr Ala Trp Ala Pro Lys Pro Tyr His Lys Phe Met Ala Phe
    1085                1090                1095

Lys Ser Phe Ala Asp Leu Gln His Lys Pro Leu Leu Val Asp Leu
    1100                1105                1110

Thr Val Glu Glu Gly Gln Arg Leu Lys Val Ile Phe Gly Ser His
    1115                1120                1125

Thr Gly Phe His Val Ile Asp Val Asp Ser Gly Asn Ser Tyr Asp
    1130                1135                1140

Ile Tyr Ile Pro Ser His Ile Gln Gly Asn Ile Thr Pro His Ala
    1145                1150                1155

Ile Val Ile Leu Pro Lys Thr Asp Gly Met Glu Met Leu Val Cys
    1160                1165                1170

Tyr Glu Asp Glu Gly Val Tyr Val Asn Thr Tyr Gly Arg Ile Thr
    1175                1180                1185

Lys Asp Val Val Leu Gln Trp Gly Glu Met Pro Thr Ser Val Ala
    1190                1195                1200

Tyr Ile His Ser Asn Gln Ile Met Gly Trp Gly Glu Lys Ala Ile
    1205                1210                1215

Glu Ile Arg Ser Val Glu Thr Gly His Leu Asp Gly Val Phe Met
    1220                1225                1230

His Lys Arg Ala Gln Arg Leu Lys Phe Leu Cys Glu Arg Asn Asp
    1235                1240                1245

Lys Val Phe Phe Ala Ser Val Arg Ser Gly Gly Ser Ser Gln Val
    1250                1255                1260

Phe Phe Met Thr Leu Asn Arg Asn Ser Met Met Asn Trp
    1265                1270                1275

<210> 16
<211> 1268
<212> PRT
<213> homo sapiens

<400> 16
   Met Ala Ser Asp Ser Pro Ala Arg Ser Leu Asp Glu Ile Asp Leu Ser
   1 5 10 15
   Ala Leu Arg Asp Pro Ala Gly Ile Phe Glu Leu Val Glu Leu Val Gly
   20 25 30
   Asn Gly Thr Tyr Gly Gln Val Tyr Lys Gly Arg His Val Lys Thr Gly
   35 40 45
   Gln Leu Ala Ala Ile Lys Val Met Asp Val Thr Gly Asp Glu Glu Glu
   50 55 60
   Glu Ile Lys Gln Glu Ile Asn Met Leu Lys Lys Tyr Ser His His Arg
   65 70 75 80
   Asn Ile Ala Thr Tyr Tyr Gly Ala Phe Ile Lys Lys Asn Pro Pro Gly
   85 90 95
   Met Asp Asp Gln Leu Trp Leu Val Met Glu Phe Cys Gly Ala Gly Ser
   100 105 110
   Val Thr Asp Leu Ile Lys Asn Thr Lys Gly Asn Thr Leu Lys Glu Glu
   115 120 125
   Trp Ile Ala Tyr Ile Cys Arg Glu Ile Leu Arg Gly Leu Ser His Leu
   130 135 140
   His Gln His Lys Val Ile His Arg Asp Ile Lys Gly Gln Asn Val Leu
   145 150 155 160
   Leu Thr Glu Asn Ala Glu Val Lys Leu Val Asp Phe Gly Val Ser Ala
   165 170 175
   Gln Leu Asp Arg Thr Val Gly Arg Arg Asn Thr Phe Ile Gly Thr Pro
   180 185 190
   Tyr Trp Met Ala Pro Glu Val Ile Ala Cys Asp Glu Asn Pro Asp Ala
   195 200 205
   Thr Tyr Asp Phe Lys Ser Asp Leu Trp Ser Leu Gly Ile Thr Ala Ile
   210 215 220
   Glu Met Ala Glu Gly Ala Pro Pro Leu Cys Asp Met His Pro Met Arg
   225 230 235 240
   Ala Leu Phe Leu Ile Pro Arg Asn Pro Ala Pro Arg Leu Lys Ser Lys
   245 250 255
   Lys Trp Ser Lys Lys Phe Gln Ser Phe Ile Glu Ser Cys Leu Val Lys
   260 265 270
   Asn His Ser Gln Arg Pro Ala Thr Glu Gln Leu Met Lys His Pro Phe
   275 280 285
   Ile Arg Asp Gln Pro Asn Glu Arg Gln Val Arg Ile Gln Leu Lys Asp
   290 295 300
   His Ile Asp Arg Thr Lys Lys Lys Arg Gly Glu Lys Asp Glu Thr Glu
   305 310 315 320
   Tyr Glu Tyr Ser Gly Ser Glu Glu Glu Glu Glu Glu Asn Asp Ser Gly
   325 330 335
   Glu Pro Ser Ser Ile Leu Asn Leu Pro Gly Glu Ser Thr Leu Arg Arg
   340 345 350
   Asp Phe Leu Arg Leu Gln Leu Ala Asn Lys Glu Arg Ser Glu Ala Leu
   355 360 365
   Arg Arg Gln Gln Leu Glu Gln Gln Gln Arg Glu Asn Glu Glu His Lys
   370 375 380
   Arg Gln Leu Leu Ala Glu Arg Gln Lys Arg Ile Glu Glu Gln Lys Glu
   385 390 395 400
   Gln Arg Arg Arg Leu Glu Glu Gln Gln Arg Arg Glu Lys Glu Leu Arg
   405 410 415
   Lys Gln Gln Glu Arg Glu Gln Arg Arg His Tyr Glu Glu Gln Met Arg
   420 425 430
   Arg Glu Glu Glu Arg Arg Arg Ala Glu His Glu Gln Glu Tyr Lys Arg
   435 440 445
   Lys Gln Leu Glu Glu Gln Arg Gln Ala Glu Arg Leu Gln Arg Gln Leu
   450 455 460
   Lys Gln Glu Arg Asp Tyr Leu Val Ser Leu Gln His Gln Arg Gln Glu
   465                 470                 475                 480
   Gln Arg Pro Val Glu Lys Lys Pro Leu Tyr His Tyr Lys Glu Gly Met
                   485                 490                 495
   Ser Pro Ser Glu Lys Pro Ala Trp Ala Lys Glu Ile Pro His Leu Val
               500                 505                 510
   Ala Val Lys Ser Gln Gly Pro Ala Leu Thr Ala Ser Gln Ser Val His
           515                 520                 525
   Glu Gln Pro Thr Lys Gly Leu Ser Gly Phe Gln Glu Ala Leu Asn Val
       530                 535                 540
   Thr Ser His Arg Val Glu Met Pro Arg Gln Asn Ser Asp Pro Thr Ser
   545                 550                 555                 560
   Glu Asn Pro Pro Leu Pro Thr Arg Ile Glu Lys Phe Asp Arg Ser Ser
                   565                 570                 575
   Trp Leu Arg Gln Glu Glu Asp Ile Pro Pro Lys Val Pro Gln Arg Thr
               580                 585                 590
   Thr Ser Ile Ser Pro Ala Leu Ala Arg Lys Asn Ser Pro Gly Asn Gly
           595                 600                 605
   Ser Ala Leu Gly Pro Arg Leu Gly Ser Gln Pro Ile Arg Ala Ser Asn
       610                 615                 620
   Pro Asp Leu Arg Arg Thr Glu Pro Ile Leu Glu Ser Pro Leu Gln Arg
   625                 630                 635                 640
   Thr Ser Ser Gly Ser Ser Ser Ser Ser Ser Thr Pro Ser Ser Gln Pro
                   645                 650                 655
   Ser Ser Gln Gly Gly Ser Gln Pro Gly Ser Gln Ala Gly Ser Ser Glu
               660                 665                 670
   Arg Thr Arg Val Arg Ala Asn Ser Lys Ser Glu Gly Ser Pro Val Leu
           675                 680                 685
   Pro His Glu Pro Ala Lys Val Lys Pro Glu Glu Ser Arg Asp Ile Thr
       690                 695                 700
   Arg Pro Ser Arg Pro Ala Asp Leu Thr Ala Leu Ala Lys Glu Leu Arg
   705                 710                 715                 720
   Glu Leu Arg Ile Glu Glu Thr Asn Arg Pro Met Lys Lys Val Thr Asp
                   725                 730                 735
   Tyr Ser Ser Ser Ser Glu Glu Ser Glu Ser Ser Glu Glu Glu Glu Glu
               740                 745                 750
   Asp Gly Glu Ser Glu Thr His Asp Gly Thr Val Ala Val Ser Asp Ile
           755                 760                 765
   Pro Arg Leu Ile Pro Thr Gly Ala Pro Gly Ser Asn Glu Gln Tyr Asn
       770                 775                 780
   Val Gly Met Val Gly Thr His Gly Leu Glu Thr Ser His Ala Asp Ser
   785                 790                 795                 800
   Phe Ser Gly Ser Ile Ser Arg Glu Gly Thr Leu Met Ile Arg Glu Thr
                   805                 810                 815
   Ser Gly Glu Lys Lys Arg Ser Gly His Ser Asp Ser Asn Gly Phe Ala
               820                 825                 830
   Gly His Ile Asn Leu Pro Asp Leu Val Gln Gln Ser His Ser Pro Ala
           835                 840                 845
   Gly Thr Pro Thr Glu Gly Leu Gly Arg Val Ser Thr His Ser Gln Glu
       850                 855                 860
   Met Asp Ser Gly Thr Glu Tyr Gly Met Gly Ser Ser Thr Lys Ala Ser
   865                 870                 875                 880
   Phe Thr Pro Phe Val Asp Pro Arg Val Tyr Gln Thr Ser Pro Thr Asp
                   885                 890                 895
   Glu Asp Glu Glu Asp Glu Glu Ser Ser Ala Ala Ala Leu Phe Thr Ser
               900                 905                 910
   Glu Leu Leu Arg Gln Glu Gln Ala Lys Leu Asn Glu Ala Arg Lys Ile
           915                 920                 925
   Ser Val Val Asn Val Asn Pro Thr Asn Ile Arg Pro His Ser Asp Thr
       930                 935                 940
   Pro Glu Ile Arg Lys Tyr Lys Lys Arg Phe Asn Ser Glu Ile Leu Cys
   945                 950                 955                 960
   Ala Ala Leu Trp Gly Val Asn Leu Leu Val Gly Thr Glu Asn Gly Leu
                   965                 970                 975
   Met Leu Leu Asp Arg Ser Gly Gln Gly Lys Val Tyr Asn Leu Ile Asn
               980                 985                 990
   Arg Arg Arg Phe Gln Gln Met Asp  Val Leu Glu Gly Leu  Asn Val Leu
           995                 1000                1005
   Val Thr  Ile Ser Gly Lys Lys  Asn Lys Leu Arg Val  Tyr Tyr Leu
       1010                1015                1020
   Ser Trp Leu Arg Asn Arg Ile Leu His Asn Asp Pro Glu Val Glu
       1025                1030                1035
   Lys Lys Gln Gly Trp Ile Thr Val Gly Asp Leu Glu Gly Cys Ile
       1040                1045                1050
   His Tyr Lys Val Val Lys Tyr Glu Arg Ile Lys Phe Leu Val Ile
       1055                1060                1065
   Ala Leu Lys Asn Ala Val Glu Ile Tyr Ala Trp Ala Pro Lys Pro
       1070                1075                1080
   Tyr His Lys Phe Met Ala Phe Lys Ser Phe Ala Asp Leu Gln His
       1085                1090                1095
   Lys Pro Leu Leu Val Asp Leu Thr Val Glu Glu Gly Gln Arg Leu
       1100                1105                1110
   Lys Val Ile Phe Gly Ser His Thr Gly Phe His Val Ile Asp Val
       1115                1120                1125
   Asp Ser Gly Asn Ser Tyr Asp Ile Tyr Ile Pro Ser His Ile Gln
       1130                1135                1140
   Gly Asn Ile Thr Pro His Ala Ile Val Ile Leu Pro Lys Thr Asp
       1145                1150                1155
   Gly Met Glu Met Leu Val Cys Tyr Glu Asp Glu Gly Val Tyr Val
       1160                1165                1170
   Asn Thr Tyr Gly Arg Ile Thr Lys Asp Val Val Leu Gln Trp Gly
       1175                1180                1185
   Glu Met Pro Thr Ser Val Ala Tyr Ile His Ser Asn Gln Ile Met
       1190                1195                1200
   Gly Trp Gly Glu Lys Ala Ile Glu Ile Arg Ser Val Glu Thr Gly
       1205                1210                1215
   His Leu Asp Gly Val Phe Met His Lys Arg Ala Gln Arg Leu Lys
       1220                1225                1230
   Phe Leu Cys Glu Arg Asn Asp Lys Val Phe Phe Ala Ser Val Arg
       1235                1240                1245
   Ser Gly Gly Ser Ser Gln Val Phe Phe Met Thr Leu Asn Arg Asn
       1250                1255                1260
   Ser Met Met Asn Trp
       1265

<210>  17
<211>  19
<212>  RNA
<213>  artificial

<220>
<223>  siRNA

<400>  17
gaacauacgg gcaaguuua                                                    19


<210>  18
<211>  19
<212>  RNA
<213>  artificial

<220>
<223>  siRNA

<400>  18
uaagcgagcu caaagguua                                                    19


<210>  19
<211>  19
<212>  RNA
<213>  artificial

<220>
<223>  siRNA

<400>  19
cgacauaccc agacugaua                                                    19


<210>  20
<211>  19
<212>  RNA
<213>  artificial

<220>
<223>  siRNA

<400>  20
gaccgaagcu cuugguuac                                                    19


<210>  21
<211>  1360
<212>  PRT
<213>  artificial

<220>
<223>  mutant TNIK

<400>  21
   Met Ala Ser Asp Ser Pro Ala Arg Ser Leu Asp Glu Ile Asp Leu Ser
   1               5                   10                  15
   Ala Leu Arg Asp Pro Ala Gly Ile Phe Glu Leu Val Glu Leu Val Gly
               20                  25                  30
   Asn Gly Thr Tyr Gly Gln Val Tyr Lys Gly Arg His Val Lys Thr Gly
           35                  40                  45
   Gln Leu Ala Ala Ile Lys Val Met Asp Val Thr Gly Asp Glu Glu Glu
       50                  55                  60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Asn Pro Pro Gly
                   85                  90                  95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Ala Ala Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
       290                 295                 300
   His Ile Asp Arg Thr Lys Lys Lys Arg Gly Glu Lys Asp Glu Thr Glu
   305                 310                 315                 320
   Tyr Glu Tyr Ser Gly Ser Glu Glu Glu Glu Glu Glu Asn Asp Ser Gly
                   325                 330                 335
   Glu Pro Ser Ser Ile Leu Asn Leu Pro Gly Glu Ser Thr Leu Arg Arg
               340                 345                 350
   Asp Phe Leu Arg Leu Gln Leu Ala Asn Lys Glu Arg Ser Glu Ala Leu
           355                 360                 365
   Arg Arg Gln Gln Leu Glu Gln Gln Gln Arg Glu Asn Glu Glu His Lys
       370                 375                 380
   Arg Gln Leu Leu Ala Glu Arg Gln Lys Arg Ile Glu Glu Gln Lys Glu
   385                 390                 395                 400
   Gln Arg Arg Arg Leu Glu Glu Gln Gln Arg Arg Glu Lys Glu Leu Arg
                   405                 410                 415
   Lys Gln Gln Glu Arg Glu Gln Arg Arg His Tyr Glu Glu Gln Met Arg
               420                 425                 430
   Arg Glu Glu Glu Arg Arg Arg Ala Glu His Glu Gln Glu Tyr Ile Arg
           435                 440                 445
   Arg Gln Leu Glu Glu Glu Gln Arg Gln Leu Glu Ile Leu Gln Gln Gln
       450                 455                 460
   Leu Leu His Glu Gln Ala Leu Leu Leu Glu Tyr Lys Arg Lys Gln Leu
   465                 470                 475                 480
   Glu Glu Gln Arg Gln Ala Glu Arg Leu Gln Arg Gln Leu Lys Gln Glu
                   485                 490                 495
   Arg Asp Tyr Leu Val Ser Leu Gln His Gln Arg Gln Glu Gln Arg Pro
               500                 505                 510
   Val Glu Lys Lys Pro Leu Tyr His Tyr Lys Glu Gly Met Ser Pro Ser
           515                 520                 525
   Glu Lys Pro Ala Trp Ala Lys Glu Val Glu Glu Arg Ser Arg Leu Asn
       530                 535                 540
   Arg Gln Ser Ser Pro Ala Met Pro His Lys Val Ala Asn Arg Ile Ser
   545                 550                 555                 560
   Asp Pro Asn Leu Pro Pro Arg Ser Glu Ser Phe Ser Ile Ser Gly Val
                   565                 570                 575
   Gln Pro Ala Arg Thr Pro Pro Met Leu Arg Pro Val Asp Pro Gln Ile
               580                 585                 590
   Pro His Leu Val Ala Val Lys Ser Gln Gly Pro Ala Leu Thr Ala Ser
   595 600 605
   Gln Ser Val His Glu Gln Pro Thr Lys Gly Leu Ser Gly Phe Gln Glu
   610 615 620
   Ala Leu Asn Val Thr Ser His Arg Val Glu Met Pro Arg Gln Asn Ser
   625 630 635 640
   Asp Pro Thr Ser Glu Asn Pro Pro Leu Pro Thr Arg Ile Glu Lys Phe
   645 650 655
   Asp Arg Ser Ser Trp Leu Arg Gln Glu Glu Asp Ile Pro Pro Lys Val
   660 665 670
   Pro Gln Arg Thr Thr Ser Ile Ser Pro Ala Leu Ala Arg Lys Asn Ser
   675 680 685
   Pro Gly Asn Gly Ser Ala Leu Gly Pro Arg Leu Gly Ser Gln Pro Ile
   690 695 700
   Arg Ala Ser Asn Pro Asp Leu Arg Arg Thr Glu Pro Ile Leu Glu Ser
   705 710 715 720
   Pro Leu Gln Arg Thr Ser Ser Gly Ser Ser Ser Ser Ser Ser Thr Pro
   725 730 735
   Ser Ser Gln Pro Ser Ser Gln Gly Gly Ser Gln Pro Gly Ser Gln Ala
   740 745 750
   Gly Ser Ser Glu Arg Thr Arg Val Arg Ala Asn Ser Lys Ser Glu Gly
   755 760 765
   Ser Pro Val Leu Pro His Glu Pro Ala Lys Val Lys Pro Glu Glu Ser
   770 775 780
   Arg Asp Ile Thr Arg Pro Ser Arg Pro Ala Ser Tyr Lys Lys Ala Ile
   785 790 795 800
   Asp Glu Asp Leu Thr Ala Leu Ala Lys Glu Leu Arg Glu Leu Arg Ile
   805 810 815
   Glu Glu Thr Asn Arg Pro Met Lys Lys Val Thr Asp Tyr Ser Ser Ser
   820 825 830
   Ser Glu Glu Ser Glu Ser Ser Glu Glu Glu Glu Glu Asp Gly Glu Ser
   835 840 845
   Glu Thr His Asp Gly Thr Val Ala Val Ser Asp Ile Pro Arg Leu Ile
   850 855 860
   Pro Thr Gly Ala Pro Gly Ser Asn Glu Gln Tyr Asn Val Gly Met Val
   865 870 875 880
   Gly Thr His Gly Leu Glu Thr Ser His Ala Asp Ser Phe Ser Gly Ser
                   885                 890                 895
   Ile Ser Arg Glu Gly Thr Leu Met Ile Arg Glu Thr Ser Gly Glu Lys
               900                 905                 910
   Lys Arg Ser Gly His Ser Asp Ser Asn Gly Phe Ala Gly His Ile Asn
           915                 920                 925
   Leu Pro Asp Leu Val Gln Gln Ser His Ser Pro Ala Gly Thr Pro Thr
       930                 935                 940
   Glu Gly Leu Gly Arg Val Ser Thr His Ser Gln Glu Met Asp Ser Gly
   945                 950                 955                 960
   Thr Glu Tyr Gly Met Gly Ser Ser Thr Lys Ala Ser Phe Thr Pro Phe
                   965                 970                 975
   Val Asp Pro Arg Val Tyr Gln Thr Ser Pro Thr Asp Glu Asp Glu Glu
               980                 985                 990
   Asp Glu Glu Ser Ser Ala Ala Ala Leu Phe Thr Ser Glu Leu Leu Arg
           995                 1000                1005
   Gln Glu Gln Ala Lys Leu Asn Glu Ala Arg Lys Ile Ser Val Val
       1010                1015                1020
   Asn Val Asn Pro Thr Asn Ile Arg Pro His Ser Asp Thr Pro Glu
       1025                1030                1035
   Ile Arg Lys Tyr Lys Lys Arg Phe Asn Ser Glu Ile Leu Cys Ala
       1040                1045                1050
   Ala Leu Trp Gly Val Asn Leu Leu Val Gly Thr Glu Asn Gly Leu
       1055                1060                1065
   Met Leu Leu Asp Arg Ser Gly Gln Gly Lys Val Tyr Asn Leu Ile
       1070                1075                1080
   Asn Arg Arg Arg Phe Gln Gln Met Asp Val Leu Glu Gly Leu Asn
       1085                1090                1095
   Val Leu Val Thr Ile Ser Gly Lys Lys Asn Lys Leu Arg Val Tyr
       1100                1105                1110
   Tyr Leu Ser Trp Leu Arg Asn Arg Ile Leu His Asn Asp Pro Glu
       1115                1120                1125
   Val Glu Lys Lys Gln Gly Trp Ile Thr Val Gly Asp Leu Glu Gly
       1130                1135                1140
   Cys Ile His Tyr Lys Val Val Lys Tyr Glu Arg Ile Lys Phe Leu
       1145                1150                1155
   Val Ile Ala Leu Lys Asn Ala Val Glu Ile Tyr Ala Trp Ala Pro
       1160                1165                1170
   Lys Pro Tyr His Lys Phe Met Ala Phe Lys Ser Phe Ala Asp Leu
       1175                1180                1185
   Gln His Lys Pro Leu Leu Val Asp Leu Thr Val Glu Glu Gly Gln
       1190                1195                1200
   Arg Leu Lys Val Ile Phe Gly Ser His Thr Gly Phe His Val Ile
       1205                1210                1215
   Asp Val Asp Ser Gly Asn Ser Tyr Asp Ile Tyr Ile Pro Ser His
       1220                1225                1230
   Ile Gln Gly Asn Ile Thr Pro His Ala Ile Val Ile Leu Pro Lys
       1235                1240                1245
   Thr Asp Gly Met Glu Met Leu Val Cys Tyr Glu Asp Glu Gly Val
       1250                1255                1260
   Tyr Val Asn Thr Tyr Gly Arg Ile Thr Lys Asp Val Val Leu Gln
       1265                1270                1275
   Trp Gly Glu Met Pro Thr Ser Val Ala Tyr Ile His Ser Asn Gln
       1280                1285                1290
   Ile Met Gly Trp Gly Glu Lys Ala Ile Glu Ile Arg Ser Val Glu
       1295                1300                1305
   Thr Gly His Leu Asp Gly Val Phe Met His Lys Arg Ala Gln Arg
       1310                1315                1320
   Leu Lys Phe Leu Cys Glu Arg Asn Asp Lys Val Phe Phe Ala Ser
       1325                1330                1335
   Val Arg Ser Gly Gly Ser Ser Gln Val Phe Phe Met Thr Leu Asn
       1340                1345                1350
   Arg Asn Ser Met Met Asn Trp
       1355                1360

<210>  22
<211>  4083
<212>  DNA
<213>  artificial

<220>
<223>  mutant TNIK

<400> 22
atggcgagcg actccccggc tcgaagcctg gatgaaatag atctctcggc tctgagggac 60

cctgcaggga tctttgaatt ggtggaactt gttggaaatg gaacatacgg gcaagtttat 120

aagggtcgtc atgtcaaaac gggccagctt gcagccatca aggttatgga tgtcacaggg 180

gatgaagagg aagaaatcaa acaagaaatt aacatgttga agaaatattc tcatcaccgg 240

aatattgcta catactatgg tgcttttatc aaaaagaacc caccaggcat ggatgaccaa 300

ctttggttgg tgatggagtt ttgtggtgct ggctctgtca ccgacctgat caagaacaca 360

aaaggtaaca cgttgaaaga ggagtggatt gcatacatct gcagggaaat cttacggggg 420

ctgagtcacc tgcaccagca taaagtgatt catgcagcta ttaaagggca aaatgtcttg 480

ctgactgaaa atgcagaagt taaactagtg gactttggag tcagtgctca gcttgatcga 540

acagtgggca ggaggaatac tttcattgga actccctact ggatggcacc agaagttatt 600

gcctgtgatg aaaacccaga tgccacatat gatttcaaga gtgacttgtg gtctttgggt 660

atcaccgcca ttgaaatggc agaaggtgct ccccctctct gtgacatgca ccccatgaga 720

gctctcttcc tcatcccccg gaatccagcg cctcggctga agtctaagaa gtggtcaaaa 780

aaattccagt catttattga gagctgcttg gtaaagaatc acagccagcg accagcaaca 840

gaacaattga tgaagcatcc atttatacga gaccaaccta atgagcgaca ggtccgcatt 900

caactcaagg accatattga tagaacaaag aagaagcgag gagaaaaaga tgagacagag 960

tatgagtaca gtggaagtga ggaagaagag gaggagaatg actcaggaga gcccagctcc 1020

atcctgaatc tgccagggga gtcgacgctg cggagggact ttctgaggct gcagctggcc 1080

aacaaggagc gttctgaggc cctacggagg cagcagctgg agcagcagca gcgggagaat 1140

gaggagcaca agcggcagct gctggccgag cgtcagaagc gcatcgagga gcagaaagag 1200

cagaggcggc ggctggagga gcaacaaagg cgagagaagg agctgcggaa gcagcaggag 1260

agggagcagc gccggcacta tgaggagcag atgcgccggg aggaggagag gaggcgtgcg 1320

gagcatgaac aggaatacat caggcgacag ttagaggagg agcagagaca gttagagatc 1380

ttgcagcagc agctactgca tgaacaagct ctacttctgg aatataagcg caaacaattg 1440

gaagaacaga gacaagcaga aagactgcag aggcagctaa agcaagaaag agactactta 1500

gtttcccttc agcatcagcg gcaggagcag aggcctgtgg agaagaagcc actgtaccat 1560

tacaaagaag gaatgagtcc tagtgagaag ccagcatggg ccaaggaggt agaagaacgg 1620

tcaaggctca accggcaaag ttcccctgcc atgcctcaca aggttgccaa caggatatct 1680

gaccccaacc tgcccccaag gtcggagtcc ttcagcatta gtggagttca gcctgctcga 1740

acacccccca tgctcagacc agtcgatccc cagatcccac atctggtagc tgtaaaatcc 1800

cagggacctg ccttgaccgc ctcccagtca gtgcacgagc agcccacaaa gggcctctct 1860

gggtttcagg aggctctgaa cgtgacctcc caccgcgtgg agatgccacg ccagaactca 1920

gatcccacct cggaaaatcc tcctctcccc actcgcattg aaaagtttga ccgaagctct 1980

tggttacgac aggaagaaga cattccacca aaggtgcctc aaagaacaac ttctatatcc 2040

ccagcattag ccagaaagaa ttctcctggg aatggtagtg ctctgggacc cagactagga 2100
tctcaaccca tcagagcaag caaccctgat ctccggagaa ctgagcccat cttggagagc 2160
cccttgcaga ggaccagcag tggcagttcc tccagctcca gcacccctag ctcccagccc 2220
agctcccaag gaggctccca gcctggatca caagcaggat ccagtgaacg caccagagtt 2280
cgagccaaca gtaagtcaga aggatcacct gtgcttcccc atgagcctgc caaggtgaaa 2340
ccagaagaat ccagggacat tacccggccc agtcgaccag ctagctacaa aaaagctata 2400
gatgaggatc tgacggcatt agccaaagaa ctaagagaac tccggattga agaaacaaac 2460
cgcccaatga agaaggtgac tgattactcc tcctccagtg aggagtcaga aagtagcgag 2520
gaagaggagg aagatggaga gagcgagacc catgatggga cagtggctgt cagcgacata 2580
cccagactga taccaacagg agctccaggc agcaacgagc agtacaatgt gggaatggtg 2640
gggacgcatg ggctggagac ctctcatgcg gacagtttca gcggcagtat ttcaagagaa 2700
ggaaccttga tgattagaga gacgtctgga gagaagaagc gatctggcca cagtgacagc 2760
aatggctttg ctggccacat caacctccct gacctggtgc agcagagcca ttctccagct 2820
ggaaccccga ctgagggact ggggcgcgtc tcaacccatt cccaggagat ggactctggg 2880
actgaatatg gcatggggag cagcaccaaa gcctccttca ccccctttgt ggaccccaga 2940
gtataccaga cgtctcccac tgatgaagat gaagaggatg aggaatcatc agccgcagct 3000
ctgtttacta gcgaacttct taggcaagaa caggccaaac tcaatgaagc aagaaagatt 3060
tcggtggtaa atgtaaaccc aaccaacatt cggcctcata gcgacacacc agaaatcaga 3120
aaatacaaga aacgattcaa ctcagaaata ctttgtgcag ctctgtgggg tgtaaacctt 3180
ctggtgggga ctgaaaatgg cctgatgctt ttggaccgaa gtgggcaagg caaagtctat 3240
aatctgatca accggaggcg atttcagcag atggatgtgc tagagggact gaatgtcctt 3300
gtgacaattt caggaaagaa gaataagcta cgagtttact atctttcatg gttaagaaac 3360
agaatactac ataatgaccc agaagtagaa aagaaacaag gctggatcac tgttggggac 3420
ttggaaggct gtatacatta taaagttgtt aaatatgaaa ggatcaaatt tttggtgatt 3480
gccttaaaga atgctgtgga aatatatgct tgggctccta aaccgtatca taaattcatg 3540
gcatttaagt cttttgcaga tctccagcac aagcctctgc tagttgatct cacggtagaa 3600
gaaggtcaaa gattaaaggt tatttttggt tcacacactg gtttccatgt aattgatgtt 3660
gattcaggaa actcttatga tatctacata ccatctcata ttcagggcaa tatcactcct 3720
catgctattg tcatcttgcc taaaacagat ggaatggaaa tgcttgtttg ctatgaggat 3780
gagggggtgt atgtaaacac ctatggccgg ataactaagg atgtggtgct ccaatgggga 3840
gaaatgccca cgtctgtggc ctacattcat tccaatcaga taatgggctg gggcgagaaa 3900
gctattgaga tccggtcagt ggaaacagga catttggatg gagtatttat gcataagcga 3960
gctcaaaggt taaagtttct atgtgaaaga aatgataagg tattttttgc atccgtgcga 4020
tctggaggaa gtagccaagt gtttttcatg accctcaaca gaaattccat gatgaactgg 4080
taa 4083

<210>  23
<211>  1360
<212>  PRT
<213>  artificial

<220>
<223>  mutant TNIK

<400>  23
   Met Ala Ser Asp Ser Pro Ala Arg Ser Leu Asp Glu Ile Asp Leu Ser
   1               5                   10                  15
   Ala Leu Arg Asp Pro Ala Gly Ile Phe Glu Leu Val Glu Leu Val Gly
               20                  25                  30
   Asn Gly Thr Tyr Gly Gln Val Tyr Lys Gly Arg His Val Lys Thr Gly
           35                  40                  45
   Gln Leu Ala Ala Ile Ala Val Met Asp Val Thr Gly Asp Glu Glu Glu
       50                  55                  60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Asn Pro Pro Gly
                   85                  90                  95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
       290                 295                 300
   His Ile Asp Arg Thr Lys Lys Lys Arg Gly Glu Lys Asp Glu Thr Glu
   305                 310                 315                 320
   Tyr Glu Tyr Ser Gly Ser Glu Glu Glu Glu Glu Glu Asn Asp Ser Gly
                   325                 330                 335
   Glu Pro Ser Ser Ile Leu Asn Leu Pro Gly Glu Ser Thr Leu Arg Arg
               340                 345                 350
   Asp Phe Leu Arg Leu Gln Leu Ala Asn Lys Glu Arg Ser Glu Ala Leu
           355                 360                 365
   Arg Arg Gln Gln Leu Glu Gln Gln Gln Arg Glu Asn Glu Glu His Lys
       370                 375                 380
   Arg Gln Leu Leu Ala Glu Arg Gln Lys Arg Ile Glu Glu Gln Lys Glu
   385                 390                 395                 400
   Gln Arg Arg Arg Leu Glu Glu Gln Gln Arg Arg Glu Lys Glu Leu Arg
                   405                 410                 415
   Lys Gln Gln Glu Arg Glu Gln Arg Arg His Tyr Glu Glu Gln Met Arg
               420                 425                 430
   Arg Glu Glu Glu Arg Arg Arg Ala Glu His Glu Gln Glu Tyr Ile Arg
           435                 440                 445
   Arg Gln Leu Glu Glu Glu Gln Arg Gln Leu Glu Ile Leu Gln Gln Gln
       450                 455                 460
   Leu Leu His Glu Gln Ala Leu Leu Leu Glu Tyr Lys Arg Lys Gln Leu
   465                 470                 475                 480
   Glu Glu Gln Arg Gln Ala Glu Arg Leu Gln Arg Gln Leu Lys Gln Glu
                   485                 490                 495
   Arg Asp Tyr Leu Val Ser Leu Gln His Gln Arg Gln Glu Gln Arg Pro
               500                 505                 510
   Val Glu Lys Lys Pro Leu Tyr His Tyr Lys Glu Gly Met Ser Pro Ser
           515                 520                 525
   Glu Lys Pro Ala Trp Ala Lys Glu Val Glu Glu Arg Ser Arg Leu Asn
       530                 535                 540
   Arg Gln Ser Ser Pro Ala Met Pro His Lys Val Ala Asn Arg Ile Ser
   545                 550                 555                 560
   Asp Pro Asn Leu Pro Pro Arg Ser Glu Ser Phe Ser Ile Ser Gly Val
                   565                 570                 575
   Gln Pro Ala Arg Thr Pro Pro Met Leu Arg Pro Val Asp Pro Gln Ile
               580                 585                 590
   Pro His Leu Val Ala Val Lys Ser Gln Gly Pro Ala Leu Thr Ala Ser
           595                 600                 605
   Gln Ser Val His Glu Gln Pro Thr Lys Gly Leu Ser Gly Phe Gln Glu
       610                 615                 620
   Ala Leu Asn Val Thr Ser His Arg Val Glu Met Pro Arg Gln Asn Ser
   625                 630                 635                 640
   Asp Pro Thr Ser Glu Asn Pro Pro Leu Pro Thr Arg Ile Glu Lys Phe
                   645                 650                 655
   Asp Arg Ser Ser Trp Leu Arg Gln Glu Glu Asp Ile Pro Pro Lys Val
               660                 665                 670
   Pro Gln Arg Thr Thr Ser Ile Ser Pro Ala Leu Ala Arg Lys Asn Ser
           675                 680                 685
   Pro Gly Asn Gly Ser Ala Leu Gly Pro Arg Leu Gly Ser Gln Pro Ile
       690                 695                 700
   Arg Ala Ser Asn Pro Asp Leu Arg Arg Thr Glu Pro Ile Leu Glu Ser
   705                 710                 715                 720
   Pro Leu Gln Arg Thr Ser Ser Gly Ser Ser Ser Ser Ser Ser Thr Pro
                   725                 730                 735
   Ser Ser Gln Pro Ser Ser Gln Gly Gly Ser Gln Pro Gly Ser Gln Ala
               740                 745                 750
   Gly Ser Ser Glu Arg Thr Arg Val Arg Ala Asn Ser Lys Ser Glu Gly
           755                 760                 765
   Ser Pro Val Leu Pro His Glu Pro Ala Lys Val Lys Pro Glu Glu Ser
       770                 775                 780
   Arg Asp Ile Thr Arg Pro Ser Arg Pro Ala Ser Tyr Lys Lys Ala Ile
   785                 790                 795                 800
   Asp Glu Asp Leu Thr Ala Leu Ala Lys Glu Leu Arg Glu Leu Arg Ile
                   805                 810                 815
   Glu Glu Thr Asn Arg Pro Met Lys Lys Val Thr Asp Tyr Ser Ser Ser
               820                 825                 830
   Ser Glu Glu Ser Glu Ser Ser Glu Glu Glu Glu Glu Asp Gly Glu Ser
           835                 840                 845
   Glu Thr His Asp Gly Thr Val Ala Val Ser Asp Ile Pro Arg Leu Ile
       850                 855                 860
   Pro Thr Gly Ala Pro Gly Ser Asn Glu Gln Tyr Asn Val Gly Met Val
   865                 870                 875                 880
   Gly Thr His Gly Leu Glu Thr Ser His Ala Asp Ser Phe Ser Gly Ser
                   885                 890                 895
   Ile Ser Arg Glu Gly Thr Leu Met Ile Arg Glu Thr Ser Gly Glu Lys
               900                 905                 910
   Lys Arg Ser Gly His Ser Asp Ser Asn Gly Phe Ala Gly His Ile Asn
           915                 920                 925
   Leu Pro Asp Leu Val Gln Gln Ser His Ser Pro Ala Gly Thr Pro Thr
       930                 935                 940
   Glu Gly Leu Gly Arg Val Ser Thr His Ser Gln Glu Met Asp Ser Gly
   945                 950                 955                 960
   Thr Glu Tyr Gly Met Gly Ser Ser Thr Lys Ala Ser Phe Thr Pro Phe
                   965                 970                 975
   Val Asp Pro Arg Val Tyr Gln Thr Ser Pro Thr Asp Glu Asp Glu Glu
               980                 985                 990
   Asp Glu Glu Ser Ser Ala Ala Ala  Leu Phe Thr Ser Glu  Leu Leu Arg
           995                 1000                1005
   Gln Glu  Gln Ala Lys Leu Asn  Glu Ala Arg Lys Ile  Ser Val Val
       1010                1015                1020
   Asn Val  Asn Pro Thr Asn Ile  Arg Pro His Ser Asp  Thr Pro Glu
       1025                1030                1035
   Ile Arg  Lys Tyr Lys Lys Arg  Phe Asn Ser Glu Ile  Leu Cys Ala
       1040                1045                1050
   Ala Leu Trp Gly Val Asn Leu Leu Val Gly Thr Glu Asn Gly Leu
   1055 1060 1065
   Met Leu Leu Asp Arg Ser Gly Gln Gly Lys Val Tyr Asn Leu Ile
   1070 1075 1080
   Asn Arg Arg Arg Phe Gln Gln Met Asp Val Leu Glu Gly Leu Asn
   1085 1090 1095
   Val Leu Val Thr Ile Ser Gly Lys Lys Asn Lys Leu Arg Val Tyr
   1100 1105 1110
   Tyr Leu Ser Trp Leu Arg Asn Arg Ile Leu His Asn Asp Pro Glu
   1115 1120 1125
   Val Glu Lys Lys Gln Gly Trp Ile Thr Val Gly Asp Leu Glu Gly
   1130 1135 1140
   Cys Ile His Tyr Lys Val Val Lys Tyr Glu Arg Ile Lys Phe Leu
   1145 1150 1155
   Val Ile Ala Leu Lys Asn Ala Val Glu Ile Tyr Ala Trp Ala Pro
   1160 1165 1170
   Lys Pro Tyr His Lys Phe Met Ala Phe Lys Ser Phe Ala Asp Leu
   1175 1180 1185
   Gln His Lys Pro Leu Leu Val Asp Leu Thr Val Glu Glu Gly Gln
   1190 1195 1200
   Arg Leu Lys Val Ile Phe Gly Ser His Thr Gly Phe His Val Ile
   1205 1210 1215
   Asp Val Asp Ser Gly Asn Ser Tyr Asp Ile Tyr Ile Pro Ser His
   1220 1225 1230
   Ile Gln Gly Asn Ile Thr Pro His Ala Ile Val Ile Leu Pro Lys
   1235 1240 1245
   Thr Asp Gly Met Glu Met Leu Val Cys Tyr Glu Asp Glu Gly Val
   1250 1255 1260
   Tyr Val Asn Thr Tyr Gly Arg Ile Thr Lys Asp Val Val Leu Gln
   1265 1270 1275
   Trp Gly Glu Met Pro Thr Ser Val Ala Tyr Ile His Ser Asn Gln
   1280 1285 1290
   Ile Met Gly Trp Gly Glu Lys Ala Ile Glu Ile Arg Ser Val Glu
   1295 1300 1305
   Thr Gly His Leu Asp Gly Val Phe Met His Lys Arg Ala Gln Arg
   1310                    1315                    1320
   Leu Lys  Phe Leu Cys Glu Arg  Asn Asp Lys Val Phe  Phe Ala Ser
       1325                    1330                    1335
   Val Arg  Ser Gly Gly Ser Ser  Gln Val Phe Phe Met  Thr Leu Asn
       1340                    1345                    1350
   Arg Asn  Ser Met Met Asn Trp
       1355                    1360

<210>  24
<211>  4083
<212>  DNA
<213>  artificial

<220>
<223>  mutant TNIK

<400>  24
atggcgagcg actccccggc tcgaagcctg gatgaaatag atctctcggc tctgagggac      60
cctgcaggga tctttgaatt ggtggaactt gttggaaatg gaacatacgg gcaagtttat     120
aagggtcgtc atgtcaaaac gggccagctt gcagccatcg cggttatgga tgtcacaggg     180
gatgaagagg aagaaatcaa acaagaaatt aacatgttga agaaatattc tcatcaccgg     240
aatattgcta catactatgg tgcttttatc aaaaagaacc caccaggcat ggatgaccaa     300
ctttggttgg tgatggagtt ttgtggtgct ggctctgtca ccgacctgat caagaacaca     360
aaaggtaaca cgttgaaaga ggagtggatt gcatacatct gcagggaaat cttacggggg     420
ctgagtcacc tgcaccagca taaagtgatt catcgagata ttaaagggca aaatgtcttg     480
ctgactgaaa atgcagaagt taaactagtg gactttggag tcagtgctca gcttgatcga     540
acagtgggca ggaggaatac tttcattgga actccctact ggatggcacc agaagttatt     600
gcctgtgatg aaaacccaga tgccacatat gatttcaaga gtgacttgtg gtctttgggt     660
atcaccgcca ttgaaatggc agaaggtgct ccccctctct gtgacatgca ccccatgaga     720
gctctcttcc tcatcccccg gaatccagcg cctcggctga agtctaagaa gtggtcaaaa     780
aaattccagt catttattga gagctgcttg gtaaagaatc acagccagcg accagcaaca     840
gaacaattga tgaagcatcc atttatacga gaccaaccta atgagcgaca ggtccgcatt     900
caactcaagg accatattga tagaacaaag aagaagcgag gagaaaaaga tgagacagag     960
tatgagtaca gtggaagtga ggaagaagag gaggagaatg actcaggaga gcccagctcc    1020
atcctgaatc tgccagggga gtcgacgctg cggagggact ttctgaggct gcagctggcc    1080
aacaaggagc gttctgaggc cctacggagg cagcagctgg agcagcagca gcgggagaat    1140
gaggagcaca agcggcagct gctggccgag cgtcagaagc gcatcgagga gcagaaagag    1200
cagaggcggc ggctggagga gcaacaaagg cgagagaagg agctgcggaa gcagcaggag    1260
agggagcagc gccggcacta tgaggagcag atgcgccggg aggaggagag gaggcgtgcg    1320
gagcatgaac aggaatacat caggcgacag ttagaggagg agcagagaca gttagagatc    1380

ttgcagcagc agctactgca tgaacaagct ctacttctgg aatataagcg caaacaattg 1440
gaagaacaga gacaagcaga aagactgcag aggcagctaa agcaagaaag agactactta 1500
gtttcccttc agcatcagcg gcaggagcag aggcctgtgg agaagaagcc actgtaccat 1560
tacaaagaag gaatgagtcc tagtgagaag ccagcatggg ccaaggaggt agaagaacgg 1620
tcaaggctca accggcaaag ttcccctgcc atgcctcaca aggttgccaa caggatatct 1680
gaccccaacc tgcccccaag gtcggagtcc ttcagcatta gtggagttca gcctgctcga 1740
acacccccca tgctcagacc agtcgatccc cagatcccac atctggtagc tgtaaaatcc 1800
cagggacctg ccttgaccgc ctcccagtca gtgcacgagc agcccacaaa gggcctctct 1860
gggtttcagg aggctctgaa cgtgacctcc caccgcgtgg agatgccacg ccagaactca 1920
gatcccacct cggaaaatcc tcctctcccc actcgcattg aaaagtttga ccgaagctct 1980
tggttacgac aggaagaaga cattccacca aaggtgcctc aaagaacaac ttctatatcc 2040
ccagcattag ccagaaagaa ttctcctggg aatggtagtg ctctgggacc cagactagga 2100
tctcaaccca tcagagcaag caaccctgat ctccggagaa ctgagcccat cttggagagc 2160
cccttgcaga ggaccagcag tggcagttcc tccagctcca gcacccctag ctcccagccc 2220
agctcccaag gaggctccca gcctggatca caagcaggat ccagtgaacg caccagagtt 2280
cgagccaaca gtaagtcaga aggatcacct gtgcttcccc atgagcctgc caaggtgaaa 2340
ccagaagaat ccagggacat tacccggccc agtcgaccag ctagctacaa aaaagctata 2400
gatgaggatc tgacggcatt agccaaagaa ctaagagaac tccggattga agaaacaaac 2460
cgcccaatga agaaggtgac tgattactcc tcctccagtg aggagtcaga aagtagcgag 2520
gaagaggagg aagatggaga gagcgagacc catgatggga cagtggctgt cagcgacata 2580
cccagactga taccaacagg agctccaggc agcaacgagc agtacaatgt gggaatggtg 2640
gggacgcatg ggctggagac ctctcatgcg gacagtttca gcggcagtat ttcaagagaa 2700
ggaaccttga tgattagaga gacgtctgga gagaagaagc gatctggcca cagtgacagc 2760
aatggctttg ctggccacat caacctccct gacctggtgc agcagagcca ttctccagct 2820
ggaaccccga ctgagggact ggggcgcgtc tcaacccatt cccaggagat ggactctggg 2880
actgaatatg gcatggggag cagcaccaaa gcctccttca ccccctttgt ggaccccaga 2940
gtataccaga cgtctcccac tgatgaagat gaagaggatg aggaatcatc agccgcagct 3000
ctgtttacta gcgaacttct taggcaagaa caggccaaac tcaatgaagc aagaaagatt 3060
tcggtggtaa atgtaaaccc aaccaacatt cggcctcata gcgacacacc agaaatcaga 3120
aaatacaaga aacgattcaa ctcagaaata ctttgtgcag ctctgtgggg tgtaaacctt 3180
ctggtgggga ctgaaaatgg cctgatgctt ttggaccgaa gtgggcaagg caaagtctat 3240
aatctgatca accggaggcg atttcagcag atggatgtgc tagagggact gaatgtcctt 3300
gtgacaattt caggaaagaa gaataagcta cgagtttact atctttcatg gttaagaaac 3360
agaatactac ataatgaccc agaagtagaa aagaaacaag gctggatcac tgttggggac 3420

ttggaaggct gtatacatta taaagttgtt aaatatgaaa ggatcaaatt tttggtgatt 3480

gccttaaaga atgctgtgga aatatatgct tgggctccta aaccgtatca taaattcatg 3540

gcatttaagt cttttgcaga tctccagcac aagcctctgc tagttgatct cacggtagaa 3600

gaaggtcaaa gattaaaggt tatttttggt tcacacactg gtttccatgt aattgatgtt 3660

gattcaggaa actcttatga tatctacata ccatctcata ttcagggcaa tatcactcct 3720

catgctattg tcatcttgcc taaaacagat ggaatggaaa tgcttgtttg ctatgaggat 3780

gagggggtgt atgtaaacac ctatggccgg ataactaagg atgtggtgct ccaatgggga 3840

gaaatgccca cgtctgtggc ctacattcat tccaatcaga taatgggctg gggcgagaaa 3900

gctattgaga tccggtcagt ggaaacagga catttggatg gagtatttat gcataagcga 3960

gctcaaaggt taaagtttct atgtgaaaga aatgataagg tattttttgc atccgtgcga 4020

tctggaggaa gtagccaagt gtttttcatg accctcaaca gaaattccat gatgaactgg 4080

taa 4083

<210> 25
<211> 1360
<212> PRT
<213> artificial

<220>
<223> mutant TNIK

<400> 25
   Met Ala Ser Asp Ser Pro Ala Arg Ser Leu Asp Glu Ile Asp Leu Ser
   1 5 10 15
   Ala Leu Arg Asp Pro Ala Gly Ile Phe Glu Leu Val Glu Leu Val Gly
   20 25 30
   Asn Gly Thr Tyr Gly Gln Val Tyr Lys Gly Arg His Val Lys Thr Gly
   35 40 45
   Gln Leu Ala Ala Ile Lys Val Met Asp Val Thr Gly Asp Glu Glu Glu
   50 55 60
   Glu Ile Lys Gln Glu Ile Asn Met Leu Lys Lys Tyr Ser His His Arg
   65 70 75 80
   Asn Ile Ala Thr Tyr Tyr Gly Ala Phe Ile Lys Lys Asn Pro Pro Gly
   85 90 95
   Met Asp Asp Gln Leu Trp Leu Val Met Glu Phe Cys Gly Ala Gly Ser
   100 105 110
   Val Thr Asp Leu Ile Lys Asn Thr Lys Gly Asn Thr Leu Lys Glu Glu
   115 120 125
   Trp Ile Ala Tyr Ile Cys Arg Glu Ile Leu Arg Gly Leu Ser His Leu
   130 135 140
   His Gln His Lys Val Ile His Arg Asp Ile Lys Gly Gln Asn Val Leu
   145                 150                 155                 160
   Leu Thr Glu Asn Ala Glu Val Lys Leu Val Ala Ala Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
       290                 295                 300
   His Ile Asp Arg Thr Lys Lys Lys Arg Gly Glu Lys Asp Glu Thr Glu
   305                 310                 315                 320
   Tyr Glu Tyr Ser Gly Ser Glu Glu Glu Glu Glu Glu Asn Asp Ser Gly
                   325                 330                 335
   Glu Pro Ser Ser Ile Leu Asn Leu Pro Gly Glu Ser Thr Leu Arg Arg
               340                 345                 350
   Asp Phe Leu Arg Leu Gln Leu Ala Asn Lys Glu Arg Ser Glu Ala Leu
           355                 360                 365
   Arg Arg Gln Gln Leu Glu Gln Gln Gln Arg Glu Asn Glu Glu His Lys
       370                 375                 380
   Arg Gln Leu Leu Ala Glu Arg Gln Lys Arg Ile Glu Glu Gln Lys Glu
   385                 390                 395                 400
   Gln Arg Arg Arg Leu Glu Glu Gln Gln Arg Arg Glu Lys Glu Leu Arg
                   405                 410                 415
   Lys Gln Gln Glu Arg Glu Gln Arg Arg His Tyr Glu Glu Gln Met Arg
               420                 425                 430
   Arg Glu Glu Glu Arg Arg Arg Ala Glu His Glu Gln Glu Tyr Ile Arg
           435                 440                 445
   Arg Gln Leu Glu Glu Glu Gln Arg Gln Leu Glu Ile Leu Gln Gln Gln
       450                 455                 460
   Leu Leu His Glu Gln Ala Leu Leu Leu Glu Tyr Lys Arg Lys Gln Leu
   465                 470                 475                 480
   Glu Glu Gln Arg Gln Ala Glu Arg Leu Gln Arg Gln Leu Lys Gln Glu
                   485                 490                 495
   Arg Asp Tyr Leu Val Ser Leu Gln His Gln Arg Gln Glu Gln Arg Pro
               500                 505                 510
   Val Glu Lys Lys Pro Leu Tyr His Tyr Lys Glu Gly Met Ser Pro Ser
           515                 520                 525
   Glu Lys Pro Ala Trp Ala Lys Glu Val Glu Glu Arg Ser Arg Leu Asn
       530                 535                 540
   Arg Gln Ser Ser Pro Ala Met Pro His Lys Val Ala Asn Arg Ile Ser
   545                 550                 555                 560
   Asp Pro Asn Leu Pro Pro Arg Ser Glu Ser Phe Ser Ile Ser Gly Val
                   565                 570                 575
   Gln Pro Ala Arg Thr Pro Pro Met Leu Arg Pro Val Asp Pro Gln Ile
               580                 585                 590
   Pro His Leu Val Ala Val Lys Ser Gln Gly Pro Ala Leu Thr Ala Ser
           595                 600                 605
   Gln Ser Val His Glu Gln Pro Thr Lys Gly Leu Ser Gly Phe Gln Glu
       610                 615                 620
   Ala Leu Asn Val Thr Ser His Arg Val Glu Met Pro Arg Gln Asn Ser
   625                 630                 635                 640
   Asp Pro Thr Ser Glu Asn Pro Pro Leu Pro Thr Arg Ile Glu Lys Phe
                   645                 650                 655
   Asp Arg Ser Ser Trp Leu Arg Gln Glu Glu Asp Ile Pro Pro Lys Val
               660                 665                 670
   Pro Gln Arg Thr Thr Ser Ile Ser Pro Ala Leu Ala Arg Lys Asn Ser
           675                 680                 685
   Pro Gly Asn Gly Ser Ala Leu Gly Pro Arg Leu Gly Ser Gln Pro Ile
       690                 695                 700
   Arg Ala Ser Asn Pro Asp Leu Arg Arg Thr Glu Pro Ile Leu Glu Ser
   705                 710                 715                 720
   Pro Leu Gln Arg Thr Ser Ser Gly Ser Ser Ser Ser Ser Ser Thr Pro
                   725                 730                 735
   Ser Ser Gln Pro Ser Ser Gln Gly Gly Ser Gln Pro Gly Ser Gln Ala
               740                 745                 750
   Gly Ser Ser Glu Arg Thr Arg Val Arg Ala Asn Ser Lys Ser Glu Gly
           755                 760                 765
   Ser Pro Val Leu Pro His Glu Pro Ala Lys Val Lys Pro Glu Glu Ser
       770                 775                 780
   Arg Asp Ile Thr Arg Pro Ser Arg Pro Ala Ser Tyr Lys Lys Ala Ile
   785                 790                 795                 800
   Asp Glu Asp Leu Thr Ala Leu Ala Lys Glu Leu Arg Glu Leu Arg Ile
                   805                 810                 815
   Glu Glu Thr Asn Arg Pro Met Lys Lys Val Thr Asp Tyr Ser Ser Ser
               820                 825                 830
   Ser Glu Glu Ser Glu Ser Ser Glu Glu Glu Glu Glu Asp Gly Glu Ser
           835                 840                 845
   Glu Thr His Asp Gly Thr Val Ala Val Ser Asp Ile Pro Arg Leu Ile
       850                 855                 860
   Pro Thr Gly Ala Pro Gly Ser Asn Glu Gln Tyr Asn Val Gly Met Val
   865                 870                 875                 880
   Gly Thr His Gly Leu Glu Thr Ser His Ala Asp Ser Phe Ser Gly Ser
                   885                 890                 895
   Ile Ser Arg Glu Gly Thr Leu Met Ile Arg Glu Thr Ser Gly Glu Lys
               900                 905                 910
   Lys Arg Ser Gly His Ser Asp Ser Asn Gly Phe Ala Gly His Ile Asn
           915                 920                 925
   Leu Pro Asp Leu Val Gln Gln Ser His Ser Pro Ala Gly Thr Pro Thr
       930                 935                 940
   Glu Gly Leu Gly Arg Val Ser Thr His Ser Gln Glu Met Asp Ser Gly
   945                 950                 955                 960
   Thr Glu Tyr Gly Met Gly Ser Ser Thr Lys Ala Ser Phe Thr Pro Phe
   965 970 975
   Val Asp Pro Arg Val Tyr Gln Thr Ser Pro Thr Asp Glu Asp Glu Glu
   980 985 990
   Asp Glu Glu Ser Ser Ala Ala Ala Leu Phe Thr Ser Glu Leu Leu Arg
   995 1000 1005
   Gln Glu Gln Ala Lys Leu Asn Glu Ala Arg Lys Ile Ser Val Val
   1010 1015 1020
   Asn Val Asn Pro Thr Asn Ile Arg Pro His Ser Asp Thr Pro Glu
   1025 1030 1035
   Ile Arg Lys Tyr Lys Lys Arg Phe Asn Ser Glu Ile Leu Cys Ala
   1040 1045 1050
   Ala Leu Trp Gly Val Asn Leu Leu Val Gly Thr Glu Asn Gly Leu
   1055 1060 1065
   Met Leu Leu Asp Arg Ser Gly Gln Gly Lys Val Tyr Asn Leu Ile
   1070 1075 1080
   Asn Arg Arg Arg Phe Gln Gln Met Asp Val Leu Glu Gly Leu Asn
   1085 1090 1095
   Val Leu Val Thr Ile Ser Gly Lys Lys Asn Lys Leu Arg Val Tyr
   1100 1105 1110
   Tyr Leu Ser Trp Leu Arg Asn Arg Ile Leu His Asn Asp Pro Glu
   1115 1120 1125
   Val Glu Lys Lys Gln Gly Trp Ile Thr Val Gly Asp Leu Glu Gly
   1130 1135 1140
   Cys Ile His Tyr Lys Val Val Lys Tyr Glu Arg Ile Lys Phe Leu
   1145 1150 1155
   Val Ile Ala Leu Lys Asn Ala Val Glu Ile Tyr Ala Trp Ala Pro
   1160 1165 1170
   Lys Pro Tyr His Lys Phe Met Ala Phe Lys Ser Phe Ala Asp Leu
   1175 1180 1185
   Gln His Lys Pro Leu Leu Val Asp Leu Thr Val Glu Glu Gly Gln
   1190 1195 1200
   Arg Leu Lys Val Ile Phe Gly Ser His Thr Gly Phe His Val Ile
   1205 1210 1215
   Asp Val Asp Ser Gly Asn Ser Tyr Asp Ile Tyr Ile Pro Ser His
   1220 1225 1230
   Ile Gln Gly Asn Ile Thr Pro His Ala Ile Val Ile Leu Pro Lys
       1235                1240                1245
   Thr Asp Gly Met Glu Met Leu Val Cys Tyr Glu Asp Glu Gly Val
       1250                1255                1260
   Tyr Val Asn Thr Tyr Gly Arg Ile Thr Lys Asp Val Val Leu Gln
       1265                1270                1275
   Trp Gly Glu Met Pro Thr Ser Val Ala Tyr Ile His Ser Asn Gln
       1280                1285                1290
   Ile Met Gly Trp Gly Glu Lys Ala Ile Glu Ile Arg Ser Val Glu
       1295                1300                1305
   Thr Gly His Leu Asp Gly Val Phe Met His Lys Arg Ala Gln Arg
       1310                1315                1320
   Leu Lys Phe Leu Cys Glu Arg Asn Asp Lys Val Phe Phe Ala Ser
       1325                1330                1335
   Val Arg Ser Gly Gly Ser Ser Gln Val Phe Phe Met Thr Leu Asn
       1340                1345                1350
   Arg Asn Ser Met Met Asn Trp
       1355                1360

<210>  26
<211>  4083
<212>  DNA
<213>  artificial

<220>
<223>  mutant TNIK

<400>  26
atggcgagcg actccccggc tcgaagcctg gatgaaatag atctctcggc tctgagggac      60

cctgcaggga tctttgaatt ggtggaactt gttggaaatg gaacatacgg gcaagtttat     120

aagggtcgtc atgtcaaaac gggccagctt gcagccatca aggttatgga tgtcacaggg     180

gatgaagagg aagaaatcaa acaagaaatt aacatgttga agaaatattc tcatcaccgg     240

aatattgcta catactatgg tgcttttatc aaaaagaacc caccaggcat ggatgaccaa     300

ctttggttgg tgatggagtt ttgtggtgct ggctctgtca ccgacctgat caagaacaca     360

aaaggtaaca cgttgaaaga ggagtggatt gcatacatct gcagggaaat cttacggggg     420

ctgagtcacc tgcaccagca taaagtgatt catcgagata ttaaagggca aaatgtcttg     480

ctgactgaaa atgcagaagt taaactagtg gccgctggag tcagtgctca gcttgatcga     540

acagtgggca ggaggaatac tttcattgga actccctact ggatggcacc agaagttatt     600

gcctgtgatg aaaacccaga tgccacatat gatttcaaga gtgacttgtg gtctttgggt     660

atcaccgcca ttgaaatggc agaaggtgct ccccctctct gtgacatgca ccccatgaga 720
gctctcttcc tcatcccccg gaatccagcg cctcggctga agtctaagaa gtggtcaaaa 780
aaattccagt catttattga gagctgcttg gtaaagaatc acagccagcg accagcaaca 840
gaacaattga tgaagcatcc atttatacga gaccaaccta atgagcgaca ggtccgcatt 900
caactcaagg accatattga tagaacaaag aagaagcgag gagaaaaaga tgagacagag 960
tatgagtaca gtggaagtga ggaagaagag gaggagaatg actcaggaga gcccagctcc 1020
atcctgaatc tgccagggga gtcgacgctg cggagggact ttctgaggct gcagctggcc 1080
aacaaggagc gttctgaggc cctacggagg cagcagctgg agcagcagca gcgggagaat 1140
gaggagcaca agcggcagct gctggccgag cgtcagaagc gcatcgagga gcagaaagag 1200
cagaggcggc ggctggagga gcaacaaagg cgagagaagg agctgcggaa gcagcaggag 1260
agggagcagc gccggcacta tgaggagcag atgcgccggg aggaggagag gaggcgtgcg 1320
gagcatgaac aggaatacat caggcgacag ttagaggagg agcagagaca gttagagatc 1380
ttgcagcagc agctactgca tgaacaagct ctacttctgg aatataagcg caaacaattg 1440
gaagaacaga gacaagcaga aagactgcag aggcagctaa agcaagaaag agactactta 1500
gtttcccttc agcatcagcg gcaggagcag aggcctgtgg agaagaagcc actgtaccat 1560
tacaaagaag gaatgagtcc tagtgagaag ccagcatggg ccaaggaggt agaagaacgg 1620
tcaaggctca accggcaaag ttcccctgcc atgcctcaca aggttgccaa caggatatct 1680
gaccccaacc tgcccccaag gtcggagtcc ttcagcatta gtggagttca gcctgctcga 1740
acacccccca tgctcagacc agtcgatccc cagatcccac atctggtagc tgtaaaatcc 1800
cagggacctg ccttgaccgc ctcccagtca gtgcacgagc agcccacaaa gggcctctct 1860
gggtttcagg aggctctgaa cgtgacctcc caccgcgtgg agatgccacg ccagaactca 1920
gatcccacct cggaaaatcc tcctctcccc actcgcattg aaaagtttga ccgaagctct 1980
tggttacgac aggaagaaga cattccacca aaggtgcctc aaagaacaac ttctatatcc 2040
ccagcattag ccagaaagaa ttctcctggg aatggtagtg ctctgggacc cagactagga 2100
tctcaaccca tcagagcaag caaccctgat ctccggagaa ctgagcccat cttggagagc 2160
cccttgcaga ggaccagcag tggcagttcc tccagctcca gcacccctag ctcccagccc 2220
agctcccaag gaggctccca gcctggatca caagcaggat ccagtgaacg caccagagtt 2280
cgagccaaca gtaagtcaga aggatcacct gtgcttcccc atgagcctgc caaggtgaaa 2340
ccagaagaat ccagggacat tacccggccc agtcgaccag ctagctacaa aaaagctata 2400
gatgaggatc tgacggcatt agccaaagaa ctaagagaac tccggattga agaaacaaac 2460
cgcccaatga agaaggtgac tgattactcc tcctccagtg aggagtcaga aagtagcgag 2520
gaagaggagg aagatggaga gagcgagacc catgatggga cagtggctgt cagcgacata 2580
cccagactga taccaacagg agctccaggc agcaacgagc agtacaatgt gggaatggtg 2640
gggacgcatg ggctggagac ctctcatgcg gacagtttca gcggcagtat ttcaagagaa 2700
ggaaccttga tgattagaga gacgtctgga gagaagaagc gatctggcca cagtgacagc 2760

aatggctttg ctggccacat caacctccct gacctggtgc agcagagcca ttctccagct 2820

ggaaccccga ctgagggact ggggcgcgtc tcaacccatt cccaggagat ggactctggg 2880

actgaatatg gcatggggag cagcaccaaa gcctccttca ccccctttgt ggaccccaga 2940

gtataccaga cgtctcccac tgatgaagat gaagaggatg aggaatcatc agccgcagct 3000

ctgtttacta gcgaacttct taggcaagaa caggccaaac tcaatgaagc aagaaagatt 3060

tcggtggtaa atgtaaaccc aaccaacatt cggcctcata gcgacacacc agaaatcaga 3120

aaatacaaga aacgattcaa ctcagaaata ctttgtgcag ctctgtgggg tgtaaacctt 3180

ctggtgggga ctgaaaatgg cctgatgctt ttggaccgaa gtgggcaagg caaagtctat 3240

aatctgatca accggaggcg atttcagcag atggatgtgc tagagggact gaatgtcctt 3300

gtgacaattt caggaaagaa gaataagcta cgagtttact atctttcatg gttaagaaac 3360

agaatactac ataatgaccc agaagtagaa aagaaacaag gctggatcac tgttggggac 3420

ttggaaggct gtatacatta taaagttgtt aaatatgaaa ggatcaaatt tttggtgatt 3480

gccttaaaga atgctgtgga aatatatgct tgggctccta aaccgtatca taaattcatg 3540

gcatttaagt cttttgcaga tctccagcac aagcctctgc tagttgatct cacggtagaa 3600

gaaggtcaaa gattaaaggt tatttttggt tcacacactg gtttccatgt aattgatgtt 3660

gattcaggaa actcttatga tatctacata ccatctcata ttcagggcaa tatcactcct 3720

catgctattg tcatcttgcc taaaacagat ggaatggaaa tgcttgtttg ctatgaggat 3780

gagggggtgt atgtaaacac ctatggccgg ataactaagg atgtggtgct ccaatgggga 3840

gaaatgccca cgtctgtggc ctacattcat tccaatcaga taatgggctg gggcgagaaa 3900

gctattgaga tccggtcagt ggaaacagga catttggatg gagtatttat gcataagcga 3960

gctcaaaggt taaagtttct atgtgaaaga aatgataagg tattttttgc atccgtgcga 4020

tctggaggaa gtagccaagt gtttttcatg accctcaaca gaaattccat gatgaactgg 4080

taa 4083

<210> 27
<211> 11786
<212> DNA
<213> Artificial

<220>
<223> Ascl2 locus

<400> 27
acaagcttgc ttccatttgg gggatcgcca tctgactatt ccacttgtct tagggtgggg 60

cagagattag gtgatgtgga ggggcttctc tacatggccc ccctgcccca gctctgaggg 120

gtagcaccag agtgggtttc accagcgtag ggcacgtagg ccccgccatg aacagggccc 180

caaccttggt ttaatgcttt gctactgcca tcttaaagtt ctttttttat tttttatttt 240

gctttatttt ttattagaga tggggtctcc cagtgttgcc caggctggtc ttgaactcct 300

ggctcaagca atcctccggc ctcagcctcc caaagcactg ggatgacacg tgtgagccac 360

cttgcctggc ctttggaatc tgactacttt tatcttctaa cttgttttgc aggtgcaggc 420
caacggcata cagcagcact cacataagca aaggagagcg tgcacaaggc gccaaatgta 480
tatccaccct cactcgtccc cccacttgag tagcgcatcc acgatgccca cagacaccag 540
gccacacaga aaaggtgcca gggacccaca gcagtgcaag gcagcgtgtc acacctacgc 600
atgagcaagc cgggcgctga tggccaccga gcagccacgt tttccattca aatccgcact 660
tgctaaggat gcagcaggaa gccagtggtg ttctaacaaa cgtgcaggac ccgggaacct 720
gtcatgtcct ttcttacttg tgcgacttct ctgtgttagc cgaggtctct tgctgatgga 780
tctacccaca gtgccttttg tctttgaact tgtcccttcc ctccttcctc gcccatcagc 840
gagcaggagg tggagggtgc tggtggaaca agcctgcgtc aaggagtgaa atcagctgat 900
ttcatttttg tgcagtttcc actgttctag tagcaaatga aatagagacg cctgtgccag 960
gacaaaacac acactgtgtc attccagtga ttccgcatag aagttaaatg ctcttatgct 1020
tgcattttaa actggcatca cataatataa agatggataa ctacattcac gctagtcact 1080
taaattccta atctttctta ctcagaatgg cattaaatag tgagtataaa ataagaagta 1140
taaaatagta agtcaagagg ttgactatag aagaaagaaa aatgctttat attttagcac 1200
cttgaacatg acatcacgat caccttctcc ctggaatcag tttctaactt ccaggtgggg 1260
actaggcctg gaccatgagc tcctagcaga gccctgctgc ccccacagca gagcccagga 1320
caggctggca cctgggccag gtgaggctct gtccaggctc actgatctca aatgctgaac 1380
tgctaaggat gtcatgtccc caaaggagcc gccaggctca gcctcacttc ctggaaggcg 1440
tgaacattgc aagaatgtgg aagtgaaaga gtccagggct taaatctcaa ttctcatcat 1500
tttcaagctg agtccaaggg agagaagaca gtcatggatt cttagtttct gtttctggtt 1560
gagccagcag ggtcccttcc tcatccctct tttctgctta tcactagaga cagaaactaa 1620
aaccatgact ttaggctgct gagagcctaa aacaaaacga cagcaagaga aggtgggttg 1680
gaccagcttg cctgtgactt caggcacttc atctttactg ggcactgggt gaatgacagt 1740
gtggggaggg gtcttcataa cacggcaatc agcagcccac tgtgcccagg agactcgcct 1800
gtggtcctgg ttatcaacca cagccctttc cagtctcaaa aatgtccccg ctgggacagc 1860
aagttacatc gtcgctacaa gtcctgtctc ctgggagatg cagtccagca gcactacatc 1920
ctctgagcag caggtgccaa gtgggatgaa ctggataagg actgcattcg gggaaacgcc 1980
cgtgtgaaag gaaatacaca ggaaggaggt ggcaacgggt gggaagccac tagaccacga 2040
cgcgattctg ccccagtgaa ggcgagggga tagcctgggc ctagatcgct gtgaggtcta 2100
tggaagtttc cacaagcttg ctgggtagtt ctcgaggcaa actcggaaag ggagtccctt 2160
gtctccctgg aacggatctt tcttggcatc tctgtcacac tcattaggtg ggcctggtgt 2220
caaccccatt tgcaggccac cccaaacttg atcaaaggtc cgcttctggc accccatacc 2280
ctgtcctaca ggaaatacag ggacaggctc ccaataacaa cacccagcac ggtgccatca 2340
acaccaccac gcacacgggg gctcaacgga acagacatct ccgcttcttc aatgaagaca 2400
ctggagggaa attgcttaca aggcgcttaa gagacctatt aagcaaactt gatgtgtgga 2460

cctgcggcgg atcccgattc tataaggcca actgcacaaa accacgagac cccctgagga 2520
ctgcgccatt ggctgggtcc ccgatgatat gaaagaacgg tggttcattt gagcgggtga 2580
tgtttttgcg gtttccttta gaggcacacg tgaaacatga cgggtgaaag gattcaaagt 2640
ctgggatttg cttcaaagca acgcagggat ggcgtggggg atggatgggg caggaagggc 2700
cttgaaactg gtgctggagg cttcccaggg ctgccctgga gcccagtgcg tcctccaccg 2760
gccagactgt acaacggttg gatcctgtgt ccactgctag gacccaggct ccacgagcac 2820
gggcttgtgt ggcacacgga tgcaccctaa gtcctggcac agagactgct caacaaaggc 2880
ctcggtgctt ttgtgtatgt ttgaaatttt ccataataaa atgaaaaatg ggaaaatggg 2940
aaaacaaaaa tggcagcact acttaccctc tgcagagttt tgtccgcttc acgccagtgg 3000
gtggcagtcg tttcctctgc cctggccttc catcgtttcc cccctaccct cttcacccac 3060
ccaacagccc cctgtggtcc tggcagctgt gggcctttcc ttgaggtcaa ggtgtggagt 3120
cctggggagg gctcagggag gccaccgacc cgggtgtgga ttctgggaga agcctgtggg 3180
atgtccctcc ctgggtgacc acggcaatgt gccccctcct gtcccttggc caaggccagt 3240
tccctgagcc ctgcagcccc aagccacagc tggtccactg accccagttg agcctggtcc 3300
tcatcagacc agctgacccc tttgaccccc gctacagact cggctttgac cttggctgct 3360
gaggagcccc cacctggact gaggctgcag ctggcgagag aggagccctg agctcctctg 3420
ataagaaggg acctggccag cctgacgttt gagacccagg catcccggta gcctgggtgt 3480
cctgttgccg tggttattca ggagccaccc actctgggac aacaccagct gctcccacct 3540
cgcagggctc ccacggctct gtcccaacca ctcctttctg aaggaagggg tgcctctgcg 3600
ccctaaagaa accgggggag ccccacaacc cctcccccac caggacacta aaaggcagct 3660
ttcggtacag tgagacatca aagcctccta ggccctgagt caaaggtata gccgtgtaat 3720
atcccagtgc cagctctccg gctgcgggga gcctggcgca aagcttccaa gccttccttg 3780
ttcctttcaa gagccgctct tagaattcag gtgagcggag acctgcaggg cctccccagt 3840
gcgggcaaaa cccaaagcta gcgagagggc agcctccagg cacctctcac taactcctcc 3900
cagaggccgt tgaggtgggt ctggtcaaac ccatttgcaa gttaacccac ttgccctggg 3960
ctgcccagct gccacgttag tggagatctg agcgtggtgg cctgcgcagg agcccatgcc 4020
ctcagcccca cagccggtgc tctctggtca gaccacctca gcctagcccc acacccagca 4080
cttaccccag ccctcgggat gggtcagcag cctccagcct gcagcttcca agccagcgag 4140
tagccctgtc tggacaaccc accagcccac cacctcctgg aggatgcccc cagcctcaca 4200
aggtgtccca atggctccgc tatcaacggc ctggctgcac tccagatctc acccagaccc 4260
accctacgga ggaggcagca gggtttgagg agtagtgacc acggaagtct ggccgtcacc 4320
tgggaagtgt aggtgatagg agccactggt aaacagaact gatttattta taaagttcac 4380
gctcccttga agaggtgtgc cccacacagg cttctcccta gcagagcagc agtgcccaca 4440
aacccacccc agggtgggct gtcacggggg cctcacgcca gggaccccgc ccctcaggga 4500

ctgctcgtgt ccagatcttg gccagcatgg aaaactccag atagtggggg caggggtcca 4560
ggtcatcttt attacgcccc aggtcaaggg ttctttgtac aaaaataggt ctccgtttgc 4620
cagcagtgtc cctccagcag ctcaagttaa tgtgtagaaa atggattctc tgtgccctta 4680
gaaaatcctc tcccctccgg aaaaatctcc aagtgttggt gccccccgcc ccactgcagt 4740
cgagaagctg tggggagggg cggcgtcgga ggaagccgcc agcccttatg ggccagctc 4800
caagcccgtt tccaccgcgg cattggtcag gctgggccgg acgaacgagg cggcgtcggc 4860
ggtgcggggg gtggtgggtg ggtccccggc tcgctggggg cggagcgcgg gccggtccac 4920
ctggcgggct ccccggcgat gagcgcgccg gccgctcgct cggcttccgg ggctgaggct 4980
gcggggggaa ggtggggaac caaacgcgcg tcaacgcggg cgcgggcccg gggcagaccc 5040
cgcccgggcc ggccctgccc gcacctcccc caagcgaagt cggcagtttc gtttgctcgg 5100
ttggttttgg agtcttgagt ccgtgggtgc cgcgactcgg tctgagacac ggcgggggcg 5160
gggcgggcgc tcggagccgc ggtgagtcag ggctccgcgc ccgccgactc atttctgccg 5220
ccccggcccg ggagcgcgat ttgcaatgca aagtcacccc gcctccagca ccccaatctg 5280
ccccaggatc cgccagcact agagacctca acggcccgac ggccgctccc ctcccctcgt 5340
ctacccctcc ctcgtcggcg gctgagccgc gaggggaagt tttgcaatcc cggacaaaca 5400
aacgccggtc ttgcacgggc ttgaaaaact ttgggggaaa tgaagagtga gcgaaatcga 5460
agccatcgct cgggcctggc gctcggctcc gcgggctcct gggggcgcga cccgccgggc 5520
ctgcccaccc cgtccctcca ccccggcccc cggccctccc tcctccctgc ctcccggctg 5580
ttacctcata ggtcgagggc gctcagtagc cccctaacca gctggagaag tcgagtagct 5640
cgcgctccgc aggactcagc gcgccttcgc agccgctgtc gtccgacgag taggcggaac 5700
gcggggagcc gggctccgag ctgcccccgc ggcccgggga cgaagaagcg cgggagggcg 5760
aggcggcgac cggggtggtc cctggcggcc cgcggggcgc agacggccgc acggcctgcg 5820
gcctcagccc tcccgccagc gcgttgcgca cggcgtcgtg ctcggccagc aggcgctgca 5880
gcgcgcggat gtactccacg gctgagcgca gcgtctccac cttgctcagc ttcttgctgg 5940
cgccgccgtg cggcacgtgc tgccgcagcg cctggaagcc caagttcacc agcttcacgc 6000
ggttgcgctc gcgctcattg cgccgcgcta cggccgctgc gccgcctccg gtctctgcgg 6060
tggccggtcg ccgccgccgg ctgcagcgca acagttccgg ggacgcgggt ctccgccggg 6120
cagcgcagcc gacagggacg gggggcgcag gggcgcggga cctgggcagt gtgccgccgt 6180
ccatcgcgcc tgcatccacc cgcccgctcc aggtcccggc gcgccgcagg aaggtgcagg 6240
cagaggaacc ggaggcgacg gggaaaactg tggcgcccca agggggcttc tggcacggcg 6300
ccgccaggca actccccagg gcacgcgtcc taggtcgtct ggagcccggg gataggaggc 6360
ctagtggtgg caggccgtac gcgccaggga gcgtgggacg ctcgtgtccc gcgcgtgcgg 6420
ccggactctc ccaggtctcc gcaggcgcgg cgcaggcggc tggtttttaa atgtatagat 6480
aaccctcctc cgcgccgccg ccgtcgcctt tctcacgccc tccttccttc gcctcgccct 6540
cccgccacgc ttcgccctcc ccctcgcgcg atcacattct gtaaggccca aagcgtgcgc 6600

atgtccccct agcccatccc ccggacgcag tccacagatc cccagtgcgc ccaactggcg 6660
aaatctgcga gttcccggtg cgccccctgc tcccggcagg tgcttagtgc gcccccaaag 6720
caaggtacgc aggtcctggg ttgagccttc ccgtaccccc accctaaccc cgcgcgcagc 6780
cccgccagtc ccaagagccg ccagaccttc gcacgcgcag cgcgcgctgt gggagggaag 6840
gcgcggccct ggcgacaaca cggctgttcg ggaggcgcgc aagatccccg ggggcagcac 6900
gcgccgcgca gcccacaccc acgccccacc ctcctggggc cgaggaggcg ggggccaggg 6960
tctcagccaa tcgtggccca cccgtttggc caatcgcgca gggcgcggct ccacgcccgg 7020
ccccattgag gaagcgcgta cgcgtggcgc gtggctcacg gggagcatcg ctaacaaagc 7080
tgggttcctg ctgggccccg ccctgctcct cgcccccgcg actgggctgg gcgcgctgtc 7140
ccctagcgca gctatgtccc gagcgcgccc ccacctgtgc gttaatctac tgggaatggg 7200
ggtggactgc gccttacctg gggcggggtg gggcttaagg agtggtcgag actgaggcgg 7260
ggtgggaggt tcaggttccc ggggcgcctt ccccaacccg ccccgctttc cccgtccctc 7320
cacgcgcacc ctgcctgtgg tttccgtgcg cccccggcct gagggctctg ggcggcacct 7380
taacccggag ggcctggagg tctgcacccg accgccttgt gccaggacgg tcaggtccac 7440
gccctccccc accgtggctc cctccatctg cagtatcccc cacctccagc ccgtcctgcc 7500
ctcctgttct ccgtctcgct tcccgtcggt gcctccggga tctcacagcc ctcgcacctc 7560
ttttgtgacc caggctgttt ttctgcaccc ccctctcccc tgagggcact gagattgggc 7620
cattggcctg aaggtctctg ggagcagcac ccttccaggg gaggtgggac gtcgagaact 7680
tctccctaag agatgcgggg aaatggtggg gcctgagagt gcaaacactg cagaaatgcg 7740
aaaaatgtag tgttaacgga agagtttagg tcctgcctca ctgtccggga aacgcgtgcc 7800
ctcgggggag cctttgccaa gccggttttt cccgaaggtg accagatgct cctgggccac 7860
tgcctctgag acctcaggga acggagattt ttgtggaccc agctgcctgg agctgctttc 7920
ctgttccggc cggaggaggt gaggcccaag acccctcctg ggagcctggg ggcagatagc 7980
cagtgtttac tgccagcctc ggggtgccca cctgctccca ttaccctgca ggatgctgct 8040
ggctggccca cctgggcccc cagcacacct gtgtctcgag tacgcctggc cctcctgcct 8100
tgggaggggc cggaagagta gcacctgcct gggagctggt ggtctgcggt ctctatttgg 8160
cagatgagga agccgacttg gagagaaccc tggatgtgtc cacagtcact cctccgccca 8220
gtggagcgat ccaggcagaa atcggggccc tgagtctgaa tccgggttct gcaaccaggg 8280
cagatgcggg cttgcctctg ctccctgtcc ctggtctgag agcccattct tcccagatgg 8340
tcacttggca aatcacagcc tggcatggat tgttctgccc tccttctgct gcctccctcc 8400
ttccccttgt caaggctgca agaccaggat ctaggaacga tcctggagcc ctgcaaacta 8460
ggccttggaa atccctgctg gatttccacc tcccgggctg ggagcccctc ggtcatctgt 8520
tgctgtgtaa ggagccacca ggattttagc ggtctgaaca acgatgtatt atttctcagg 8580
attctgtgac ttgatgggtg ggccctctgc tgctctgggt gtggctgcat acaccccggg 8640

ggtcaacagg gacgagcggt acagcggctg ggttgctctc tacccggtct tcgtccaagc 8700
ccctccacag ctggtaagat ctccggagca ggacctgcaa gccctcttca gatcacccca 8760
gaacttcctg tctaaaaact gaagcctctc actgcccagg catggcttct tgctaccctg 8820
ccctcaggca cagtcctgca cccacctgcg tctgctgtgc catgtccagg ccagtccccc 8880
cccaccacca acacctctct ctatcttcat cctcttccca atctggtcct cccaccgctg 8940
tggaaacccc gtctgccccc aaagcctagc ttaaaaataa ttccctaggg acctgtgtct 9000
ctccctgcct cggcccctcc ttcattcctg ggtgcctccg gctgtgcagc atttgacact 9060
gcagcacccc ccttaattcg gaagcatgct gtctcctgga ctggtgagtc tccacactat 9120
ctgagccgtc ttctctggaa ctcttggcct ctcagtccgt tctgagaata cagccttggt 9180
aagcacggtg cccacatgaa tgtttccagc agcaggattc aaaatagcca catgtccatc 9240
aacagatgag tggataaaca aaacatggtc cagaataatg gaagattact cagccctaaa 9300
aagagacgaa gctggtgaac ctcgagaaca cgaggccgcg tgaacgaagc cagacaccga 9360
ggaccacgta gcgtgagact ctcagtctat gaaatgtgcg gagtcgataa attcacagag 9420
acagaaagga gattcacggt tgccaggggc tggggagtga caacagaggg atgggggtga 9480
ctgtgaaagg gtacgtggtt tctttcccag aggataagaa cgttctaaca tggcctgtcc 9540
tgttggcttc acagctctgt acaacacaca aaaaaaccat tgaaatgtac actttgtgga 9600
atgtgaactg tatcttgata aagcagttag aagaccttcg aacataagca tgcggcctca 9660
tggggccttt gcctgggcac cctggcacct ctcccaggct ctacctatct ccgacttcat 9720
tcctgagctc ttgaacaggg gtaaggcaaa ctttttctgc aaaggaacac gtggtaagta 9780
ttttcggcct tgacggtcac atgtctctgc cacgagtcgt ctgccttggg gcgcaaatgc 9840
aggcttgggc agggaagaaa taacaaaact tgcttcctgg tcactgaaac atgaagtcca 9900
ggtcacactc actgttacaa aatactccga attttcagac tgtggttcaa tacacatgac 9960
ataaaatgga ccttcttaac catttgtaag tgcacggttc cgtggaattc agtatattca 10020
tgtggctgtg caatcatcac caccatccat ctccaaaagt ttctcatttt cccaaaccga 10080
aagtctgtcc ccattaaaca gcagcttccc atgacccttc ccccagcccc tggcacccac 10140
catccactct gtgtctgtag atttgactgc tctggagacc tcctgtaagt ggaatcctac 10200
agcatctgtc tttttgtgga ccggcttctt acactgatgc tgatgccctc gagcttcatc 10260
catgtcgtag cctgcataag gatttcctct ctttttatgg gtgaataata ttccactgta 10320
tgggtagacc acggtgttga tccgttcctc cgtcagtgga tgctggggtg gtttccaccc 10380
ttgggctacc gtcagtgacg ctactgtgga catgggggta caaatatctc tttgagatcc 10440
tgctttcagt tcttttgggg atagacggag aagcggagtt gccaggtcat acggcaaacc 10500
tctgtttaac cttttgaggg accaccatgt tgttttccgc agtggctgcc cacagtacat 10560
tcctgctgcg cacgaggttc tgatgtctcc acatccccgc ccacacttgg tgctttctgg 10620
gtttgtttcg tttcgttttg tttttgtttg tttttgagac ggagtctcgc tctgtctccc 10680
aggctggagt gcagtggcgc aatcttggct cactgcgact tctgcctccc gagttccagc 10740

cattctctag tttcagcctc ccgagtagct gagactacag atacgtgcca ccatgcccgg 10800

ccaaattttt attttttgta gagatagagt ctgactatgt tgcccagcct ggctgaggtg 10860

ataatagttt tttgatgata gctaatgggt atggatttta attttttaac cacttaagaa 10920

tttaaagaaa attcctagct tttgggcaat acaaaagcag gccaggggct ggatctggcc 10980

catgggcctc ggtctgctga cagctgctcc agaggactgg tatgtccacg tgacacctgg 11040

cccgaccccc atcctcctgc agctcctcaa actcaacttg ttgcaggttg aactcggcct 11100

cctttcctct aaggaaagat cccctccgca gcagagaaca ccaggtcggc agtgtgggca 11160

ctgcccttcc tctcccctgc cctctgctgt acgtcagccc agccgcttct ccagccaggt 11220

ccccatcttg ccttggacac tgcccctgcc tctgccctgg tctcctgggt tctcagtttg 11280

ctgcttctgt ctgtgcaccg cctggaagtg ggggggcctt acccagcatc cagcccagct 11340

agatcatgtc cgggccctcg gggttcaggc ccagcaccct cacgtgccat cactcactgc 11400

ctcctctcca gctcggacgt tgtatctcct ggaagccttc cctgatccca gtggcctcct 11460

gaagcctcct cgcccctgtg ctccacaggg agctgtgctg cccgggcctg ctctgtccaa 11520

taggctaacc tgacctgctc cttcgacatc taaggtgctg ctcatgtgta ttcatgacct 11580

gggtggatgt tggggagccc aggcccagca aagaggggca ggagcaggca gttccggggt 11640

tggcgatggc ccaggggaag ctttcggcct ggttggtcag agctcctggt gaccaagggt 11700

gacttcaaag tcaacgtgag cctcactcac atgagatgag cctagagcgt ccaagaacag 11760

ctctgtagct ggccagccgg gagctg 11786

<210> 28
<211> 19
<212> DNA
<213> Artificial

<220>
<223> primer

<400> 28
gttacctgga agacgaagg 19

<210> 29
<211> 18
<212> DNA
<213> Artificial

<220>
<223> primer

<400> 29
tctggaggcg ttcagttg 18

<210> 30
<211> 20
<212> DNA
<213> Artificial

<220>
<223> primer

<400>  30
gccagagtca agccagtagt                                                    20


<210>  31
<211>  20
<212>  DNA
<213>  Artificial

<220>
<223>  primer

<400>  31
tagcctaatg tggagtggat                                                    20


<210>  32
<211>  18
<212>  DNA
<213>  Artificial

<220>
<223>  primer

<400>  32
ccaccaagac ctacatac                                                      18


<210>  33
<211>  18
<212>  DNA
<213>  Artificial

<220>
<223>  primer

<400>  33
ccactcctca catattcc                                                      18


<210>  34
<211>  26
<212>  DNA
<213>  Artificial

<220>
<223>  primer

<400>  34
ctaccgtgtt actctgacta ttcttc                                             26


<210>  35
<211>  18
<212>  DNA
<213>  Artificial

<220>
<223>  primer

<400>  35
gcggctcagt ggctaagg                                                      18


<210>  36
<211>  19
<212>  DNA
<213>  Artificial

<220>
<223>  primer

<400>  36
actcagtctg ggtggaagg                                                    19


<210>  37
<211>  26
<212>  DNA
<213>  Artificial

<220>
<223>  primer

<400>  37
ggaatgatag aggcataagg agtatc                                            26


<210>  38
<211>  20
<212>  DNA
<213>  Artificial

<220>
<223>  primer

<400>  38
cagcaagata gcagaggaag                                                   20


<210>  39
<211>  21
<212>  DNA
<213>  Artificial

<220>
<223>  primer

<400>  39
tggagatgga ttgaatgaag g                                                 21


<210>  40
<211>  21
<212>  DNA
<213>  Artificial

<220>
<223>  primer

<400>  40
ctcactggaa cttacaatct g                                                 21


<210>  41
<211>  18
<212>  DNA
<213>  Artificial

<220>
<223>  primer

<400>  41
actcaagaac tggtagac                                                     18


<210>  42

<211>  18
<212>  DNA
<213>  Artificial

<220>
<223>  primer

<400>  42
ctaagtgggt aaggtagg                                                    18


<210>  43
<211>  22
<212>  DNA
<213>  Artificial

<220>
<223>  primer

<400>  43
aagtttgaca agttcaagga cc                                               22


<210>  44
<211>  22
<212>  DNA
<213>  Artificial

<220>
<223>  primer

<400>  44
tggcaccatg cttctttaag tc                                               22


<210>  45
<211>  20
<212>  DNA
<213>  Artificial

<220>
<223>  primer

<400>  45
tggagttaca ggcagtggtg                                                  20


<210>  46
<211>  25
<212>  DNA
<213>  Artificial

<220>
<223>  primer

<400>  46
attgatttct ttaggttggg tggag                                            25


<210>  47
<211>  46
<212>  DNA
<213>  Artificial

<220>
<223>  primer

<400>  47
ccagcataaa gtgattcatg cagctattaa agggcaaaat gtcttg                     46

<210>  48
<211>  46
<212>  DNA
<213>  Artificial

<220>
<223>  primer

<400>  48
caagacattt tgccctttaa tagctgcatg aatcacttta tgctgg                    46


<210>  49
<211>  37
<212>  DNA
<213>  Artificial

<220>
<223>  primer

<400>  49
gccagcttgc agccatcgcg gttatggatg tcacagg                              37


<210>  50
<211>  37
<212>  DNA
<213>  Artificial

<220>
<223>  primer

<400>  50
cctgtgacat ccataaccgc gatggctgca agctggc                              37


<210>  51
<211>  40
<212>  DNA
<213>  Artificial

<220>
<223>  primer

<400>  51
gcagaagtta aactagtggc cgctggagtc agtgctcagc                           40


<210>  52
<211>  40
<212>  DNA
<213>  Artificial

<220>
<223>  primer

<400>  52
gctgagcact gactccagcg gccactagtt taacttctgc                           40